(19) Europäisches Patentamt
European Patent Office
Office européen des brevets

(11) EP 0 789 077 B1

(12) # FASCICULE DE BREVET EUROPEEN

(45) Date de publication et mention de la délivrance du brevet:
**26.09.2007 Bulletin 2007/39**

(21) Numéro de dépôt: **96420265.9**

(22) Date de dépôt: **02.08.1996**

(51) Int Cl.:
*C12N 15/48* (2006.01) *C12N 7/00* (2006.01)
*C07K 14/15* (2006.01) *C12Q 1/68* (2006.01)
*C07K 16/10* (2006.01) *G01N 33/569* (2006.01)
*A61K 39/21* (2006.01) *A61K 39/42* (2006.01)
*A61K 48/00* (2006.01)

(54) **Matériel viral et fragments nucléotidiques associés à la sclérose en plaques, à des fins de diagnostic, prophylactiques et thérapeutiques**

Virales Material und Multiple-Sklerose assoziierte Nukleotidfragmente mit diagnostischen, prophylaktischen und therapeutischen Verwendungen

Viral material and nucleotide fragments associated with multiple sclerose having diagnostic, prophylactic and therapeutical uses

(84) Etats contractants désignés:
**AT BE CH DE DK ES FI FR GB GR IE IT LI LU MC NL PT SE**

(30) Priorité: **03.08.1995 FR 9509643**

(43) Date de publication de la demande:
**13.08.1997 Bulletin 1997/33**

(73) Titulaire: **BIO MERIEUX**
**69280 Marcy l'Etoile (FR)**

(72) Inventeurs:
- **Perron, Hervé 69005 Lyon (FR)**
- **Paranhos-Baccala, Glaucia 69003 Lyon (FR)**
- **Beseme, Frédéric 38090 Villefontaine (FR)**
- **Komurian-Pradel, Florence 69450 St Cyr Au Mont d'Or (FR)**
- **Bedin, Frédéric 69002 Lyon (FR)**
- **Jolivet-Reynaud, Colette 69500 Bron (FR)**
- **Mandrand, Bernard 69100 Villeurbanne (FR)**

(74) Mandataire: **Maureau, Philippe et al**
**Cabinet GERMAIN & MAUREAU,**
**12, rue Boileau,**
**BP 6153**
**69466 Lyon Cedex 06 (FR)**

(56) Documents cités:
**WO-A-94/28138**

- **RESEARCH IN VIROLOGY, vol. 143, no. 5, 1992, pages 337-350, XP000569296 H. PERRON ET AL.: "In vitro transmission and antigenicity of a retrovirus isolated from a multiple sclerosis patient "**
- **LANCET THE, vol. 337, 6 Avril 1991, LONDON GB, pages 862-863, XP002001596 H. PERRON ET AL.: "Isolation of retrovirus from patients with multiple sclerosis"**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 789 077 B1

Printed by Jouve, 75001 PARIS (FR)

**LISTE DE SEQUENCES**

(1) INFORMATIONS GENERALES:

(i) DEPOSANT:

(A) NOM: BIOMERIEUX
(B) RUE: AUCUNE
(C) VILLE: MARCY L'ETOILE
(E) PAYS: FRANCE
(F) CODE POSTAL: 69280

(ii) TITRE DE L'INVENTION: Matériel viral et fragments nucléotidiques associés à la sclérose en plaques, à des fins de diagnostic, prophylactiques et thérapeutiques.

(iii) NOMBRE DE SEQUENCES: 92

(iv) FORME DECHIFFRABLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS (D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:

(A) LONGUEUR: 1158 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 1:

```
CCCTTTGCCA CTACATCAAT TTTAGGAGTA AGGAAACCCA ACGGACAGTG GAGGTTAGTG   60
CAAGAACTCA GGATTATCAA TGAGGCTGTT GTTCCTCTAT ACCCAGCTGT ACCTAACCCT  120
TATACAGTGC TTTCCCAAAT ACCAGAGGAA GCAGAGTGGT TTACAGTCCT GGACCTTAAG  180
GATGCCTTTT TCTGCATCCC TGTACGTCCT GACTCTCAAT TCTTGTTTGC CTTTGAAGAT  240
CCTTTGAACC CAACGTCTCA ACTCACCTGG ACTGTTTTAC CCCAAGGGTT CAGGGATAGC  300
CCCCATCTAT TTGGCCAGGC ATTAGCCCAA GACTTGAGTC AATTCTCATA CCTGGACACT  360
CTTGTCCTTC AGTACATGGA TGATTTACTT TTAGTCGCCC GTTCAGAAAC CTTGTGCCAT  420
CAAGCCACCC AAGAACTCTT AACTTTCCTC ACTACCTGTG GCTACAAGGT TTCCAAACCA  480
AAGGCTCGGC TCTGCTCACA GGAGATTAGA TACTNAGGGC TAAAATTATC CAAAGGCACC  540
AGGGCCCTCA GTGAGGAACG TATCCAGCCT ATACTGGCTT ATCCTCATCC CAAAACCCTA  600
AAGCAACTAA GAGGGTTCCT TGGCATAACA GGTTTCTGCC GAAAACAGAT TCCCAGGTAC  660
ASCCCAATAG CCAGACCATT ATATACACTA ATTANGGAAA CTCAGAAAGC CAATACCTAT  720
TTAGTAAGAT GGACACCTAC AGAAGTGGCT TTCCAGGCCC TAAAGAAGGC CCTAACCCAA  780
GCCCCAGTGT TCAGCTTGCC AACAGGGCAA GATTTTTCTT TATATGCCAC AGAAAAAACA  840
GGAATAGCTC TAGGAGTCCT TACGCAGGTC TCAGGGATGA GCTTGCAACC CGTGGTATAC  900
CTGAGTAAGG AAATTGATGT AGTGGCAAAG GGTTGGCCTC ATNGTTTATG GGTAATGGNG  960
GCAGTAGCAG TCTNAGTATC TGAAGCAGTT AAAATAATAC AGGGAAGAGA TCTTNCTGTG 1020
TGGACATCTC ATGATGTGAA CGGCATACTC ACTGCTAAAG GAGACTTGTG GTTGTCAGAC 1080
AACCATTTAC TTAANTATCA GGCTCTATTA CTTGAAGAGC CAGTGCTGNG ACTGCGCACT 1140
TGTGCAACTC TTAAACCC                                                1158
```

(2) INFORMATIONS POUR LA SEQ ID NO: 2:

(i) CARACTERISTIQUES DE LA SEQUENCE:

(A) LONGUEUR: 297 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 2:

```
CCCTTTGCCA CTACATCAAT TTTAGGAGTA AGGAAACCCA ACGGACAGTG GAGGTTAGTG   60
CAAGAACTCA GGATTATCAA TGAGGCTGTT GTTCCTCTAT ACCCAGCTGT ACCTAACCCT  120
TATACAGTGC TTTCCCAAAT ACCAGAGGAA GCAGAGTGGT TTACAGTCCT GGACCTTAAG  180
GATGCCTTTT TCTGCATCCC TGTACGTCCT GACTCTCAAT TCTTGTTTGC CTTTGAAGAT  240
CCTTTGAACC CAACGTCTCA ACTCACCTGG ACTGTTTTAC CCCAAGGGTT CAAGGGA     297
```

(2) INFORMATIONS POUR LA SEQ ID NO: 3:

(i) CARACTERISTIQUES DE LA SEQUENCE:

(A) LONGUEUR: 85 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 3:

```
GTTTAGGGAT ANCCCTCATC TCTTTGGTCA GGTACTGGCC CAAGATCTAG GCCACTTCTC   60
AGGTCCAGSN ACTCTGTYCC TTCAG                                         85
```

(2) INFORMATIONS POUR LA SEQ ID NO: 4:

(i) CARACTERISTIQUES DE LA SEQUENCE:

(A) LONGUEUR: 86 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 4:

```
GTTCAGGGAT AGCCCCCATC TATTTGGCCA GGCACTAGCT CAATACTTGA GCCAGTTCTC   60
ATACCTGGAC AYTCTYGTCC TTCGGT                                        86
```

(2) INFORMATIONS POUR LA SEQ ID NO: 5:

(i) CARACTERISTIQUES DE LA SEQUENCE:

(A) LONGUEUR: 85 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 5:

```
GTTCARRGA TAGCCCCCATC TATTTGGCCW RGYATTAGCC CAAGACTTGA GYCAATTCTC   60
ATACCTGGA CACTCTTGTCC TTYRG                                         85
```

(2) INFORMATIONS POUR LA SEQ ID NO: 6:

(i) CARACTERISTIQUES DE LA SEQUENCE:

(A) LONGUEUR: 85 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 6:

```
GTTCAGGGAT AGCTCCCATC TATTTGGCCT GGCATTAACC CGAGACTTAA GCCAGTTCTY   60
ATACGTGGAC ACTCTTGTCC TTTGG                                         85
```

(2) INFORMATIONS POUR LA SEQ ID NO: 7:

(i) CARACTERISTIQUES DE LA SEQUENCE:

(A) LONGUEUR: 111 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 7:

```
GTGTTGCCAC AGGGGTTTAR RGATANCYCY CATCTMTTTG GYCWRGYAYT RRCYCRAKAY    60
YTRRGYCAVT TCTYAKRYSY RGSNAYTCTB KYCCTTYRGT ACATGGATGA C            111
```

(2) INFORMATIONS POUR LA SEQ ID NO: 8:

(i) CARACTERISTIQUES DE LA SEQUENCE:

(A) LONGUEUR: 645 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 8:

```
TCAGGGATAG CCCCCATCTA TTTGGCCAGG CATTAGCCCA AGACTTGAGT CAATTCTCAT    60
ACCTGGACAC TCTTGTCCTT CAGTACATGG ATGATTTACT TTTAGTCGCC CGTTCAGAAA   120
CCTTGTGCCA TCAAGCCACC CAAGAACTCT TAACTTTCCT CACTACCTGT GGCTACAAGG   180
TTTCCAAACC AAAGGCTCGG CTCTGCTCAC AGGAGATTAG ATACTNAGGG CTAAAATTAT   240
CCAAAGGCAC CAGGGCCCTC AGTGAGGAAC GTATCCAGCC TATACTGGCT TATCCTCATC   300
CCAAAACCCT AAAGCAACTA AGAGGGTTCC TTGGCATAAC AGGTTTCTGC CGAAAACAGA   360
TTCCCAGGTA CASCCCAATA GCCAGACCAT TATATACACT AATTANGGAA ACTCAGAAAG   420
CCAATACCTA TTTAGTAAGA TGGACACCTA CAGAAGTGGC TTTCCAGGCC CTAAAGAAGG   480
CCCTAACCCA AGCCCCAGTG TTCAGCTTGC CAACAGGGCA AGATTTTTCT TTATATGCCA   540
CAGAAAAAAC AGGAATAGCT CTAGGAGTCC TTACGCAGGT CTCAGGGATG AGCTTGCAAC   600
CCGTGGTATA CCTGAGTAAG GAAATTGATG TAGTGGCAAA GGGTT                   645
```

**(2) INFORMATIONS POUR LA SEQ ID NO: 9:**

(i) CARACTERISTIQUES DE LA SEQUENCE:

(A) LONGUEUR: 741 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 9:

```
CAAGCCACCC AAGAACTCTT AAATTTCCTC ACTACCTGTG GCTACAAGGT TTCCAAACCA    60
AAGGCTCAGC TCTGCTCACA GGAGATTAGA TACTTAGGGT TAAAATTATC CAAAGGCACC   120
AGGGGCCTCA GTGAGGAACG TATCCAGCCT ATACTGGGTT ATCCTCATCC CAAAACCCTA   180
AAGCAACTAA GAGGGTTCCT TAGCATGATC AGGTTTCTGC CGAAAACAAG ATTCCCAGGT   240
ACAACCAAAA TAGCCAGACC ATTATATACA CTAATTAAGG AAACTCAGAA AGCCAATACC   300
TATTTAGTAA GATGGACACC TAAACAGAAG GCTTTCCAGG CCCTAAAGAA GGCCCTAACC   360
CAAGCCCCAG TGTTCAGCTT GCCAACAGGG CAAGATTTTT CTTTATATGG CACAGAAAAA   420
ACAGGAATCG CTCTAGGAGT CCTTACACAG GTCCGAGGGA TGAGCTTGCA ACCCGTGGCA   480
TACCTGAATA AGGAAATTGA TGTAGTGGCA AAGGGTTGGC CTCATNGTTT ATGGGTAATG   540
GNGGCAGTAG CAGTCTNAGT ATCTGAAGCA GTTAAAATAA TACAGGGAAG AGATCTTNCT   600
GTGTGGACAT CTCATGATGT GAACGGCATA CTCACTGCTA AAGGAGACTT GTGGTTGTCA   660
GACAACCATT TACTTAANTA TCAGGCTCTA TTACTTGAAG AGCCAGTGCT GNGACTGCGC   720
ACTTGTGCAA CTCTTAAACC C                                             741
```

(2) INFORMATIONS POUR LA SEQ ID NO: 10:

(i) CARACTERISTIQUES DE LA SEQUENCE:

(A) LONGUEUR: 93 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 10:

```
TGGAAAGTGT TGCCACAGGG CGCTGAAGCC TATCGCGTGC AGTTGCCGGA TGCCGCCTAT    60
AGCCTCTACA TGGATGACAT CCTGCTGGCC TCC                                 93
```

(2) INFORMATIONS POUR LA SEQ ID NO: 11:

(i) CARACTERISTIQUES DE LA SEQUENCE:

(A) LONGUEUR: 96 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 11:

```
TTGGATCCAG TGYTGCCACA GGGCGCTGAA GCCTATCGCG TGCAGTTGCC GGATGCCGCC    60
TATAGCCTCT ACGTGGATGA CCTSCTGAAG CTTGAG                              96
```

(2) INFORMATIONS POUR LA SEQ ID NO: 12:

(i) CARACTERISTIQUES DE LA SEQUENCE:

(A) LONGUEUR: 748 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 12:

```
TGCAAGCTTC ACCGCTTGCT GGATGTAGGC CTCAGTACCG GNGTGCCCCG CGCGCTGTAG    60
TTCGATGTAG AAAGCGCCCG GAAACACGCG GGACCAATGC GTCGCCAGCT TGCGCGCCAG   120
CGCCTCGTTG CCATTGGCCA GCGCCACGCC GATATCACCC GCCATGGCGC CGGAGAGCGC   180
CAGCAGACCG GCGGCCAGCG GCGCATTCTC AACGCCGGGC TCGTCGAACC ATTCGGGGGC   240
GATTTCCGCA CGACCGCGAT GCTGGTTGGA GAGCCAGGCC CTGGCCAGCA ACTGGCACAG   300
GTTCAGGTAA CCCTGCTTGT CCCGCACCAA CAGCAGCAGG CGGGTCGGCT TGTCGCGCTC   360
GTCGTGATTG GTGATCCACA CGTCAGCCCC GACGATGGGC TTCACGCCCT TGCCACGCGC   420
TTCCTTGTAG ANGCGCACCA GCCCGAAGGC ATTGGCGAGA TCGGTCAGCG CCAAGGCGCC   480
CATGCCATCT TTGGCGGCAG CCTTGACGGC ATCGTCGAGA CGGACATTGC CATCGACGAC   540
GGAATATTCG GAGTGGAGAC GGAGGTGGAC GAAGCGCGGC GAATTCATCC GCGTATTGTA   600
ACGGGTGACA CCTTCCGCAA AGCATTCCGG ACGTGCCCGA TTGACCCGGA GCAACCCCGC   660
ACGGCTGCGC GGGCAGTTAT AATTTCGGCT TACGAATCAA CGGGTTACCC CAGGGCGCTG   720
AAGCCTATCG CGTGCAGTTG CCGGATGC                                      748
```

(2) INFORMATIONS POUR LA SEQ ID NO: 13:

(i) CARACTERISTIQUES DE LA SEQUENCE:

(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 13:
GCATCCGGCA ACTGCACG 18

(2) INFORMATIONS POUR LA SEQ ID NO: 14:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 14:
GTAGTTCGAT GTAGAAAGCG 20

(2) INFORMATIONS POUR LA SEQ ID NO: 15:

(i) CARACTERISTIQUES DE LA SEQUENCE:

(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 15:
GCATCCGGCA ACTGCACG 18

(2) INFORMATIONS POUR LA SEQ ID NO: 16:

(i) CARACTERISTIQUES DE LA SEQUENCE:

(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 16:
AGGAGTAAGG AAACCCAACG GAC 23

(2) INFORMATIONS POUR LA SEQ ID NO: 17:

(i) CARACTERISTIQUES DE LA SEQUENCE:

(A) LONGUEUR: 19 paires de bases
(B) TYPE: nucléotide

(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 17:
TAAGAGTTGC ACAAGTGCG 19

(2) INFORMATIONS POUR LA SEQ ID NO: 18:

(i) CARACTERISTIQUES DE LA SEQUENCE:

(A) LONGUEUR: 21 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 18:
TCAGGGATAG CCCCCATCTA T 21

(2) INFORMATIONS POUR LA SEQ ID NO: 19:

(i) CARACTERISTIQUES DE LA SEQUENCE:

(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 19:
AACCCTTTGC CACTACATCA ATTT 24

(2) INFORMATIONS POUR LA SEQ ID NO: 20:

(i) CARACTERISTIQUES DE LA SEQUENCE:

(A) LONGUEUR: 15 paires de bases
(B) TYPE: nucléotide
(C) NOMBRE DE BRINS: simple
(D) CONFIGURATION: linéaire

(ii) TYPE DE MOLECULE: ADNc
(ix) CARACTERISTIQUES:

(B) EMPLACEMENT: 5, 7, 10, 13
(D) AUTRES INFORMATIONS: G représente l'inosine (i)

(xi) DESCRIPTION DE LA SEQUENCE: SEQ ID NO: 20:
GGTCGTGCCG CAGGG 15

(2) INFORMATIONS POUR LA SEQ ID NO: 21:

(i) CARACTERISTIQUES DE LA SEQUENCE:

(A) LONGUEUR: 21 paires de bases
(B) TYPE: nucléotide
(C) NOMBRE DE BRINS: simple

(D) CONFIGURATION: linéaire

(ii) TYPE DE MOLECULE: ADNc
(xi) DESCRIPTION DE LA SEQUENCE: SEQ ID NO: 21:
TTAGGGATAG CCCTCATCTC T 21

(2) INFORMATIONS POUR LA SEQ ID NO: 22:

(i) CARACTERISTIQUES DE LA SEQUENCE:

(A) LONGUEUR: 21 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 22:
TCAGGGATAG CCCCCATCTA T 21

(2) INFORMATIONS POUR LA SEQ ID NO: 23:

(i) CARACTERISTIQUES DE LA SEQUENCE:

(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 23
AACCCTTTGC CACTACATCA ATTT 24

(2) INFORMATIONS POUR LA SEQ ID NO: 24:

(i) CARACTERISTIQUES DE LA SEQUENCE:

(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 24
GCGTAAGGAC TCCTAGAGCT ATT 23

(2) INFORMATIONS POUR LA SEQ ID NO: 25:

(i) CARACTERISTIQUES DE LA SEQUENCE:

(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 25
TCATCCATGT ACCGAAGG 18

(2) INFORMATIONS POUR LA SEQ ID NO: 26:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 26
ATGGGGTTCC CAAGTTCCCT 20

(2) INFORMATIONS POUR LA SEQ ID NO: 27:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 27
GCCGATATCA CCCGCCATGG 20

(2) INFORMATIONS POUR LA SEQ ID NO: 28:

(i) CARACTERISTIQUES DE LA SEQUENCE:

(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 28
GCATCCGGCA ACTGCACG 18

(2) INFORMATIONS POUR LA SEQ ID NO: 29:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 29
CGCGATGCTG GTTGGAGAGC 20

(2) INFORMATIONS POUR LA SEQ ID NO: 30:

(i) CARACTERISTIQUES DE LA SEQUENCE:

(A) LONGUEUR: 20 paires de bases
(B) TYPE: nucléotide

(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 30
TCTCCACTCC GAATATTCCG 20

(2) INFORMATIONS POUR LA SEQ ID NO: 31:

(i) CARACTERISTIQUES DE LA SEQUENCE:

(A) LONGUEUR: 26 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 31
GATCTAGGCC ACTTCTCAGG TCCAGS 26

(2) INFORMATIONS POUR LA SEQ ID NO: 32:

(i) CARACTERISTIQUES DE LA SEQUENCE:

(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc
(ix) CARACTERISTIQUES:

(B) EMPLACEMENT: 6, 12, 19
(D) AUTRES INFORMATIONS: G représente l'inosine (i)

(xi) DESCRIPTION DE LA SEQUENCE: SEQ ID NO: 32
CATCTGTTTG GGCAGGCAGT AGC 23

2) INFORMATIONS POUR LA SEQ ID NO: 33:

(i) CARACTERISTIQUES DE LA SEQUENCE:

(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 33
CTTGAGCCAG TTCTCATACC TGGA 24

2) INFORMATIONS POUR LA SEQ ID NO: 34:

(i) CARACTERISTIQUES DE LA SEQUENCE:

(A) LONGUEUR: 22 paires de bases
(B) TYPE: nucléotide
(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 34

AGTGYTRCCM CARGGCGCTG AA 22

2) INFORMATIONS POUR LA SEQ ID NO: 35:

(i) CARACTERISTIQUES DE LA SEQUENCE:

(A) LONGUEUR: 22 paires de bases

(B) TYPE: nucléotide

(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 35

GMGGCCAGCA GSAKGTCATC CA 22

2) INFORMATIONS POUR LA SEQ ID NO: 36:

(i) CARACTERISTIQUES DE LA SEQUENCE:

(A) LONGUEUR: 22 paires de bases

(B) TYPE: nucléotide

(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 36

GGATGCCGCC TATAGCCTCT AC 22

2) INFORMATIONS POUR LA SEQ ID NO: 37:

(i) CARACTERISTIQUES DE LA SEQUENCE:

(A) LONGUEUR: 22 paires de bases

(B) TYPE: nucléotide

(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 37

AAGCCTATCG CGTGCAGTTG CC 22

(2) INFORMATIONS POUR LA SEQ ID NO: 38:

(i) CARACTERISTIQUES DE LA SEQUENCE:

(A) LONGUEUR: 40 paires de bases

(B) TYPE: nucléotide

(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 38:

TAAAGATCTA GAATTCGGCT ATAGGCGGCA TCCGGCAAGT 40

(2) INFORMATIONS POUR LA SEQ ID NO: 39:

(i) CARACTERISTIQUES DE LA SEQUENCE:

(A) LONGUEUR: 50 acides aminés
(B) TYPE: acides aminés

(ii) TYPE DE MOLECULE: peptide
(xi) DESCRIPTION DE LA SEQUENCE: SEQ ID NO: 39:

```
Asp Ala Phe Phe Cys Ile Pro Val Arg Pro Asp Ser Gln Phe Leu Phe
 1               5                  10                  15
Ala Phe Glu Asp Pro Leu Asn Pro Thr Ser Gln Leu Thr Trp Thr Val
            20                  25                  30
Leu Pro Gln Gly Phe Arg Asp Ser Pro His Leu Phe Gly Gln Ala Leu
        35                  40                  45
Ala Gln
    50
```

(2) INFORMATIONS POUR LA SEQ ID NO: 40:

(i) CARACTERISTIQUES DE LA SEQUENCE:

(A) LONGUEUR: 150 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 40:

```
GATGCCTTTT TCTGCATCCC TGTACGTCCT GACTCTCAAT TCTTGTTTGC CTTTGAAGAT   60
CCTTTGAACC CAACGTCTCA ACTCACCTGG ACTGTTTTAC CCCAAGGGTT CAGGGATAGC  120
CCCCATCTAT TTGGCCAGGC ATTAGCCCAA                                   150
```

(2) INFORMATIONS POUR LA SEQ ID NO: 41:

(i) CARACTERISTIQUES DE LA SEQUENCE:

(A) LONGUEUR: 11 acides aminés
(B) TYPE: acides aminés

(ii) TYPE DE MOLECULE: peptide
(xi) DESCRIPTION DE LA SEQUENCE: SEQ ID NO: 41:

```
Cys Ile Pro Val Arg Pro Asp Ser Gln Phe Leu
 1               5                  10
```

(2) INFORMATIONS POUR LA SEQ ID NO: 42:

(i) CARACTERISTIQUES DE LA SEQUENCE:

(A) LONGUEUR: 17 acides aminés

(B) TYPE: acides aminés

(ii) TYPE DE MOLECULE: peptide

(xi) DESCRIPTION DE LA SEQUENCE: SEQ ID NO: 42:

```
Val Leu Pro Gln Gly Phe Arg Asp Ser Pro His Leu Phe Gly Glu Ala
 1               5                   10                  15
Leu
 17
```

(2) INFORMATIONS POUR LA SEQ ID NO: 43:

(i) CARACTERISTIQUES DE LA SEQUENCE:

(A) LONGUEUR: 8 acides aminés

(B) TYPE: acides aminés

(ii) TYPE DE MOLECULE: peptide

(xi) DESCRIPTION DE LA SEQUENCE: SEQ ID NO: 43:

```
Leu Phe Ala Phe Glu Asp Pro Leu
 1               5           8
```

(2) INFORMATIONS POUR LA SEQ ID NO: 44:

(i) CARACTERISTIQUES DE LA SEQUENCE:

(A) LONGUEUR: 8 acides aminés

(B) TYPE: acides aminés

(ii) TYPE DE MOLECULE: peptide

(xi) DESCRIPTION DE LA SEQUENCE: SEQ ID NO: 44:

```
Phe Ala Phe Glu Asp Pro Leu Asn
 1               5           8
```

(2) INFORMATIONS POUR LA SEQ ID NO: 45:

(i) CARACTERISTIQUES DE LA SEQUENCE:

(A) LONGUEUR: 25 paires de bases

(B) TYPE: nucléotide

(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 45:

GTGCTGATTG GTGTATTTAC AATCC 25

(2) INFORMATIONS POUR LA SEQ ID NO: 46:

(i) CARACTERISTIQUES DE LA SEQUENCE:

(A) LONGUEUR: 1859 paires de bases

(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 46:

```
GTGCTGATTG GTGTATTTAC AATCCTTTAT CTAATCCGAA ATGCCCATGT TGCAATATGG   60
AAAGAAAGGG AGTTCCTAAC CTCTGGGGGA ACCCCCATTA AATACCACAA GTAAATCATG  120
GAGTTATTGC ACACAGTGCA AAAACTCAAG GAGGTGGAAG TCTTACACTG CCAAAGCCAT  180
CAGAAAAGGG AAGAGGGGAG AAGAGCAGCA TAAGTGGCTA CAGAGGCAAG GAAAGACTAG  240
CAGAAAGGAA AGAGAGAAAG AGACAGAAAG TCAGAGAGAG AGAGAGGAAG AGACAGAGCA  300
CAAAGAGGGA GTCAGAGAGA GAGAGAGACA GAGAGTCAGA GAGAAGGAAA GAGAGAGAGG  360
AAGAGACAAA GAATGAATCA AACAGAGAGA CAGAAAGTCA GAGAGAGAGA GAGAGAGGAA  420
GAGACAGAGA AAAAGAGGGA GTCAGAAAAA GAGAGACCAA AGAAGAAGTC CAAAGAGAAA  480
GAAAGAGAGA TGGAAGTAGT AAAGGAAAAA CAGTGTACCC TATTCCTTTA AAAGCCGGGG  540
TAAATTTAAA ACCTATAATT GATAACTGAA GGTCTTCTCT GTAACCCTGT AACACTCCAA  600
TACCACCTTG TTGTCAAGTG TAAACAAGGG CGTAGCCCAA AAGCACTGAG GCCACTAACA  660
ACCCATAGCC TTCCTATCAA AATTCCTTAA CCCAGCAGGT TTCCTAACAG GGGATCTAAA  720
TCTTAATTAA TTACCATACA ATGGTCCAAC CAGACTTAGG AGGAATTCCC TTCAGGACGG  780
GAAGATAGAT GCTTCCTCCC AGGCGATTAA GGGAGAAAGA CACAATGGGT ATTCAGTAAG  840
TGCCAAGGGG AACACTTGTA GAAGCAAAGT TAGGAAAATT GCCAAATAAT TGGTTTGCTC  900
AAGAGTTGTT TGCACTCAGC CAAACCTTGA AGTACTTGCA GAATCAGAAA GGAGCCATCT  960
ATACCAATTC TAAGTTAATA TGGACTGAAG GAGGTTTTAT TAATACCAAA GAGAAATTAA 1020
AATCCCAAAC TTATAAGGTT TTCAACCAAA GTAAAGTTTG CTAAAAGTTA ACAGCGTAAC 1080
ATGTATTATC CTACTACCAC ACACTCTCAA AGGATTTCTC AGACAGTTTG CAAGAAATAA 1140
TGATATCTAT CCTTACTCTA CAATCCCAAA TAGACTCTTT GGCAGCAGTG ACTCTCCAAA 1200
ACCGTCAAGG CCTAGACCTC CTCACTGCTG AGAAAGGAGG ACTCTGCACC TTCTTAAGGG 1260
AAGAGTGTTG TCTTTACACT AACCAGTCAG GGATAGTATG AGATGCTGCC CGGCATTTAC 1320
AGAAAAAGGC TTCTGAAATC AGACAACGCC TTTCAAATTC CTATACCAAC CTCTGGAGTT 1380
GGGCAACATG GTTTCTTCCC TTTCTATGTC CCATGGCTGC CATCTTGCTA TTACTCGCCT 1440
TTGGGCCCTG TATTTTTAAC CTCCTTGTCA AATTTGTTTC TTCTAGGATC GAGGCCATCA 1500
AGCTACAGAT GGTCTTACAA ATGGAACCCC AAATGAGCTC AACTATCAAC TTCTACTGAG 1560
GACCCCTAGA CCAACCCCCT GGCCCTTTCA CTGGCCTAAA GAGTTCCCCT CTGGAGGACA 1620
CTACCACTGC AGGGCCCCAT CTTTGCCCCT ATCCAGAAGG AAGTAGCTAG AGCAGTCATT 1680
GCCCAATTCC CAAGAGCAGC TGGGGTGTCC CGTTTAGAGT GGGGATTGAG AGGTGAAGCC 1740
AGCTGGACTT CTGGGTCGGG TGGGGACTTG GAGAACTTTT GTGTCTAGCT AAAGGATTGT 1800
AAATGCAACA ATCAGTGCTC TGTGTCTAGC TAAAGGATTG TAAATACACC AATCAGCAC  1859
```

(2) INFORMATIONS POUR LA SEQ ID NO: 47:

(i) CARACTERISTIQUES DE LA SEQUENCE:

(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 47:
TGATGTGAAC GGCATACTCA CTG 23

(2) INFORMATIONS POUR LA SEQ ID NO: 48:

(i) CARACTERISTIQUES DE LA SEQUENCE:

(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 48:
CCCAGAGGTT AGGAACTCCC TTTC 24

(2) INFORMATIONS POUR LA SEQ ID NO: 49:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 49:
GCTAAAGGAG ACTTGTGGTT GTCAG 25

(2) INFORMATIONS POUR LA SEQ ID NO: 50:

(i) CARACTERISTIQUES DE LA SEQUENCE:

(A) LONGUEUR: 22 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 50:
CAACATGGGC ATTTCGGATT AG 22

(2) INFORMATIONS POUR LA SEQ ID NO: 51:

(i) CARACTERISTIQUES DE LA SEQUENCE:

(A) LONGUEUR: 400 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 51:

```
GGCTGCTAAA GGAGACTTGT GGTTGTCAGA CAATCGCCTA CTTAGGTACC AGGCCTTATT   60
ACTTGAGGGA CTGGTGCTTC AGATGCGCAC TTGTGCAGCT CTTAACCCAA ACTTATGCTG  120
CCCAGAAGGA TCTTTTAGAG GTCCCCTTAG CCAACCCTGA CCTCAACCTA TATATATACT  180
GATGGAAGTT CGTTTGTAGA AAAGGGATTA CAAAGGGNAG GATATNCCAT AGGTTAGTGA  240
TAAAGCAGTA CTTGAAAGTA AGCCTCTTCC CCCCAGGGAC CAGCGCCCCC GTTAGCAGAA  300
CTAGTGGCAC TGACCCCGAG CCTTAGAACT TGGAAAGGGA GGAGGATAAA TGTGTATACA  360
GATAGCAAGT ATGCTTATCT AATCCGAAAT GCCCATGTTG                         400
```

(2) INFORMATIONS POUR LA SEQ ID NO: 52:

(i) CARACTERISTIQUES DE LA SEQUENCE:

(A) LONGUEUR: 2389 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 52:

```
TCAGGGATAG CCCCCATCTA TTTGGTCAGG CACTGGCCCA AGATCTAGGG ACATGCCACT   60
TTTAAGAGCC ATTTCTCAAG TCCAGGTACT CTGGTCCTTC GGTATGTGGA TGATTTACTT  120
TTGGCTACCA GTTCAGTAGC CTCATGCCAG CAGGCTACTC TAGATCTCTT GAACTTTCTA  180
GCTAATCAAG GGTACAAGGC ATCTAGGTTG AAGGCCCAGC TTTGCCTACA GCAGGTCAAA  240
TATCTAGGCC TAATCTTAGC CAGAGGGACC AGGGCACTCA GCAAGGAACA AATACAGCCT  300
ATACTGGCTT ATCCTCACCC TAAGACATTA AAACAGTTGC GGGGGTTCCT TGGAATCACT  360
GGCTTTTTGG TGACTATGGA TTCCCAGATA CAGCAAGATT GGCAGGCCCC TCTATACTGT  420
AATCAAGGAG ACTCACGAGG GCAAGTACTC ATCTAGTAGA ATGGGAACTA GGGACAGAAA  480
CAGCCTTCAA AACCTTAAAG CAGGCCCTAG TACAATCTCC AGCTTTAAGC CTTCCCACAG  540
GACAAAACTT CTCTTTATAC ATCACAGAGA GGGCAGAGAT AGCTCTTGGT GTCCTTATTC  600
AGACTCATGG GACTACCCCA CAACCAGTGG CACACCTAAG TAAGGAAATT GATGTAGTAG  660
CAAAAGGCTG GCCTCACTGT TTATGGGTAG CTGTGGTGGT GGCTGTCTTA GTGTCAGAAG  720
CTATCAAAAT AATACAAGGA AAGGATCTCA CTGTCTGGAC TACTCATGAT GTAATGGCAT  780
ACTAGGTGCC AAAAGAAGTT TATGGGTATC AGACAACCAC CTGCTTAGAT ACCAGGGACT  840
ACTCCTGGAG GATTGGGCTT CAAGTGCGTT TTTTGTGGCC TCAACCCTGC CACTTTTCCT  900
CCAGAGGATG GAGAGCCGCT TGAGCATGCT TGCCAACAGG TTGTAGGCCA GAATTATTCC  960
ACCCGAGATG ATCTCTTAGA GTACCCTTAG CTAATCCTGA CCTTAACCTA TATACCAATG 1020
GAAGTTCATT TGTGGAAAAC GGGATATGAA GGGCAGGTTA TGTCATAGTT AGTGATGTAA 1080
TCATACTTGC AAGTAAGCCT CTTACCCCAG GGGCCAGCAC TCAGTTAGCA GAACTAGTCA 1140
CACTTACCTT AACCTTAGAA CTGGGAAAGG GAAAAAGAAT AAATATGTAT ACAGATAGTA 1200
AGTATGCTTA TCTAATCCTA CATGCCCATG CTGCAATATG GAAGGAAAGG GAGTTCCTAA 1260
CCCCTGGGGG AACCCCCATT AAATACCACA AGGYAAATCA TGGAGTTATT GCACGCAGTG 1320
CAAAAACTCA AGGAGGTGGC AGTCTTACAC TGCCGAAGCY ATCAAAAAGG GGAAGGAGAG 1380
GGGAGAACAG CAGCATAAGT GGTTGGCAGA GGCAGTGAAA GACCAGCAGA GAGAAGGAGA 1440
GAGACAACGT CAACGACAGA AGGAAAGAAG AGGAGGAGAC AGAGAGGAAG AGACAGAGAG 1500
ACAGTTAGTC CAAGAGAGAG ACAGAGAGAG GAAGAGACAG ACAGAAAGTC CAAGAGAGAA 1560
GGAAAGAGAG GAAGAGACCA AGGAGTCCNA GAGAGAGAAA GAGATAGAAG TAGTAAAGAA 1620
AAAACATTGT ACCCTATTCC TTTAAAAGCC GGGGTATATT TAAAACCTAT AATTGATAAT 1680
TGAGTTCTTG CACCCTCCTC CAGGGGATYG CTGGGAGGAA ACCCTCAACC GATATGTGAA 1740
AATTGTGGGT CGTCCCTATG TCTCAATTAC CAGCCAATAC CCCCTTGTTT TTAGTGTGAA 1800
CGAGGGTGTA GAGCGCAGAC AGGGAGACCT CTGACAATCC ATACCCTTCC TATCCAAAAT 1860
CCTTAACCCA GCAGGTTTTC TAAAAGGGGA TCTAAATCTT AATTAATTAC CATACAAAGG 1920
TCAAACCAGA TCTAGGAGGA ACTTCCTTCA GGACAGGATG ATAGATGGTT CCTCCCAGGC 1980
GATTAAAGAA AATAAAAAGA CACATGGGCA GCCAGTAAGT GATAAGGGAA CACTAGTAGA 2040
AGCAGTTAGG AGAAGTTGCC TAATAATTGG TCTACTCCAA ATGTGTGAGT TGTTCGCACT 2100
CAGCCCAAAT CTTAAAGTAC TTACAGAATT AGGGAGGAGC CATTTACACC AATTCTAAGT 2160
TAATATGGAC TGGATGAGGT TTTATTAATA GCGAAGGAGA ATTAAATCCT AAACTNACAA 2220
GGTTTTCAAC TAAAGTAAAT TTTACTAAAA GCTAACAGTG TAACATGCAT TATCCTACTA 2280
CAACACACTC TCANAGGATT CCTCAGACAG TTTACAAGAA ATAACAAAAT CTATCTGGTA 2340
AGGATAGTAA CTACAATCCC AAATACATTC TTTGGCAGCA GTGACTCTC             2389
```

(2) INFORMATIONS POUR LA SEQ ID NO: 53:

(i) CARACTERISTIQUES DE LA SEQUENCE:

(A) LONGUEUR: 2448 paires de bases

(B) TYPE: nucléotide

(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 53:

```
TCAGGGATAG CCCCCATCTA TTTGATCAGG CACTAGCCCA AGATCTAGGC CACTTCTGAA   60
GTCCAGGCAT TCTAGTCCTT CAGTATGTGG ATGATTTACT TTTGGCTACC AGTTTGGAAG  120
CCTCATGCCA GCAGGCTACT TGAGATCTCT TGAACTTTCT AGCTAATCAA GGGTGTATGG  180
CATCTAAATT GAAAGTCCAG CTCTGCCTAC AACAAGTCAA ATATCTAGGC CTAATCTTAG  240
ATAGAAGAAC CAGGGCCCTC AGCAAGGAAT GAATAAAGCC TATGCTGGCT TATCGGCACC  300
CTAAGACATT AAAACAATTG TGGGGGTTCC TTGGAATCAC TGGCTTTTGC CGACTATGGA  360
TCCCTGGATA GAGTGAGATA GCCAGGCCCC CTCTATTACT CTTATCAAGG AGACCCAGAG  420
GGCAAATACT TATCTAGTAT TATGGGNACC AGAGGCAGAA AAAGCCTTCC AAACCTTAAA  480
GGAGACCCTA GTACAAGCTC CAGCTTTAAG CCTTCCCACA GGACAAANCT TCTCTTTATA  540
TGTCACAGAG AGAGCAGGAA TAGCTCCTGG AGTCCTTACT CAGACTTTTG GACGACCCCA  600
CGGCCAGTGG CRTACCTAAG TAAGGAAATT GATGTAGTAG CAAAAGGCTG GCCTCACTGT  660
TTATGGGTAG TTGCGGCTGT GGCAGTCTTA CTGTCAAAGG CTATCAAAAT AATACAAGGA  720
AAGGATTTCA CTATCTGGAC TACTCATGAG GAAAATGGCA TATTAGGTGC CAAAGGAAGT  780
TTTTGGCTAT CAGACAACCA CCTGCTCAGA TTCCAGGCAC TACTGATTGA GAGACCAGTG  840
CTTTAAATAT GTATGTGTGT GTGTGGCCCT CAACCCTGCC ACTGTTCTCC CAGAAGATGG  900
AGAACCAATG AAGCATTACT GTCAACAAAT TAGAGTCCAG AGTTATGCTG CCTGAGAGGA  960
TCTCTTAGAA GTCCCCTTAG CTAATCCTGA CCTTAACCTA TATGCTGATG GAAGTTCACT 1020
TGTGGAGAAT GGGATACGAA AAGCACATTA TGCCATAGTT AGTGAGGTAA CAGTACTTGA 1080
AAGTAAGCCT ATTCCCCCAT GGACCAGAGC CCAGTTAGCA GAACTAGTGG CACTTACCCA 1140
AGCCTTAGAA CTAGGAAAGG GAAAAATAAT AAATGTGTAT ACAGATAGCA AGTATGCTTA 1200
TCTAATCCTA CATGCCCATG CTGCAGTATG GAAAGAAAGG GAGTTCCTAA CCTCTGGGGG 1260
AACCCCCATT AAATACCACA AGGCAAATCA TGGAGTTATT GCATGTAGTG CAAAACCTCA 1320
AGTAGGTGGC AGTTTTACAC TGCCTGAAGC TATGGGGAAG GAGAGAGGAG AACAGCAGCA 1380
TAAGTGGCTA GCAGAGGCAG CGAAAGACTA GCAGAGAGGA GAGGTAGGGG AAAGACAGAA 1440
AGTCAAAGAA AAGAAGTCAA AGACAGACAG AGAAAGAGAC AGAGGGAGCC AGAGAGAAAG 1500
AAAAGAGAGA ACGAAAGAGA CAGAATGTCA AAGAACAGAA GAGAGAGGCA GCGCCAGAAG 1560
AGTTAAGAAA GTGAGAAAGA GAGATGGAAA TAGTAAAGAA AAAACAGTGT ACCCTATTCC 1620
TTTAAAAGCC AGGGTAAATT TAAAACGTAT AATTTTATAA TTGGAAGGTC TTCTCCATAA 1680
CCCTATAACA TTAAAATACC ACCTTGTTGT CAGTGTAAAC AAGAGCATAG CCCAAAAGCA 1740
CTGAGGCCAC TGACAACCCA TAGCCTTCCT ATCAAAAATC CTTAACTCTG CAGGTTTCCT 1800
AACAGGGGAT CTAAATCTCA ACTAATCACC ATACAATGGT CCGACCAGAC CTAGGAGCGA 1860
CTCCCCTCAG GACAGAAGGA TGGATGGTTC CTCCCAGGCC ATTAAGGGAA AGAGACACAA 1920
TGGGTATTCA GTAAGTGATA AGGGAACTCT TGTAGAAGCA GTTAGGAAGA TTGCCTAATA 1980
TTTGGTCTGC TCAAATGTGC CAGCTGTTTG CACTCAGCTA AACCTTAAAT TACTTACAGA 2040
ATTAGGAAGG AGCCATCTAT ACCAATTCTG AGTTAATATG AGCTGAACAA GTTCTTATTA 2100
ATAGCAAAGA ATCATTGAAA TCTCAAACTT GCAAAGTTTT CAACAAAAGT AAAGTTTGCT 2160
GAAAGTTAGC AGTGTAACAT GTATTATCCT AACTTCTAAT CTTGTGGAAA TCAGACCCTA 2220
TCAGTGCCCC TCAAAGCTGA AGTCCATCAG CATATGGCCA TACAACTAAT ACCCCTATTT 2280
ATAGGGTTAG GAATGGCCAC TGCTACAGGA ATGGGAGTAA CAGGTTTATC TACTTCATTA 2340
TCCTATTACC ACACACTCTT AAAGGATTTC TCAGACAGTT TACAAGAAAT AACAAAATCT 2400
ATCCTTACTC TNTARTCCCA AATAGRTTCT TTGGCAGCAG TGACTCTC             2448
```

(2) INFORMATIONS POUR LA SEQ ID NO: 54:

(i) CARACTERISTIQUES DE LA SEQUENCE:

(A) LONGUEUR: 21 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 54:
CCTGAGTTCT TGCACTAACC C 21

(2) INFORMATIONS POUR LA SEQ ID NO: 55:

(i) CARACTERISTIQUES DE LA SEQUENCE:

(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 55:

```
GTCCGTTGGG TTTCCTTACT CCT          23
```

(2) INFORMATIONS POUR LA SEQ ID NO: 56:

(i) CARACTERISTIQUES DE LA SEQUENCE:

(A) LONGUEUR: 1196 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 56:

```
TTCCTGAGTT CTTGCACTAA CCTCAAATGA GAGAAGTGCC GCCATAACTG CAACCCAAGA    60
GTTTGGCGAT CCCTGGTATC TCAGTCAGGT CAATGACAGG ATGACAACAG AGGAAAGATA   120
ATGATTCCCC ACAGGCCAGC AGGCAGTTCC CAGTGTAGAC CCTCATTAGG ACACAGAATC   180
AGAACATGGA GATTGGTGCC GCAGACATTT GCTAACTTGC GTGCTAGAAG GACTAAGGAA   240
AACTAGGAAG ATATGAATTA TTCAATGATG TCCACTATAA CACAGGGGAA AGGAAGAAAA   300
TCCTACTGCC TTTCTGGAGA GACTAAGGGA GGCATTGAGG AAGCATACCA GGCAAGTGGA   360
CATTGGAGGC TCTGGAAAAG GGAAAAGTTG GGAAAAGTAT ATGTCTAATA GGGCTTGCTT   420
CCAGTGTGGT CTACAAGGAC ACTTTAAAAA AGATTGTCCA ATAGAAATAA GCCACCACCT   480
CGTCCATGCC CCTTATGTCA AGGGAATCAC TGGAAGGCCC ACTGCCCCAG GGGATGAAGG   540
TCCTCTGAGT CAGAAGCCAC TAACCAGATG ATCCAGCAGC AGGACTGAGG GTGCCCGGGG   600
CAAGCGCCAG CCCATGCCAT CACCCTCACA GAGCCCCAGG TATGCTTGAC CATTGAGGGT   660
CAGAAGGGTA CTGTCTCCTG GACACTGGCG GGCCTTCTCA GTCTTACTTT CCTGTCCTGG   720
ACAACTGTCC TCCAGATCTG TCACTGTCCG AGGGGTCCTA GGACAGCCAG TCACTAGATA   780
CTTCTCCCAG CCACTAAGTT GTGACTGGGG AACTTTACTC TTCCACATGC TTTTCTAATT   840
ATGCCTGAAA GCCCCACTCT CTTGTTAGGG GAGAGACATT CTAGCAAAAG CAGGGGCCAT   900
TATACATGTG AATATAGGAG AAGGAACAAC TGTTTGTTGT CCCCTGCTTG AGGAAGGAAT   960
TAATCCTGAA GTCCGGGCAA CAGAAGGACA ATATGGACAA GCAAAGAATG CCCGTCCTGT  1020
TCAAGTTAAA CTAAAGGATT CCACCTCCTT TCCCTACCAA AGGCAGTACC CCCTCAGACC  1080
CGAGACCCAA CAAGAACTCC AAAAGATTGT AAAGGACCTA AAAGCCCAAG GCCTAGTAAA  1140
ACCAAGCAAT AGCCCTTGCA AGACTCCAAT TTTAGGAGTA AGGAAACCCA ACGGAC      1196
```

(2) INFORMATIONS POUR LA SEQ ID NO: 57:

(i) CARACTERISTIQUES DE LA SEQUENCE:

(A) LONGUEUR: 2391 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 57:

```
ATGATCCAGC AGCAGGACNG AGGGTGCCCG GGGCAAGCGC CAGCCCATGC CATCACCCTC   60
ACAGAGCCCC AGGTATGCTT GACCATTGAG GGTCAGAAGG GTNACTGTCT CCTGGACACT  120
GGCGGNGCCT TCTCAGTCTT ACTTTCCTGT CCTGGACAAC TGTCCTCCAG ATCTGTCACT  180
GTCCGAGGGG TCCTAGGACA GCCAGTCACT AGATACTTCT CCCAGCCACT AAGTTGTGAC  240
TGGGGAACTT TACTCTTCCC ACATGCTTTT CTAATTATGC CTGAAAGCCC CACTCTCTTG  300
TTGGGGAGAG ACATTCTAGC AAAAGCAGGG GCCATTATAC ATGTGAATAT AGGAGAAGGA  360
ACAACTGTTT GTTGTCCCCT GCTTGAGGAA GGAATTAATC CTGAAGTCCG GGCAACAGAA  420
GGACAATATG GACAAGCAAA GAATGCCCGT CCTGTTCAAG TTAAACTAAA GGATTCCACC  480
TCCTTTCCCT ACCAAAGGCA GTACCCCCTC AGACCCGAGA CCCAACAAGA ACTCCAAAAG  540
ATTGTAAAGG ACCTAAAAGC CCAAGGCCTA GTAAAACCAA GCAATAGCCC TTGCAAGACT  600
CCAATTTTAG GAGTAAGGAA ACCCAACGGA CAGTGGAGGT TAGTGCAAGA ACTCAGGATT  660
ATCAATGAGG CTGTTGTTCC TCTATACCCA GCTGTACCTA ACCCTTATAC AGTGCTTTCC  720
CAAATACCAG AGGAAGCAGA GTGGTTTACA GTCCTGGACC TTAAGGATGC CTTTTTCTGC  780
ATCCCTGTAC GTCCTGACTC TCAATTCTTG TTTGCCTTTG AAGATCCTTT GAACCCAACG  840
TCTCAACTCA CCTGGACTGT TTTACCCCAA GGGTTCAGGG ATAGCCCCCA TCTATTTGGC  900
CAGGCATTAG CCCAAGACTT GAGTCAATTC TCATACCTGG ACACTCTTGT CCTTCAGTAC  960
ATGGATGATT TACTTTTAGT CGCCCGTTCA GAAACCTTGT GCCATCAAGC CACCCAAGAA 1020
CTCTTAACTT TCCTCACTAC CTGTGGCTAC AAGGTTTCCA AACCAAAGGC TCGGCTCTGC 1080
TCACAGGAGA TTAGATACTN AGGGCTAAAA TTATCCAAAG GCACCAGGGC CCTCAGTGAG 1140
GAACGTATCC AGCCTATACT GGCTTATCCT CATCCCAAAA CCCTAAAGCA ACTAAGAGGG 1200
TTCCTTGGCA TAACAGGTTT CTGCCGAAAA CAGATTCCCA GGTACASCCC AATAGCCAGA 1260
CCATTATATA CACTAATTAN GGAAACTCAG AAAGCCAATA CCTATTTAGT AAGATGGACA 1320
CCTACAGAAG TGGCTTTCCA GGCCCTAAAG AAGGCCCTAA CCCAAGCCCC AGTGTTCAGC 1380
TTGCCAACAG GGCAAGATTT TTCTTTATAT GCCACAGAAA AAACAGGAAT AGCTCTAGGA 1440
GTCCTTACGC AGGTCTCAGG GATGAGCTTG CAACCCGTGG TATACCTGAG TAAGGAAATT 1500
GATGTAGTGG CAAAGGGTTG GCCTCATNGT TTATGGGTAA TGGNGGCAGT AGCAGTCTNA 1560
GTATCTGAAG CAGTTAAAAT AATACAGGGA AGAGATCTTN CTGTGTGGAC ATCTCATGAT 1620
GTGAACGGCA TACTCACTGC TAAAGGAGAC TTGTGGTTGT CAGACAACCA TTTACTTAAN 1680
TATCAGGCTC TATTACTTGA AGAGCCAGTG CTGNGACTGC GCACTTGTGC AACTCTTAAA 1740
CCCAAACTTA TGCTGCCCAG AAGGATCTTT NTAGAGGTCC CCTTAGCCAA CCCTGACCTC 1800
AACTATATAT ATACTGATGG AAGTTCGTTT GTAGAAAAGG GATTACAAAG GGNAGGATAT 1860
NCCATAGGTG TTAGTGATAA AGCAGTACTT GAAAGTAAGC CTCTTCCCCC CCAGGGACCA 1920
GCGCCCCCGT TAGCAGAACT AGTGGCACTG ACCCCGCGAG CCTTAGAACT TTGGAAAGGG 1980
AGGAGGATAA ATGTGTATAC AGATAGCAAG TATGCTTATC TAATCCGAAA TGCCCATGTT 2040
GTTTATCTAA TCCGAAATGC CCATGTTGCA ATATGGAAAG AAAGGGAGTT CCTAACCTCT 2100
GGGGGAACCC CCATTAAATA CCACAAGTTA ATCATGGAGT TATTGCACAC AGTGCAAAAA 2160
CTCAAGGAGG TGGAAGTCTT ACACTGCCAA AGCCATCAGA AAAGGGAAAG GGGAGAAGAG 2220
CAGCATAAGT GGCTACAGAG GCAAGGAAAG ACTAGCAGAA AGGAAAGAGA GAAAGAGACA 2280
GAAAGTCAGA GAGAGAGAGA GGAAGAGACA GAGCACAAAG AGGGAGTCAG AGAGAGAGAG 2340
AGACAGAGAG TCAGAGAGAA GGAAAGAGAG AGAGGAAGAG ACAAAGAATG A          2391
```

(2) INFORMATIONS POUR LA SEQ ID NO: 58:

(i) CARACTERISTIQUES DE LA SEQUENCE:

(A) LONGUEUR: 1722 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 58:

```
TGGAGAATAG CAGCATAAGT TGGCTGGCAG AAGTAGGGAA AGACAGCAAG AAGTAAAGAA   60
AAAAARGAGA AAGTCAGAGA AAGAAAAAAA GAGAGGAAGA AACAAAGAAG AACTTGAAGA  120
GAGAAAGAAG TAGTAAAGAA AAAACAGTAT ACCCTATTCC TTTAAAAGCC AGGGTAAATT  180
TCTGTCTACC TAGCCAAGGC ATATTCTTCT TATGTGGAAC ATCAACCTAT ATCTGCCTCC  240
CCACTAACTG GACAGGCACC TGAACCTTAG TCTTTCTAAG TCCCAACATT AACATTGCCC  300
CAGGAAATCA GACCCTATTG GTACCTGTCA AAGCTAAAGT CCCGTCAGTG CAGAGCCATA  360
```

```
CAACTAATAT CCCTATTTAT AGGGTTAGGA ATGGCTACTG CTACAGGAAC TGGAATAGCC  420
GGTTTATCTA CTTCATTATC CTACTACCAT ACACTCTCAA AGAATTTCTC AGACAGTTTG  480
CAAGAAATAA TGAAATCTAT TCTTACTTTA CAATCCCAAT TAGACTCTTT GGCAGCAATG  540
ACTCTCCAAA ACCGCCGAGG CCCACACCTC CTCACTGCTG AGAAAGGAGG ACTCTGCACC  600
TTCTTAGGGG AAGAGTGTTG TTTTTACACT AACCAGTCAG GGATAGTACG AGATGCCACC  660
TGGCATTTAC AGGAAAGGGC TTCTGATATC AGACAATGCC TTTCAAACTC TTATACCAAC  720
CTCTGGAGTT GGGCAACATG GCTTCTTCCA TTTCTAGGTC CCATGGCAGC CATCTTGCTG  780
TTACTCACCT TTGGGCCCTG TATTTTTAAG CTTCTTGTCA AATTTGTTTC CTCTAGGATC  840
GAAGCCATCA AGCTACAGAT GGTCTTACAA ATGGAACCCC AAATGAGTTC AACTAACAAC  900
TTCTACCAAG GACCCCTGGA ACGATCCACT GGCACTTCCA CTAGCCTAGA GATTCCCCTC  960
TGGAAGACAC TACAACTGCA GGGCCCCTTC TTTGCCCCTA TCCAGCAGGA AGTAGCTAGA 1020
GCGGTCATCG GCCAAATTCC CAACAGCAGT TGGGGTGTCC TGTTTAGAGG GGGGATTGAA 1080
GAGGTGACAG CCTGCTGGCA GCCTCACAGC CCTCGTTGGY TCTCAGTGCC TCCTCAGCCT 1140
TGGTGCCCAC TCTGGCCGTG CTTGAGGAGC CCTTCAGCCT GCCACTGCAC TGTGGGAGCC 1200
TCTTTCTGGG CTGGACAAGG CCGGAGCCAG CTCCCTCAGC TTGCAGGGAG GTATGGAGGG 1260
AGAGATGCAG GCGGGAACCA GGGCTGCGCA TGGCGCTTGC GGGCCAGCAT GAGTTCCAGG 1320
TGGGCGTGGG CTCGGCGGGC CCCACACTCG GGCAGTGAGG GGCTTAGCAC CTGGGCCAGA 1380
CAGATGCTGT GCTCAACTTC TTCGCTGGGC CTTAGCTGCC TTCCCCGTGG GGCAGGGCTY 1440
CGGGAACMTG CAGCCTGCCC ATGCTTGAGC CCCCCACCCC GCCGTGGGTT CYTGCACAGC 1500
CCAAGCTTCC CGGACAAGCA CCACCCCTTA TCCACGGTGC CCAGTCCCAT CAACCACCCA 1560
AGGGTTGAGG AGTGCGGGCA CACAGCGCGG GATTGGCAGG CAGTTCCACT TGCGGCCTTG 1620
GTGCGGGATC CACTGCGTGA AGCCAGCTGG GCTCCTGAGT CTGGTGGGGA CTTGGAGAAT 1680
CTTTATGTCT AGCTAAGGGA TTGTAAATAC ACCAATCAGC AC                   1722
```

(2) INFORMATIONS POUR LA SEQ ID NO: 59:

(i) CARACTERISTIQUES DE LA SEQUENCE:

(A) LONGUEUR: 495 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 59:

```
CTTCCCCAAC TAATAAGGAC CCCCCTTTCA ACCCAAACAG TCCAAAAGGA CATAGACAAA   60
GGAGTAAACA ATGAACCAAA GAGTGCCAAT ATTCCCTGGT TATGCACCCT CCAAGCGGTG  120
GGAGAAGAAT TCGGCCCAGC CAGAGTGCAT GTACCTTTTT CTCTCTCACA CTTGAAGCAA  180
ATTAAAATAG ACNTAGGTNA ATTNTCAGAT AGCCCTGATG GYTATATTGA TGTTTTACAA  240
GGATTAGGAC AATCCTTTGA TCTGACATGG AGAGATATAA TATTACTGCT AAATCAGACG  300
CTAACCTCAA ATGAGAGAAG TGCTGCCATA ACTGGAGCCC GAGAGTTTGG CAATCTCTGG  360
TATCTCAGTC AGGTCAATGA TAGGATGACA ACGGAGGAAA GAGAACGATT CCCCACAGGG  420
CAGCAGGCAG TTCCCAGTGT AGCTCCTCAT TGGGACACAG AATCAGAACA TGGAGATTGG  480
TGCCGCAGAC ATTTA                                                 495
```

(2) INFORMATIONS POUR LA SEQ ID NO: 60:

(i) CARACTERISTIQUES DE LA SEQUENCE:

(A) LONGUEUR: 2503 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 60:

```
CCAAGAACCC ACCAATTCCG GANCACATTT TGGCGACCAC GAAGGGACTT TCGCATATCG   60
CCAAGCGGTG AGACAATAGC CGAGCGGTGA GACCTTTCCC AATCGCCAAG CAGTGAGTAC  120
CATCAGACCC CTTTCACTTG CTATTCTGTC CTATCTTTCT TTAGAATTCG GGGGCTAAAT  180
ACCGGGCATC TGTCAGCCAT TTAAAAGTGA CTAGCGGGCC GCCGGACTAA AGACACGGGT  240
```

```
GTCAAGCTTT CTGGGAAAGG GCTCTCTAAC AACCCCCAAC TCTTTGGAGT TGGGACCGTT  300
GGTTTGCCTA GAACCAGCTT CCGCTTTTCC TGTACTTCTG GGCTGAGCCG TGGGTTGACA  360
GTGAAGGAAA GCCATGCATC TCCGGGGTCT CGMCAACATG TTGGTTGACC CTGCGGCCAT  420
GAGTGGAACT CTCAAAAGCA TGTCGCCCAA GCGACACTCG CCTATCTATC CTATCTATCC  480
TGACCCTTGC CCTCTGGGTC CTAATGCCTG CCAGACAAAC TTCCTCTCGC CTCTCTTCTC  540
TGAAGCTAGA ACCGCTTCTA AAAATTGCTA CCTGGTCTCT GGTGCTTTTC CTARTTTCTC  600
CTATAAAGAA TGAWTTCTAG TATTAAACTC CAGGACTCTG TTACCTTCTT TAGGCACCCG  660
GGCTCACCAA TCAGAAAGAC ACAGTTTTTG CCCAAGGCCC CATCGTAGTG GGGACTACCT  720
GGAATTTTAG GATCCCTCCT CAGACTAACA GGCCTAACAA AAGTTATTCC TGAAGCTAGG  780
ATATGGGGAG CCTCAGAAAT TGTATCCCTC CTATTCATAT AAGTGAGAAC AAAAGGTGTC  840
ACTCTTCCAA CCCTGAAGAT CCCCTCCCTC CCTCAGGGTA TGGCCCTCCA TTTCATTTTT  900
GTGGCATAAC ATCTTTATAG GATGGGGTAA AGTCCCAATA CTAACAGGAG AATGCTTAGG  960
ACTCTAACAG GTTTTTGAGA ATGCGTCAGT AAGGGCCACT AAATCTGATT TTTCTCAGTC 1020
GGTCCTCCTT GTGGTCTAGG AGGACAGGCA AGGTTGTGCA GGTTTTCGAG AATGCGTCAG 1080
TAAGGACCAC TAAATCCGAC CTTCCTCGGT CCTCCATGTG GTCTGGGAGG AAAACTAGTG 1140
TTTCTGCTGC TGCGTCGGTG AGCGCAACTA TTCAAGTCAG CAGGGTCCAG GGACCGTTGC 1200
AGGTTCTTGG GCAGGGGTTG TTTCTGCTGC TGCATTGGTG AATGCAACTA TTCTGATCAG 1260
CAGGGTCCCA GGACCATTGC AGGTCCTTGG GCAGGGAGAG AAACAAAACA AACCAAAACT 1320
GTGGGCGGTT TTGTCTTTCA TATGGGAAAC ACTCAGGCAT CAACAGGTTC ACCCTTGAAA 1380
TGCATCCTAA GCCATTGGGA CCAATTTGAC CCACAAACCC TGAAAAAGAG GAGGCTCATT 1440
TTTTCCTGCA CTACGGCTTG GCCCCAATAT TCTCTTTYTG ATGGGGAAAA ATGGCCACCT 1500
GAGGGAAGCA CAAATTACAA TAYTATCCTA CAGCYTGATC TTTTCTGTAA GAGGGAAGGC 1560
AAATGGAGTG AATACCTTAT GTCCAAGCTT TCTTTTCATT GAGGGAGAAT ACACAACTAT 1620
GCAAAGCTTG CAATTTACAT CCCACAGGAG GACCCTTCAG CTTACCCCCA TATCCTAGCC 1680
TCCCTATAGC TTCCCTTCCT ATTGATGATA CTCCTCCTCT AATCTCCCCT GCCCAGAAGG 1740
AAATAAGCAA AGAAATCTCC AAAGGTCCAC AAAAACCCCC GGGCTATCGG TTATGTCCCT 1800
TCAAGYTGTA GGGGGAGGGG AATTTGGCCC AACCCGGGTG CATGTCCCTT CTCCCTCTCT 1860
GATTTAAAGC AGATCAAGGC AGACCTGGGG AAGTTTTCAG ATGATCCTGA TAGGTACATA 1920
GATGTCCTAC AGGGTCTAGG GCAAACCTTT GACCTCACTT GGAGAGACGT CATGCTACTG 1980
TTAGATCAAA CCCTGGCCTT TAATGAAAAG AATGCGGCTT TAGCTGCAGC CTGAGAGTTT 2040
GGAGATACCT GGTATCCTAG TCAAGTAAAT GAAAGAATGA CAGCCGAAGA AAGGGACAAC 2100
TTCCTTACTG GTCAGCAACC CATCCCCAGT ATGGATCCCC ACTGGGACTT TGACTCAGAT 2160
CATGGGGACT GGAGTCGTAA ACATCTGTTG ATCTGTGTTC TGGAAGGACT AAGGAGAATT 2220
GGGAAAAAGC CCATGAATTA TTCAATGATA TCCACCATAA CCCAGGGAAA GGAAGAAAAT 2280
CCTTCTGCCT TCCTCGAGCG GCTACAAGAG GCCTTAAGAA AATATACTCC CCTGTCACCC 2340
GAATCACTCG AGGGTCAATT GATTCTAAAA GATAAGTTTA TTACCCAATC AGCCACAGAT 2400
ATCAGGAGAA AGCTCCAAAA GCAAGCCCTG AGCCTGAACA AAATCTAGAG ACATTATTAA 2460
ACCTGGCAAC CTTGGTGTTC TATAATAGGG ACCAAGAGGA ACA                   2503
```

(2) INFORMATIONS POUR LA SEQ ID NO: 61:

(i) CARACTERISTIQUES DE LA SEQUENCE:

(A) LONGUEUR: 1167 paires de bases

(B) TYPE: nucléotide

(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 61:

```
AAGGAAACTC AGAAAGCCAA TACCCATTTA GTAAGATGGA CACCAGAAGC AGAAGCAGCT   60
TTCCAGGCCC TAAAGAAATC CCTAACCCAA GCCCCAGTGT TAAGCTTGCC AACGGGGCAA  120
GACTTTTCTT TATATGTCAC AGAAAAACAG GAATAGCTCT AGGAGTCCTT ACACAGGTCC  180
AAGGGACAAG CTTGCAACCT GTGGCATACC TGAGTAAGGA AACTGATGTA NTGGCAAAGG  240
GTTGGCCTCA TTGTTTACAG GTAGGGCAGC AGTAGCAGTC TTAGTTTCTG AAACAGTTAA  300
AATAATACAG GGAAGAGATC TTACTGTGTG GACATCTCAT GATGTGAACG GCATACTCAC  360
TGCTAAAGAG GACTTGTGGC TGTCAGACAA CCATTTACTT AAATAGCAGG TTCTATTACT  420
TGAAGTGCCA GTGCTGCGAC TGCACATTTG TGCAACTCTT AACCCAGCCA CATTTCTTCC  480
AGACAATGAA GAAAAGATAG AACATAACTG TCAACAAGTA ATTGCTCAAA CCTATGCTGC  540
TCGAGGGGAC CTTCTAGAGG TTCCCTTGAC TGATCCCGAC CTCAACTTGT ATACTGATGG  600
AAGTTCCTTG GCAGAAAAAG GACTTTGAAA AGCGGGGTAT GCAGTGATCA GTGATAATGG  660
```

```
AATACTTGAA AGTAATCGCC TCACTCCAGG AACTAGTGCT CACCTGGCAG AACTAATAGC  720
CCTCACTTGG GCACTAGAAT TAGGAGAAGG AAAAAGGGTA AATATATATT CAGACTCTAA  780
GTATGCTTAC CTAGTCCTCC ATGCCCATGC AGCAATATGG AGAGAGAGGG AATTCCTAAC  840
TTCTGAGGGA ACACCTATCA ACCATCAGGG AAGCCATTAG GAGATTATTA TTGGCTGTAC  900
AGAAACCTAA AGAGGTGGCA GTCTTACACT GCCAGGGTCA TCAGGAAGAA GAGGAAAGGG  960
AAATAGAAGG CAATCGCCAA GCGGATATTG AAGCAAAAAA AGCCGCAAGG CAGGACTCTC 1020
CATTAGAAAT GCTTATAGAA GGACCCCTAG TATGGGGTAA TCCCCTCTGG GAAACCAAGC 1080
CCCAGTACTC AGCAGGAAAA ATAGAATAGG AAACCTCACA AGGACATACT TTCCTCCCCT 1140
CCAGATGGCT AGCCACTGAG GAAGGAA                                     1167
```

(2) INFORMATIONS POUR LA SEQ ID NO: 62:

(i) CARACTERISTIQUES DE LA SEQUENCE:

(A) LONGUEUR: 78 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 62:

```
TCCAAAGGCA CCAGGGCCCT CAGTGAGGAA CGTATCCAGC CTATACTGGC TTATCCTCAT  60
CCCAAAACCC TAAAGCAA                                                78
```

(2) INFORMATIONS POUR LA SEQ ID NO: 63:

(i) CARACTERISTIQUES DE LA SEQUENCE:

(A) LONGUEUR: 26 résidus d'acide aminé
(B) TYPE: acides aminés

(ii) TYPE DE MOLECULE: peptide
(xi) DESCRIPTION DE LA SEQUENCE: SEQ ID NO: 63:

```
Ser Lys Gly Thr Arg Ala Leu Ser Glu Glu Arg Ile Gln Pro Ile Leu
 1               5                  10                  15
Ala Tyr Pro His Pro Lys Thr Leu Lys Gln
            20                  25
```

(2) INFORMATIONS POUR LA SEQ ID NO: 64:

(i) CARACTERISTIQUES DE LA SEQUENCE:

(A) LONGUEUR: 28 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 64:
AAATGTCTGC GGCACCAATC TCCATGTT 28

(2) INFORMATIONS POUR LA SEQ ID NO: 65:

(i) CARACTERISTIQUES DE LA SEQUENCE:

(A) LONGUEUR: 30 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 65:
AAGGGGCATG GACGAGGTGG TGGCTTATTT 30

(2) INFORMATIONS POUR LA SEQ ID NO: 66:

(i) CARACTERISTIQUES DE LA SEQUENCE:

(A) LONGUEUR: 21 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 66:
GGAGAAGAGC AGCATAAGTG G 21

(2) INFORMATIONS POUR LA SEQ ID NO: 67:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 67:
GTGCTGATTG GTGTATTTAC AATCC 25

(2) INFORMATIONS POUR LA SEQ ID NO: 68:

(i) CARACTERISTIQUES DE LA SEQUENCE:

(A) LONGUEUR: 34 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 68:
GACTCGCTGC AGATCGATTT TTTTTTTTTT TTTT 34

(2) INFORMATIONS POUR LA SEQ ID NO: 69:

(i) CARACTERISTIQUES DE LA SEQUENCE:

(A) LONGUEUR: 30 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 69:
GCCATCAAGC CACCCAAGAA CTCTTAACTT 30

(2) INFORMATIONS POUR LA SEQ ID NO: 70:

(i) CARACTERISTIQUES DE LA SEQUENCE:

(A) LONGUEUR: 30 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 70:
CCAATAGCCA GACCATTATA TACACTAATT 30

(2) INFORMATIONS POUR LA SEQ ID NO: 71:

(i) CARACTERISTIQUES DE LA SEQUENCE:

(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 71:
GCCATAACTG CAACCCAAGA GTT 23

(2) INFORMATIONS POUR LA SEQ ID NO: 72:

(i) CARACTERISTIQUES DE LA SEQUENCE:

(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 72:
GGACGAGGTG GTGGCTTATT TCT 23

(2) INFORMATIONS POUR LA SEQ ID NO: 73:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 73:
AACTTGCGTG CTAGAAGGAC TAAGG 25

(2) INFORMATIONS POUR LA SEQ ID NO: 74:

(i) CARACTERISTIQUES DE LA SEQUENCE:

(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 74:
AACTTTTCCC TTTTCCAGAT CCTC 24

(2) INFORMATIONS POUR LA SEQ ID NO: 75:

(i) CARACTERISTIQUES DE LA SEQUENCE:

(A) LONGUEUR: 22 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 75:
GCATACCAGG CAAGTGGACA TT 22

(2) INFORMATIONS POUR LA SEQ ID NO: 76:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 76:
CTGTCCGTTG GGTTTCCTTA CTCCT 25

(2) INFORMATIONS POUR LA SEQ ID NO: 77:

(i) CARACTERISTIQUES DE LA SEQUENCE:

(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 77:
GAGGCTCTGG AAAAGGGAAA AGTT 24

(2) INFORMATIONS POUR LA SEQ ID NO: 78:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 78:
CTGTCCGTTG GGTTTCCTTA CTCCT 25

(2) INFORMATIONS POUR LA SEQ ID NO: 79:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 79:
AGGAGTAAGG AAACCCAACG GACAG 25

(2) INFORMATIONS POUR LA SEQ ID NO: 80:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 80:
TGTATATAAT GGTCTGGCTA TTGGG 25

(2) INFORMATIONS POUR LA SEQ ID NO: 81:

(i) CARACTERISTIQUES DE LA SEQUENCE:

(A) LONGUEUR: 25 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 81:
AGGAGTAAGG AAACCCAACG GACAG 25

(2) INFORMATIONS POUR LA SEQ ID NO: 82:

(i) CARACTERISTIQUES DE LA SEQUENCE:

(A) LONGUEUR: 26 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 82:
TTCGGCAGAA ACCTGTTATG CCAAGG 26

(2) INFORMATIONS POUR LA SEQ ID NO: 83:

(i) CARACTERISTIQUES DE LA SEQUENCE:

(A) LONGUEUR: 22 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 83:
CTCGATTTCT TGCTGGGCCT TA 22

(2) INFORMATIONS POUR LA SEQ ID NO: 84:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 84:
GTTGATTCCC TCCTCAAGCA 20

(2) INFORMATIONS POUR LA SEQ ID NO: 85:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 85:
CTCTACCAAT CAGCATGTGG 20

(2) INFORMATIONS POUR LA SEQ ID NO: 86:

(i) CARACTERISTIQUES DE LA SEQUENCE:

(A) LONGUEUR: 19 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 86:

TGTTCCTCTT GGTCCCTAT 19

(2) INFORMATIONS POUR LA SEQ ID NO: 87:

(i) CARACTERISTIQUES DE LA SEQUENCE:

(A) LONGUEUR: 433 résidus d'acide aminé
(B) TYPE: acides aminés

(ii) TYPE DE MOLECULE: peptide
(xi) DESCRIPTION DE LA SEQUENCE: SEQ ID NO: 87:

```
Met Ala Thr Ala Thr Gly Thr Gly Ile Ala Gly Leu Ser Thr Ser Leu
1               5                   10                  15
Ser Tyr Tyr His Thr Leu Ser Lys Asn Phe Ser Asp Ser Leu Gln Glu
            20                  25                  30
Ile Met Lys Ser Ile Leu Thr Leu Gln Ser Gln Leu Asp Ser Leu Ala
        35                  40                  45
Ala Met Thr Leu Gln Asn Arg Arg Gly Pro His Leu Leu Thr Ala Glu
    50                  55                  60
Lys Gly Gly Leu Cys Thr Phe Leu Gly Glu Glu Cys Cys Phe Tyr Thr
65                  70                  75                  80
Asn Gln Ser Gly Ile Val Arg Asp Ala Thr Trp His Leu Gln Glu Arg
                85                  90                  95
Ala Ser Asp Ile Arg Gln Cys Leu Ser Asn Ser Tyr Thr Asn Leu Trp
            100                 105                 110
Ser Trp Ala Thr Trp Leu Leu Pro Phe Leu Gly Pro Met Ala Ala Ile
        115                 120                 125
Leu Leu Leu Leu Thr Phe Gly Pro Cys Ile Phe Lys Leu Leu Val Lys
    130                 135                 140
Phe Val Ser Ser Arg Ile Glu Ala Ile Lys Leu Gln Met Val Leu Gln
145                 150                 155                 160
Met Glu Pro Gln Met Ser Ser Thr Asn Asn Phe Tyr Gln Gly Pro Leu
                165                 170                 175
Glu Arg Ser Thr Gly Thr Ser Thr Ser Leu Glu Ile Pro Leu Trp Lys
            180                 185                 190
Thr Leu Gln Leu Gln Gly Pro Phe Phe Ala Pro Ile Gln Gln Glu Val
        195                 200                 205
Ala Arg Ala Val Ile Gly Gln Ile Pro Asn Ser Ser Trp Gly Val Leu
    210                 215                 220
Phe Arg Gly Gly Ile Glu Glu Val Thr Ala Cys Trp Gln Pro His Ser
225                 230                 235                 240
Pro Arg Trp Xaa Ser Val Pro Pro Gln Pro Trp Cys Pro Leu Trp Pro
                245                 250                 255
Cys Leu Arg Ser Pro Ser Ala Cys His Cys Thr Val Gly Ala Ser Phe
            260                 265                 270
Trp Ala Gly Gln Gly Arg Ser Gln Leu Pro Gln Leu Ala Gly Arg Tyr
        275                 280                 285
Gly Gly Arg Asp Ala Gly Gly Asn Gln Gly Cys Ala Trp Arg Leu Arg
    290                 295                 300
Ala Ser Met Ser Ser Arg Trp Ala Trp Ala Arg Arg Ala Pro His Ser
305                 310                 315                 320
Gly Ser Glu Gly Leu Ser Thr Trp Ala Arg Gln Met Leu Cys Ser Thr
                325                 330                 335
Ser Ser Leu Gly Leu Ser Cys Leu Pro Arg Gly Ala Gly Leu Arg Glu
            340                 345                 350
Xaa Ala Ala Cys Pro Cys Leu Ser Pro Pro Pro Arg Arg Gly Phe Leu
        355                 360                 365
His Ser Pro Ser Phe Pro Asp Lys His His Pro Leu Ser Thr Val Pro
    370                 375                 380
Ser Pro Ile Asn His Pro Arg Val Glu Glu Cys Gly His Thr Ala Arg
385                 390                 395                 400
```

```
Asp Trp Gln Ala Val Pro Leu Ala Ala Leu Val Arg Asp Pro Leu Arg
                405                 410                 415
Glu Ala Ser Trp Ala Pro Glu Ser Gly Gly Asp Leu Glu Asn Leu Tyr
            420                 425                 430
Val
433
```

(2) INFORMATIONS POUR LA SEQ ID NO: 88:

(i) CARACTERISTIQUES DE LA SEQUENCE:

(A) LONGUEUR: 693 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 88:

```
CTTCCCCAAC TAATAAGGAC CCCCCTTTCA ACCCAAACAG TCCAAAAGGA CATAGACAAA 60
GGAGTAAACA ATGAACCAAA GAGTGCCAAT ATTCCCTGGT TATGCACCCT CCAAGCGGTG 120
GGAGAAGAAT TCGGCCCAGC CAGAGTGCAT GTACCTTTTT CTCTCTCACA CTTGAAGCAA 180
ATTAAAATAG ACNTAGGTNA ATTNTCAGAT AGCCCTGATG GYTATATTGA TGTTTTACAA 240
GGATTAGGAC AATCCTTTGA TCTGACATGG AGAGATATAA TATTACTGCT AAATCAGACG 300
CTAACCTCAA ATGAGAGAAG TGCTGCCATA ACTGGAGCCC GAGAGTTTGG CAATCTCTGG 360
TATCTCAGTC AGGTCAATGA TAGGATGACA ACGGAGGAAA GAGAACGATT CCCCACAGGG 420
CAGCAGGCAG TTCCCAGTGT AGCTCCTCAT TGGGACACAG AATCAGAACA TGGAGATTGG 480
TGCCGCAGAC ATTTACTAAC TTGCGTGCTA GAAGGACTAA GGAAAACTAG GAAGACTATG 540
AATTATTCAA TGATGTCCAC TATAACACAG GGGAAAGGAA GAAAATCCTA CTGCCTTTCT 600
GGAGAGACTA AGGGAGGCAT TGAGGAAGCA TACCAGGCAA GTGGACATTG GAGGCTCTGG 660
AAAAGGGAAA AGTTGGGCAA ATTGAATGCC TAA                              693
```

(2) INFORMATIONS POUR LA SEQ ID NO: 89:

(i) CARACTERISTIQUES DE LA SEQUENCE:

(A) LONGUEUR: 1577 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 89:

```
AACTTGCGTG CTAGAAGGAC TAAGGAAAAC TAGGAAGACT ATGAATTATT CAATGATGTC   60
CACTATAACA CAGGGGAAAG GAAGAAAATC CTACTGCCTT TCTGGAGAGA CTAAGGGAGG  120
CATTGAGGAA GCATACCAGG CAAGTGGACA TTGGAGGCTC TGGAAAAGGG AAAAGTTGGG  180
CAAATTGAAT GCCTAATAGG GCTTGCTTCC AGTGCAGTCT ACAAGGACGC TTTAGAAAAG  240
ATTGTCCAAG TAGAAATAAG CCGCCCCTCG TCCATGCCCC TTATGTCAAG GGAATCACTG  300
GAAGGCCTAC TGCCCCAGGG GACGAAGGTC CTCTGAGTCA GAAGCCACTA ACCTGATGAT  360
CCAGCAGCAG GACTGAGGGT GCCCGGGGCA AGTGCCAGCC CATGCCATCA CCCTCAGAGC  420
CCCGGGTATG TTTGACCATT GAGAGCCAGG AAGTTAACTG TCTCCTGGAC ACTGGCGCAG  480
CCTTCTCAGT CTTACTTTCC TGTCCCAGAC AATTGTCCTC CAGATCTGTC ACTATCCGAG  540
GGGTCCTAAG ACAGCCAGTC ACTACATACT TCTCTCAGCC ACTAAGTTGT GACTGGGGAA  600
CTTTACTCTT TTCACATGCT TTTCTAATTA TGCCTGAAAG CCCCACTCCC TTGTTAGGGA  660
GAGACATTTT AGCAAAAGCA GGGGCCATTA TACACCTGAA CATAGGAAAA GGAATACCCA  720
TTTGCTGTCC CCTGCTTGAG GAAGGAATTA ATCCTGAAGT CTGGGCAATA GAAGGACAAT  780
ATGGACAAGC AAAGAATGCC CGTCCTGTTC AAGTTAAACT AAAGGATTCT GCCTCCTTTC  840
CCTACCAAAG GAAGTACCCT CTTAGACCCG AGGCCCTACA AGGACTCAAA AGATTGTTAA  900
GGACCTAAAA GCCCAAGGCC TAGTAAAACC ATGCAGTAGC CCCTGCAATA CTCCAATTTT  960
AGGAGTAAGG AAACCCAACG GACAGTGGAG GTTAGTGCAA GATCTCAGGA TTATTAATGA 1020
GGCTGTTTTT CCTCTATACC CAGCTGTATC TAGCCCTTAT ACTCTGCTTT CCCTAATACC 1080
```

```
AGAGGAAGCA GAGTAGTTTA CAGTCCTGGA CCTTAAGGAT GCCTCTTTCT GCATCCCTGT 1140
ACATCCTGAT TCTCAATTCT TGTTTGTCTT TGAAGATCCT TTGAACCCAA TGTCTCAATT 1200
CACCTGGACT GTTTTACCCC AGGGGTTCCG GGATAGCCCC CATCTATTTG GCCAGGCATT 1260
AGCCCAAGAC TTGAGCCAAT TCTCATACCT GGACATCTTG TCCTTCGGTA TGGGATGATT 1320
TAATTTTAGC CACCCGTTCA GAAACCTTGT GCCATCAAGC CACCCAAGCG TTCTTAAATT 1380
TCCTCACTCC GTGTGGCTAC AAGGTTTCCA AACCAAAGGC TCAGCTCTGC TCACAGCAGG 1440
TTAAATACTT AGGGTTAAAA TTATCCAAAG GCACCAGGGC CCTCTGTGAG GAATGTATCC 1500
AACCTGTACT GGCTTATCTT CATCCCAAAA CCCTAAAGCA ACTAAGAAGG TCCTTGGCAT 1560
AACAGGTTTC TGCCGAA                                                1577
```

(2) INFORMATIONS POUR LA SEQ ID NO: 90:

(i) CARACTERISTIQUES DE LA SEQUENCE:

(A) LONGUEUR: 182 résidus d'acide aminé
(B) TYPE: acides aminés

(ii) TYPE DE MOLECULE: peptide
(xi) DESCRIPTION DE LA SEQUENCE: SEQ ID NO: 90:

```
Ser Ser Ser Arg Thr Glu Gly Ala Arg Gly Lys Cys Gln Pro Met Pro
1               5                   10                  15
Ser Pro Ser Glu Pro Arg Val Cys Leu Thr Ile Glu Ser Gln Glu Val
            20                  25                  30
Asn Cys Leu Leu Asp Thr Gly Ala Ala Phe Ser Val Leu Leu Ser Cys
        35                  40                  45
Pro Arg Gln Leu Ser Ser Arg Ser Val Thr Ile Arg Gly Val Leu Arg
    50                  55                  60
Gln Pro Val Thr Thr Tyr Phe Ser Gln Pro Leu Ser Cys Asp Trp Gly
65                  70                  75                  80
Thr Leu Leu Phe Ser His Ala Phe Leu Ile Met Pro Glu Ser Pro Thr
                85                  90                  95
Pro Leu Leu Gly Arg Asp Ile Leu Ala Lys Ala Gly Ala Ile Ile His
            100                 105                 110
Leu Asn Ile Gly Lys Gly Ile Pro Ile Cys Cys Pro Leu Leu Glu Glu
        115                 120                 125
Gly Ile Asn Pro Glu Val Trp Ala Ile Glu Gly Gln Tyr Gly Gln Ala
    130                 135                 140
Lys Asn Ala Arg Pro Val Gln Val Lys Leu Lys Asp Ser Ala Ser Phe
145                 150                 155                 160
Pro Tyr Gln Arg Lys Tyr Pro Leu Arg Pro Glu Ala Leu Gln Gly Leu
                165                 170                 175
Lys Arg Leu Leu Arg Thr
            180
```

(2) INFORMATIONS POUR LA SEQ ID NO: 91:

(i) CARACTERISTIQUES DE LA SEQUENCE:

(A) LONGUEUR: 36 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 91: 36

(2) INFORMATIONS POUR LA SEQ ID NO: 92:

(i) CARACTERISTIQUES DE LA SEQUENCE:

(A) LONGUEUR: 22 paires de bases
(B) TYPE: nucléotide
(C) NOMBRE DE BRINS: simple
(D) CONFIGURATION: linéaire

(ii) TYPE DE MOLECULE: ADNc
(xi) DESCRIPTION DE LA SEQUENCE: SEQ ID NO: 92:
AGATCTGCAG AATTCGATAT CA 22

## Description

La Sclérose en Plaques (SEP) est une maladie démyélinisante du système nerveux central (SNC) dont la cause complète reste encore inconnue.

De nombreux travaux ont étayé l'hypothèse d'une étiologie virale de la maladie, mais aucun des virus connus testés ne s'est avéré être l'agent causal recherché: une revue des virus recherchés depuis des années dans la SEP a été faite par E. Norrby **(1)** et R.T. Johnson **(2)**.

Récemment, un rétrovirus, différent des rétrovirus humains connus a été isolé chez des patients atteints de SEP **(3,4 et 5)**. Les auteurs ont aussi pu montrer que ce rétrovirus pouvait être transmis in vitro, que des patients atteints de SEP produisaient des anticorps susceptibles de reconnaître des protéines associées à l'infection des cellules leptoméningées par ce rétrovirus, et que l'expression de ce dernier pouvait être fortement stimulée par les gènes immédiats-précoces de certains herpesvirus **(6)**.

Tous ces résultats plaident en faveur du rôle dans la SEP d'au moins un rétrovirus inconnu ou d'un virus ayant une activité transcriptase inverse détectable selon la méthode publiée par H. Perron **(3)** et qualifiée d'activité "RT de type LM7". Le contenu de la publication identifiée par (3) est incorporé à la présente description, par référence.

Récemment, les travaux de la Demanderesse ont permis d'obtenir deux lignées continues de cellules infectées par des isolats naturels provenant de deux patients différents atteints de SEP, par un procédé de culture tel que décrit dans le document WO-A-93 20188, dont le contenu est incorporé par référence à la présente description. Ces deux lignées dérivées de cellules de plexus-choroïdes humains, dénommées LM7PC et PLI-2 ont été déposées à l'E.C.A.C.C. respectivement le 22 juillet 1992 et le 8 janvier 1993, sous les numéros 92 072201 et 93 010817, conformément aux dispositions du Traité de Budapest. Par ailleurs, les isolats viraux possédant une activité RT de type LM7 ont également été déposés à l'E.C.A.C.C. sous la dénomination globale de "souches". La "souche" ou isolat hébergé par la lignée PLI-2, dénommée POL-2, a été déposée auprès de l'E.C.A.C.C. le 22 juillet 1992 sous le n° V92072202. La "souche" ou isolat hébergé par la lignée LM7PC, dénommée MS7PG, a été déposée auprès de l'E.C.A.C.C. le 8 janvier 1993 sous le n° V93010816.

A partir des cultures et des isolats précités, caractérisés par des critères biologiques et morphologiques, on s'est ensuite attaché à caractériser le matériel nucléique associé aux particules virales produites dans ces cultures.

Les portions de génome déjà caractérisées ont été utilisées pour mettre au point des tests de détection moléculaire du génome viral et des tests immunosérologiques, utilisant les séquences d'aminoacides codés par les séquences nucléotidiques du génome viral, pour détecter la réponse immunitaire dirigée contre des épitopes associés à l'infection et/ou l'expression virale.

Ces outils ont déjà permis de confirmer une association entre la SEP et l'expression des séquences identifiées dans les brevets cités plus loin. Cependant, le système viral découvert par la Demanderesse, s'apparente à un système rétroviral complexe. En effet, les séquences retrouvées encapsidées dans les particules virales extracellulaires produites par les différentes cultures de cellules de patients atteints de SEP, montrent clairement qu'il y a co-encapsidation de génomes rétroviraux apparentés, mais différents du génome rétroviral "sauvage" qui produit les particules virales infectantes. Ce phénomène a été observé entre des rétrovirus réplicatifs et des rétrovirus endogènes appartenant à la même famille, voire même hétérologues. La notion de rétrovirus endogène est très importante dans le contexte de notre découverte car, dans le cas de MSRV-1, on a observé que des séquences rétrovirales endogènes comprenant des séquences homologues au génome MSRV-1, existent dans l'ADN humain normal. L'existence d'éléments rétroviraux endogènes (ERV) apparentés à MSRV-1 par tout ou partie de leur génome, explique le fait que l'expression du rétrovirus MSRV-1 dans les cellules humaines puisse interagir avec des séquences endogènes proches. Ces interactions sont retrouvées dans le cas de rétrovirus endogènes pathogènes et/ou infectieux (par exemple certaines souches écotropes du Murine Leukaemia virus), dans le cas de rétrovirus exogènes dont la séquence nucléotidique peut être retrouvée partiellement ou en totalité, sous forme d'ERVs, dans le génome de l'animal hôte (ex. virus exogène de la tumeur mammaire de la souris transmis par le lait). Ces interactions consistent principalement en (i) une transactivation ou co-activation d'ERVs par le rétrovirus réplicatif, (ii) une encapsidation "illégitime" d'ARN apparentés d'ERVS, ou d'ERVs -voire d'ARN cellulaires- possédant simplement des séquences d'encapsidation compatibles, dans les particules rétrovirales produites par l'expression de la souche réplicative, parfois transmissibles et parfois avec une pathogènicité propre, et (iii) des recombinaisons plus ou moins importantes entre les génomes co-encapsidés, notamment dans les phases de transcription inverse, qui conduisent à la formation de génomes hybrides, parfois transmissibles et parfois avec une pathogénicité propre.

Ainsi,(i) différentes séquences apparentées à MSRV-1 ont été retrouvées dans les particules virales purifiées; (ii) l'analyse moléculaire des différentes régions du génome rétroviral MSRV-1 doit être faite en analysant systématiquement les séquences co-encapsidées, interférantes et/ou recombinées qui sont générées par l'infection et/ou l'expression de MSRV-1, de plus, certains clones peuvent avoir des parties de séquences défectives produites par la réplication rétrovirale et les erreurs de matrice et/ou de transcription de la transcriptase inverse; (iii) les familles de séquences apparentées à une même région génomique rétrovirale sont les supports d'une détection diagnostique globale qui peut être optimisée par l'identification de régions invariables parmi les clones exprimés et par l'identification de trames de lectures responsables de la production de polypeptides antigéniques et/ou pathogènes qui peuvent n'être produits que par une partie, voire un seul, des clones exprimés et dans ces conditions, l'analyse systématique des clones exprimés dans une région d'un gène donné permet d'évaluer la fréquence de variation et/ou de recombinaison du génome MSRV-1 dans cette région et de définir les séquences optimales pour les applications, notamment diagnostiques; (iv) la pathologie provoquée par un rétrovirus tel que MSRV-1 peut être un effet direct de son expression et des protéines ou peptides produits de ce fait, mais aussi un effet de l'activation, de l'encapsidation, de la recombinaison de génomes apparentés ou hétérologues et des protéines ou peptides produits de ces faits ; ainsi ces génomes associés à l'expression de et/ou l'infection par MSRV-1 sont-ils une partie intégrante de la pathogénicité potentielle de ce virus et donc, constituent des supports de détection diagnostique et des cibles thérapeutiques particulières. De même tout agent associé à, ou, co-facteur de ces interactions responsables de la pathogénie en cause, tel que MSRV-2 ou le facteur gliotoxique décrits dans la demande de brevet publiée sous le N° FR-2 716 198, peut participer à l'élaboration d'une stratégie globale et très efficace de diagnostic, de pronostic, de suivi thérapeutique et/ou de thérapeutique intégrée de la SEP notamment, mais aussi de toute autre maladie associée aux mêmes agents.

Dans ce contexte, on a fait une découverte parallèle dans une autre maladie autoimmune, la polyarthrite rhumatoïde (PR), qui a été, décrite dans la demande de brevet français déposée sous le N°95 02960. Cette découverte montre que, en appliquant des approches méthodologiques similaires à celles qui furent utilisées dans les travaux de la Demanderesse sur la SEP, on a pu identifier un rétrovirus exprimé dans la PR qui partage les séquences décrites pour MSRV-1 dans la SEP et aussi, la co-existence d'une séquence associée MSRV-2 également décrite dans la SEP. En ce qui concerne MSRV-1, les séquences détectées communément dans la SEP et la PR, concernent les gènes *pol* et *gag.* En l'état actuel des connaissances, on peut associer les séquences *gag* et *pol* décrites aux souches MSRV-1 exprimées dans ces deux maladies.

La présente demande de brevet a pour objet différents résultats, supplémentaires par rapport à ceux déjà protégés par les demandes de brevet français :

- N° 92 04322 du 03-04.1992, publiée sous le N° 2 689 519 ;
- N° 92 13447 du 03.11.1992, publiée sous le N° 2 689 521 ;
- N° 92 13443 du 03.11.1992, publiée sous le N° 2 689 520 ;
- N° 94 01529 du 04.02.1994, publiée sous le N° 2 715 936 ;
- N° 94 01531 du 04.02.1994, publiée sous le N° 2 715939 ;
- N° 94 01530 du 04.02.1994, publiée sous le N° 2 715 936 ;
- N° 94 01532 du 04.02.1994, publiée sous le N° 2 715 937 ;
- N° 94 14322 du 24.11.1994, publiée sous le N° 2 727 428 ; et
- N° 94 15810 du 23.12.1994, publiée sous le N° 2 728 585.

La présente invention concerne tout d'abord différents fragments nucléotidiques, comprenant, ou consistant en une séquence nucléotidique choisie dans le groupe constistant en :

(a) les séquences des clones FBd3 (SEQ ID NO 46), t pol (SEQ ID NO 51), JLBc1 (SEQ ID NO 52), JLBc2 (SEQ ID NO 53), GM3 (SEQ ID NO 56),

(b) les séquences complémentaires auxdites séquences génomiques.

Par séquences génomiques, on inclut toutes séquences associées par co-encapsidation ou par co-expression, ou recombinées.

L'invention concerne aussi toute sonde nucléique de détection d'un agent pathogène et/ou infectant associé à la SEP, susceptible de s'hybrider spécifiquement sur tout fragment tel que précédemment défini, appartenant ou compris dans le génome dudit agent pathogène.

En particulier, la séquence nucléotidique d'une sonde nucléique de l'invention est choisie parmi les séquences de 100 monomères contigus d'un fragment choisi parmi les fragments dont les séquences nucléotidiques sont identifiées en SEQ ID NO 46, SEQ ID NO 51, SEQ ID NO 52, SEQ ID NO 53, parmi les séquences de 30 ou 100 monomères contigus du fragment dont la séquence nucléotidique est identifiée en SEQ ID NO 56 ; et parmi les séquences nucléotidiques complémentaires desdites séquences.

L'invention concerne aussi une amorce pour l'amplification par polymérisation d'un ARN ou d'un ADN d'un matériel viral, comprenant une séquence nucléotidique identique à au moins une partie de la séquence nucléotidique de tout fragment tel que défini précédemment, notamment une séquence nucléotidique consistant en une séquence de 30 monomères contigus de SEQ ID n° 56 ou sa séquence complémentaire.

De préférence, la séquence de l' amorce est identique à SEQ ID n° 49.

De manière générale, l'invention embrasse également tout ARN ou ADN, et notamment vecteur de réplication, comprenant un fragment nucléotidique tel que précédemment défini.

L'invention s'intéresse également aux différents peptides codés par tout cadre de lecture ouvert appartenant à un fragment nucléotidique tel que précédemment défini, notamment tout polypeptide, par exemple tout oligopeptide formant ou comprenant un déterminant antigénique reconnu par des sera de patients infectés par le virus MSRV-1, et/ou chez lesquels le virus MSRV-1 a été réactivé. Préférentiellement, ce polypeptide est antigénique, et est codé par le cadre de lecture ouvert commençant dans le sens 5'-3', au nucléotide 181, et finissant au nucléotide 330 de SEQ ID NO 1.

A titre particulier, un polypeptide antigénique, reconnu par les sera de patients infectés par le virus MSRV-1, et/ou chez lesquels le virus MSRV-1 a été réactivé, a une séquence peptidique identique, partiellement ou totalement, ou équivalente à la séquence définie par SEQ ID NO 39, SEQ ID NO 63 et SEQ ID NO 87 ; une telle séquence est identique par exemple à toute séquence choisie dans le groupe incluant les séquences SEQ ID NO 41 à SEQ ID NO 44, SEQ ID NO 63 et SEQ ID NO 87.

Ainsi, la présente invention propose également des anticorps mono- ou polyclonaux, dirigés contre le virus MSRV-1, obtenus par réaction immunologique d'un organisme humain ou animal, à un agent immunogène constitué par un polypeptide antigénique tel que défini précédemment.

L'invention s'intéresse ensuite :

- aux réactifs de détection du virus MSRV-1, ou d'une exposition à ce dernier, comprenant à titre de substance réactive, un peptide, notamment antigénique, tel que précédemment défini, ou un anticorps dudit peptide ;
- à toutes compositions diagnostiques, prophylactiques, ou thérapeutiques, comprenant un ou plusieurs peptides, notamment antigéniques, tels que précédemment définis, ou un ou plusieurs anticorps des peptides, considérés précédemment ; une telle composition est préférentiellement, et à titre d'exemple, une composition vaccinale.

L'invention s'intéresse également à toute composition diagnostique, prophylactique, ou thérapeutique, notamment pour inhiber l'expression d'au moins un agent pathogène et/ou infectant associé à la SEP, comprenant un fragment nucléotidique tel que précédemment défini.

Selon l'invention, ces mêmes fragments, ou polynucléotides, notamment oligonucléotides, peuvent entrer dans toutes compositions appropriées, pour détecter, selon tout procédé ou méthode convenable, un agent pathologique, et/ou infectant, associé respectivement à la SEP et à la PR, dans un échantillon biologique. Dans un tel procédé, on met en contact un ARN et/ou un ADN présumé appartenir ou provenant dudit agent pathologique et/ou infectant, et/ou leur ARN et/ou ADN complémentaire, avec une telle composition.

La présente invention concerne également tout procédé pour détecter la présence ou l'exposition à un tel agent pathologique et/ou infectant, dans un échantillon biologique, en mettant en contact cet échantillon avec un peptide, notamment antigénique, tel que précédemment défini, ou un anti-ligand, notamment anticorps de ce peptide, tel que précédemment défini.

En pratique, et par exemple, un dispositif de détection du virus MSRV-1 comprend un réactif tel que précédemment défini, supporté par un support solide, immunologiquement compatible avec, le réactif, et un moyen de mise en contact de l'échantillon biologique, par exemple un échantillon de sang ou de liquide céphalo-rachidien, susceptible de contenir des anticorps anti-MSRV-1, avec ce réactif, dans des conditions permettant une éventuelle réaction immunologique, tout ceci avec des moyens de détection du complexe immun formé avec ce réactif.

Pour terminer, l'invention concerne également la détection d'anticorps anti-MSRV-1, dans un échantillon biologique, par exemple un échantillon de sang ou de liquide céphalo-rachidien, selon lequel on met en contact cet échantillon avec un réactif tel que précédemment défini, consistant en un anticorps, dans des conditions permettant leur éventuelle réaction immunologique, et on détecte ensuite la présence du complexe immun ainsi formé avec le réactif.

L'invention concerne aussi les utilisations suivantes :

Utilisation d'une sonde nucléique pour la détection in vitro d'un agent pathogène et/ou infectant associé à la sclérose en plaques, ladite sonde comprenant de 10 à 100 monomères contigus d'un fragment tel que défini précédemment.
Utilisation d'une amorce pour l'amplification par polymérisation d'un ARN ou d'un ADN d'un matériel viral associé à la sclérose en plaques, ladite amorce comprenant une séquence nucléotidique de 10 à 30 monomères contigus, identique à la séquence nucléotidique d'un fragment tel que défini précédemment.

Avant de détailler l'invention, différents termes utilisés dans la description et les revendications sont à présent définis :

- par souche ou isolat, on entend toute fraction biologique infectante et/ou pathogène, contenant par exemple des virus et/ou des bactéries et/ou des parasites, générant un pouvoir pathogène et/ou antigénique, hébergée par une culture ou un hôte vivant ; à titre d'exemple, une souche virale selon la définition précédente peut contenir un agent co-infectant, par exemple un protiste pathogène,

- le terme "MSRV" utilisé dans la présente description désigne tout agent pathogène et/ou infectant, associé à la SEP, notamment une espèce virale, les souches atténuées de ladite espèce virale, ou les particules défectives interférentes ou contenant des génomes co-encapsidés ou encore des génomes recombinés avec une partie du génome MSRV-1, dérivées de cette espèce. Il est connu que les virus et particulièrement les virus contenant de l'ARN ont une variabilité, consécutive notamment à des taux relativement élevés de mutation spontanée (7), dont il sera tenu compte ci-après pour définir la notion d'équivalence,
- par virus humain, on entend un virus susceptible d'infecter ou d'être hébergé par l'être humain,
- il doit être tenu compte de toutes les variations et/ou recombinaison naturelles ou induites, pouvant être rencontrées dans la pratique de la présente invention,
- le variant d'un virus ou d'un agent pathogène et/ou infectant, selon l'invention, comprend au moins un antigène reconnu par au moins un anticorps dirige contre au moins un antigène correspondant dudit virus et/ou dudit agent pathogène et/ou infectant, et/ou un génome dont toute partie est détectée par au moins une sonde d'hybridation, et/ou au moins une amorce d'amplification nucléotidique spécifique dudit virus et/ou agent pathogène et/ou infectant, comme par exemple pour le virus dit MSRV-1, celles ayant une séquence nucléotidique choisie parmi SEQ ID N° 20 à SEQ ID N° 24, SEQ ID N° 26, SEQ ID N° 16 à SEQ ID N° 19, SEQ ID N° 31 à SEQ ID N° 33, SEQ ID N° 45, SEQ ID N° 47, SEQ ID N° 48, SEQ ID N° 49, SEQ ID N° 50, SEQ ID N° 45 et leurs séquences complémentaires, dans des conditions d'hybridation déterminées bien connues de l'homme de l'art,
- selon l'invention, un fragment nucléotidique ou un oligonucléotide ou un polynucléotide est un enchaînement de monomères, ou un biopolymère, caractérisé par la séquence informationnelle des acides nucléiques naturels, susceptible de s'hybrider à tout autre fragment nucléotidique dans des conditions prédéterminées, l'enchaînement pouvant contenir des monomères de structures chimiques différentes et être obtenu à partir d'une molécule d'acide nucléique naturelle et/ou par recombinaison génétique et/ou par synthèse chimique ; un fragment nucléotidique peut être identique à un fragment génomique du virus MSRV-1 considéré par la présente invention, notamment un gène de ce dernier, par exemple pol ou env dans le cas dudit virus ;
- ainsi un monomère peut être un nucléotide naturel d'acide nucléique, dont les éléments constitutifs sont un sucre, un groupement phosphate et une base azotée; dans l'ARN le sucre est le ribose, dans l'ADN le sucre est le désoxy-2-ribose; selon qu'il s'agit de l'ADN ou l'ARN, la base azotée est choisie parmi l'adénine, la guanine, l'uracile, la cytosine, la thymine; ou le nucléotide peut être modifié dans l'un au moins des trois éléments constitutifs ; à titre d'exemple, la modification peut intervenir au niveau des bases, générant des bases modifiées telles que l'inosine, la méthyl-5-désoxycytidine, la désoxyuridine, la diméthylamino-5-désoxyuridine, la diamino-2,6-purine, la bromo-5-désoxyuridine et toute autre base modifiée favorisant l'hybridation; au niveau du sucre, la modification peut consister dans le remplacement d'au moins un désoxyribose par un polyamide (8), et au niveau du groupement phosphate, la modification peut consister dans son remplacement par des esters, notamment choisis parmi les esters de diphosphate, d'alkyl et arylphosphonate et de phosphorothioate,
- par "séquence informationnelle", on entend toute suite ordonnée de monomères, dont la nature chimique et l'ordre dans un sens de référence, constituent ou non une information fonctionnelle de même qualité que celle des acides nucléiques naturels,
- par hybridation, on entend le processus au cours duquel, dans des conditions opératoires appropriées, deux fragments nucléotidiques, ayant des séquences suffisamment complémentaires, s'apparient pour former une structure complexe, notamment double ou triple, de préférence sous forme d'hélice,
- une sonde comprend un fragment nucléotidique synthétisé par voie chimique ou obtenu par digestion ou coupure enzymatique d'un fragment nucléotidique plus long, comprenant au moins six monomères, avantageusement de 10 à 100 monomères, de préférence 10 à 30 monomères, et possédant une spécificité d'hybridation dans des conditions déterminées ; de préférence, une sonde possédant moins de 10 monomères n'est pas utilisée seule, mais l'est en présence d'autres sondes de taille aussi courte ou non ; dans certaines conditions particulières, il peut être utile d'utiliser des sondes de taille supérieure à 100 monomères ; une sonde peut notamment être utilisée à des fins de diagnostic et il s'agira par exemple de sondes de capture et/ou de détection,
- la sonde de capture peut être immobilisée sur un support solide par tout moyen approprié, c'est-à-dire directement ou indirectement, par exemple par covalence ou adsorption passive,
- la sonde de détection peut être marquée au moyen d'un marqueur choisi notamment parmi les isotopes radioactifs, des enzymes notamment choisis parmi la péroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine,
- les sondes utilisées à des fins de diagnostic de l'invention peuvent être mises en oeuvre dans toutes les techniques d'hybridation connues, et notamment les techniques dites "DOT-BLOT" **(9)**, "SOUTHERN BLOT" **(10)**, "NORTHERN BLOT" qui est une technique identique à la technique "SOUTHERN BLOT" mais qui utilise de l'ARN comme cible, la technique SANDWICH (11) ; avantageusement, on utilise la technique SANDWICH dans la présente invention, comprenant une sonde de capture spécifique et/ou une sonde de détection spécifique, étant entendu que la sonde de capture et la sonde de détection doivent présenter une séquence nucléotidique au moins partiellement différente,

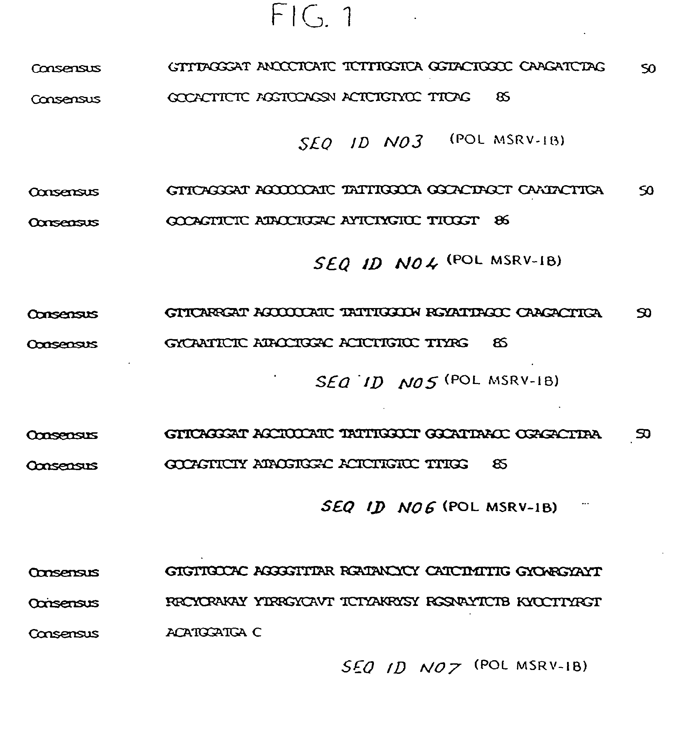

- toute sonde selon la présente invention peut s'hybrider in vivo ou in vitro sur l'ARN et/ou sur l'ADN, pour bloquer les phénomènes de réplication, notamment traduction et/ou transcription, et/ou pour dégrader ledit ADN et/ou ARN,
- une amorce est une sonde comprenant au moins six monomères, et avantageusement de 10 à 30 monomères, possédant une spécificité d'hybridation dans des conditions déterminées, pour l'initiation d'une polymérisation enzymatique, par exemple dans une technique d'amplification telle que la PCR (Polymerase Chain Reaction), dans un procédé d'élongation, tel que le séquençage, dans une méthode de transcription inverse ou analogue,
- deux séquences nucléotidiques ou peptidiques sont dites équivalentes ou dérivées l'une par rapport à l'autre, ou par rapport à une séquence de référence, si fonctionnellement les biopolymères correspondants peuvent jouer sensiblement le même rôle, sans être identiques, vis-à-vis de l'application ou utilisation considérée, ou dans la technique dans laquelle elles interviennent ; sont notamment équivalentes deux séquences obtenues du fait de la variabilité naturelle, notamment mutation spontanée de l'espèce à partir de laquelle elles ont été identifiées, ou induite, ainsi que deux séquences homologues, l'homologie étant définie ci-après,
- par "variabilité", on entend toute modification, spontanée ou induite d'une séquence, notamment par substitution, et/ou insertion, et/ou délétion de nucléotides et/ou de fragments nucléotidiques, et/ou extension et/ou racourcissement de la séquence à l'une au moins des extrémités; une variabilité non naturelle peut résulter des techniques de génie génétique utilisées, par exemple du choix des amorces de synthèse dégénérées ou non, retenues pour amplifier un acide nucléique; cette variabilité peut se traduire par des modifications de toute séquence de départ, considérée comme référence, et pouvant être exprimées par un degré d'homologie par rapport à ladite séquence de référence,
- l'homologie caractérise le degré d'identité de deux fragments nucléotidiques ou peptidiques comparés ; elle se mesure par le pourcentage d'identité qui est notamment déterminé par comparaison directe de séquences nucléotidiques ou peptidiques, par rapport à des séquences nucléotidiques ou peptidiques de référence,
- ce pourcentage d'identité a été spécifiquement déterminé pour les fragments nucléotidiques, notamment clones relevant de la présente invention, homologues aux fragments identifiés pour le virus MSRV-1 par SEQ ID N° 1 à N° 9, SEQ ID NO 46, SEQ ID NO 51 à SEQ ID NO 53, SEQ ID NO 40, SEQ ID NO 56 et SEQ ID NO 57, ainsi que pour les sondes et amorces homologues aux sondes et amorces identifiées par SEQ ID NO 20 à SEQ ID NO 24, SEQ ID NO 26, SEQ ID NO 16 à SEQ ID NO 19, SEQ ID NO 31 à SEQ ID NO 33, SEQ ID NO 45, SEQ ID NO 47, SEQ ID NO 48, SEQ ID NO 49, SEQ ID NO 50, SEQ ID NO 55, SEQ ID NO 40, SEQ ID NO 56 et SEQ ID NO 57 ; à titre d'exemple, le plus faible pourcentage d'identité observé entre les différents consensus généraux en acides nucléiques obtenus à partir de fragments d'ARN viral de MSRV-1, issu des lignées LM7PC et PLI-2 selon un protocole détaillé plus loin, est de 67% dans la région décrite à la figure 1,
- tout fragment nucléotidique est dit équivalent ou dérivé d'un fragment de référence, s'il présente une séquence nucléotidique équivalente à la séquence du fragment de référence ; selon la définition précédente, sont notamment équivalents à un fragment nucléotidique de référence :

  (a) tout fragment susceptible de s'hybrider au moins partiellement avec le complément du fragment de référence,
  (b) tout fragment dont l'alignement avec le fragment de référence conduit à mettre en évidence des bases contigues identiques, en nombre plus important qu'avec tout autre fragment provenant d'un autre groupe taxonomique,
  (c) tout fragment résultant ou pouvant résulter de la variabilité naturelle de l'espèce, à partir de laquelle il est obtenu,
  (d) tout fragment pouvant résulter des techniques de génie génétique appliquées au fragment de référence,
  (e) tout fragment, comportant au moins huit nucléotides contigus, codant pour un peptide homologue ou identique au peptide codé par le fragment de référence,
  (f) tout fragment différent du fragment de référence, par insertion, délétion, substitution d'au moins un monomère, extension, ou raccourcissement à l'une au moins de ses extrémités ; par exemple, tout fragment correspondant au fragment de référence, flanqué à l'une au moins de ses extrémités par une séquence nucléotidique ne codant pas pour un polypeptide,

- par polypeptide, on entend notamment tout peptide d'au moins deux acides aminés, notamment oligopeptide, protéine, extrait, séparé, ou substantiellement isolé ou synthétisé, par l'intervention de la main de l'homme, notamment ceux obtenus par synthèse chimique, ou par expression dans un organisme recombinant,
- par polypeptide codé de manière partielle par un fragment nucléotidique, on entend un polypeptide présentant au moins trois acides aminés codés par au moins neuf monomères contigus compris dans ledit fragment nucléotidique,
- un acide aminé est dit analogue à un autre acide aminé, lorsque leur caractéristiques physico-chimiques respectives, telles que polarité, hydrophobicité, et/ou basicité, et/ou acidité, et/ou neutralité, sont sensiblement les mêmes ; ainsi, une leucine est analogue à une isoleucine.

- tout polypeptide est dit équivalent ou dérivé d'un polypeptide de référence, si les polypeptides comparés ont sensiblement les mêmes propriétés, et notamment les mêmes propriétés antigéniques, immunologiques, enzymologiques et/ou de reconnaissance moléculaire ; est notamment équivalent à un polypeptide de référence :

  (a) tout polypeptide possédant une séquence dont au moins un acide aminé a été substitué par un acide aminé analogue,
  (b) tout polypeptide ayant une séquence peptidique équivalente, obtenue par variation naturelle ou induite dudit polypeptide de référence, et/ou du fragment nucléotidique codant pour ledit polypeptide,
  (c) un mimotope dudit polypeptide de référence,
  (d) tout polypeptide dans la séquence duquel un ou plusieurs acides aminés de la série L sont remplacés par un acide aminé de la série D, et vice versa,
  (e) tout polypeptide dans la séquence duquel on a introduit une modification des chaînes latérales des acides aminés, telle que par exemple une acétylation des fonctions amines, une carboxylation des fonctions thiol, une estérification des fonctions carboxyliques,
  (f) tout polypeptide dans la séquence duquel une ou des liaisons peptidiques ont été modifiées, comme par exemple les liaisons carba, rétro, inverso, rétro-inverso, réduites, et méthylène-oxy,
  (g) tout polypeptide dont au moins un antigène est reconnu par anticorps dirigé contre un polypeptide de référence,

- le pourcentage d'identité caractérisant l'homologie de deux fragments peptidiques comparés est selon la présente invention d'au moins 50% et de préférence au moins 70 %.

Etant donné qu'un virus possédant une activité enzymatique transcriptase inverse peut être génétiquement caractérisé aussi bien sous forme d'ARN que d'ADN, il sera fait mention aussi bien de l'ADN que de l'ARN viral pour caractériser les séquences relatives à un virus possédant une telle activité transcriptase inverse, dit MSRV-1 selon la présente description.

Les expressions d'ordre utilisées dans la présente description et les revendications, telles que "première séquence nucléotidique" ne sont pas retenues pour exprimer un ordre particulier, mais pour définir plus clairement l'invention.

Par détection d'une substance ou agent, on entend ci-après aussi bien une identification, qu'une quantification, ou une séparation ou isolement de ladite substance ou dudit agent.

L'invention sera mieux comprise à la lecture de la description détaillée qui va suivre faite en référence aux figures annexées dans lesquelles :

- la figure 1 représente des consensus généraux en acides nucléiques des clones MSRV-1B amplifiés par la technique PCR dans la région "pol" définie par Shih (12), à partir d'ADN viral issu des lignées LM7PC et PLI-2, et identifiés sous les références SEQ ID NO 3, SEQ ID NO 4, SEQ ID NO 5, et SEQ ID NO 6, et le consensus commun avec amorces d'amplification portant la référence SEQ ID NO 7,
- la figure 2 donne la définition d'une trame de lecture fonctionnelle pour chaque famille de type MSRV-1B/"PCR pol", lesdites familles A à D étant définies respectivement par les séquences nucléotidiques SEQ ID NO 3, SEQ ID NO 4, SEQ ID NO 5, et SEQ ID NO 6, décrites à la figure 1,
- la figure 3 donne un exemple de consensus des séquences de MSRV-2B, identifié par SEQ ID NO 11,
- la figure 4 est une représentation de l'activité transcriptase inverse (RT) en dpm (désintégration par minute), dans les fractions de saccharose prélevées sur un gradient de purification des virions produits par les lymphocytes B en culture d'un patient atteint de SEP,
- la figure 5 donne, dans les mêmes conditions exprimentales qu'à la figure 4, le dosage de l'activité transcriptase inverse dans la culture d'une lignée de lymphocytes B obtenue à partir d'un témoin exempt de SEP,
- la figure 6 représente la séquence nucléotidique du clone PSJ17 (SEQ ID NO 9),
- la figure 7 représente la séquence nucléotidique SEQ ID NO 8, du clone dénommé M003-P004,
- la figure 8 représente la séquence nucléotidique SEQ ID NO 2 du clone F11-1 ; la partie repérée entre les deux flèches dans la région de l'amorce correspond à une variabilité imposée par le choix de l'amorce ayant servi au clonage de F11-1 ; sur cette même figure, la traduction en acides aminés est représentée,
- la figure 9 représente la séquence nucléotidique SEQ ID NO 1, et une trame fonctionnelle de lecture possible en acides aminés de SEQ ID NO 1; sur cette séquence, les séquences consensus du gène pol sont soulignées,
- les figures 10 et 11 donnent les résultats d'une PCR, sous forme de photographie sous lumière ultra-violette d'un gel d'agarose imprégné de bromure d'éthidium, des produits d'amplification obtenus à partir des amorces identifiées par SEQ ID NO 16, SEQ ID NO 17, SEQ ID NO 18, et SEQ ID NO 19.

- la figure 12 donne une représentation matricielle de l'homologie entre SEQ ID NO 1 de MSRV-1 et celle d'un rétrovirus endogène dénommé HSERV9 ; cette homologie d'au moins 65 % est mise en évidence par un trait plein, l'absence de trait signifiant une homologie inférieure à 65 % ;
- la figure 13 représente la séquence nucléotidique SEQ ID NO 46 du clone FBd3,
- la figure 14 représente l'homologie de séquence entre le clone FBd3 et le rétrovirus HSERV-9 ;
- la figure 15 représente la séquence nucléotidique SEQ ID NO 51 du clone t pol ;
- les figures 16 et 17 représentent respectivement les séquences nucléotidiques SEQ ID NO 52 et SEQ ID NO 53, des clones JLBc1 et JLBc2 respectivement ;
- la figure 18 représente l'homologie de séquence entre le clone JLBc1 et le clone FBd3,
- et la figure 19 l'homologie de séquence entre le clone JLBc2 et le clone FBd3 ;
- la figure 20 représente l'homologie de séquence entre les clones JLBc1 et JLBc2 ;
- les figures 21 et 22 représentent l'homologie de séquence entre le rétrovirus HSERV-9, et respectivement les clones JLBc1 et JLBc2 ;
- la figure 23 représente la séquence nucléotidique SEQ ID NO 56 du clone GM3 ;
- la figure 24 représente l'homologie de séquence entre le rétrovirus HSERV-9 et le clone GM3 ;
- la figure 25 représente la localisation des différents clones étudiés, par rapport au génome du rétrovirus connu ERV9 ;
- la figure 26 représente la position des clones F11-1, M003-P004, MSRV-1B et PSJ17 dans la région dénommée ci-après MSRV-1 pol * ;

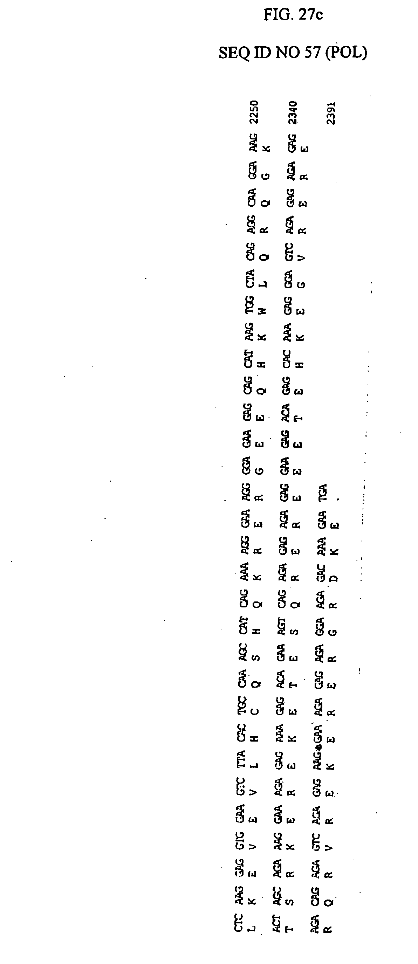

- la figure 27, éclatée en trois figures successives 27a, 27b, 27c représente un cadre de lecture possible, couvrant l'ensemble du gène pol ;
- la figure 28 représente selon SEQ ID NO 40, la séquence nucléotidique codant pour le fragment peptidique POL2B, ayant la séquence en acides aminés identifiée par SEQ ID NO 39 ;
- la figure 29 représente les valeur de DO (tests ELISA) à 492 nm obtenues pour 29 sérums de patients SEP et 32 sérums de témoins sains testés avec un anticorps anti-IgG ;
- la figure 30 représente les valeurs de DO (tests ELISA) à 492 nm obtenues pour 36 sérums de patients SEP et 42 sérums de témoins sains testés avec un anticorps anti-IgM ;

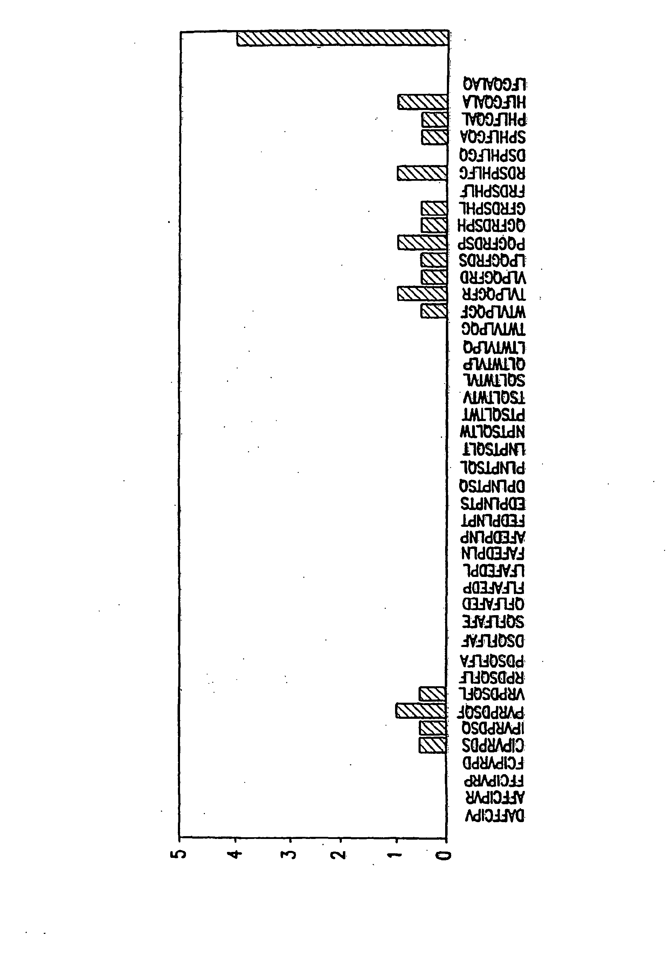

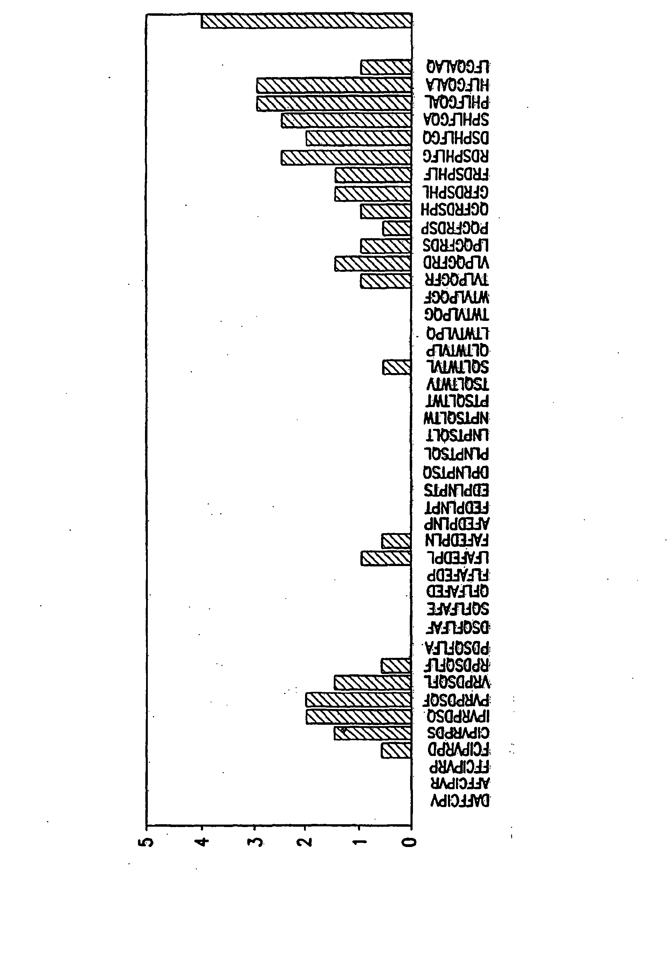

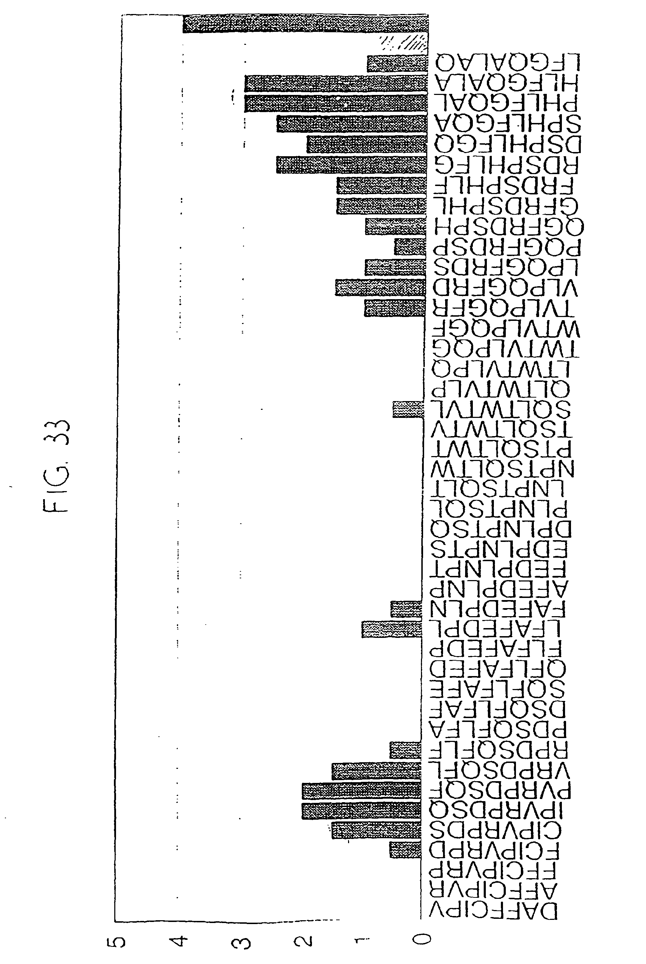

- les figures 31 à 33 représentent les résultats obtenus (intensité relative des spots) pour 43 octapeptides chevauchants couvrant la séquence en acides aminés 61-110, selon la technique Spotscan, respectivement avec un pool de sérums de SEP, avec un pool de sérums témoins et avec le pool de sérums SEP après déduction d'un bruit de fond correspondant au signal maximum détecté sur au moins un octapeptide avec le sérum témoin (intensité = 1), étant entendu que ces sérums ont été dilués au 1/50ème. La barre à l'extrême droite représente un standard d'échelle graphique sans rapport avec le test sérologique ;
- la figure 34 représente les SEQ ID NO 41 et SEQ ID NO 42 de deux polypeptides comprenant immuno-dominantes, tandis que SEQ ID NO 43 et 44 représentent des polypeptides immuno-réactifs et spécifiques à la SEP ;
- la figure 35 représente la séquence nucléotidique SEQ ID NO 59 du clone LB19 et trois trames de lecture potentielles en acides aminés de SEQ ID NO 59 ;
- la figure 36 représente la séquence nucléotidique SEQ ID NO 88 (GAG*) et une trame de lecture potentielle en acides aminés de SEQ ID NO 88 ;
- la figure 37 représente l'homologie de séquence entre le clone FBd13 et le rétrovirus HSERV-9 ; selon cette représentation, le trait plein signifie un pourcentage d'homologie supérieur ou égal à 70% et l'absence de trait signifie un pourcentage d'homologie inférieur ;
- la figure 38 représente la séquence nucléotidique SEQ ID NO 61 du clone FP6 et trois trames de lecture potentielles en acides aminés de SEQ ID NO 61 ;
- la figure 39 représente la séquence nucléotidique SEQ ID NO 89 du clone G+E+A et trois trames de lecture potentielles en acides aminés de SEQ ID NO 89 ;
- la figure 40 représente un cadre de lecture trouvé dans la région E et codant pour une protéase rétrovirale MSRV-1 identifiée par SEQ ID NO 90 ;
- la figure 41 représente la réponse de chaque sérum de patients atteints de SEP indiqué par le symbole (+) et de patients sains symbolisé par (-), testé avec un anticorps anti-IgG exprimée en densité optique nette à 492 nm;
- la figure 42 représente la réponse de chaque sérum de patients atteints de SEP indiqué par les symboles (+) et (QS) et de patients sains (-), testé avec un anticorps anti-IgM exprimée en densité optique nette à 492 nm.

**EXEMPLE 1 : OBTENTION DE CLONES DENOMMES MSRV-1B ET MSRV-2B, DEFINISSANT RESPECTIVEMENT UN RETROVIRUS MSRV-1 ET UN AGENT CO-INFECTANT MSRV2, PAR AMPLIFICATION PCR "NICHEE" DES REGIONS POL CONSERVEES DES RETROVIRUS, SUR DES PREPARATIONS DE VIRIONS ISSUES DES LIGNEES LM7PC ET PLI-2**

Une technique PCR dérivée de la technique publiée par Shih **(12)** a été utilisée. Cette technique permet, par traitement de tous les composants du milieu réactionnel par la DNase, d'éliminer toute trace d'ADN contaminant. Elle permet parallèlement, par l'utilisation d'amorces différentes mais chevauchantes dans deux séries successives de cycles d'amplifications PCR, d'augmenter les chances d'amplifier un ADNc synthétisé à partir d'une quantité d'ARN faible au départ et encore réduite dans l'échantillon par l'action parasite de la DNAse sur l'ARN. En effet, la DNase est utilisée dans des conditions d'activité en excès qui permettent d'éliminer toute trace d'ADN contaminant, avant inactivation de cette enzyme restant dans l'échantillon par chauffage à 85°C pendant 10 minutes. Cette variante de la technique PCR décrite par Shih **(12)**, a été utilisée sur un ADNc synthétisé à partir des acides nucléiques de fractions de particules infectantes purifiées sur gradient de saccharose, selon la technique décrite par H. Perron **(13)**, à partir de l'isolat "POL-2" (ECACC n°V92072202) produit par la lignée PLI-2 (ECACC n°92072201) d'une part, et à partir de l'isolat MS7PG (ECACC n°V93010816) produit par la lignée LM7PC (ECACC n°93010817) d'autre part. Ces cultures ont été obtenues selon les méthodes ayant fait l'objet des demandes de brevet publiées sous les n° WO 93/20188 et WO 93/20189.

Après clonage avec le TA Cloning Kit® des produits amplifiés par cette technique et analyse de la séquence à l'aide d'un séquençeur automatique "Automatic Sequencer, modèle 373A" d'Applied Biosystems, les séquences ont été analysées à l'aide du logiciel Geneworks®, sur la dernière version disponible de la banque de données Genebank®.

Les séquences clonées et séquencées à partir de ces échantillons correspondent notamment à deux types de séquences: un premier type de séquence, retrouvé dans la majorité des clones (55 % des clones issus des isolats POL-2 des culture PLI-2, et, 67 % des clones issus des isolats MS7PG des cultures LM7PC), qui correspond à une famille de séquences "pol" proches mais différentes du rétrovirus endogène humain dénommé ERV-9 ou HSERV-9, et un second type de séquence qui correspond à des séquences très fortement homologues à une séquence attribuée à un autre agent infectant et/ou pathogène dénommé MSRV-2.

Le premier type de séquences représentant la majorité des clones est constituée de séquences dont la variabilité permet de définir quatre sous-familles de séquences. Ces sous-familles sont suffisamment proches entre elles pour qu'on puisse les considérer comme des quasi-espèces provenant d'un même rétrovirus, tel que cela est bien connu pour le rétrovirus HIV-1 **(14)**, ou comme le résultat de l'interférence avec plusieurs provirus endogènes co-régulés dans les cellules productrices. Ces éléments endogènes plus ou moins défectifs sont sensibles aux mêmes signaux de régulation générés éventuellement par un provirus réplicatif, puisqu'ils appartiennent à la même famille de rétrovirus endogènes **(15)**. Cette nouvelle famille de rétrovirus endogènes ou, alternativement, cette nouvelle espèce rétrovirale dont on a obtenu en culture la génération de quasi-espèces, et qui contient un consensus des séquences décrites ci-dessous est dénommée MSRV-1B.

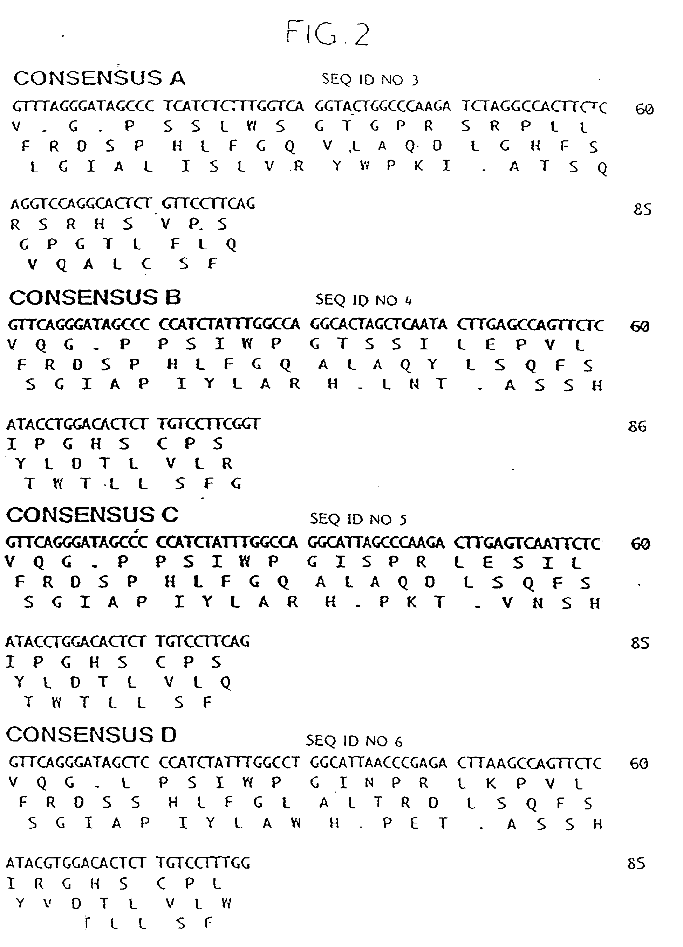

Dans la figure 1 sont présentés les consensus généraux des séquences des différents clones MSRV-1B séquencés lors de cette expérience, ces séquences étant respectivement identifiées par SEQ ID NO 3, SEQ ID NO 4, SEQ ID NO 5, et SEQ ID NO 6. Ces séquences présentent une homologie en acides nucléiques allant de 70 % à 88 % avec la séquence HSERV9 référencée X57147 et M37638 dans la base de données Genebank®. Quatre séquences nucléiques "consensus" représentatives de différentes quasi-espèces d'un rétrovirus MSRV-1B éventuellement exogène, ou de différentes sous-familles d'un rétrovirus endogène MSRV-1B, ont été définies. Ces consensus représentatifs sont présentés dans la figure 2, avec la traduction en acides aminés. Une trame de lecture fonctionnelle existe pour chaque sous-famille de ces séquences MSRV-1B, et l'on peut voir que la trame de lecture ouverte fonctionnelle correspond à chaque fois à la séquence en acides aminés venant en deuxième ligne sous la séquence en acide nucléiques. Le consensus général de la séquence MSRV-1B, identifié par SEQ ID NO 7 et obtenu par cette technique PCR dans la région "pol" est présenté dans la figure 1.

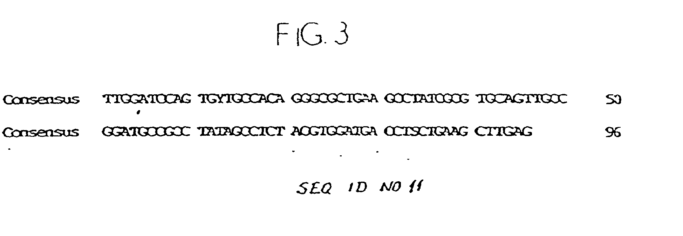

Le deuxième type de séquence représentant la majorité des clones séquencés est représenté par la séquence MSRV-2B présentée dans la figure 3 et identifiée par SEQ ID NO 11. Les différences observées dans les séquences correspondant aux amorces PCR s'expliquent par l'utilisation d'amorces dégénérées en mélange utilisées dans des conditions techniques différentes.

La séquence MSRV-2B (SEQ ID NO 11) est suffisamment divergente des séquences rétrovirales déjà décrites dans les banques de données pour qu'on puisse avancer qu'il s'agit d'une région de séquence appartenant à un nouvel agent infectant, dénommé MSRV-2. Cet agent infectant s'apparenterait à priori, d'après l'analyse des premières séquences obtenues, à un rétrovirus, mais, étant donnée la technique utilisée pour obtenir cette séquence, il pourrait aussi s'agir d'un virus à ADN dont le génome code pour une enzyme qui possède accessoirement une activité transcriptase inverse comme c'est le cas, par exemple, pour le virus de l'hépatite B, HBV (12). De plus, le caractère aléatoire des amorces dégénérées utilisées pour cette technique d'amplification PCR peut très bien avoir permis, du fait d'homologies de séquences imprévues ou de sites conservés dans le gène d'une enzyme apparentée, l'amplification d'un acide nucléique provenant d'un agent pathogène et/ou co-infectant prokaryote ou eukaryote (protiste).

**EXEMPLE 2 : OBTENTION DE CLONES DENOMMES MSRV-1B ET MSRV-2B, DEFINISSANT UNE FAMILLE MSRV-1 et MSRV2, PAR AMPLIFICATION PCR "NICHEE" DES REGIONS POL CONSERVEES DES RETROVIRUS, SUR DES PREPARATIONS DE LYMPHOCYTES B D'UN NOUVEAU CAS DE SEP**

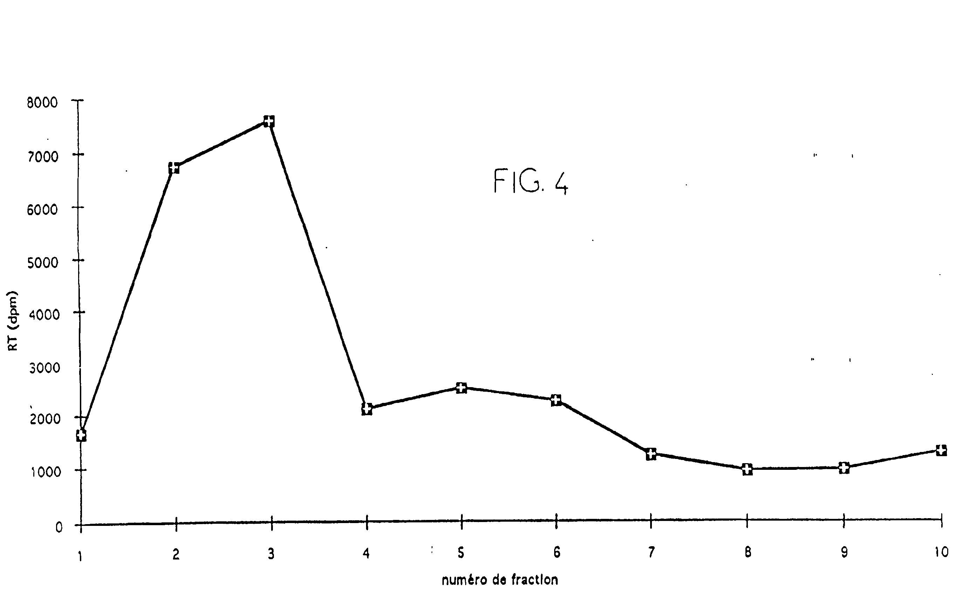

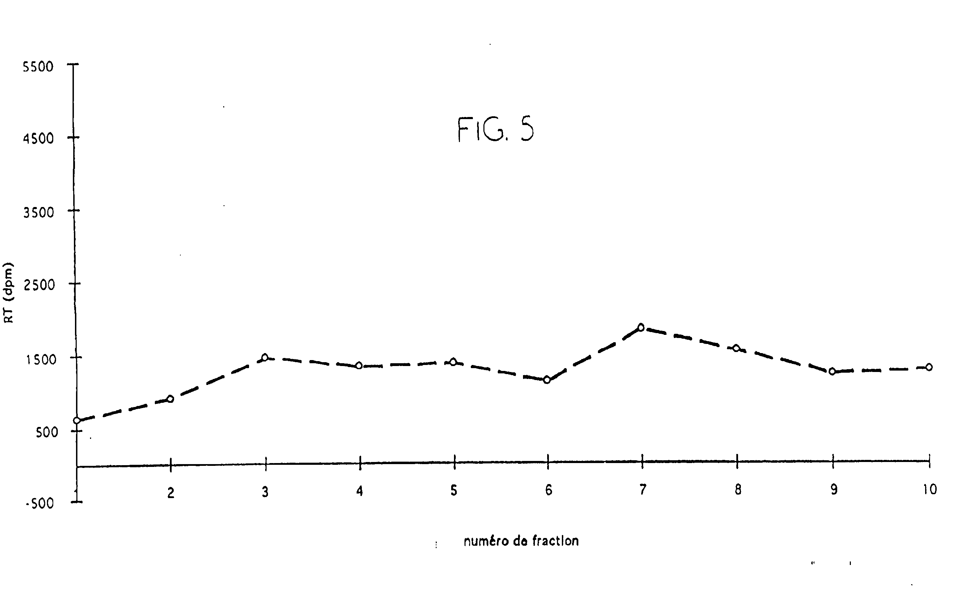

La même technique PCR modifiée d'après la technique de Shih (12) a été utilisée pour amplifier et séquencer le matériel nucléique ARN présent dans une fraction de virions purifiée au pic d'activité transcriptase inverse "de type LM7", sur gradient de saccharose selon la technique décrite par H. Perron **(13)**, et selon les protocoles mentionnés dans l'exemple 1, à partir d'une lignée lymphoblastoïde spontanée, obtenue par auto-immortalisation en culture de lymphocytes B d'un patient SEP séropositif pour le virus d'Epstein-Barr (EBV), après mise en culture des cellules lymphoïdes sanguines dans un milieu de culture approprié contenant une concentration appropriée de cyclosporine A. Une représentation de l'activité transcriptase inverse dans les fractions de saccharose prélevées sur un gradient de purification des virions produits par cette lignée est présentée dans la figure 4. De même, les surnageants de culture d'une lignée B obtenue dans les mêmes conditions à partir d'un témoin exempt de SEP ont été traités dans les mêmes conditions, et le dosage de l'activité transcriptase inverse dans les fractions du gradient de saccharose s'est avéré négatif partout (bruit de fond) et est présenté dans la figure 5. La fraction 3 du gradient correspondant à la lignée B de SEP et la même fraction sans activité transcriptase inverse du gradient témoin non-SEP, ont été analysées par la même technique RT-PCR que précédemment, dérivée de Shih **(12)**, suivie des mêmes étapes de clonage et de séquençage tels que décrites dans l'exemple 1.

Il est tout à fait notable que les séquences de type MSRV-1 et MSRV-2 soient retrouvées dans le seul matériel associé à un pic d'activité transcriptase inverse "de type LM7" provenant de la lignée lymphoblastoïde B de SEP. Ces séquences n'ont pas été retrouvées avec le matériel de la lignée lymphoblastoïde B témoin (non-SEP) dans 26 clones recombinants pris au hasard. Seules les séquences contaminantes de type Mo-MuLV provenant de la transcriptase inverse commerciale utilisée pour l'étape de synthèse de l'ADNc et des séquences sans analogie rétrovirale particulière ont été retrouvées chez ce témoin, du fait de l'amplification "consensus" des séquences de polymérases homologues que réalise cette technique PCR. De plus, l'absence de cible concentrée qui fait compétition pour la réaction d'amplification dans l'échantillon témoin permet l'amplification de contaminants dilués. La différence de résultats est à l'évidence hautement significative (chi-2, $p<0,001$).

**EXEMPLE 3 : OBTENTION D'UN CLONE PSJ17, DEFINISSANT UN RETROVIRUS MSRV-1, PAR REACTION DE TRANSCRIPTASE INVERSE ENDOGENE SUR UNE PREPARATION DE VIRION ISSUE DE LA LIGNEE PLI-2.**

Cette approche vise à obtenir des séquences d'ADN rétrotranscrites à partir de l'ARN supposé rétroviral dans l'isolat, en utilisant l'activité transcriptase inverse présente dans ce même isolat. Cette activité transcriptase inverse ne peut théoriquement fonctionner qu'en présence d'un ARN rétroviral, lié à un ARNt amorce ou hybridé à des brins courts d'ADN déjà rétro-transcrits dans les particules rétrovirales **(16)**. Ainsi l'obtention de séquences rétrovirales spécifiques dans un matériel contaminé par des acides nucléiques cellulaires, a été optimisée selon ces auteurs grâce à l'amplification enzymatique spécifique des portions d'ARN viraux par une activité transcriptase inverse virale. Les auteurs ont pour cela, déterminé les conditions physico-chimiques particulières dans lesquelles cette activité enzymatique de transcription inverse sur des ARN contenus dans des virions pouvait être effective in vitro. Ces conditions correspondent à la description technique des protocoles présentés ci-dessous (réaction de RT endogène, purification, clonage et séquençage).

L'approche moléculaire a consisté à utiliser une préparation de virion concentré, mais non purifié, obtenu à partir des surnageants de culture de la lignée PLI-2 préparés selon la méthode suivante : les surnageants de culture sont collectés deux fois par semaine, pré-centrifugés à 10 000 trs/min pendant 30 minutes pour éliminer les débris cellulaires, et ensuite, congelés à -80°C ou utilisés tels quels pour les étapes suivantes. Les surnageants frais ou décongelés sont centrifugés sur un coussin de PBS-glycérol 30% à 100 000g (ou 30 000 trs/min dans un rotor LKB-HITACHI de type 45 T) pendant 2h à 4 °C. Après élimination du surnageant, le culot sédimenté est repris dans un petit volume de PBS et constitue la fraction de virion concentré, mais non purifié. Cet échantillon viral concentré mais non purifié a été utilisé pour effectuer une réaction dite de transcription inverse endogène, telle que décrite ci-après.

Un volume de 200 µl de virion purifié selon le protocole décrit ci-dessus et contenant une activité transcriptase inverse d'environ 1-5 millions de dpm est décongelé à 37°C jusqu'à apparition d'une phase liquide, puis placé sur de la glace. Un tampon 5 fois concentré a été préparé avec les composants suivants: Tris-HCl pH 8,2, 500 mM; NaCl 75 mM; $MgCl_2$ 25 mM; DTT 75 mM et NP 40 0,10 % ; 100 µl de tampon 5X + 25 µl d'une solution de dATP 100mM + 25 µl d'une solution de dTTP 100 mM + 25 µl d'une solution de dGTP 100 mM + 25 µl d'une solution de dCTP 100 mM + 100 µl d'eau distillée stérile + 200 µl de la suspension de virions (activité T.I. de 5 millions de DPM) dans le PBS ont été mélangés et incubés à 42°C pendant 3 heures. Après cette incubation le mélange réactionnel est directement ajouté à un mélange tamponné phénol/chloroforme/alcool isoamylique (Sigma ref. P 3803); la phase aqueuse est collectée et un volume d'eau distillée stérile est ajouté à la phase organique pour ré-extraire le matériel nucléique résiduel. Les phases aqueuses collectées sont regroupées et les acides nucléiques contenus sont précipités par addition d'acétate de sodium 3M, pH 5,2 au 1/10 de volume + 2 volumes d'éthanol + 1 µl de glycogène (Boehringer-Mannheim ref. 901 393) et mise à -20°C de l'échantillon pendant 4h ou la nuit à +4°C. Le précipité obtenu après centrifugation est ensuite lavé avec de l'éthanol à 70% et resuspendu dans 60 ml d'eau distillée. Les produits de cette réaction ont ensuite été purifiés, clonés et séquencés selon le protocole décrit ci-après: des ADN bouts francs avec des adénines non-appariées aux extrémités ont été générés: une réaction de "remplissage" a d'abord été effectuée: 25 µl de la solution d'ADN précédemment purifiée ont été mélangés avec 2 µl d'une solution 2,5 mM contenant, en quantité equimolaire, dATP + dGTP + dTTP + dCTP / 1 µl d'ADN polymérase T4 (Boehringer-Mannheim ref. 1004 786) / 5 µl de 10X "incubation buffer for restriction enzyme" (Boehringer-Mannheim ref. 1417 975) / 1 µl d'une solution à 1 % de sérum-albumine bovine / 16 µl d'eau distillée stérile. Ce mélange a été incubé 20 minutes à 11°C. 50 µl de tampon TE et 1 µl de glycogène (Boehringer-Mannheim ref. 901 393) y ont été ajoutés avant extraction des acides nucléiques avec du phénol/chloroforme/alcool isoamylique (Sigma ref. P 3803), et précipitation à l'acétate de sodium comme décrit précédemment. L'ADN précipité après centrifugation est resuspendu dans 10 µl de tampon 10 mM Tris pH 7,5. Puis, 5 µl de cette suspension ont été mélangés avec 20 µl de tampon Taq 5X, 20 µl de 5mM dATP, 1 µl (5U) de Taq ADN polymérase (Amplitaq™) et 54 µl d'eau distillée stérile. Ce mélange est incubé 2 h à 75°C avec un film d'huile à la surface de la solution. L'ADN en suspension dans la solution aqueuse prélevée en dessous du film d'huile après incubation est précipité comme décrit précédemment et resuspendu dans 2 µl d'eau distillée stérile. L'ADN obtenu a été inséré dans un plamide à l'aide du kit TA Cloning™. Les 2 µl de solution d'ADN ont été mélangés avec 5 µl d'eau distillée stérile, 1µl d'un tampon de ligation 10 fois concentré "10X LIGATION BUFFER", 2 µl de "pCR™ VECTOR" (25 ng/ml) et 1 µl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" (17). La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA cloning kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer, modèle 373 A" Applied Biosystems, selon les instructions du fabricant.

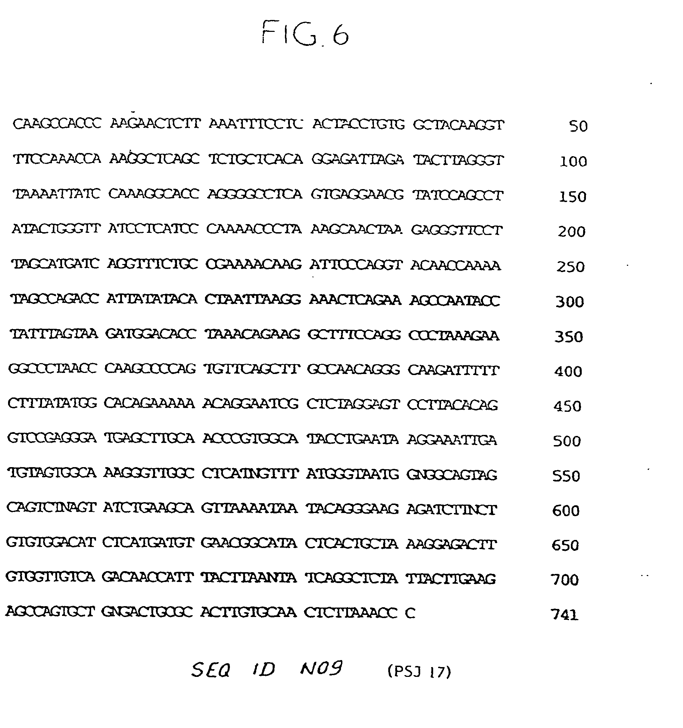

L'analyse discriminante sur les banques de données informatiques des séquences clonées à partir des fragments d'ADN présents dans le mélange réactionnel a permis de mettre en évidence une séquence de type rétroviral. Le clone correspondant PSJ17 a été entièrement séquencé et la séquence obtenue, présentée dans la figure 6 et identifiée apr SEQ ID N°9, a été analysée à l'aide du logiciel "Geneworks®" sur les banques de données actualisées "Genebank®". L'analyse des banques de données n'a pas permis de trouver de séquence identique déjà décrite. Seule une homologie partielle a pu être retrouvée avec certains éléments rétroviraux connus. L'homologie relative la plus intéressante concerne un rétrovirus endogène dénommé ERV-9, ou HSERV-9, selon les références **(18)**.

**<u>EXEMPLE 4</u> : AMPLIFICATION PCR DE LA SEQUENCE NUCLEIQUE CONTENUE ENTRE LA REGION 5' DEFINIE PAR LE CLONE "POL MSRV-1B" ET LA REGION 3' DEFINIE PAR LE CLONE PSJ17.**

Cinq oligonucléotides, M001, M002-A, M003-BCD, P004 et P005, ont été définis pour amplifier de l'ARN provenant de virions purifiés POL-2. Des réactions de contrôle ont été effectuées de manière à contrôler la présence de contaminants (réaction sur de l'eau). L'amplification consiste en une étape de RT-PCR selon le protocole décrit dans l'Exemple 2, suivie d'une PCR "nichée" selon le protocole PCR décrit dans le document EP-A-0 569 272. Dans le premier cycle RT-PCR, les amorces M001 et P004 ou P005 sont utilisées. Dans le deuxième cycle PCR, les amorces M002-A ou M003-BCD, et l'amorce POO4 sont utilisées. Les amorces sont positionnées comme suit :

```
                M002-A
                M003-BCD
          M001  ___                            P004      P005
```

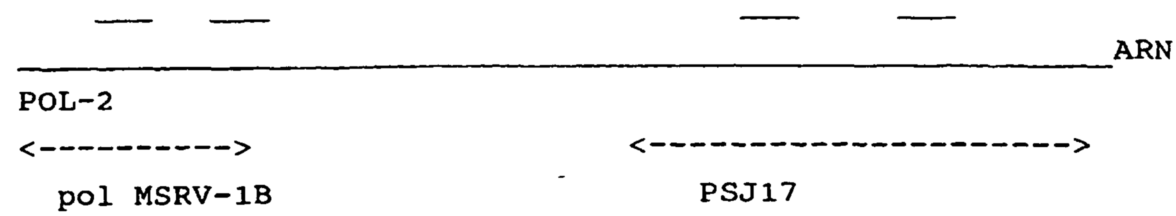


Leur composition est :

amorce M001: GGTCITICCICAIGG (SEQ ID NO 20)
amorce M002-A: TTAGGGATAGCCCTCATCTCT (SEQ ID NO 21)
amorce M003-BCD: TCAGGGATAGCCCCCATCTAT(SEQ ID NO 22)
amorce P004: AACCCTTTGCCACTACATCAATTT (SEQ ID NO 23)
amorce P005: GCGTAAGGACTCCTAGAGCTATT (SEQ ID NO 24)

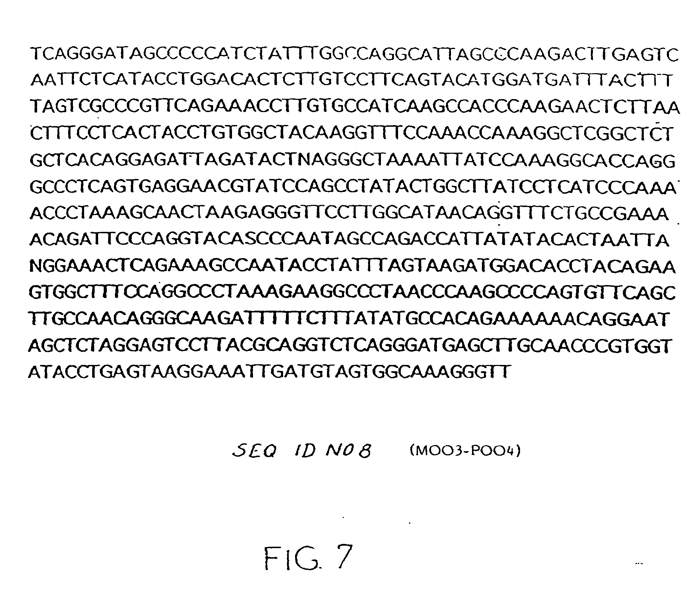

Le produit d'amplification "nichée" obtenu et dénommé M003-P004 est présenté dans la figure 7, et correspond à la séquence SEQ ID NO 8.

**<u>EXEMPLE 5</u> : AMPLIFICATION ET CLONAGE D'UNE PORTION DU GENOME RETROVIRAL MSRV-1 A L'AIDE D'UNE SEQUENCE DEJA IDENTIFIEE, DANS UN ECHANTILLON DE VIRUS PURIFIE AU PIC D'ACTIVITE TRANSCRIPTASE INVERSE**

Une technique PCR dérivée de la technique publiée par Frohman (19) a été utilisée. La technique dérivée permet, à l'aide d'une amorce spécifique en 3' du génome à amplifier, d'élonguer la séquence vers la région 5' du génome à analyser. Cette variante technique est décrite dans la documentation de la firme "Clontech Laboratories Inc., (Palo-Alto California, USA) fournie avec son produit "5'-AmpliFINDER™ RACE Kit", qui a été utilisé sur une fraction de virion purifié comme décrit précédemment.

Les amorces 3' spécifiques utilisées dans le protocole du kit pour la synthèse du cDNA et l'amplification PCR sont respectivement complémentaires aux séquences MSRV-1 suivantes :

| | | |
|---|---|---|
| cDNA : | TCATCCATGTACCGAAGG | (SEQ ID N°25) |
| amplification : | ATGGGGTTCCCAAGTTCCCT | (SEQ ID N°26) |

Les produits issus de la PCR ont été purifiés après purification sur gel d'agarose selon les méthodes conventionnelles (17), puis resuspendus dans 10 ml d'eau distillée. Une des propriétés de la polymérase Taq consistant à ajouter une adénine à l'extémité 3' de chacun des deux brins d'ADN, l'ADN obtenu a été directement inséré dans un plamide à l'aide du kit TA Cloning™ (British Biotechnology). Les 2 µl de solution d'ADN ont été mélangés avec 5 µl d'eau distillée stérile, 1 µl d'un tampon de ligation 10 fois concentré "10X LIGATION BUFFER", 2 µl de "pCR™ VECTOR" (25 ng/ml) et 1 µl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" (17). La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer, modèle 373 A" Applied Biosystems selon les instructions du fabricant.

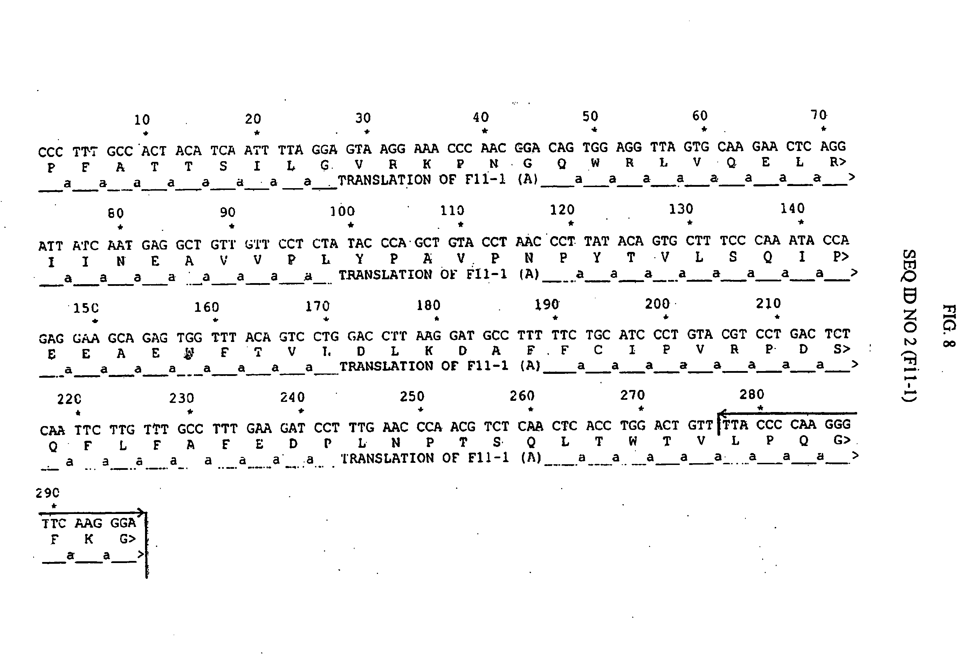

Cette technique a d'abord été appliquée à deux fractions de virion purifié comme décrit ci-après, sur saccharose à partir de l'isolat "POL-2" produit par la lignée PLI-2 d'une part, et à partir de l'isolat MS7PG produit par la lignée LM7PC d'autre part. Les surnageants de cultures sont collectés deux fois par semaine, pré-centrifugés à 10 000 trs/min pendant 30 minutes pour éliminer les débris cellulaires, et ensuite, congelés à -80°C ou utilisés tels quels pour les étapes suivantes. Les surnageants frais ou décongelés sont centrifugés sur un coussin de PBS-glycérol 30% à 100 000g (ou 30 000 trs/min dans un rotor LKB-HITACHI de type 4,5 T) pendant 2 h à 4°C. Après élimination du surnageant, le culot sédimenté est repris dans un petit volume de PBS et constitue la fraction de virions concentrés, mais non purifiés. Le virus concentré est ensuite déposé sur un gradient de sucrose dans un tampon PBS stérile (15 à 50 % poids/poids) et ultracentrifugé à 35 000 trs/min (100 000g) pendant 12 h à +4°C dans un rotor à godets. 10 fractions sont recueillies et 20 µl sont prélevés dans chaque fraction après homogénéisation pour y doser l'activité transcriptase inverse selon la technique décrite par H. Perron (3). Les fractions contenant le pic d'activité RT "de type LM7" sont ensuite diluées dans du tampon PBS stérile et ultracentrifugées une heure à 35 000 trs/min (100 000g) pour sédimenter les particules virales. Le culot de virion purifié ainsi obtenu est alors repris dans un petit volume d'un tampon adéquat pour l'extraction d'ARN. La réaction de synthèse de cDNA mentionnée plus haut est réalisée sur cet ARN extrait de virion extracellulaire purifié. L'amplification PCR selon la technique citée plus-haut a permis d'obtenir le clone F1-11 dont la séquence identifiée par SEQ ID NO 2, est présentée dans la figure 8.

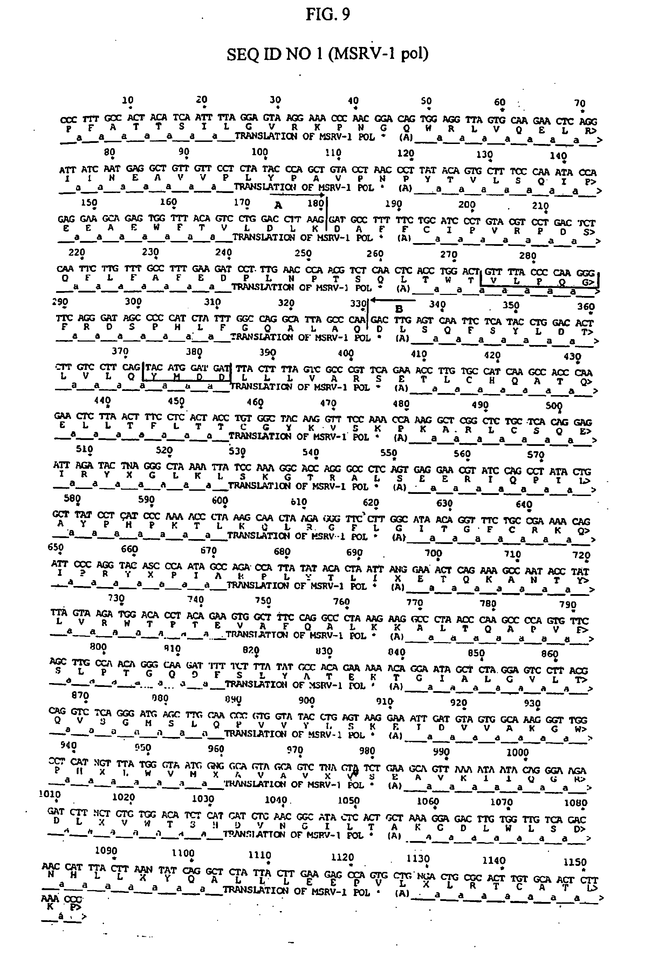

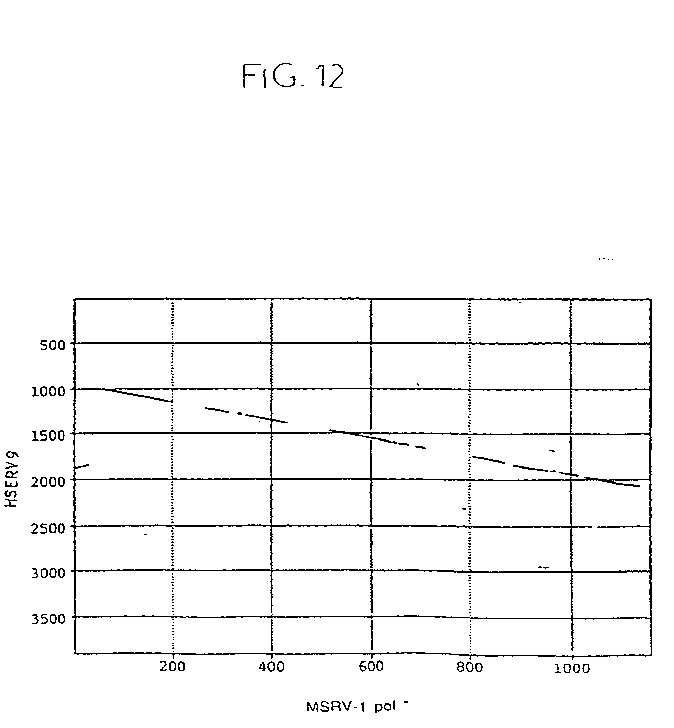

Ce clone permet de définir, avec les différents clones préalablement séquences, une région de longueur importante (1,2 kb) représentative du gène "pol" du rétrovirus MSRV-1, telle que présentée dans la figure 9. Cette séquence dénommée SEQ ID NO 1 est reconstituée à partir de différents clones se recouvrant à leurs extrémités, en corrigeant les artéfacts liés aux amorces et aux techniques d'amplification ou de clonage, qui interromperaient artificiellement la trame de lecture de l'ensemble. Cette séquence sera identifiée ci-après sous la dénomination "région MSRV-1 pol *". Son degré d'homologie avec la séquence HSERV-9 est représenté à la figure 12.

Dans la figure 9, la trame de lecture potentielle avec sa traduction en acides aminés est présentée en-dessous de la séquence en acides nucléiques.

**<u>EXEMPLE 6</u> : DETECTION DE SEQUENCES SPECIFIQUES MSRV-1 et MSRV-2 DANS DIFFERENTS ECHANTILLONS DE PLASMA PROVENANT DE PATIENTS ATTEINTS DE SEP OU DE TEMOINS.**

Une technique PCR a été utilisée pour détecter les génomes MSRV-1 et MSRV-2 dans des plasmas obtenus après prélèvement de sang sur EDTA de patients atteints de SEP et de témoins non-SEP.

L'extraction des ARN de plasma a été effectuée selon la technique décrite par P. Chomzynski (20), après addition d'un volume du tampon contenant du guanidinium thiocyanate à 1 ml de plasma gardé congelé à -80°C après recueil.

Pour MSRV-2, la PCR a été effectuée dans les mêmes conditions et avec les amorces suivantes :

- amorce 5', identifiée par SEQ ID NO 14
  5' GTAGTTCGATGTAGAAAGCG 3' ;
- amorce 3', identifiée par SEQ ID NO 15
  5' GCATCCGGCAACTGCACG 3'.

Cependant des résultats similaires ont aussi été obtenus avec les amorces PCR suivantes dans deux amplifications successives par PCR "nichée" sur échantillons d'acides nucléiques non-traités par la DNase.

Les amorces utilisées pour cette première étape de 40 cycles avec une température d'hybridation de 48°C sont les suivantes :

- amorce 5', identifiée par SEQ ID NO 27
  5' GCCGATATCACCCGCCATGG 3', correspondant à une amorce PCR MSRV-2 5', pour une première PCR sur prélèvement de patients,
- amorce 3', identifiée par SEQ ID NO 28
  5' GCATCCGGCAACTGCACG 3', correspondant à une amorce PCR MSRV-2 3', pour une première PCR sur prélèvement de patients.

Après cette étape, 10µl du produit d'amplification sont prélevés et utilisés pour réaliser une deuxième amplification PCR dite "nichée" avec des amorces situées à l'intérieur de la région déjà amplifiée. Cette deuxième étape se déroule sur 35 cycles, avec une température d'hybridation des amorces ("annealing") de 50°C. Le volume réactionnel est de 100µl.

Les amorces utilisées pour cette deuxième étape sont les suivantes :

- amorce 5', identifiée par SEQ ID NO 29
  5' CGCGATGCTGGTTGGAGAGC 3', correspondant à une amorce PCR MSRV-2 5', pour une PCR-nichée sur prélèvement de patients,
- amorce 3', identifiée par SEQ ID NO 30
  5' TCTCCACTCCGAATATTCCG 3', correspondant à une amorce PCR MSRV-2 3', pour une PCR-nichée sur prélèvement de patients.

Pour MSRV-1, l'amplification a été effectuée en deux étapes. De plus, l'échantillon d'acides nucléiques est préalablement traité par la DNase et un contrôle PCR sans RT (transcriptase inverse AMV) est effectué sur les deux étapes d'amplification, de manière à vérifier que l'amplification RT-PCR provient exclusivement de l'ARN MSRV-1. En cas de contrôle sans RT positif, l'échantillon aliquoté d'ARN de départ est à nouveau traité par la DNase et amplifié à nouveau.

Le protocole de traitement par la DNase dépourvue d'activité RNAse est le suivant: l'ARN extrait est aliquoté en présence de "RNAse inhibitor" (Boehringer-Mannheim) dans de l'eau traitée au DEPC à une concentration finale de 1 $\mu$g dans 10 $\mu$l; à ces 10 $\mu$l, est ajouté 1$\mu$l de "RNAse-free DNAse" (Boehringer-Mannheim) et 1, 2 $\mu$l de tampon à pH 5 contenant 0,1 M/l d'acétate de sodium et 5 mM/l de $MgSO_4$ le mélange est incubé 15 min. à 20°C et porté à 95°C pendant 1,5 min. dans un "thermocycler".

La première étape de RT-PCR MSRV-1 est effectuée selon une variante du procédé d'amplification d'ARN tel que décrit dans la demande de brevet n° EP-A-0 569 272. Notamment, l'étape de synthèse d'ADNc est effectuée à 42°C pendant une heure; l'amplification PCR se déroule sur 40 cycles, avec une température d'hybridation des amorces ("annealing") de 53°C. Le volume réactionnel est de 100$\mu$l.

Les amorces utilisées pour cette première étape sont les suivantes :

- amorce 5', identifiée par SEQ ID NO 16
  5' AGGAGTAAGGAAACCCAACGGAC 3' ;
- amorce 3', identifiée par SEQ ID NO 17
  5' TAAGAGTTGCACAAGTGCG 3'.

Après cette étape, 10$\mu$l du produit d'amplification sont prélevés et utilisés pour réaliser une deuxième amplification PCR dite "nichée" avec des amorces situées à l'intérieur de la région déjà amplifiée. Cette deuxième étape se déroule sur 35 cycles, avec une température d'hybridation des amorces ("annealing") de 53°C. Le volume réactionnel est de 100$\mu$l.

Les amorces utilisées pour cette deuxième étape sont les suivantes :

- amorce 5', identifiée par SEQ ID NO 18
  5' TCAGGGATAGCCCCCATCTAT 3' ;
- amorce 3', identifiée par SEQ ID NO 19
  5' AACCCTTTGCCACTACATCAATTT 3'.

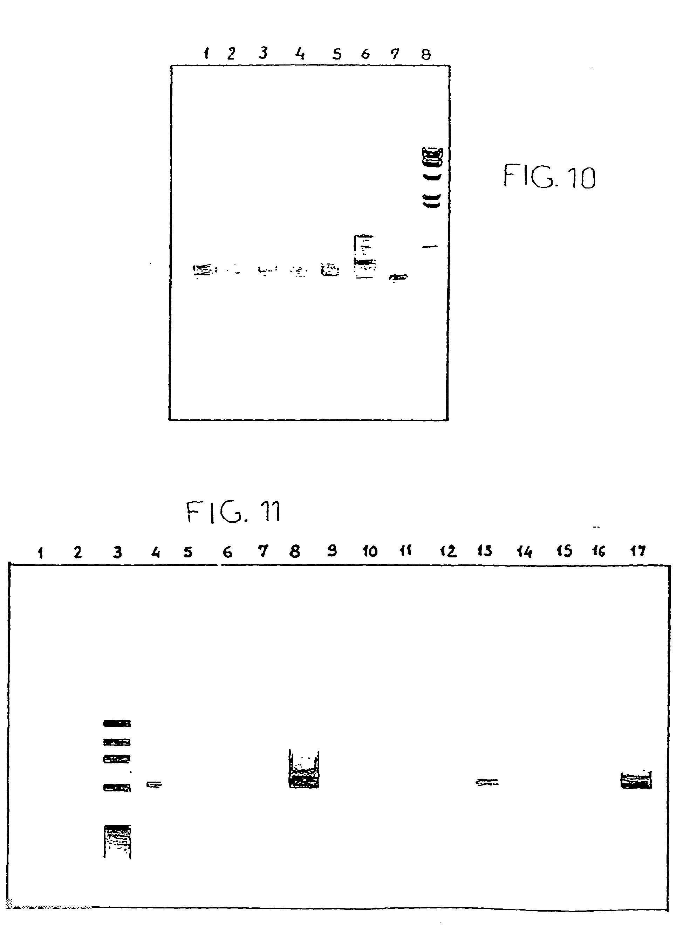

Dans les figures 10 et 11, sont présentés les résultats de PCR sous forme de photographies sous lumière ultra-violette de gels d'agarose imprégnés de bromure d'éthidium, dans lesquels on a effectué une électrophorèse des produits d'amplification PCR déposés séparément dans les différents puits.

La photographie du haut (figure 10) représente le résultat de l'amplification spécifique MSRV-2.

Le puits numéro 8 contient un mélange de marqueurs de poids moléculaire ADN et les puits 1 à 7 représentent, dans l'ordre, les produits amplifiés à partir des ARN totaux de plasmas provenant de 4 témoins sains exempts de SEP (puits 1 à 4) et de 3 patients atteints de SEP, à différents stades de la maladie (puits 5 à 7).

Dans cette série, du matériel nucléique MSRV-2 est détecté dans le plasma d'un cas de SEP sur les 3 testés et dans aucun des 4 plasmas témoins. D'autres résultats obtenus sur des séries plus étendues confirment ces résultats.

La photographie du bas (figure 11) représente le résultat de l'amplification spécifique par RT-PCR "nichée" MSRV-1:

le puits n°1 contient le produit PCR effectué avec de l'eau seule, sans addition de transcriptase inverse AMV; le puits n°2 contient le produit PCR effectué avec de l'eau seule, avec addition de transcriptase inverse AMV; le puits numéro 3 contient un mélange de marqueurs de poids moléculaire ADN; les puits 4 à 13 contiennent, dans l'ordre, les produits amplifiés à partir des ARN totaux extraits de fractions de gradient de saccharose (collectées du haut vers le bas) sur lequel un culot de virion provenant d'un surnageant de culture infectée par MSRV-1 et MSRV-2 a été centrifugé à l'équilibre selon le protocole décrit par H. Perron (13); dans le puits 14 rien n'a été déposé; dans les puits 15 à 17 , on a déposé les produits amplifiés d'ARN extrait de plasmas provenant de 3 patients différents atteints de SEP, à différents stades de la maladie.

Le génome rétroviral MSRV-1 est bien retrouvé dans la fraction du gradient de saccharose contenant le pic d'activité transcriptase inverse mesurée selon la technique décrite par H. Perron (3), avec une très forte intensité (fraction 5 du gradient, déposée dans le puits n°8). Une légère amplification a eu lieu dans la première fraction (puits n°4) correspondant vraisemblablement à de l'ARN libéré par des particules lysées qui flottaient à la surface du gradient; de même, des débris aggrégés ont sédimenté dans la dernière fraction (fond de tube) entraînant quelques copies de génome MSRV-1 qui ont donné lieu à une amplification de faible intensité.

Sur les 3 plasmas de SEP testés dans cette série, l'ARN MSRV-1 a été retrouvé dans un cas, produisant une amplification très intense (puits n°17).

Dans cette série, le génome ARN rétroviral MSRV-1 correspondant vraisemblablement à des particules de virus extracellulaire présentes dans le plasma en nombre extrêmement faible, a été détecté par RT-PCR "nichée" dans un cas de SEP sur les 3 testés. D'autres résultats obtenus sur des séries plus étendues confirment ces résultats.

De plus, la spécificité des séquences amplifiées par ces techniques PCR peut être vérifiée et évaluée par la technique "ELOSA" telle que décrite par F. Mallet (21) et dans le document FR-A-2 663 040.

Pour MSRV-1 les produits de la PCR nichée sus-décrite peuvent être testés dans deux systèmes ELOSA permettant de détecter séparément un consensus A et un consensus B+C+D de MSRV-1, correspondant aux sous-familles décrites dans l'exemple 1 et les figures 1 et 2. En effet, les séquences proches du consensus B+C+D sont retrouvées essentiellement dans les échantillons d'ARN provenant de virions MSRV-1 purifiés à partir de cultures ou amplifiés dans des liquides biologiques extra-celluaires de patients SEP, alors que les séquences proches du consensus A sont essentiellement retrouvées dans l'ADN cellulaire humain normal.

Le système ELOSA/MSRV-1 pour la capture et l'hybridation spécifique des produits PCR de la sous-famille A utilise un oligonucléotide de capture cpV1A avec une liaison amine en 5' et un oligonucléotide de détection biotinylé dpV1A ayant respectivement pour séquence:

- cpV1A identifié par SEQ ID NO 31
  5' GATCTAGGCCACTTCTCAGGTCCAGS 3', correspondant à l'oligonucléotide de capture ELOSA des produits PCR-nichée MSRV-1 effectuée avec les amorces identifiées par SEQ ID NO 16 et SEQ ID NO 17, suivie éventuellement de l'amplification avec les amorces identifiées par SEQ ID NO 18 et SEQ ID NO 19 sur prélèvement de patients ;
- dpV1A identifié par SEQ ID NO 32
  5' CATCTITTTGGICAGGCAITAGC 3' correspondant à l'oligonucléotide de capture ELOSA de la sous-famille A des produits PCR-"nichée" MSRV-1 effectuée avec les amorces identifiées par SEQ ID NO 16 et SEQ ID NO 17, suivie éventuellement de l'amplification avec les amorces identifiées par SEQ ID NO18 et SEQ ID NO 19 sur prélèvement de patients.

Le système ELOSA/MSRV-1 pour la capture et l'hybridation spécifique des produits PCR de la sous-famille B+C+D utilise le même oligonucléotide de détection biotinylé dpV1A et un oligonucléotide de capture cpV1B avec une liaison amine en 5'ayant pour séquence :

- dpV1B identifié par SEQ ID N°33
  5' CTTGAGCCAGTTCTCATACCTGGA 3', correspondant à l'oligonucléotide de capture ELOSA de la sous-famille B + C + D des produits PCR-"nichée" MSRV-1 effectuée avec les amorces identifiées par SEQ ID NO 16 et SEQ ID NO 17, suivie éventuellement de l'amplification avec les amorces identifiées par SEQ ID NO 18 et SEQ ID NO 19 sur prélèvement de patients.

Ce système de détection ELOSA a permis de vérifier qu'aucun des produits PCR ainsi amplifiés à partir de plasmas de patients SEP traités par la DNase ne contenait de séquence de la sous-famille A et que tous étaient positifs avec le consensus des sous-familles B, C et D.

Pour MSRV-2, une technique ELOSA similaire a été évaluée sur des isolats provenant de cultures cellulaires infectées en utilisant les amorces d'amplification PCR suivantes :

- amorce 5', identifiée par SEQ ID NO 34
  5' AGTGYTRCCMCARGGCGCTGAA 3', correspondant à une amorce PCR MSRV-2 5', pour PCR sur prélèvement de cultures,
- amorce 3', identifiée par SEQ ID NO 35
  5' GMGGCCAGCAGSAKGTCATCCA 3', correspondant à une amorce PCR MSRV-2 3', pour PCR sur prélèvement de cultures,

et les oligonucléotides de capture avec une liaison amine en 5' cpV2, et de détection biotinylé dpV2, ayant pour séquences respectives :

- cpV2 identifiée par SEQ ID NO 36
  5 GGATGCCGCCTATAGCCTCTAC 3', correspondant à un oligonucléotide de capture ELOSA des produits de la PCR MSRV-2 effectuée avec les amorces SEQ ID NO 34 et SEQ ID NO 35, ou éventuellement avec les amorces dégénérées définies par Shih (12),

- dpV2 identifié par SEQ ID NO 37
  5' AAGCCTATCGCGTGCAGTTGCC 3', correspondant à un oligonucléotide de détection ELOSA des produits de la PCR MSRV-2 effectuée avec les amorces SEQ ID NO 34 et SEQ ID NO 35, ou éventuellement avec les amorces dégénérées définies par Shih (12).

Ce système d'amplification PCR avec un couple d'amorces différent de ceux qui ont été précédemment décrits pour l'amplification sur les échantillons de patients, a permis de confirmer l'infection par MSRV-2 de cultures in vitro et d'échantillons d'acides nucléiques utilisés pour les travaux de biologie moléculaire.

En fin de compte, les premiers résultats de détection PCR du génome d'agents pathogènes et/ou infectants, montrent qu'il est vraisemblable que du "virus" libre peut circuler dans le flux sanguin de patients en phase de poussée aigüe, en dehors du système nerveux. Ceci est compatible avec l'existence quasi-systématique de "brèches" dans la barrière hémato-encéphalique des patients en phase active de SEP.

## <u>EXEMPLE 7</u> : OBTENTION DE SEQUENCES DU GENE "env" DU GENOME RETROVIRAL MSRV-1.

Ainsi que cela a déjà été décrit dans l'exemple 5, une technique PCR dérivée de la technique publiée par Frohman (19) a été utilisée. La technique dérivée permet, à l'aide d'une amorce spécifique en 3' du génome à amplifier, d'élonguer la séquence vers la région 5' du génome à analyser. Cette variante technique est décrite dans la documentation de la firme "Clontech Laboratories Inc., (Palo-Alto California, USA) fournie avec son produit "5'-AmpliFINDER™ RACE Kit", qui a été utilisé sur une fraction de virion purifié comme décrit précédemment.

Afin de réaliser une amplification de la région 3' du génome rétroviral MSRV-1 en englobant la région du gène "env", une étude a été réalisée pour déterminer une séquence consensus dans les régions LTR de même type que celles du rétrovirus endogène défectif HSERV-9 (18,24), avec lequel le rétrovirus MSRV-1 présente des homologies partielles.

La même amorce 3' spécifique a été utilisée dans le protocole du kit pour la synthèse du ADNc et l'amplification PCR ; sa séquence est la suivante :

GTGCTGATTGGTGTATTTACAATCC (SEQ ID NO 45)

La synthèse de l'ADN complémentaire (ADNc) et l'amplification PCR monodirectionelle avec l'amorce ci-dessus, ont été réalisées en une étape, selon le procédé décrit dans le brevet EP-A-0 569 272.

Les produits issus de la PCR ont été extraits après purification sur gel d'agarose selon les méthodes conventionnelles **(17)**, puis resuspendus dans 10 ml d'eau distillée. Une des propriétés de la polymérase Taq consistant à ajouter une adénine à l'extémité 3' de chacun des deux brins d'ADN, l'ADN obtenu a été directement inséré dans un plamide à l'aide du kit TA Cloning™ (British Biotechnology). Les 2 μl de solution d'ADN ont été mélangés avec 5 μl d'eau distillée stérile, 1 μl d'un tampon de ligation 10 fois concentré "10X LIGATION BUFFER", 2 μl de "pCR™ VECTOR" (25 ng/ml) et 1 μl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" **(17)**. La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "automatic sequencer, modèle 373 A Applied Biosystems selon les instructions du fabricant.

Cette approche technique a été appliquée à un échantillon de virion concentré comme décrit ci-après, à partir d'un mélange de surnageants de culture produits par des lignées lymphoblastoïdes B telles que décrites dans l'exemple 2, établies à partir des lymphocytes de patients atteints de SEP et présentant une activité transcriptase inverse détectable selon la technique décrite par Perron et coll. (3): les surnageants de cultures sont collectés deux fois par semaine, précentrifugés à 10 000 trs/min pendant 30 minutes pour éliminer les débris cellulaires, et ensuite, congelés à -80°C ou utilisés tels quels pour les étapes suivantes. Les surnageants frais ou décongelés sont centrifugés sur un coussin de PBS-glycérol 30% à 100 000g pendant 2 h à 4°C. Après élimination du surnageant, le culot sédimenté constitue l'échantillon de virions concentrés, mais non purifiés. Le culot ainsi obtenu est alors repris dans un petit volume d'un tampon adéquat pour l'extraction d'ARN. La réaction de synthèse de ADNc mentionnée plus haut est réalisée sur cet ARN extrait de virion extracellulaire concentré.

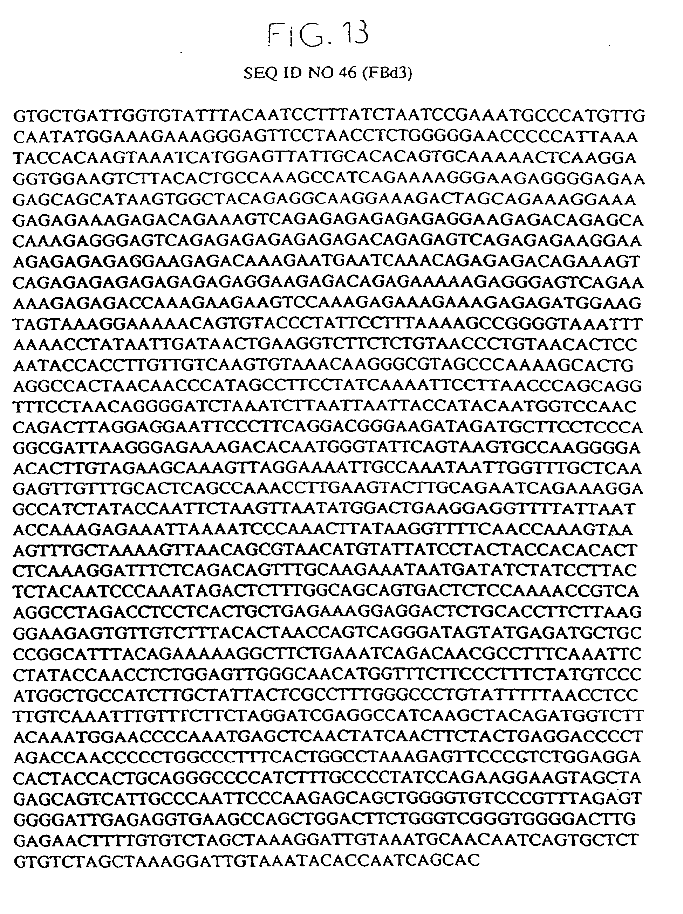

L’amplification RT-PCR selon la technique citée plus-haut a permis d’obtenir le clone FBd3 dont la séquence identifiée par SEQ ID NO 46, est présentée dans la figure 13.

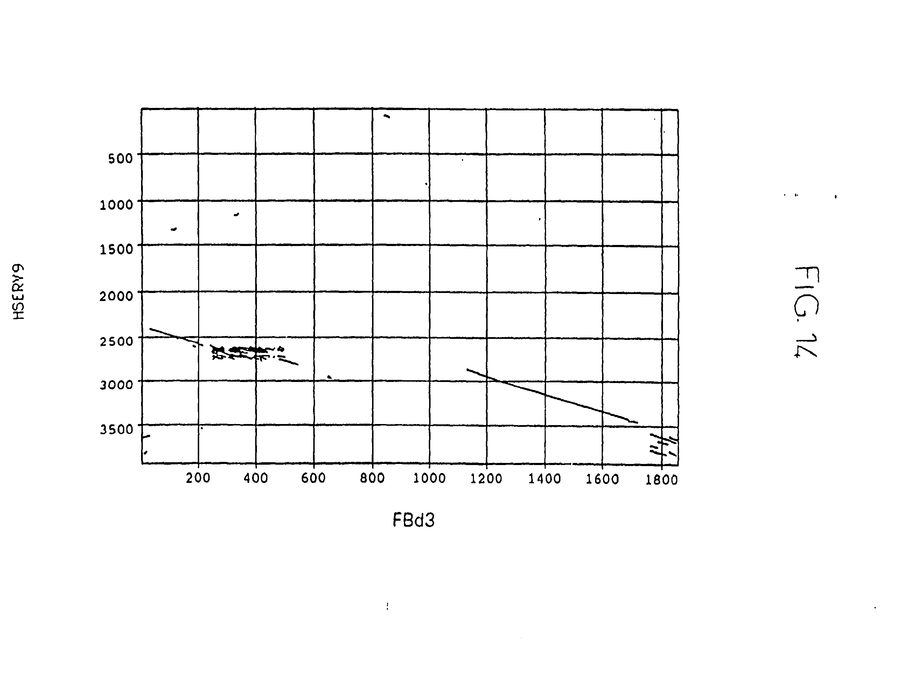

Dans la figure 14, l’homologie de séquence entre le clone FBd3 et le rétrovirus HSERV-9 est représentée sur le tableau matriciel par un trait plein, pour toute homologie partielle supérieure ou égale à 65 %. On peut constater que des homologies existent dans les régions flanquantes du clone (avec le gène pol en 5’ et avec le gène env puis le LTR en 3’), mais que la région interne est totalement divergente et ne présente aucune homologie, même faible, avec le gène "env" d’HSERV9. De plus, il apparaî*t que le clone FBd3 contient une région "env" plus longue que celle qui est décrite pour l’endogène défectif HSERV-9 ; on peut ainsi constater que la région divergente interne constitue un "insert" entre les régions d’homologie partielle avec les gènes défectifs HSERV-9.

**<u>EXEMPLE 8</u> : AMPLIFICATION, CLONAGE ET SEQUENCAGE DE LA REGION DU GENOME RETROVIRAL MSRV-1 SITUEE ENTRE LES CLONES PSJ17 ET FBd3.**

Quatre oligonucléotides, F1, B4, F6 et B1, ont été définis pour amplifier de l’ARN provenant de virions concentrés des souches POL2 et MS7PG. Des réactions de contrôle ont été effectuées de manière à contrôler la présence de contaminants (réaction sur de l’eau). L’amplification consiste en une première étape de RT-PCR selon le protocole décrit dans la demande de brevet EP-A-0 569 272, suivie d’une deuxième étape de PCR effectuée sur 10 μl de produit de la première étape avec des amorces internes à la première région amplifiée (PCR "nichée"). Dans le premier cycle RT-PCR, les amorces F1 et B4 sont utilisées. Dans le deuxième cycle PCR, les amorces F6 et l’amorce B1 sont utilisées. Les amorces sont positionnées comme suit :

| | | | | |
|---|---|---|---|---|
| F1 | F6 | B1 | B4 | |
| | | | | ARN |
| MSRV-1 | | | | |
| PSJ17 | | | FBd3 | |
| -----> | | <-----/--- | | |
| 5’pol MSRV-1 | | 3’ pol | MSRV-1 | / |
| 5’env. | | | | |

Leur composition est :

amorce F1 : TGATGTGAACGGCATACTCACTG (SEQ ID NO 47)
amorce B4 : CCCAGAGGTTAGGAACTCCCTTTC (SEQ ID NO 48)
amorce F6 : GCTAAAGGAGACTTGTGGTTGTCAG(SEQ ID NO 49)
amorce B1 : CAACATGGGCATTTCGGATTAG (SEQ ID NO 50)

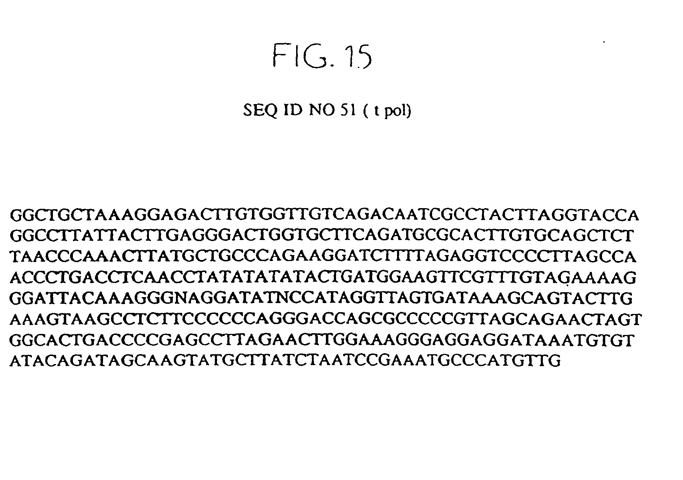

Le produit d’amplification "nichée" obtenu et dénommé "t pol" est présenté dans la figure 15, et correspond à la séquence SEQ ID NO 51.

**<u>EXEMPLE 9</u> : OBTENTION DE NOUVELLES SEQUENCES, EXPRIMEES EN ARN DANS LES CELLULES EN CULTURE PRODUISANT MSRV-1, ET COMPRENANT UNE REGION "env" DU GENOME RETROVIRAL MSRV-1.**

Une banque d’ADNc a été réalisée selon la procédure décrite par le fabricant des kits "cDNA synthesis module, cDNA rapid adaptator ligation module, cDNA rapid cloning module, lambda gt10 in vitro packaging module" (Amersham, ref RPN1256Y/Z, RPN1712, RPN1713, RPN1717, N334Z) à partir de l’ARN messager extrait de cellules d’une lignée lymphoblastoïde B telle que décrite dans l’exemple 2, établie à partir des lymphocytes d’un patient atteint de SEP et présentant une activité transcriptase inverse détectable selon la technique décrite par Perron et coll. (3).

Des oligonucléotides ont été définis pour amplifier de l’ADNc cloné dans la banque nucléique entre la région 3’ du clone PSJ17 (pol) et la région 5’(LTR) du clone FBd3.Des réactions de contrôle ont été effectuées de manière à contrôler la présence de contaminants (réaction sur de l’eau). Des réactions PCR effectuées sur les acides nucléiques clonés dans la banque avec différents couples d’amorces ont permis d’amplifier une série clones reliant des séquences pol aux séquences env ou LTR de type MSRV-1.

Deux clones sont représentatifs des séquences obtenues dans la banque de cDNA cellulaire :

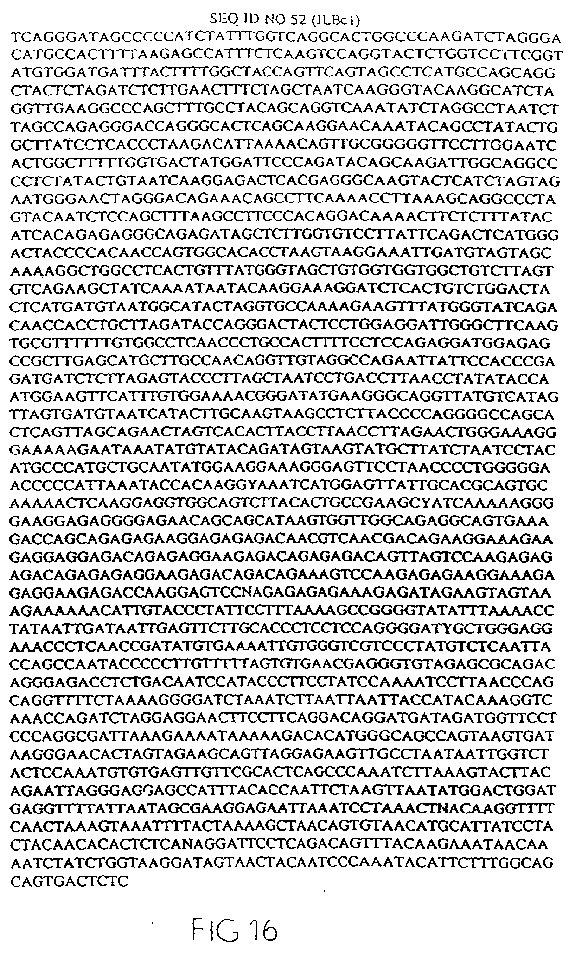

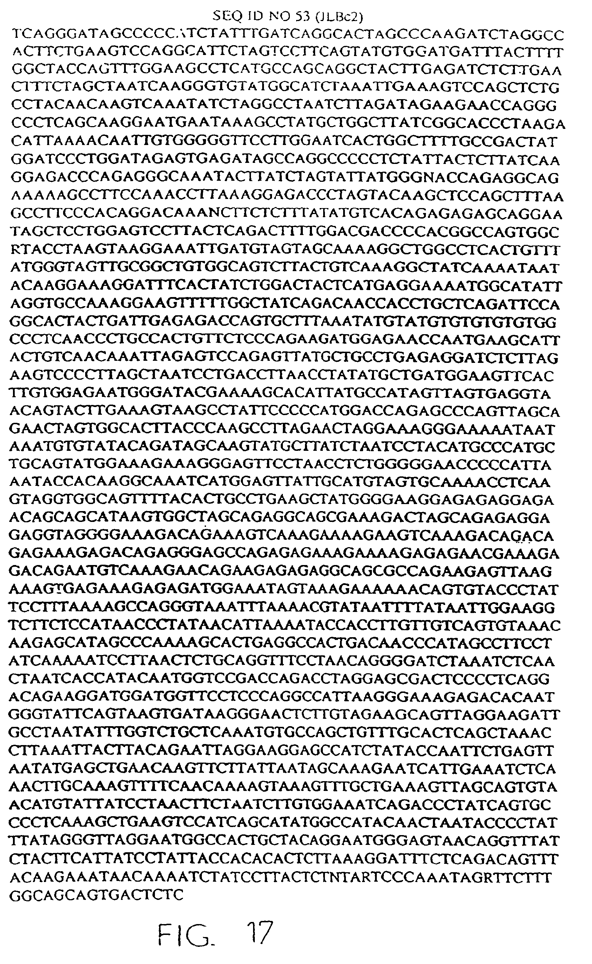

- le clone JLBc1, dont la séquence SEQ ID NO 52 est présentée dans la figure 16 ;
- le clone JLBc2, dont la séquence SEQ ID NO 53 est présentée dans la figure 17.

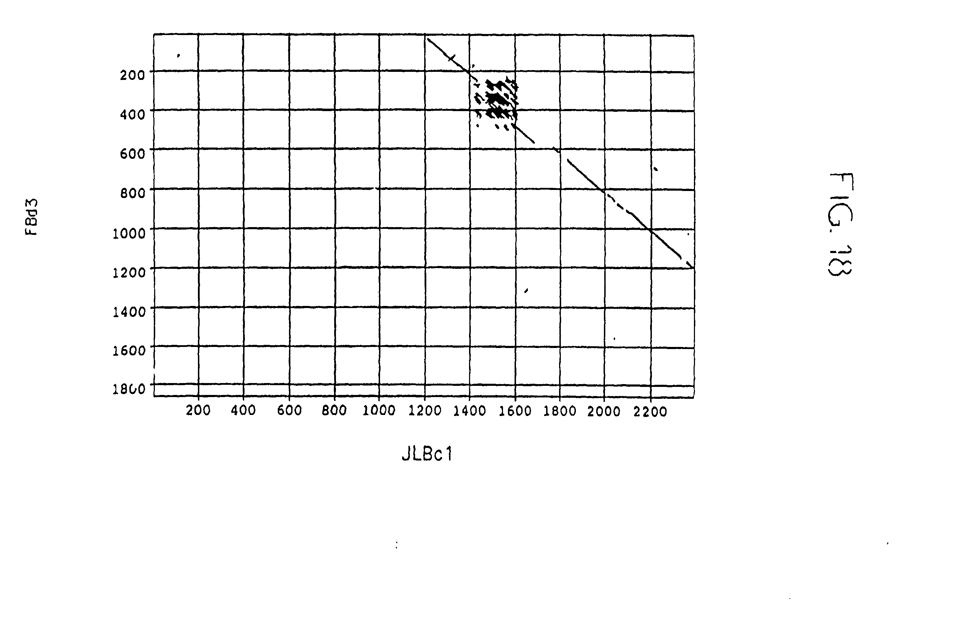

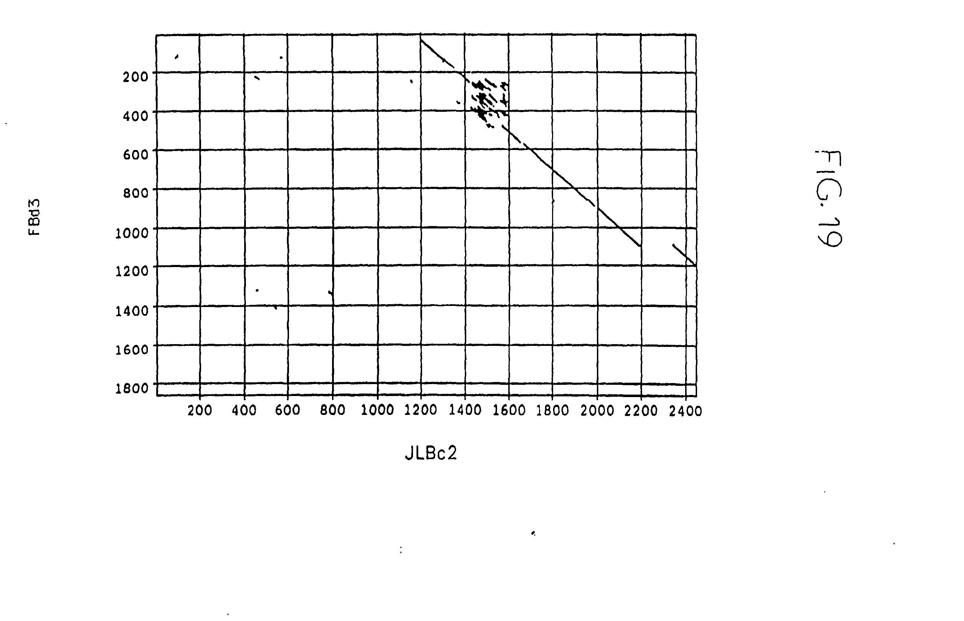

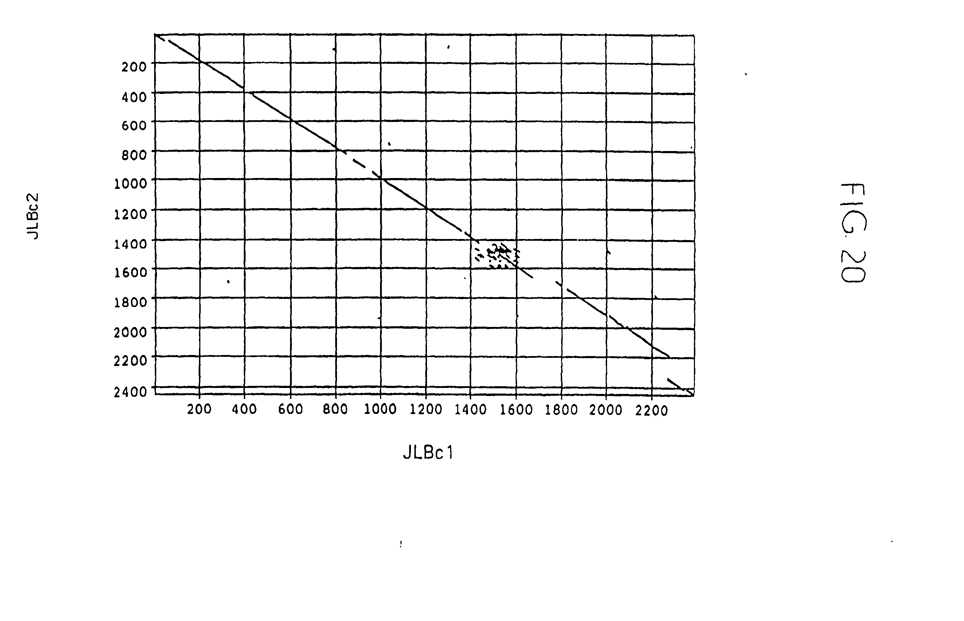

Les séquences des clones JLBc1 et JLBc2 sont homologues à celle du clone FBd3, ainsi que cela apparait dans les figures 18 et 19. L'homologie entre le clone JLBc1 et le clone JLBc2 est représentée dans la figure 20.

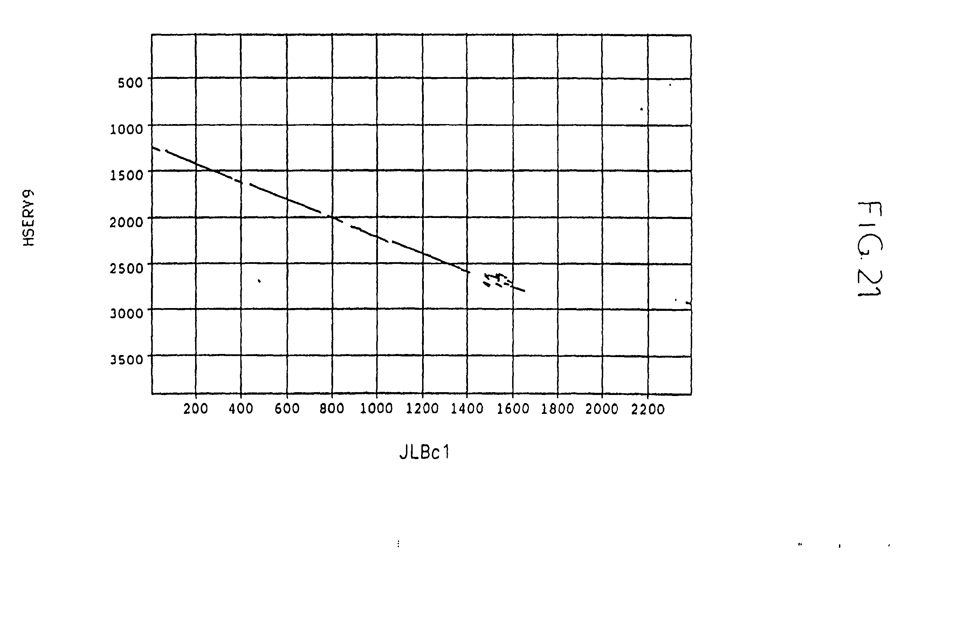

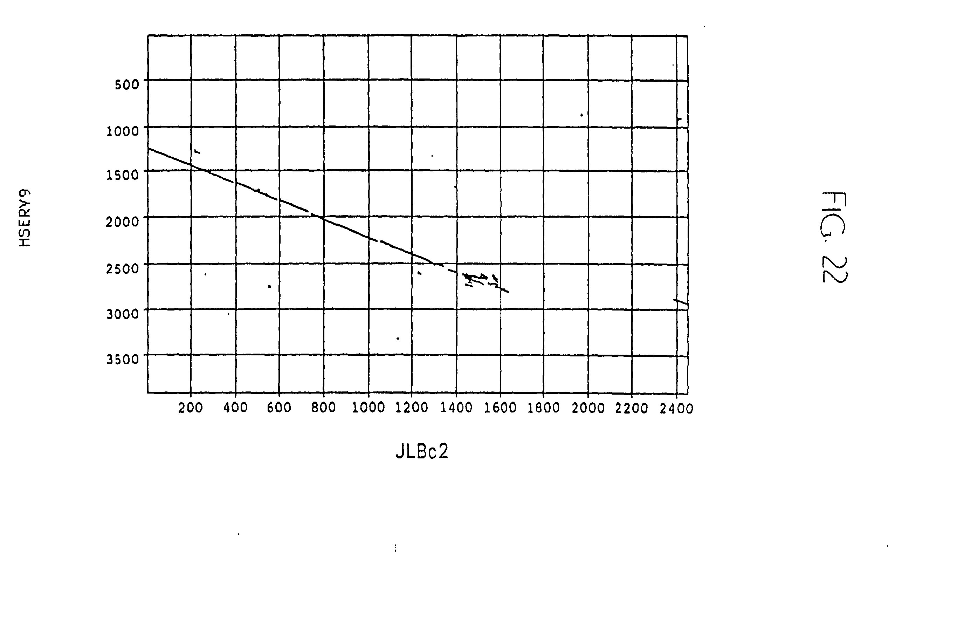

Les homologies entre les clones JLBc1 et JLBc2 d'une part, et la séquence HSERV9 d'autre part, sont présentées respectivement dans les figures 21 et 22.

On remarque que la région d'homologie entre JLB1, JLB2 et FBd3 comprend, avec quelques variations de séquence et de taille de "l'insert", la séquence supplémentaire absente ("insérée") dans la séquence env HSERV-9, telle que décrite dans l'exemple 8.

On remarque aussi que la région "pol" clonée est très homologue à HSERV-9, ne possède pas de cadre de lecture (en tenant compte des erreurs de séquences induites par les techniques utilisées, jusqu'au séquenceur automatique inclus) et diverge des séquences MSRV-1 obtenues à partir de virions. Etant donné que ces séquences ont été clonées à partir de l'ARN de cellules exprimant des particules MSRV-1, il est probable qu'elles proviennent d'éléments rétroviraux endogènes apparentés à la famille ERV9 ; ce, d'autant plus que les gènes pol et env sont présent sur un même ARN qui n'est à l'évidence pas l'ARN génomique MSRV-1. Certains de ces éléments ERV9 possèdent des LTR fonctionnels activables par des virus réplicatifs codant pour des transactivateurs homologues ou hétérologues. Dans ces conditions, la parenté entre MSRV-1 et HSERV-9 rend probable la transactivation des éléments ERV9 endogènes défectifs (ou non) par des protéines transactivatrices MSRV-1 homologues, voire identiques.

Un tel phénomène peut induire une interférence virale entre l'expression de MSRV-1 et les éléments endogènes apparentés. Une telle interférence conduit généralement à une expression dite "défective-interférente", dont certaines caractéristiques ont été retrouvées dans les cultures étudiées infectées par MSRV-1. De plus, un tel phénomène n'est pas sans générer l'expression de polypeptides, voire de protéines rétrovirales endogènes qui ne sont pas nécessairement tolérées par le système immunitaire. Un tel schéma d'expression aberrante d'éléments endogènes apparentés à MSRV-1 et induite par ce dernier est susceptible de multiplier les antigènes aberrants et, donc, de contribuer à l'induction de processus autoimmuns tels qu'on les observe dans la SEP.

Il est cependant essentiel de noter que les clones JLBc1 et JLBc2 diffèrent de la séquence ERV9 ou HSERV9 déjà décrite, en ce qu'ils possèdent une région env plus longue comprenant une région supplémentaire totalement divergente d'ERV9. On peut donc définir leur parenté avec la famille endogène ERV9, mais ils constituent à l'évidence des éléments originaux, jamais décrits à ce jour. En effet, l'interrogation des banques de données de séquences nucléiques disponibles dans la version n° 15 (1995) du logiciel "Entrez" (NCBI, NIH, Bethesda, USA) n'a pas permis d'identifier de séquence homologue connue dans la région env de ces clones.

**<u>EXEMPLE 10</u> : OBTENTION DES SEQUENCES SITUEES DANS LA REGION 5' pol et 3' gag DU GENOME RETROVIRAL MSRV-1.**

Ainsi que cela a déjà été décrit dans l'exemple 5, une technique PCR dérivée de la technique publiée par Frohman (19) a été utilisée. La technique dérivée permet, à l'aide d'une amorce spécifique en 3' du génome à amplifier, d'élonguer la séquence vers la région 5' du génome à analyser. Cette variante technique est décrite dans la documentation de la firme "Clontech Laboratories Inc., (Palo-Alto California, USA) fournie avec son produit "5'-AmpliFINDER™ RACE Kit", qui a été utilisé sur une fraction de virion purifié comme décrit précédemment.

Afin de réaliser une amplification de la région 5' du génome rétroviral MSRV-1 partant de la séquence pol déjà séquencée (clone F11-1) et s'étendant vers le gène gag, des amorces spécifiques MSRV-1 ont été définies.

Les amorces 3' spécifiques utilisées dans le protocole du kit pour la synthèse du ADNc et l'amplification PCR sont respectivement complémentaires aux séquences MSRV-1 suivantes :

ADNc : (SEQ ID NO 54)
CCTGAGTTCTTGCACTAACCC
amplification : (SEQ ID NO 55)
GTCCGTTGGGTTTCCTTACTCCT

Les produits issus de la PCR ont été extraits après purification sur gel d'agarose selon les méthodes conventionnelles (17), puis resuspendus dans 10 ml d'eau distillée. Une des propriétés de la polymérase Taq consistant à ajouter une adénine à l'extémité 3' de chacun des deux brins d'ADN, l'ADN obtenu a été directement inséré dans un plamide à l'aide du kit TA Cloning™ (British Biotechnology). Les 2 μl de solution d'ADN ont été mélangés avec 5 μl d'eau distillée stérile, 1 μl d'un tampon de ligation 10 fois concentré "10X LIGATION BUFFER", 2 μl de "pCR™ VECTOR" (25 ng/ml) et 1 μl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" (17). La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "automatic sequencer, modèle 373 A Applied Biosystems selon les instructions du fabricant.

Cette approche technique a été appliquée à un échantillon de virion concentré comme décrit ci-après, à partir d'un mélange de surnageants de culture produits par des lignées lymphoblastoïdes B telles que décrites dans l'exemple 2, établies à partir des lymphocytes de patients atteints de SEP et présentant une activité transcriptase inverse détectable selon la technique décrite par Perron et coll. (3) : les surnageants de cultures sont collectés deux fois par semaine, pré-centrifugés à 10 000 trs/min pendant 30 minutes pour éliminer les débris cellulaires, et ensuite, congelés à -80°C ou utilisés tels quels pour les étapes suivantes. Les surnageants frais ou décongelés sont centrifugés sur un coussin de PBS-glycérol 30 % à 100 000g pendant 2 h à 4°C. Après élimination du surnageant, le culot sédimenté constitue l'échantillon de virions concentrés, mais non purifiés. Le culot ainsi obtenu est alors repris dans un petit volume d'un tampon adéquat pour l'extraction d'ARN. La réaction de synthèse de ADNc mentionnée plus haut est réalisée sur cet ARN extrait de virion extracellulaire concentré.

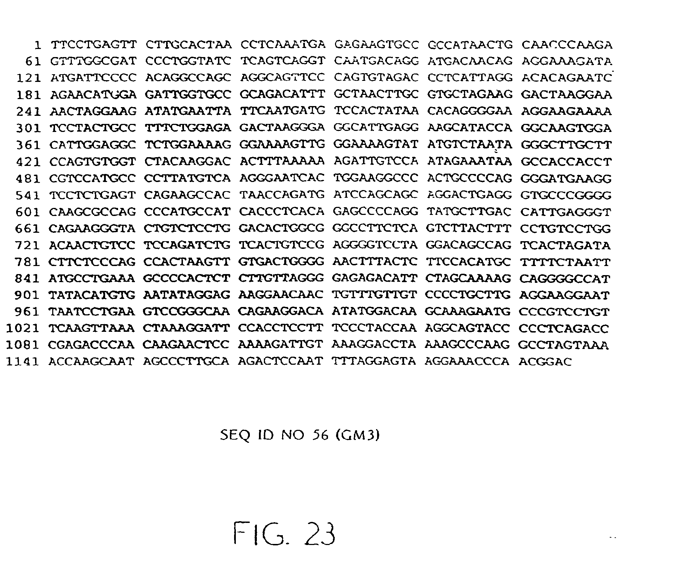

L'amplification RT-PCR selon la technique citée plus-haut a permis d'obtenir le clone GM3 dont la séquence identifiée par SEQ ID N0 56, est présentée dans la figure 23.

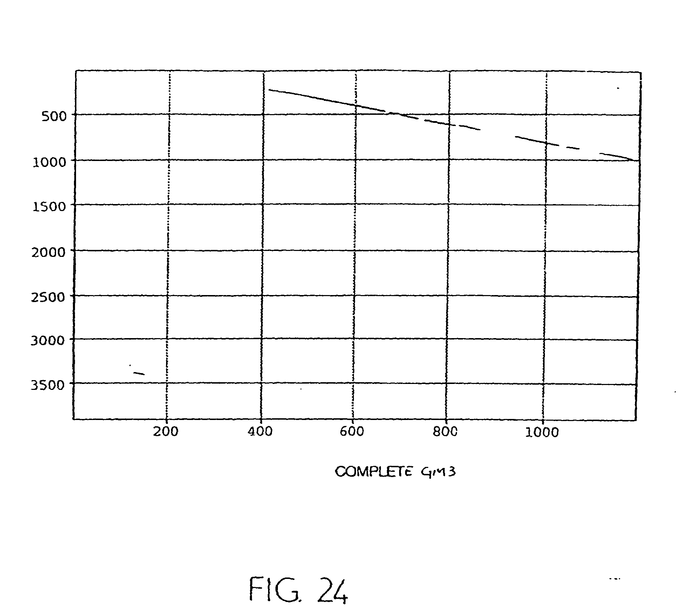

Dans la figure 24, l'homologie de séquence entre le clone GMP3 et le rétrovirus HSERV-9 est représentée sur le tableau matriciel par un trait plein, pour toute homologie partielle supérieure ou égale à 65 %.

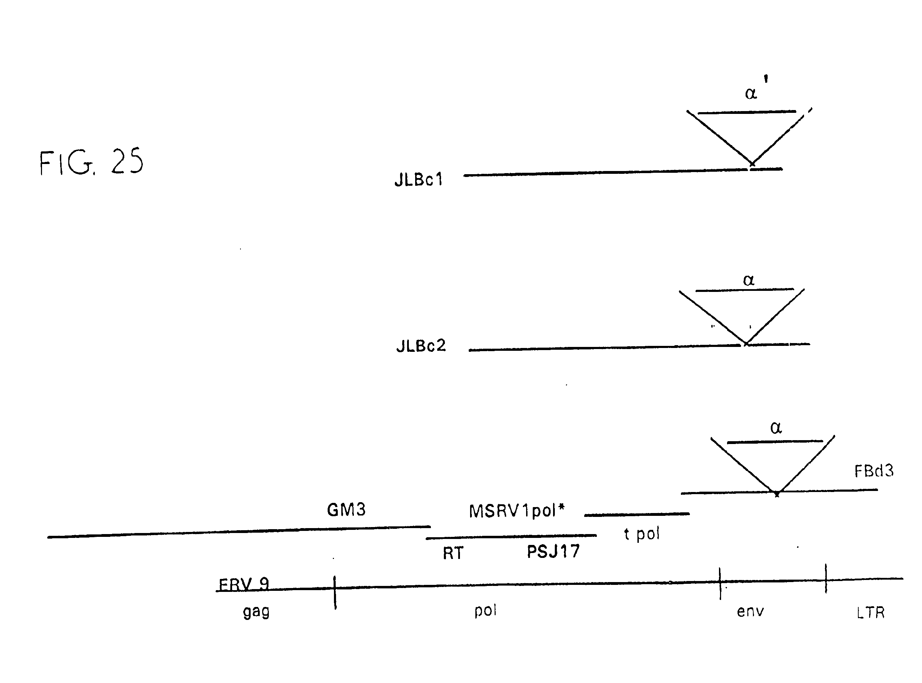

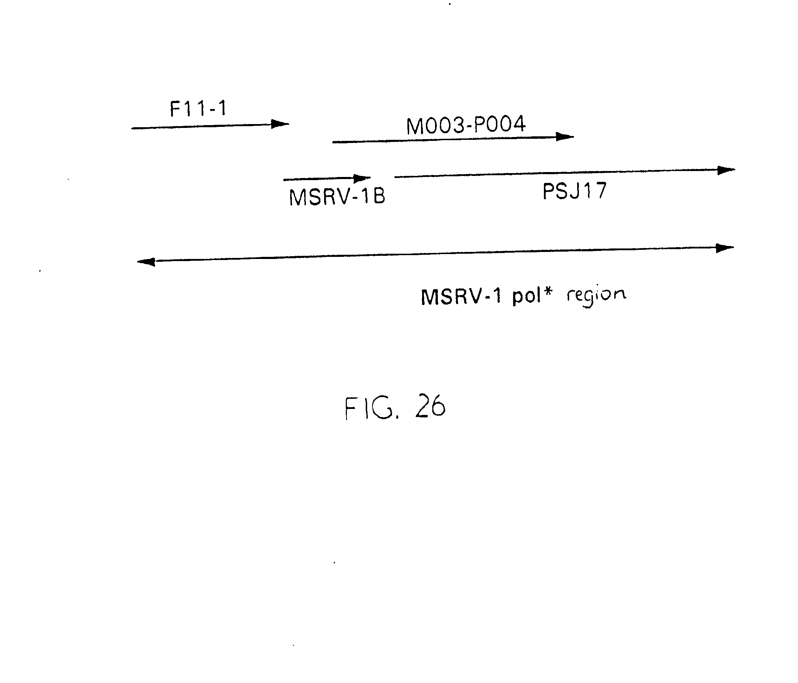

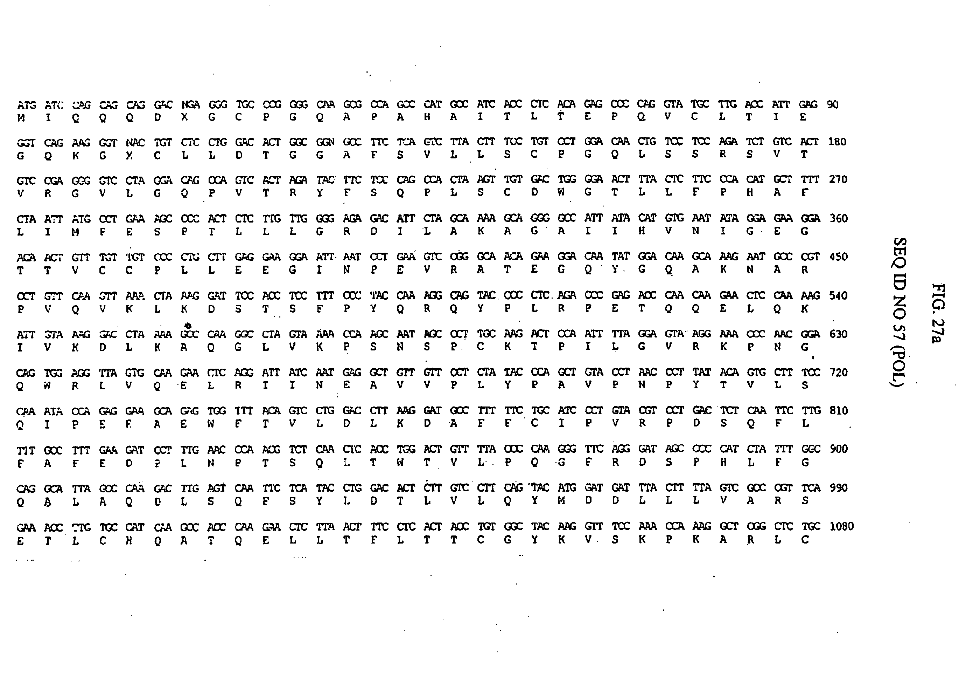

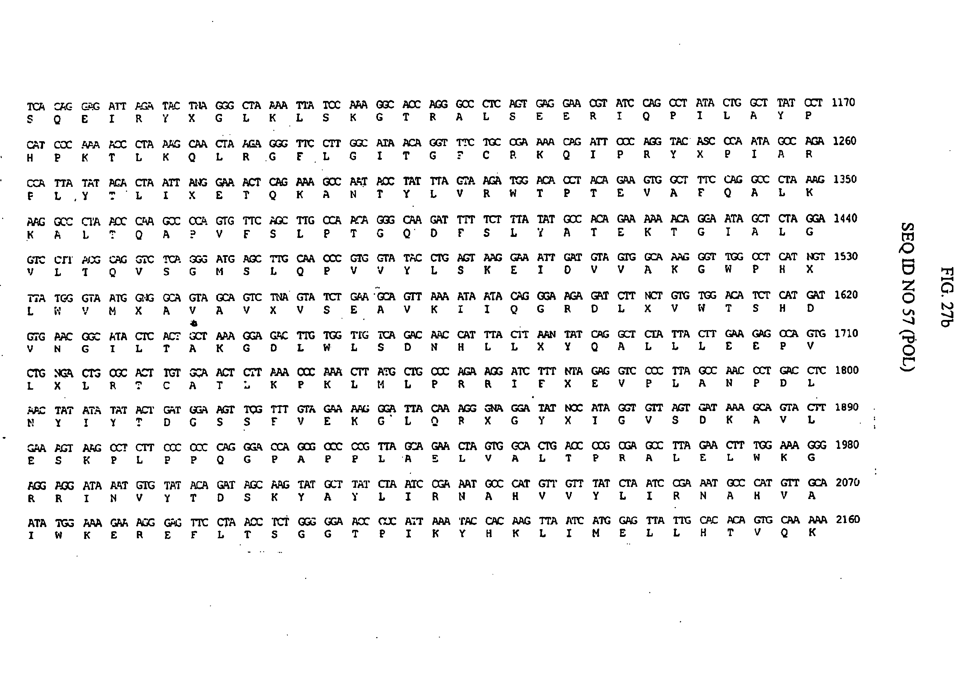

En synthèse, sur la figure 25 est représentée la localisation des différents clones précédemment étudiés, par rapport au génome ERV9 connu. Sur la figure 25, la région env MSRV-1 étant plus longue que le gène env de ERV9 de référence, la région supplémentaire est représentée au-dessus du point d'insertion selon un "V", étant entendu que le matériel inséré présente une variabilité de séquences et de taille entre les clones représentés (JLBcl, JLBc2, FBd3). Et sur la figure 26, est représentée la position de différents clones étudiés dans la région MSRV-1 pol *.

Grâce au clone GM3 précédemment décrit, on a pu définir un cadre de lecture possible, couvrant l'ensemble du gène pol, référencé selon SEQ ID NO 57, représenté aux figures successives 27a à 27c.

**EXEMPLE 11 : DETECTION D'ANTICORPS SPECIFIQUES ANTI-MSRV-1 DANS LE SERUM HUMAIN.**

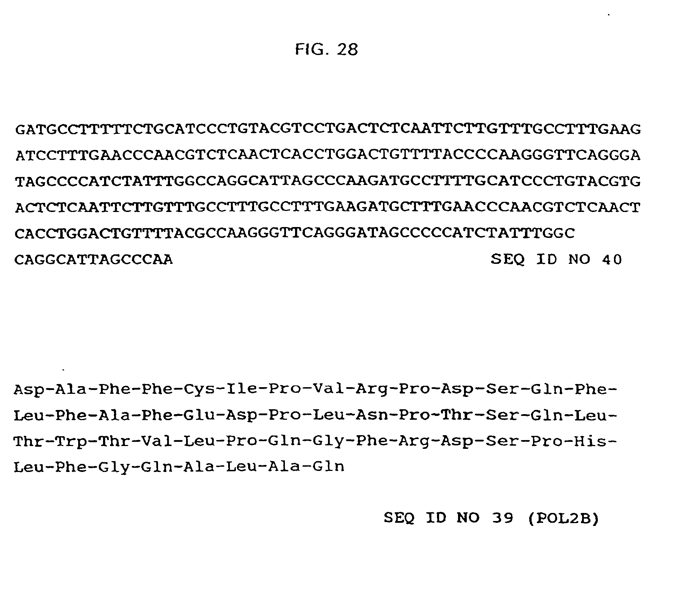

L'identification de la séquence du gène pol du rétrovirus MSRV-1 et d'un cadre de lecture ouverte de ce gène a permis de déterminer la séquence SEQ ID NO 39 en acides aminés d'une région dudit gène, référencée SEQ ID NO 40 (cf figure 28).

Différents peptides synthétiques correspondant à des fragments de la séquence protéique de la transcriptase inverse MSRV-1 codée par le gène pol, ont été testés pour leur spécificité antigénique vis à vis de sera de patients atteints de SEP et de témoins sains.

Les peptides ont été synthétisés chimiquement par synthèse en phase solide, selon la technique de Merrifield (Barany G, and Merrifielsd R.B, 1980, In the Peptides, 2, 1-284, Gross E and Meienhofer J, Eds Academic Press, New York). Les modalités pratiques sont celles décrites ci-après.

a) Synthèse des peptides:

Les peptides ont été synthétisés sur une résine phénylacétamidométhyle (PAM) /polystyrène/divinylbenzène (Applied Biosystems, Inc. Foster City, CA), en utilisant un synthétiseur automatique "Applied Biosystems 430A". Les acides aminés sont couplés sous forme d'esters d'hydroxybenzotriazole (HOBT). Les acides aminés utilisés proviennent de Novabiochem (Laüflerlfingen, Suisse) ou de Bachem (Bubendorf, Suisse).

La synthèse chimique a été effectuée en utilisant un protocole de double couplage avec la N-méthylpyrrolidone (NMP) comme solvant. Les peptides ont été coupés de la résine ainsi que les protections latérales, de manière simultanée, à l'aide d'acide fluorhydrique (HF) dans un appareil approprié (appareil de coupure de type I, Peptide Instiute, Osaka, Japon).

Pour 1g de peptidylrésine, 10ml de HF, 1ml d'anisole et 1ml de diméthylsulfure 5DMS sont utilisés. Le mélange est agité durant 45 minutes à -2°C. Le HF est alors évaporé sous vide. Après lavages intensifs à l'éther, le peptide est élué de la résine par de l'acide acétique 10%, puis lyophilisé.

Les peptides sont purifiés par chromatographie liquide haute performance préparative sur une colonne VYDAC de type C18 (250 x 21 mm) (The Séparation Group, Hesperia, CA, USA). L'élution est réalisée par un gradient d'acétonitrile à un débit de 22 ml/min. Les fractions collectées sont contrôlées par une élution en condition isocratique sur une colonne VYDAC®. C18 analytique (250 x 4,6 mm), à un débit de 1ml/min. Les fractions qui présentent le même temps de rétention sont réunies et lyophilisées. La fraction majoritaire est ensuite analysée par chromatographie liquide haute performance analytique, avec le système décrit précédemment. Le peptide qui est considéré comme de pureté acceptable se traduit par un pic unique représentant 95 % minimum du chromatogramme.

Les peptides purifiés sont ensuite analysés dans le but de contrôler leur composition en acides aminés, à l'aide d'un analyseur d'acides aminés automatique Applied Biosystems 420H. La mesure de la masse moléculaire chimique (moyenne) des peptides est obtenue en utilisant la spectrométrie de masse L.S.I.M.S. en mode d'ion positif sur un instrument à double focalisation VG. ZAB.ZSEQ relié à un système d'acquisition DEC-VAX 2000 (VG analytical Ltd, Manchester, Angleterre).

La réactivité des différents peptides a été testée contre des sera de patients atteints de SEP et contre des sera de témoins sains. Ceci a permis de sélectionner un peptide dénommé POL2B dont la séquence est représentée à la figure 28 dans l'identificateur SEQ ID NO 39 ci-dessous, codé par le gène *pol* de MSRV-1 (nucléotides 181 à 330).

b) Propriétés antigéniques :

Les propriétés antigéniques du peptide POL2B ont été mises en évidence selon le protocole ELISA décrit ci-dessous.

Le peptide POL2B lyophilisé a été dissous dans de l'eau distillée stérile à une concentration de 1mg/ml. Cette solution-mère a été aliquotée et gardée à +4°C pour usage sous quinzaine, ou congelée à -20°C, pour un usage dans les 2 mois. Un aliquot est dilué dans une solution de PBS (Phosphate Buffer Saline) afin d'obtenir une concentration finale de peptide de 1 microgramme/ml. 100 microlitres de cette dilution sont déposés dans chaque puits de plaques de microtitration (plastique "high-binding", COSTAR ref: 3590). Les plaques sont recouvertes d'un adhésif de type "plate-sealer" et maintenues une nuit à +4°C, pour la phase d'adsorption du peptide sur le plastique. L'adhésif est enlevé et les plaques sont lavées trois fois avec un volume de 300 microlitres d'une solution A (PBS 1x, 0,05% Tween 20®), puis retournées sur un tissu absorbant. Les plaques ainsi égouttées sont remplies avec 200 microlitres par puits d'une solution B (solution A + 10 % de sérum de chèvre), puis recouvertes d'un adhésif et incubées de 45 minutes à 1 heure, à 37°C. Les plaques sont ensuite lavées trois fois avec la solution A, comme décrit précédemment.

Les échantillons de sérum à tester sont préalablement dilués au 1/50ème dans la solution B et 100 microlitres de chaque sérum dilué à tester sont déposés dans les puits de chaque plaque de micrititration. Un contrôle négatif est déposé dans un puits de chaque plaque, sous la forme de 100 microlitres de tampon B. Les plaques recouvertes d'un adhésif sont alors incubées 1 à 3 heures, à 37°C. Les plaques sont ensuite lavées trois fois avec la solution A, comme décrit précédemment. Parallèlement, un anticorps de chèvre marqué à la peroxydase et dirigé contre les IgG (Sigma Immunochemicals ref. A6029) ou les IgM (Cappel ref.: 55228) humaines est dilué dans la solution B (dilution 1/5000 pour l'anti-IgG et, 1/1000 pour l'anti-IgM). 100 microlitres de la dilution adéquate de l'anticorps marqué sont alors déposés dans chaque puits des plaques de microtitration et les plaques recouvertes d'un adhésif sont incubées 1 à 2 heures, à 37°C. Un nouveau lavage des plaques est ensuite effectué comme décrit précédemment. Parallèlement, le substrat de la peroxydase est préparé selon les indications du kit "Sigma fast OPD kit" (Sigma Immunochemichals, ref. P9187). 100 microlitres de solution-substrat sont déposés dans chaque puits et les plaques sont placées à l'abri de la lumière pendant 20 à 30 minutes, à température ambiante.

Une fois la réaction colorée stabilisée, les plaques sont immédiatement placées dans un lecteur spectrophotométrique de plaques ELISA, et la densité optique (D.O.) de chaque puits est lue à une longueur d'onde de 492 nm. Alternativement, 30 microlitres d'HCL IN sont déposés dans chaque puits pour stopper la réaction et les plaques sont lues au spectrophotomètre dans les 24 heures.

Les échantillons sérologiques sont déposés en double ou en triple et la densité optique (D.O.) correspondant au sérum testé est calculée en faisant la moyenne des D.O. obtenues pour un même échantillon, à la même dilution.

La D.O. nette de chaque sérum correspond à la D.O. moyenne du sérum de laquelle est soustraite la D.O. moyenne du témoin négatif (solution B : PBS, Tween 20® 0,05%, 10% sérum de chèvre).

c) Détection d'anticorps IgG anti-MSRV-1 par ELISA :

La technique décrite ci-dessus a été utilisée avec le peptide POLB2 pour rechercher la présence d'anticorps IgG spécifiques anti-MSRV-1 dans le sérum de 29 patients, pour lesquels un diagnostic certain ou probable de SEP a été posé selon les critères de Poser (23), et de 32 témoins sains (donneurs de sang).

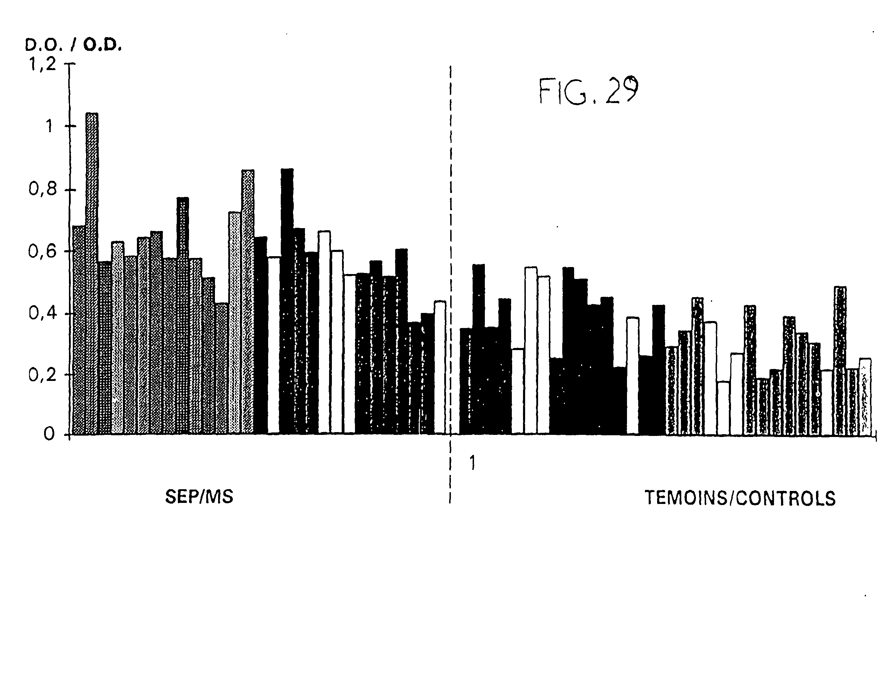

Dans la figure 29, les résultats de chaque sérum testé avec un anticorps anti-IgG, sont représentés. Chaque barre verticale représente la densité optique (D.O à 492 nm) nette d'un sérum testé. L'axe des ordonnées indique la D.O. nette au sommet des barres verticales. Les 29 premières barres verticales situées à gauche de la ligne pointillée verticale, représentent les sera de 29 cas de SEP testés et les 32 barres verticales situées à droite de la ligne pointillée verticale, représentent les sera de 32 témoins sains (donneurs de sang).

La moyenne des D.O nettes des SEP testées est de : 0,62. Le graphique permet de visualiser 5 témoins dont la D.O. nette émerge au dessus des valeurs groupées de la population témoin. Ces valeurs peuvent représenter la présence d'IgG spécifiques chez des patients séropositifs non-malades. Deux méthodes ont donc été évaluéespour déterminer le seuil statistique de positivité du test.

La moyenne des D.O. nettes des témoins, y compris les témoins avec des D.O. nettes élevées, est de 0,36. Sans les 5 témoins dont les D.O. nettes sont supérieures ou égales à 0,5, la moyenne des témoins "négatifs" est de 0,33. L'écart type des témoins négatifs est de 0,10. Un seuil de positivité théorique peut être calculé selon la formule :

valeur seuil (moyenne des D.O. nettes des témoins séronégatifs) + (2 ou 3 x écart-type des D.O. nettes des témoins séronégatifs).

Dans le premier cas, on considère qu'il existe des séropositifs non-malades et la valeur seuil est égale à : 0,33 + (2 x 0,10) = 0,53. Les résultats négatifs représentent un "bruit de fond" non spécifique de la présence d'anticorps spécifiquement dirigés contre un épitope du peptide.

Dans le deuxième cas, si l'ensemble des témoins constitués de donneurs de sang en bonne santé apparente est pris comme base de référence, sans exclure les sérums a priori séropositifs, l'écart-type des "témoins non-SEP" est de 0,116. La valeur seuil devient alors : 0,36 + (2 x 0,116) = 0,59.

Selon cette analyse, le test est spécique de la SEP. A cet égard, on constate que le test est spécifique de la SEP, puisque comme montré dans le tableau 1, aucun témoin n'a une D.O. nette au-dessus de ce seuil. En fait ce résultat reflète le fait que les titres d'anticorps chez les patients atteints de SEP sont en majorité plus élevés que chez des témoins sains ayant été en contact avec MSRV-1.

**TABLEAU N°1**

| | SEP | TEMOINS |
|---|---|---|
| | 0,681 | 0,3515 |
| | 1,0425 | 0,56 |
| | 0,5675 | 0,3565 |
| | 0,63 | 0,449 |
| | 0,588 | 0,2825 |
| | 0,645 | 0.55 |
| | 0,6635 | 0.52 |
| | 0,576 | 0,2535 |
| | 0,7765 | 0.55 |
| | 0,5745 | 0.51 |
| | 0,513 | 0,426 |
| | 0,4325 | 0,451 |
| | 0.7255 | 0.227 |
| | 0,859 | 0,3905 |
| | 0,6435 | 0,265 |
| | 0,5795 | 0,4295 |
| | 0,8655 | 0,291 |
| | 0,671 | 0,347 |
| | 0,596 | 0.4495 |
| | 0,652 | 0,3725 |
| | 0,602 | 0,181 |

(suite)

| | SEP | TEMOINS |
|---|---|---|
| | 0,525 | 0,2725 |
| | 0,53 | 0,426 |
| | 0,565 | 0,1915 |
| | 0,517 | 0,222 |
| | 0,607 | 0.395 |
| | 0,3705 | 0,34 |
| | 0,397 | 0,307 |
| | 0,4395 | 0,219 |
| | | 0,491 0.491 |
| | | 0,2265 |
| | | 0,2605 |
| | | |
| MOYENNE | 0.62 | 0,33 |
| FCART TYPE | 0.14 | 0,10 |
| VALEUR SEUIL | | 0,53 |

En fonction du premier mode de calcul et comme représenté à la figure 29 et dans le tableau correspondant 1, 26 des 29 sérums SEP donnent un résultat positif (D.O. nette supérieure ou égale à 0,50) indiquant la présence d'IgG spécifiquement dirigées contre le peptide POL2B, donc contre une partie de l'enzyme transcriptase inverse du rétrovirus MSRV-1 codée par son gène pol et, par conséquent, contre le rétrovirus MSRV-1. Ainsi, environ 90 % des patients SEP testés ont réagi contre un épitope porté par le peptide POL2B et présentent des IgG circulantes dirigées contre ce dernier.

Cinq donneurs de sang, en apparente bonne santé, sur 32 présentent un résultat positif. Ainsi, il apparaît qu'environ 15 % de la population non-malade peut avoir été en contact d'un épitope porté par le peptide POL2B dans des conditions ayant conduit à une immunisation active qui se traduit par la persistance d'IgG sériques spécifiques. Ces conditions sont compatibles avec une immunisation contre la transcriptase inverse du rétrovirus MSRV-1, lors d'une infection par le (et/ou réactivation du) rétrovirus MSRV-1. L'absence de pathologie neurologique apparente évoquant la SEP chez ces témoins séropositifs peut indiquer qu'ils sont porteurs sains, ont éliminé un virus infectieux après s'être immunisés ou qu'ils constituent une population à risque de porteurs chroniques. En effet, les données épidémiologiques montrant qu'un agent pathogène présent dans l'environnement des régions à haute prévalence de SEP, peut être la cause de cette maladie, impliquent qu'une fraction de la population exempte de SEP a nécessairement été en contact avec un tel agent pathogène. On a montré que le rétrovirus MSRV-1 constitue tout ou partie de cet "agent pathogène" à l'origine de la SEP et il est donc normal que des témoins pris dans une population saine présentent des anticorps de type IgG contre des composants du rétrovirus MSRV-1. Ainsi, la différence de séroprévalence entre les SEP et la population témoin est extrêmement significative : test "chi-2", $p<0,001$. Ces résultats sont donc en faveur d'un rôle étiopathogénique de MSRV-1 dans la SEP.

d) Détection d'anticorps IgM anti-MSRV-1 par ELISA :

La technique ELISA avec le peptide POL2B a été utilisée pour rechercher la présence d'anticorps spécifiques IgM anti-MSRV-1 dans le sérum de 36 patients, pour lesquels un diagnostic certain ou probable de SEP a été posé selon les critères de Poser (23), et de 42 témoins sains (donneurs de sang).

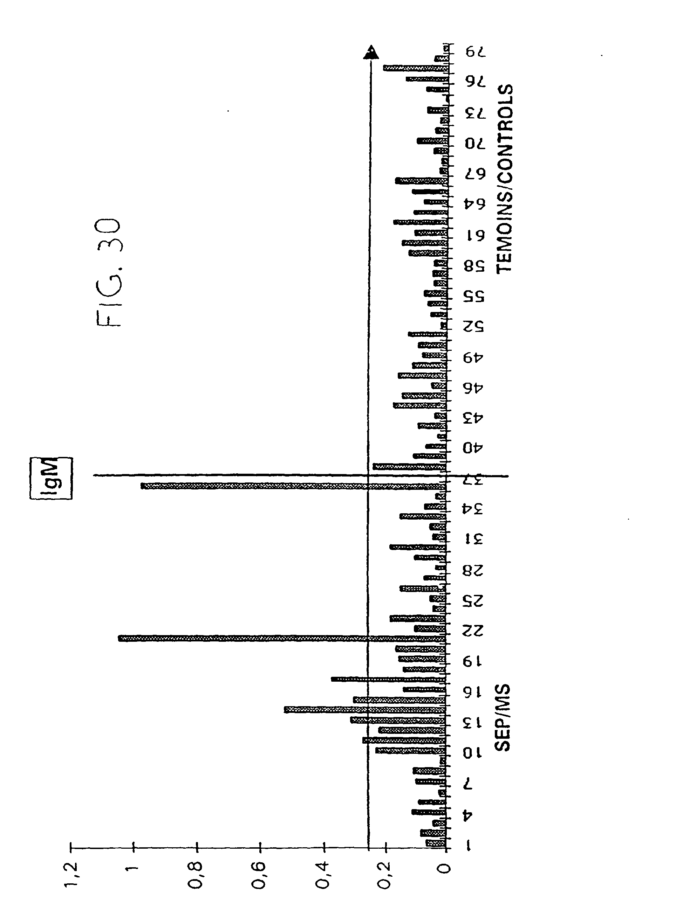

Dans la figure 30, les résultats de chaque sérum testé avec un anticorps anti-IgM, sont représentés. Chaque barre verticale représente la densité optique (D.O à 492 nm) nette d'un sérum testé. L'axe des ordonnées indique la D.O. nette au sommet des barres verticales. Les 36 premières barres verticales situées à gauche de la ligne verticale coupant l'axe des abscisses représentent les sérums de 36 cas de SEP testés et les barres verticales situées à droite de la ligne pointillée verticale, représentent les sérums de 42 témoins sains (donneurs de sang). La ligne horizontale tracée au milieu du graphique représente un seuil théorique délimitant les résultats positifs (dont le sommet de la barre est situé au dessus) et les résultats négatifs (dont le sommet de la barre est situé au dessous).

La moyenne des D.O. nettes des SEP testées est de : 0,19.

La moyenne des D.O. nettes des témoins est de : 0,09.

L'écart type des témoins négatifs est de : 0,05.

Etant donné la faible différence entre la moyenne et l'écart-type des témoins, le seuil de positivité théorique peut être calculé selon la formule :

valeur seuil = (moyenne des D.O. nettes des témoins séronégatifs) + (3 x écart-type des D.O. nettes des témoins séronégatifs).

La valeur seuil est donc égale à 0,09+ (3 x 0,05) = 0,26 ; soit, en pratique, 0,25.

Les résultats négatifs représentent un "bruit de fond" non spécifique de la présence d'anticorps spécifiquement dirigés contre un épitope du peptide.

Selon cette analyse et comme montré à la figure 30 et dans le tableau 2 correspondant, le test IgM est spécique de la SEP, puisqu'aucun témoin n'a une D.O. nette au-dessus du seuil. 7 des 36 sérums SEP produisent un résultat IgM positif ; or l'étude des données cliniques révèle que ces sérums positifs ont été prélevés lors d'une première poussée de SEP ou d'une poussée aiguë chez des malades non-traités. Il est connu que les IgM dirigées contre des agents pathogènes sont produites lors des primo-infections ou lors de réactivations suivant une phase de latence dudit agent pathogène.

La différence de séroprévalence entre les SEP et la population témoin est extrêmement significative : test "chi-2", p<0,001.

Ces résultats sont en faveur d'un rôle étiopathogénique de MSRV-1 dans la SEP.

La détection des anticorps IgM et IgG contre le peptide POL2B permet d'évaluer l'évolution d'une infection par MSRV-1 et/ou de la réactivation virale de MSRV-1.

**TABLEAU N°2**

| | SEP | TEMOINS |
|---|---|---|
| | 0,064 | 0,243 |
| | 0.087 | 0.11 |
| | 0,044 | 0,098 |
| | 0,115 | 0,028 |
| | 0,089 | 0,094 |
| | 0,025 | 0,038 |
| | 0,097 | 0.176 |
| | 0,108 | 0,146 |
| | 0,018 | 0,049 |
| | 0.234 | 0.161 |
| | 0,274 | 0,113 |
| | 0,225 | 0,079 |
| | 0,314 | 0,093 |
| | 0,522 | 0,127 |
| | 0,306 | 0,02 |
| | 0.143 | 0.052 |
| | 0.375 | 0.062 |
| | 0.142 | 0.074 |
| | 0,157 | 0,043 |

(suite)

| | SEP | TEMOINS |
|---|---|---|
| | 0,168 | 0,046 |
| | 1,051 | 0,041 |
| | 0,104 | 0,13 |
| | 0,187 | 0,153 |
| | 0,044 | 0,107 |
| | 0,053 | 0,178 |
| | 0.153 | 0,114 |
| | 0.07 | 0,078 |
| | 0,033 | 0,118 |
| | 0.104 | 0,177 |
| | 0.187 | 0.026 |
| | 0,044 | 0,024 |
| | 0,053 | 0,046 |
| | 0,153 | 0,116 |
| | 0,07 | 0,04 |
| | 0,033 | 0,028 |
| | 0.973 | 0,073 |
| | | 0,008 |
| | | 0,074 |
| | | 0,141 |
| | | 0,219 |
| | | 0,047 |
| | | 0,017 |
| | | |
| MOYENNE | 0,19 | 0,09 |
| ECARTTYPE | 0,23 | 0,05 |
| VALEUR SEUIL | | 0,26 |

e) Recherche d'épitopes immunodominants dans le peptide POL2B :

Afin de réduire le bruit de fond non-spécifique et d'optimiser la détection des réponses des anticorps anti-MSRV-1, la synthèse d'octapeptides, successivement décalés d'un aminoacide, couvrant l'ensemble de la séquence déterminée par POL2B, a été réalisée selon le protocole décrit ci-dessous.

La synthèse chimiques d'octapeptides chevauchants couvrant la séquence en acides aminés 61-110 représentée dans l'identificateur SEQ ID NO 39 a été réalisée sur membrane de cellulose activée selon la technique de BERG et al. (1989. J. Ann. Chem. Soc., 111, 8024-8026) commercialisée par Cambridge Research Biochemicals sous la dénomination commerciale Spotscan. Cette technique permet la synthèse simultanée d'un grand nombre de peptides et leur analyse.

La synthèse est réalisée avec des acides aminés estérifiés dont le groupement $\alpha$-aminé est protégé par un groupement FMOC (Nova Biochem) et les groupements latéraux par des groupements protecteurs tels que trityl, t-butyl ester ou t-butyl éther. Les acides aminés estérifiés sont solubilisés dans du N-methyl pyrrolidone (NMP) à la concentration de 300 nM, et 0, 9 $\mu$l sont déposés au niveau de taches de dépôt de bleu de bromophénol. Après incubation de 15 minutes, un nouveau dépôt d'acides aminés est réalisé suivi d'une autre incubation de 15 minutes. Si le couplage entre deux acides aminés s'est effectué correctement on observe une modification de coloration (passage du bleu au vert-jaune). Après trois lavages dans du DMF, une étape d'acétylation est effectuée par de l'anhydride acétique. Ensuite, les groupements aminés terminaux des peptides en cours de synthèse sont déprotégés par de la pipéridine 20 % dans le DMF. Les taches de dépôt sont recolorées par une solution de bleu de bromophénol à 1 % dans le DMF, lavées trois fois au méthanol et séchées. L'ensemble de ces opérations constitue un cycle d'addition d'un acide aminé et ce cycle est répété jusqu'à l'achèvement de la synthèse. Lorsque tous les acides aminés ont été ajoutés, le groupement $NH_2$-terminal du dernier acide aminé est déprotégé par de la pipéridine à 20 % dans le DMF et acétylé par de l'anhydride acétique. Les groupements protecteurs de la chaîne latérale sont enlevés par un mélange de dichlorométhane/acide trifluoroacétique/triisobutylsilane (5ml/5ml/250µl). L'immunoréactivité des peptides est ensuite testée par ELISA.

Après synthèse en double des différents octapeptides sur deux membranes différentes, ces dernières sont rincées avec du méthanol et lavées dans du TBS (Tris 0,1M pH 7,2), puis incubées une nuit à température ambiante dans un tampon de saturation. Après plusieurs lavages dans du TBS-T (Tris 0,1M pH 7,2 - 0,05% Tween 20), une membrane est incubée avec une dilution au 1/50 d'un sérum de référence provenant d'un patient atteint de SEP et l'autre membrane avec une dilution au 1/50 d'un pool de sérums de témoins sains. Les membranes sont incubées 4 heures à température ambiante. Après lavages avec du TBS-T, un conjugué anti-immunoglobulines humaines marqué à la β-galactosidase (commercialisé par Cambridge Research Biomedicals) est ajouté à une dilution au 1/200 et l'ensemble est incubé deux heures à température ambiante. Après lavages des membranes par du TBS-T 0,05% et du PBS, l'immunoréactivité au niveau des différentes taches est révélée par addition de 5-bromo-4-chloro-3-indoyl-β-D-galactopyranoside dans du potassium. L'intensité de coloration des taches est estimée qualitativement avec une valeur relative de 0 à 5 comme représenté aux figures 31 à 33 annexées.

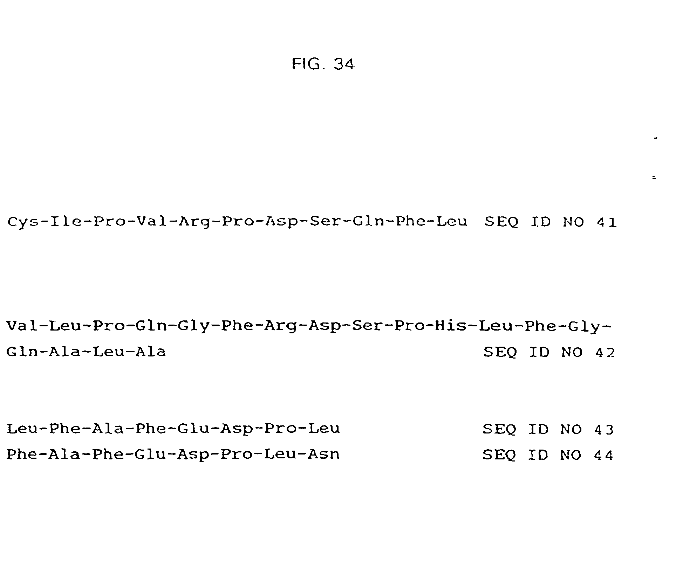

On peut ainsi déterminer deux régions immunodominantes à chaque extrémité du peptide POL2B correspondant respectivement aux séquences en acides aminés 65-75 (SEQ ID NO 41) et 92-109 (SEQ ID NO 42), selon figure 34, et comprises respectivement entre les octapeptides Phe-Cys-Ile-Pro-Val-Arg-Pro-Asp (FCIPVRPD) et Arg-Pro-Asp-Ser-Gln-Phe-Leu-Phe (RPDSQFLF), et, Thr-Val-Leu-Pro-Gln-Gly-Phe-Arg (TVLPQGFR) et Leu-Phe-Gly-Gln-Ala-Leu-Ala-Gln (LFGQALAQ) et une région moins réactive, mais apparemment plus spécifique, puisqu'elle ne produit aucun bruit de fond avec le sérum témoin, représentée par les octapeptides Leu-Phe-Ala-Phe-Glu-Asp-Pro-Leu (LFAFEDPL) (SEQ ID NO 43) et Phe-Ala-Phe-Glu-Asp-Pro-Leu-Asn (FAFEDPLN) (SEQ ID NO 44).

Ces régions permettent de définir de nouveaux peptides plus spécifiques et plus immunoréactifs selon les techniques habituelles.

Il est ainsi possible, grâce aux découvertes effectuées et aux méthodes mises au point par les inventeurs, de réaliser un diagnostic de l'infection et/ou de la réactivation MSRV-1, et d'évaluer une thérapie dans la SEP sur la base de son efficacité à "negativer" la détection de ces agents dans les fluides biologiques des patients. De plus, la détection précoce chez des personnes ne présentant pas encore de signes neurologiques de SEP, pourrait permettre d'instaurer un traitement d'autant plus efficace sur l'évolution clinique ultérieure qu'il précèderait le stade lésionnel qui correspond à l'apparition des troubles neurologiques. Or, à ce jour, un diagnostic de SEP ne peut être établi avant l'installation d'une symptomatologie neurologique lésionnelle et, donc, aucun traitement n'est instauré avant l'émergence d'une clinique évocatrice de lésions du système nerveux central déjà conséquentes. Le diagnostic d'une infection et/ou réactivation de MSRV-1 et/ou MSRV-2 chez l'homme est donc déterminant et la présente invention en fournit les moyens.

Il est ainsi possible, outre de réaliser un diagnostic de l'infection et/ou de la réactivation MSRV-1, d'évaluer une thérapie dans la SEP sur la base de son efficacité à "négativer" la détection de ces agents dans les fluides biologiques des patients.

**<u>EXEMPLE 12</u> : OBTENTION D'UN CLONE LB19 CONTENANT UNE PARTIE DU GENE GAG DU RETROVIRUS MSRV-1**

Une technique PCR dérivée de la technique publiée par Gonzalez-Quintial R et al. (19) et PLAZA et al (25) a été utilisée. A partir des ARNs totaux extrait d'une fraction de virion purifié comme décrit précédemment, la cDNA a été synthétisé à l'aide d'une amorce spécifique (SEQ ID N°64)en 3' du génome à amplifier, en utilisant la EXPAND™ REVERSE TRANSCRIPTASE (BOEHRINGER MANNHEIM).

cDNA :
AAGGGGCATG GACGAGGTGG TGGCTTATTT (SEQ ID NO°65) (anti-sens)

Après purification, une queue poly G a été ajoutée à l'extrémité 5' du cDNA à l'aide du kit "Terminal transferases kit" commercialisé par la société Boehringer Mannheim, selon le protocole du fabriquant.

Une PCR d'ancrage a été réalisée à l'aide des amorces 5' et 3'suivantes:

AGATCTGCAG AATTCGATAT CACCCCCCCC CCCCCC (SEQ ID N°91) (sens), et

AAATGTCTGC GGCACCAATC TCCATGTT (SEQ ID NO°64) (anti-sens)

Ensuite, une PCR d'ancrage semi-nichée a été réalisée avec les amorces 5' et 3'suivantes:

AGATCTGCAG AATTCGATAT CA (SEQ ID N°92) (sens), et
AAATGTCTGC GGCACCAATC TCCATGTT (SEQ ID NO° 64) (anti-sens)

Les produits issus de la PCR ont été purifiés après purification sur gel d'agarose selon les méthodes conventionnelles (17), puis resuspendus dans 10 microlitres d'eau distillée. Une des propriétés de la polymérase Taq consistant à ajouter une adénine à l'extémité 3' de chacun des deux brins d'ADN, l'ADN obtenu a été directement inséré dans un plamide à l'aide du kit TA Cloning™ (British Biotechnology). Les 2 µl de solution d'ADN ont été mélangés avec 5 µl d'eau distillée stérile, 1 µl d'un tampon de ligation 10 fois concentré "10X LIGATION BUFFER", 2 µl de "pCR™ VECTOR" (25 ng/ml) et 1 µl de "T4 DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" (17). La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer, modèle 373 A" Applied Biosystems selon les instructions du fabricant.

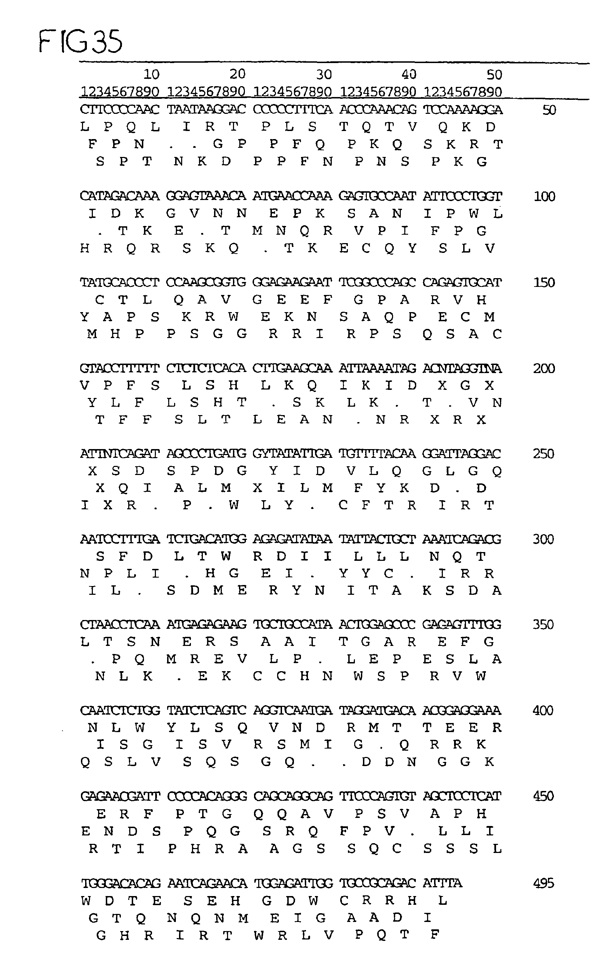

L'amplification PCR selon la technique citée plus-haut a été utilisée sur un ADNc synthétisé à partir des acides nucléiques de fractions de particules infectantes purifiées sur gradient de saccharose, selon la technique décrite par H. Perron (13), à partir de surnageants de culture de lymphocytes B d'un patient atteint de SEP, immortalisés par la souche B95 du virus d'Epstein-Barr (EBV) et exprimant des particules rétrovirales associées à une activité transcriptase inverse telle que décrite par Perron et coll. (3) et dans les demandes de brevets français SEP 10, 11 et 12. le clone LB19 dont la séquence identifiée par SEQ ID NO 59, est présentée dans la figure 35.

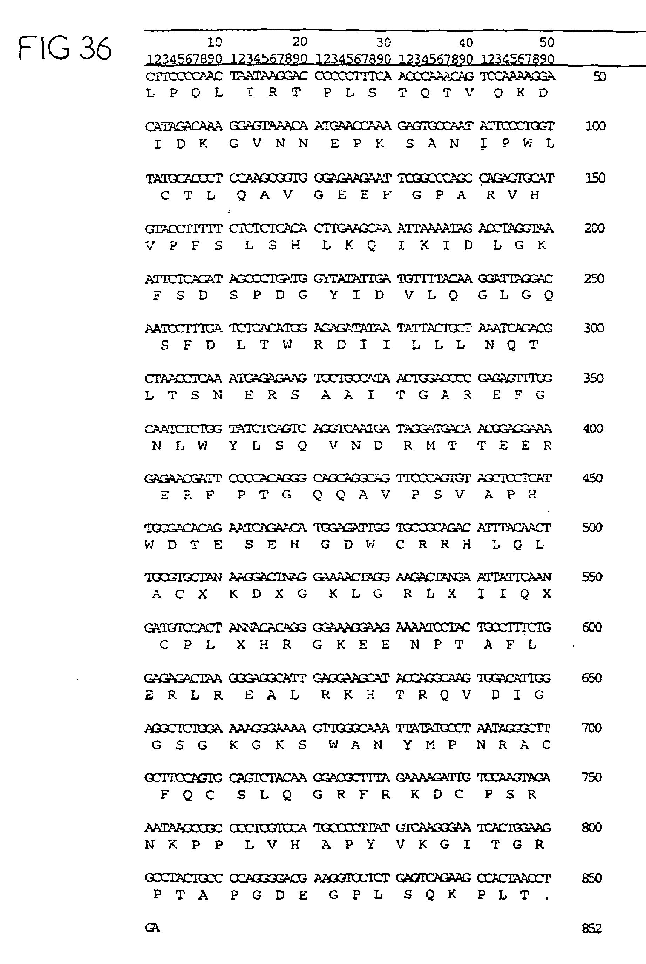

Ce clone permet de définir, avec le clone GM3 préalablement séquencé et le clone G+E+A (cf Exemple 15), une région de 690 paires de bases représentative d'une partie significative du gène gag du rétrovirus MSRV-1, telle que présentée dans la figure 36. Cette séquence dénommée SEQ ID NO 88 est reconstituée à partir de différents clones se recouvrant à leurs extrémités. Cette séquence est identifiée sous la dénomination région MSRV-1 "gag*". Dans la figure 36, une trame de lecture potentielle avec la traduction en acides aminés est présentée en-dessous de la séquence en acides nucléiques.

**<u>EXEMPLE 13</u> : OBTENTION D'UN CLONE FBd13 CONTENANT UNE REGION DE GENE *pol* APPARENTEE AU RETROVIRUS MSRV-1 ET UNE REGION ENV APPAREMMENT INCOMPLETE CONTENANT UN CADRE DE LECTURE (ORF) POTENTIEL POUR UNE GLYCOPROTEINE.**

<u>Extraction des ARN viraux</u>: les ARN ont été extraits selon la méthode brièvement décrite ci-après.

Un "pool" de surnageant de culture de lymphocytes B de patients atteints de SEP (650ml) est centrifugé 30 minutes à 10 000 g. Le culot viral obtenu est resuspendu dans 300 microlitres de PBS/10mM $MgCl_2$. Le matériel est traité par un mélange DNAse (100mg/ml)/RNAse (50mg/ml) 30 minutes à 37°C puis par de la protéinase K (50mg/ml) 30 minutes à 46°C.

Les acides nucléiques sont extraits par un volume d'un mélange phénol/SDS 0,1% (V/V) chauffé à 60°C puis réextraits par un volume de phénol/chloroforme (1/1; V/V).

La précipitation du matériel est effectué par 2,5 V d'éthanol en présence de 0,1 V d'acétate de sodium pH=5,2. Le culot obtenu après centrifugation est resuspendu dans 50 microlitres d'eau DEPC stérile.

L'échantillon est de nouveaux traité par 50mg/ml de DNAse "RNAse free" 30 minutes à température ambiante, extrait par un volume de phénol/chloroforme, et précipité en présence d'acétate de sodium et d'éthanol.

L'ARN obtenu est quantifié par un lecture de D.O. à 260 nm. La présence de MSRV-1 et l'absence de contaminant ADN est contrôlé par une PCR et une RTPCR MSRV-1 spécifique associé à un ELOSA spécifique du génome MSRV-1.

<u>Synthèse de cDNA:</u>

5 mg d'ARN sont utilisés pour synthétiser un cDNA amorcé par un oligonucléotide polydT selon les instructions du kit "cDNA Synthesis Module" (ref RPN 1256, Amersham) avec quelques modifications: la rétrotranscription est effectuée à 45°C au lieu des 42°C recommandés.

Le produit de synthèse est purifié par une double extraction et une double purification suivant les instructions du fabriquant.

La présence de MSRV-1 est vérifiée par une PCR MSRV-1 associée à un ELOSA spécifique du génome MSRV-1.

"Long Distance PCR": (LD-PCR)

500 ng de cDNA sont utilisés pour l'étape de LD-PCR (Expand Long Template System; Boehringer (ref.1681 842)).

Plusieurs couples d'oligonucléotides ont été utilisés. Parmi ceux-ci, le couple défini par les amorces suivantes :

amorce 5': GGAGAAGAGC AGCATAAGTG G (SEQ ID N°66)
amorce 3': GTGCTGATTG GTGTATTTAC AATCC (SEQ ID N°67).

Les conditions d'amplification sont les suivantes:

94°C 10 secondes
56°C 30 secondes
68°C 5 minutes ;

10 cycles puis 20 cycles avec un incrément de 20 secondes à chaque cycle sur le temps d'élongation. A l'issue de cette première amplification, 2 microlitres du produit d'amplification sont soumis à une deuxième amplification dans les mêmes conditions que précédemment.

Les LD-PCR sont conduites dans un appareil PCR Perkin modèle 9600 dans des microtubes à paroi fine (Boehringer).

Les produits d'amplification sont contrôlés par électrophorèse de 1/5 du volume d'amplification (10 microlitres) en gel d'agarose 1%. Pour le couple d'amorces décrit ci-dessus, on obtient une bande d'environ 1,7 Kb.

Clonage du fragment amplifié:

Le produit PCR a été purifié par passage sur un gel d'agarose préparatif puis sur une colonne Costar (Spin; D. Dutcher) selon les instructions du fournisseur.

2 microlitres de la solution purifiée sont raboutés avec 50 ng de vecteur PCRII selon les instructions du fournisseur (TA Cloning Kit; British Biotechnology)).

Le vecteur recombinant obtenu est isolé par transformation de bactéries DH5aF' compétentes. Les bactéries sont sélectionnées sur leur résistance à l'ampicilline et la perte du métabolisme pour le Xgal (=colonies blanches). La structure moléculaire du vecteur recombinant est confirmée par minipréparation plasmidique et hydrolyse par l'enzyme *Eco*R1.

FBd13, un clone positif pour tous ces critères a été sélectionné. Une préparation à large échelle du plasmide recombinant a été effectué à l'aide du kit Midiprep Quiagen (ref 12243) selon les instructions du fournisseur.

Le séquençage du clone FBd13 est effectué grâce au kit Prism Ready Amplitaq FS dye terminator Perkin (ref. 402119) suivant les instructions du fabriquant. Les réactions de séquences sont déposées sur un séquençeur automatique Perkin de type 377 ou 373A. La stratégie de séquençage consiste en une "marche sur le gène" réalisée sur les deux brins du clone FBd13.

La séquence du clone FBd13 est identifiée par SEQ ID NO 58.

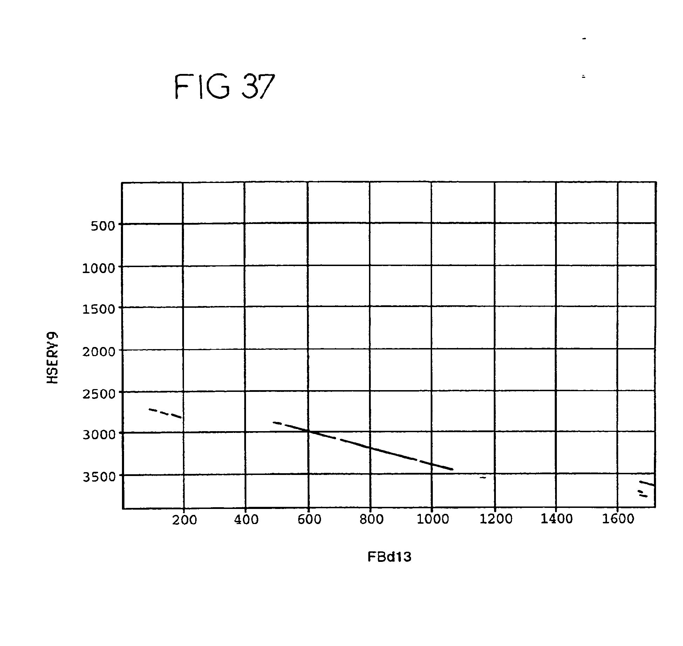

Dans la figure 37, l'homologie de séquence entre le clone FBd13 et le rétrovirus HSERV-9 est représentée sur le tableau matriciel par un trait plein, pour toute homologie partielle supérieure ou égale à 70 %. On peut constater que des homologies existent dans les régions flanquantes du clone (avec le gène *pol* en 5' et avec le gène env puis le LTR en 3'), mais que la région interne est totalement divergente et ne présente aucune homologie, même faible, avec le gène env d'HSERV-9. De plus, il apparaît que le clone FBd13 contient une région "env" plus longue que celle qui est décrite pour l'endogène défectif HSERV-9 ; on peut ainsi constater que la région divergente interne constitue un "insert" entre les régions d'homologie partielle avec les gènes défectifs HSERV-9.

Cette séquence supplémentaire détermine une orf potentielle, dénommée ORF B13, qui est représentée par sa séquence aminoacide SEQ ID NO 87.

La structure moléculaire du clone FBd13 a été analysée à l'aide du logiciel GeneWork et des banques de données Genebank et SwissProt.

5 sites de glycosylation ont été trouvés.

La protéine n'a pas d'homologie significative avec des séquences déjà connues.

Il est probable que ce clone provienne d'une recombinaison avec un élément rétroviral endogène (ERV), liée à la réplication de MSRV-1.

Un tel phénomène n'est pas sans générer l'expression de polypeptides, voire de protéines rétrovirales endogènes qui ne sont pas nécessairement tolérées par le système immunitaire. Un tel schéma d'expression aberrante d'éléments endogènes apparentés à MSRV-1 et/ou induite par ce dernier est susceptible de multiplier les antigènes aberrants et, donc, de contribuer à l'induction de processus autoimmuns tels qu'on les observe dans la SEP. Il constitue à l'évidence un élément original, jamais décrit à ce jour. En effet, l'interrogation des banques de données de séquences nucléiques disponibles dans la version n° 19 (1996) du logiciel "Entrez" (NCBI, NIH, Bethesda, USA) n'a pas permis d'identifier de séquence homologue connue comprenant l'ensemble de la région env de ce clone.

**<u>EXEMPLE 14</u> : OBTENTION D'UN CLONE FP6 CONTENANT UNE PARTIE DU GENE *pol,* AVEC UNE REGION CODANT POUR L'ENZYME TRANSCRIPTASE INVERSE HOMOLOGUE AU CLONE POL* MSRV-1, ET UNE REGION 3'pol DIVERGENTE DES SEQUENCES EQUIVALENTES DECRITES DANS LES CLONES POL*, tpol, FBd3, JLBc1 et JLBc2.**

Une 3'RACE a été effectuée sur de l'ARN total extrait de plasma d'un patient atteint de SEP. Un plasma témoin sain, traité sous les mêmes conditions, a été utilisé comme contrôle négatif. La synthèse de cDNA a été réalisée avec l'amorce oligo dT modifiée suivante:

5' GACTCGCTGC AGATCGATTT TTTTTTTTTT TTTT 3'(SEQ ID NO 68)

et la transcriptase inverse "Expand RT" de Boehringer selon les conditions préconisées par la société. Une PCR a été effectuée avec l'enzyme Klentaq (Clontech) sous les conditions suivantes : 94°C 5 min puis 93°C 1 min, 58°C 1 min, 68°C 3 min pendant 40 cycles et 68°C pendant 8 min et avec un volume réactionnel final de 50 $\mu$l.

Amorces utilisées pour la PCR :

- amorce 5', identifiée par SEQ ID NO 69
  5' GCCATCAAGC CACCCAAGAA CTCTTAACTT 3' ;
- amorce 3', identifiée par SEQ ID NO 68 (=la même que pour le cDNA)

Une deuxième PCR dite "semi-nichée" a été réalisée avec une amorce 5' située à l'intérieur de la région déjà amplifiée. Cette deuxième PCR a été effectuée sous les mêmes conditions expérimentales que celles utilisées lors de la première PCR, en utilisant 10 $\mu$l du produit d'amplification issu de la première PCR.

Amorces utilisées pour la PCR semi-nichée:

- amorce 5', identifiée par SEQ ID NO 70
  5' CCAATAGCCA GACCATTATA TACACTAATT 3' ;
- amorce 3', identifiée par SEQ ID NO 68 (=la même que pour le cDNA)

Les amorces SEQ ID NO 69 et SEQ ID NO 70 sont spécifiques de la région pol* : position n°403 à n°422 et n°641 à n°670 respectivement.

Un produit d'amplification a ainsi été obtenu à partir de l'ARN extracellulaire extrait du plasma d'un patient atteint de SEP. Le fragment correspondant n'a pas été observé pour le plasma d'un témoin sain. Ce produit d'amplification a été cloné de la façon suivante.

L'ADN amplifié a été inséré dans un plasmide à l'aide du kit TA Cloning™. Les 2 $\mu$l de solution d'ADN ont été mélangés avec 5 $\mu$l d'eau distillée stérile, 1 $\mu$l d'un tampon de ligation 10 fois concentré "10X LIGATION BUFFER", 2 $\mu$l de "pCR™ VECTOR" (25 ng/ml) et 1 $\mu$l de "TA DNA LIGASE". Ce mélange a été incubé une nuit à 12°C. Les étapes suivantes ont été réalisées conformément au instructions du kit TA cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" (17). La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA cloning kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer, modèle 373 A" Applied Biosystems, selon les instructions du fabricant.

Le clone obtenu, nommé FP6, permet de définir une région de 467 pb homologue à 89% à la région pol* du rétrovirus MSRV-1, et une région de 1167 pb homologue à 64% à la région pol d'ERV-9 (n°1634 à 2856).

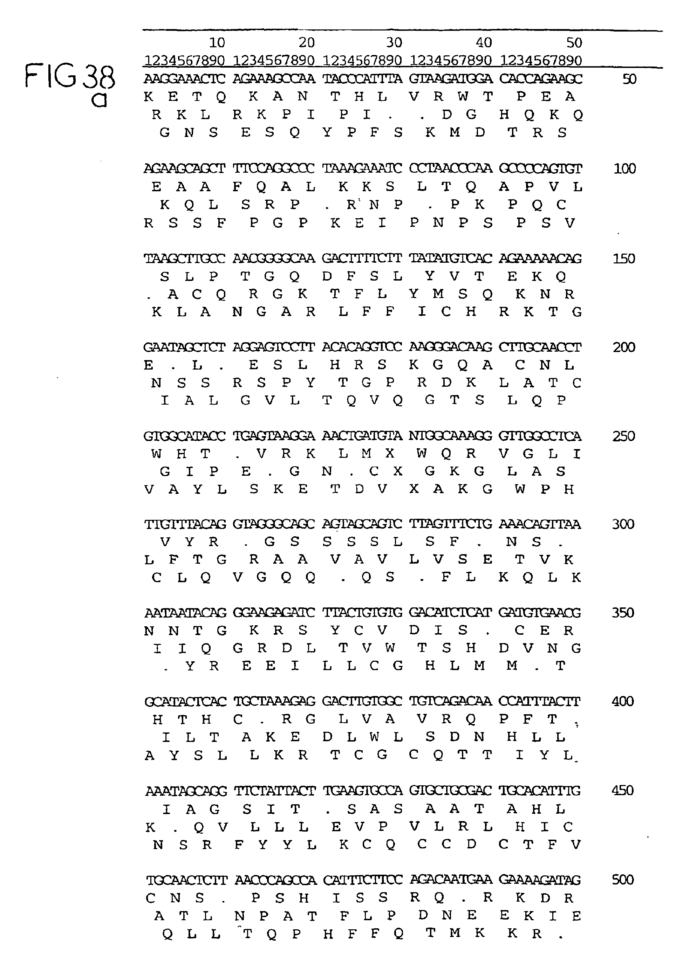

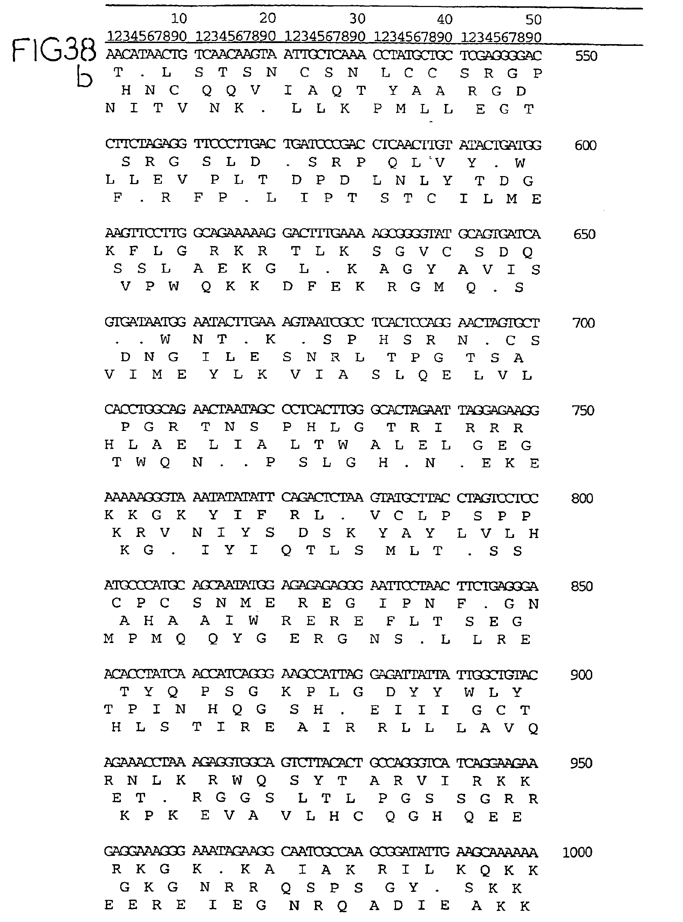

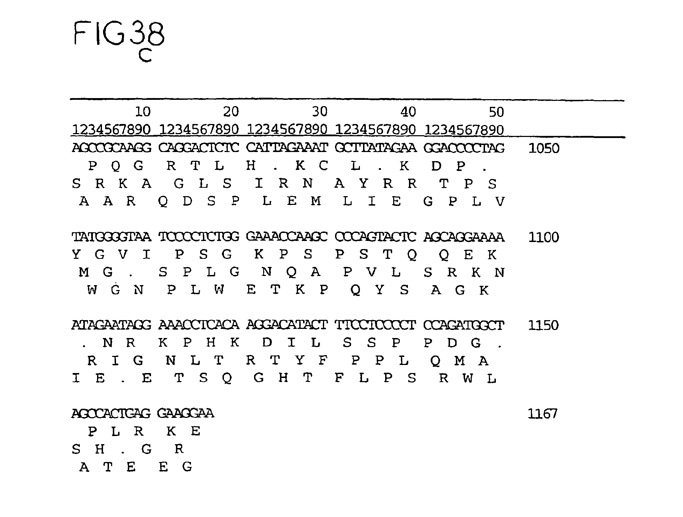

Le clone FP6 est représenté sur la figure 38 par sa séquence nucléotidique identifiée par SEQ ID NO 61. Les trois trames de lecture potentielles de ce clone sont présentées par leur séquence aminoacide sous la séquence nucléotidique.

**<u>EXEMPLE 15</u> : OBTENTION D'UNE REGION DENOMMEE G+E+A CONTENANT UNE ORF POUR UNE PROTEASE RETROVIRALE PAR AMPLIFICATION PCR DE LA SEQUENCE NUCLEIQUE CONTENUE ENTRE LA REGION 5' DEFINIE PAR LE CLONE "GM3" ET LA REGION 3' DEFINIE PAR LE CLONE POL*, A PARTIR DE L'ARN EXTRAIT D'UN POOL DE PLASMAS DE PATIENTS ATTEINTS DE SEP.**

Des oligonucléotides spécifiques des séquences MSRV-1 déjà identifiées par la Demanderesse ont été définis pour amplifier l'ARN rétroviral provenant de virions présents dans le plasma de patients atteints de SEP. Des réactions de contrôle ont été effectuées de manière à contrôler la présence de contaminants (réaction sur de l'eau). L'amplification consiste en une étape de RT-PCR , suivie d'une PCR "nichée". Des couples d'amorces ont été définis pour amplifier trois régions chevauchantes (dénommées G, E et A) sur les régions définies par les séquences des clones GM3 et pol*, préalablement décrits.

<u>RT-PCR semi nichée pour l'amplification de la région G</u>:

- dans le premier cycle RT-PCR, les amorces suivantes sont utilisées :

  amorce 1 : SEQ ID NO 71 (sens)
  amorce 2 : SEQ ID NO 72 (anti-sens)

- dans le deuxième cycle PCR, les amorces suivantes sont utilisées :

  amorce 1 : SEQ ID NO 73 (sens)
  amorce 4 : SEQ ID NO 74 (anti-sens)

<u>RT-PCR nichée pour l'amplification de la région E</u>:

- dans le premier cycle RT-PCR, les amorces suivantes sont utilisées :

  amorce 5 : SEQ ID NO 75 (sens)
  amorce 6 : SEQ ID NO 76 (anti-sens)

- dans le deuxième cycle PCR, les amorces suivantes sont utilisées :

  amorce 7 : SEQ ID NO 77 (sens)
  amorce 8 : SEQ ID NO 78 (anti-sens)

<u>RT-PCR semi nichée pour l'amplification de la région A</u>:

- dans le premier cycle RT-PCR, les amorces suivantes sont utilisées :

  amorce 9 : SEQ ID NO 79 (sens)
  amorce 10 :SEQ ID NO 80 (anti-sens)

- dans le deuxième cycle PCR, les amorces suivantes sont utilisées :

amorce 9 : SEQ ID NO 81(sens)
amorce 11 :SEQ ID NO 82 (anti-sens)

Les amorces et les régions G, E et A qu'elles définissent sont positionnées comme suit :

```
___________________________________________________________
cDNA
     1______G______4 2
              5 7____________E_____________8 6

                                         3______A_____11 10
<------------------------------><------------------------->
              GM3                          POL*
```

La séquence de la région définie par les différents clones G, E et A a été déterminée après clonage et séquençage des produits d'amplification "nichée".

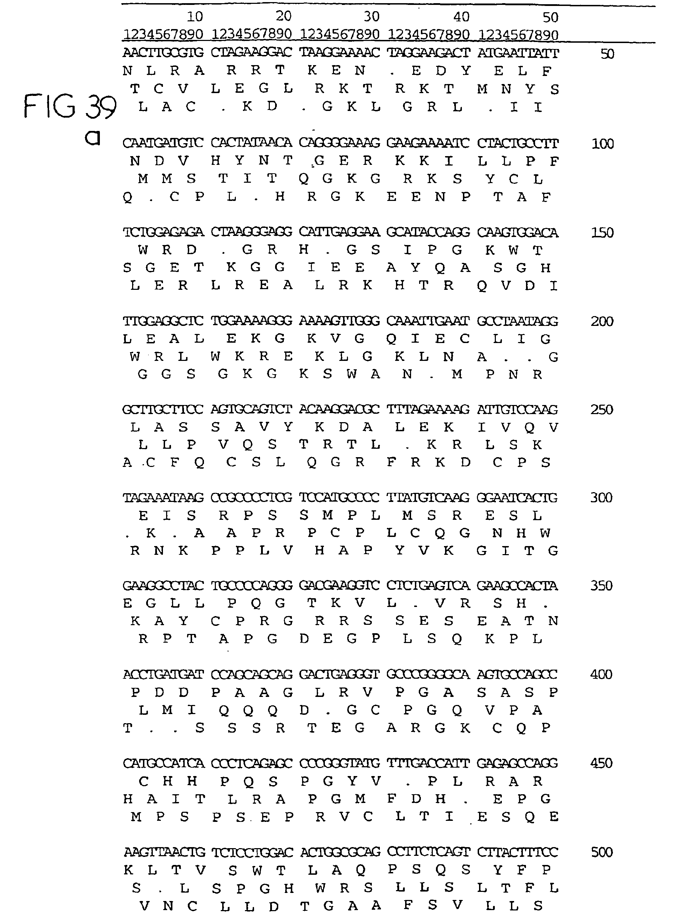

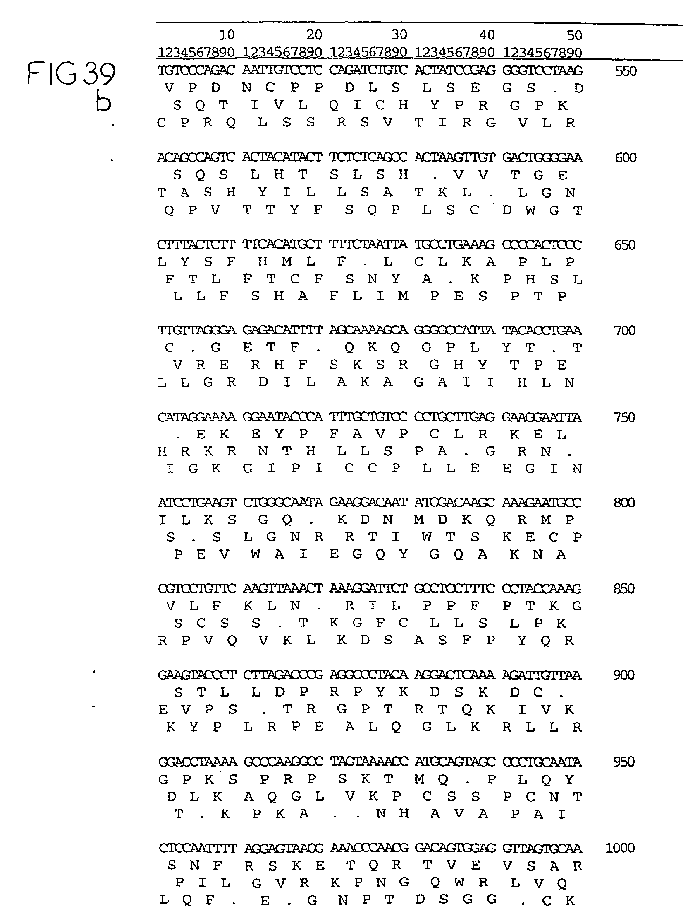

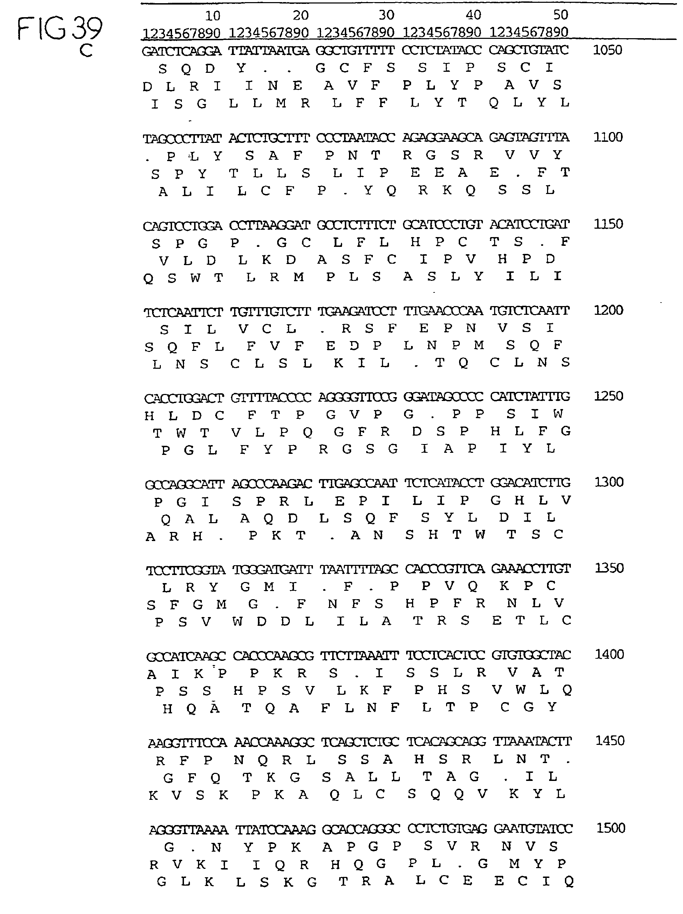

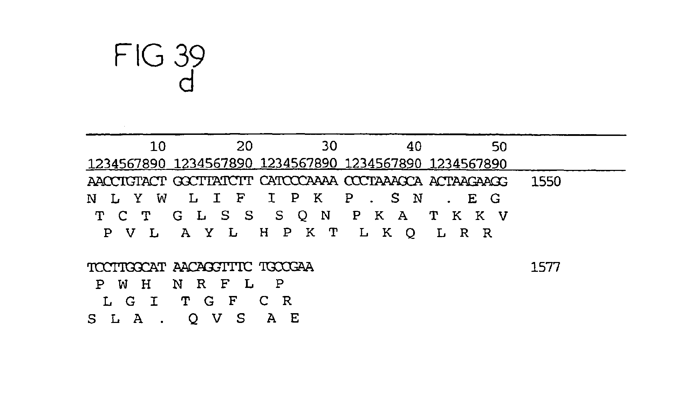

Les clones G, E et A ont été rassemblés par PCR avec les amorces 1 en 5' du fragment G et 11 en 3' du fragment A, précédemment décrites. Un fragment G+E+A d'environ 1580bp a été amplifié et inséré dans un plasmide à l'aide du kit TA Cloning (marque de commerce). La séquence du produit d'amplification correspondant à G+E+A a été déterminée et l'analyse des recouvrements G+E et E+A a été réalisée. La séquence est représentée dans la figure 39, et correspond à la séquence SEQ ID NO 89.

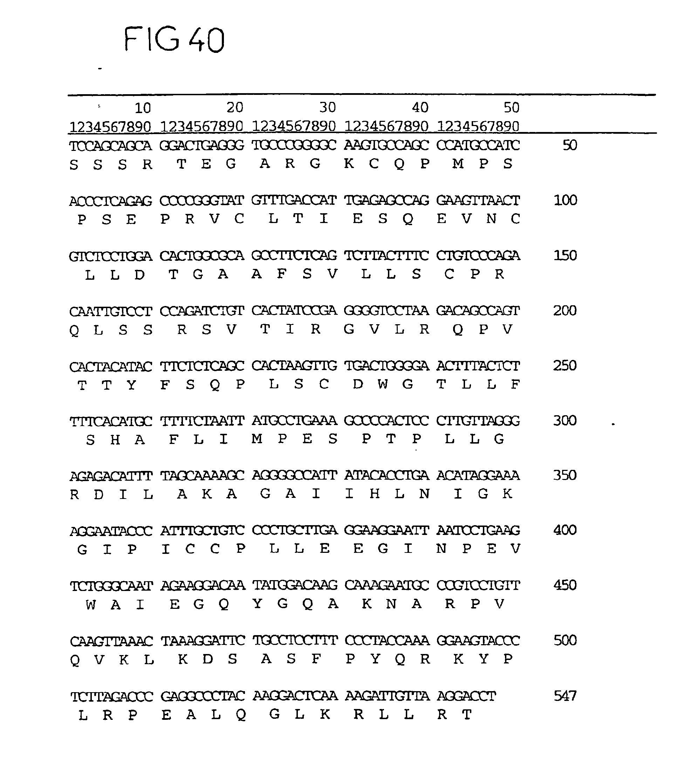

Une trame de lecture codant pour une protéase rétrovirale MSRV-1 a été trouvée dans la région E. La séquence aminoacide de la protéase, identifiée par SEQ ID NO 90, est présentée dans la figure 40.

**<u>EXEMPLE 16</u> : OBTENTION D'UN CLONE LTRGAG12, APPARENTE AU UN ELEMENT RETROVIRAL ENDOGENE (ERV) PROCHE DE MSRV-1, DANS L'ADN D'UNE LIGNEE LYMPHOBLASTOIDE DE SEP PRODUISANT DES VIRIONS ET EXPRIMANT LE RETROVIRUS MSRV-1.**

Une PCR nichée a été effectuée sur l'ADN extrait d'une lignée lymphoblastoïde (lymphocytes B immortalisés par le virus EBV, souche B95, comme décrit précédemment et comme cela est bien connu de l'homme de l'art) exprimant le rétrovirus MSRV-1 et provenant des lymphocytes du sang périphérique d'un malade atteint de SEP.

Dans la première étape PCR, les amorces suivantes sont utilisées :

amorce 4327 : CTCGATTTCT TGCTGGGCCT TA (SEQ ID NO 83)
amorce 3512 : GTTGATTCCC TCCTCAAGCA (SEQ ID NO 84)

Cette étape comprend 35 cycles d'amplification avec les conditions suivantes: 1 min à 94°C, 1 min à 54°C et 4 min à 72°C.

Dans le deuxième étape PCR, les amorces suivantes sont utilisées :

amorce 4294 : CTCTACCAAT CAGCATGTGG (SEQ ID NO 85)
amorce 3591 : TGTTCCTCTT GGTCCCTAT (SEQ ID NO 86)

Cette étape comprend 35 cycles d'amplification avec les conditions suivantes: 1 min à 94°C, 1 min à 54°C et 4 min à 72°C.

Les produits issus de la PCR ont été purifiés après purification sur gel d'agarose selon les méthodes conventionnelles (17), puis resuspendus dans 10 ml d'eau distillée. Une des propriétés de la polymérase Taq consistant à ajouter une adénine à l'extémité 3' de chacun des deux brins d'ADN, l'ADN obtenu a été directement inséré dans un plamide à l'aide du kit TA Cloning™ (British Biotechnology). Les 2 µl de solution d'ADN ont été mélangés avec 5 µl d'eau distillée stérile, 1 µl d'un tampon de ligation 10 fois concentré "10X LIGATION BUFFER", 2 µl de "pCR™ VECTOR" (25 ng/ml) et 1 µl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" (17). La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer, modèle 373 A" Applied Biosystems selon les instructions du fabricant.

Ainsi, un clone dénommé LTRGAG12 a pu être obtenu et est représenté par sa séquence interne identifiée par SEQ ID NO 60.

Ce clone est vraisemblablement représentatif d'éléments endogènes proches d'ERV-9, présent dans l'ADN humain, notamment dans l'ADN de patients atteints de SEP, et pouvant interférer avec l'expression du rétrovirus MSRV-1, donc pouvant avoir un rôle dans la pathogénie associée au rétrovirus MSRV-1 et pouvant servir de marqueur d'une expression spécifique dans la pathologie concernée.

**<u>EXEMPLE 17</u> : DETECTION D'ANTICORPS SPECIFIQUES ANTI-MSRV-1 DANS LE SERUM HUMAIN.**

L'identification de la séquence du gène *pol* du rétrovirus MSRV-1 et d'un cadre de lecture ouverte de ce gène a permis de déterminer la séquence SEQ ID NO 63 en acides aminés d'une région dudit gène, référencée par SEQ ID NO 62.

Différents peptides synthétiques correspondant à des fragments de la séquence protéique de la transcriptase inverse MSRV-1 codée par le gène pol, ont été testés pour leur spécificité antigénique vis à vis de sera de patients atteints de SEP et de témoins sains.

Les peptides ont été synthétisés chimiquement par synthèse en phase solide, selon la technique de Merrifield (22). Les modalités pratiques sont celles décrites ci-après.

a) <u>Synthèse des peptides</u>:

Les peptides ont été synthétisés sur une résine phénylacétamidométhyle (PAM)/polystyrène/divinylbenzène (Applied Biosystems, Inc. Foster City, CA), en utilisant un synthétiseur automatique "Applied Biosystems 430A". Les acides aminés sont couplés sous forme d'esters d'hydroxybenzotriazole (HOBT). Les acides aminés utilisés proviennent de Novabiochem (Laüflerlfingen, Suisse) ou de Bachem (Bubendorf, Suisse).

La synthèse chimique a été effectuée en utilisant un protocole de double couplage avec la N-méthylpyrrolidone (NMP) comme solvant. Les peptides ont été coupés de la résine ainsi que les protections latérales, de manière simultanée, à l'aide d'acide fluorhydrique (HF) dans un appareil approprié (appareil de coupure de type I, Peptide Instiute, Osaka, Japon).

Pour 1 g de peptidylrésine, 10 ml de HF, 1 ml d'anisole et 1 ml de diméthylsulfure 5DMS sont utilisés. Le mélange est agité durant 45 minutes à -2 °C. Le HF est alors évaporé sous vide. Après lavages intensifs à l'éther, le peptide est élué de la résine par de l'acide acétique 10%, puis lyophilisé.

Les peptides sont purifiés par chromatographie liquide haute performance préparative sur une colonne VYDAC de type C18 (250 x 21 mm) (The Separation Group, Hesperia, CA, USA). L'élution est réalisée par un gradient d'acétonitrile à un débit de 22 ml/min. Les fractions collectées sont contrôlées par une élution en condition isocratique sur une colonne VYDAC® C18 analytique (250 x 4,6 mm), à un débit de 1 ml/min. Les fractions qui présentent le même temps de rétention sont réunies et lyophilisées. La fraction majoritaire est ensuite analysée par chromatographie liquide haute performance analytique, avec le système décrit précédemment. Le peptide qui est considéré comme de pureté acceptable se traduit par un pic unique représentant 95 % minimum du chromatogramme.

Les peptides purifiés sont ensuite analysés dans le but de contrôler leur composition en acides aminés, à l'aide d'un analyseur d'acides aminés automatique Applied Biosystems 420H. La mesure de la masse moléculaire chimique (moyenne) des peptides est obtenue en utilisant la spectrométrie de masse L.S.I.M.S. en mode d'ion positif sur un instrument à double focalisation VG. ZAB.ZSEQ relié à un système d'acquisition DEC-VAX 2000 (VG analytical Ltd, Manchester, Angleterre).

La réactivité des différents peptides a été testée contre des sera de patients atteints de SEP et contre des sera de témoins sains. Ceci a permis de selectionner un peptide dénommé S24Q dont la séquence est identifiée par SEQ ID NO 63, codé par une séquence nucléotidique du gène *pol* de MSRV-1 (SEQ ID NO 62).

b) Propriétés antigéniques :

Les propriétés antigéniques du peptide S24Q ont été mises en évidence selon le protocole ELISA décrit ci-dessous.

Le peptide S24Q lyophilisé a été dissous à une concentration de 1 mg/ml dans de l'acide acétique à 10%. Cette solution-mère a été aliquotée et gardée à +4°C pour usage sous quinzaine, ou congelée à -20°C, pour un usage dans les 2 mois. Un aliquot est dilué dans une solution de PBS (Phosphate Buffer Saline) afin d'obtenir une concentration finale de peptide de 5 microgrammes/ml. 100 microlitres de cette dilution sont déposés dans chaque puits de plaques de microtitration Nunc Maxisorb (nom commercial). Les plaques sont recouvertes d'un adhésif de type "plate-sealer" et maintenues 2 heures à +37°C, pour la phase d'adsorption du peptide sur le plastique. L'adhésif est enlevé et les plaques sont lavées trois fois avec un volume de 300 microlitres d'une solution A (PBS 1x, 0,05% Tween 20®), puis retournées sur un tissu absorbant. Les plaques ainsi égouttées sont remplies avec 250 microlitres par puits d'une solution B (solution A + 10 % de sérum de chèvre), puis recouvertes d'un adhésif et incubées 1 heure, à 37°C. Les plaques sont ensuite lavées trois fois avec la solution A, comme décrit précédemment.

Les échantillons de sérum à tester sont préalablement dilués au 1/100ème dans la solution B et 100 microlitres de chaque sérum dilué à tester sont déposés dans les puits de chaque plaque de microtitration. Un contrôle négatif est déposé dans un puits de chaque plaque, sous la forme de 100 microlitres de tampon B. Les plaques recouvertes d'un adhésif sont alors incubées 1 heure 30 min. à 37°C. Les plaques sont ensuite lavées trois fois avec la solution A, comme décrit précédemment. Pour la réponse IgG, un anticorps de chèvre marqué à la peroxydase et dirigé contre les IgG humaines (commercialisé par Jackson Immuno Research Inc.) est dilué dans la solution B (dilution 1/10 000). 100 microlitres de la dilution adéquate de l'anticorps marqué sont alors déposés dans chaque puits des plaques de microtitration et les plaques recouvertes d'un adhésif sont incubées 1 heure, à 37°C. Un nouveau lavage des plaques est ensuite effectué comme décrit précédemment. Parallèlement, le substrat de la peroxydase est préparé selon les indications des kits bioMérieux. 100 microlitres de solution-substrat sont déposés dans chaque puits et les plaques sont placées à l'abri de la lumière pendant 20 à 30 minutes, à température ambiante.

Une fois la réaction colorée stabilisée, 50 microlitres de Color 2 (nom commercial-bioMérieux) sont déposés dans chaque puits pour stopper la réaction. Les plaques sont immédiatement placées dans un lecteur spectrophotométrique de plaques ELISA, et la densité optique (D.O.) de chaque puits est lue à une longueur d'onde de 492 nm.

Les échantillons sérologiques sont déposés en double ou en triple et la densité optique (D.O.) correspondant au sérum testé est calculée en faisant la moyenne des D.O. obtenues pour un même échantillon, à la même dilution.

La D.O. nette de chaque sérum correspond à la D.O. moyenne du sérum de laquelle est soustraite la D.O. moyenne du témoin négatif (solution B : PBS, Tween 20® 0,05%, 10% sérum de chèvre).

c) Détection d'anticorps IgG anti-MSRV-1 (S24Q) par ELISA :

La technique décrite ci-dessus a été utilisée avec le peptide S24Q pour rechercher la présence d'anticorps IgG spécifiques anti-MSRV-1 dans le sérum de 15 patients, pour lesquels un diagnostic certain de SEP a été posé selon les critères de Poser (23), et de 15 témoins sains (donneurs de sang).

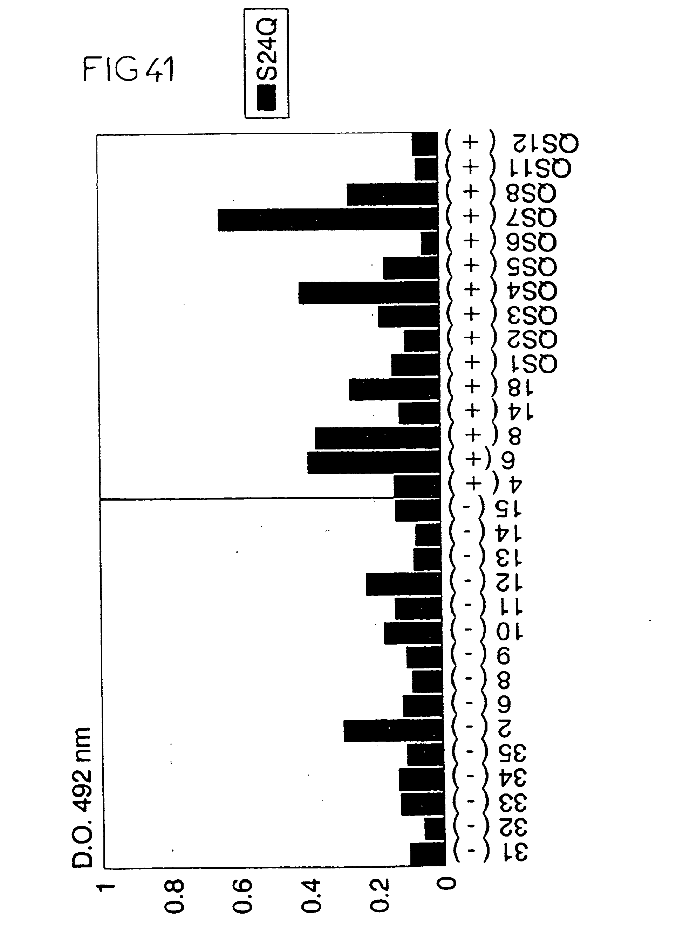

Dans la figure 41, les résultats de chaque sérum testé avec un anticorps anti-IgG, sont représentés. Chaque barre verticale représente la densité optique (D.O à 492 nm) nette d'un sérum testé. L'axe des ordonnées indique la D.O. nette au sommet des barres verticales. Les 15 premières barres verticales situées à gauche de la ligne pointillée verticale, représentent les sérums de 15 témoins sains (donneurs de sang) et les 15 barres verticales situées à droite de la ligne pointillée verticale, représentent les sérums de 15 cas de SEP testés. Le graphique permet de visualiser 2 témoins dont la D.O. émerge au dessus des valeurs groupées de la population témoin. Ces valeurs peuvent représenter la présence d'IgG spécifiques chez des patients séropositifs non-malades. Deux méthodes ont donc été évaluées pour déterminer le seuil statistique de positivité du test.

La moyenne des D.O. nettes des témoins, y compris les témoins avec des D.O. nettes élevées, est de 0,129 et l'écart type est de 0,06. Sans les 2 témoins dont les D.O. sont supérieures à 0,2, la moyenne des témoins "négatifs" est de 0,107 et l'écart-type de 0,03. Un seuil de positivité théorique peut être calculé selon la formule :

valeur seuil (moyenne des D.O. nettes des témoins égatifs) + (2 ou 3 x écart-type des D.O. nettes des témoins négatifs).

Dans le premier cas, on considère qu'il existe des séropositifs non-malades et la valeur seuil est égale à : 0,11 + (3 x 0,03) = 0,20. Les résultats négatifs représentent un "bruit de fond" non spécifique de la présence d'anticorps spécifiquement dirigés contre un épitope du peptide.

Dans le deuxième cas, si l'ensemble des témoins constitués de donneurs de sang en bonne santé apparente est pris comme base de référence, sans exclure les sérums a priori séropositifs, l'écart-type des "témoins non-SEP" est de 0,116. La valeur seuil devient alors : 0,13 + (3 x 0,06) = 0,31.

Selon cette dernière analyse, le test est spécique de la SEP. A cet égard, on constate que le test est spécifique de la SEP, puisque comme montré dans le tableau 1, aucun témoin n'a une D.O. nette au-dessus de ce seuil. En fait ce résultat reflète le fait que les titres d'anticorps chez les patients atteints de SEP sont en majorité plus élevés que chez des témoins sains ayant été en contact avec MSRV-1.

En fonction du premier mode de calcul et comme représenté à la figure 41 et dans le tableau 3, 6 des 15 sérums SEP donnent un résultat positif (D.O. supérieure ou égale à 0,2) indiquant la présence d'IgG spécifiquement dirigées contre le peptide S24Q, donc contre une partie de l'enzyme transcriptase inverse du rétrovirus MSRV-1 codée par son gène pol et, par conséquent, contre le rétrovirus MSRV-1.

Ainsi, environ 40 % des patients SEP testés ont réagi contre un épitope porté par le peptide S24Q et présentent des IgG circulantes dirigées contre ce dernier.

2 donneurs de sang, en apparente bonne santé, sur 15 présentent un résultat positif. Ainsi, il apparaît qu'environ 13 % de la population non-malade peut avoir été en contact d'un épitope porté par le peptide S24Q dans des conditions ayant conduit à une immunisation active qui se traduit par la persistance d'IgG sériques spécifiques. Ces conditions sont compatibles avec une immunisation contre la transcriptase inverse du rétrovirus MSRV-1, lors d'une infection par le (et/ou réactivation du) rétrovirus MSRV-1. L'absence de pathologie neurologique apparente évoquant la SEP chez ces témoins séropositifs peut indiquer qu'ils sont porteurs sains, ont éliminé un virus infectieux après s'être immunisés ou qu'ils constituent une population à risque de porteurs chroniques. En effet, les données épidémiologiques montrant qu'un agent pathogène présent dans l'environnement des régions à haute prévalence de SEP, peut être la cause de cette maladie, impliquent qu'une fraction de la population exempte de SEP a nécessairement été en contact avec un tel agent pathogène. On a montré que le rétrovirus MSRV-1 constitue tout ou partie de cet "agent pathogène" à l'origine de la SEP et il est donc normal que des témoins pris dans une population saine présentent des anticorps de type IgG contre des composants du rétrovirus MSRV-1.

Enfin, la détection d'anticorps anti-S24Q chez seulement une SEP sur deux testée ici, peut refléter le fait que ce peptide ne représente pas un épitope immunodominant MSRV-1, que des variations interindividuelles de souches peuvent induire une immunisation contre un motif peptidique divergent dans la même région, ou encore que l'évolution de la maladie et les traitements suivis peuvent moduler dans le temps la réponse anticorps contre le peptide S24Q.

**TABLEAU N°3**

| TEMOINS | SEP |
|---|---|
| 0,101 | 0,136 |
| 0,058 | 0,391 |
| 0,126 | 0,37 |
| 0,131 | 0,119 |
| 0,105 | 0,267 |
| 0,294 | 0,141 |
| 0,116 | 0,102 |
| 0,088 | 0,18 |
| 0,105 | 0,411 |
| 0,172 | 0,164 |
| 0,137 | 0,049 |
| 0,223 | 0,644 |
| 0,08 | 0,268 |
| 0,073 | 0,065 |
| 0,132 | 0,074 |
| | |

(suite)

| | TEMOINS | SEP |
|---|---|---|
| **Moyenne** | **0,129** | |
| **Ecart Type** | **0,06** | |
| **Seuil** | **0,31** | |

d) Détection d'anticorps IgM anti-MSRV-1 par ELISA :

La technique ELISA avec le peptide S24Q a été utilisée pour rechercher la présence d'anticorps spécifiques IgM anti-MSRV-1 dans les mêmes sérums que précédemment.

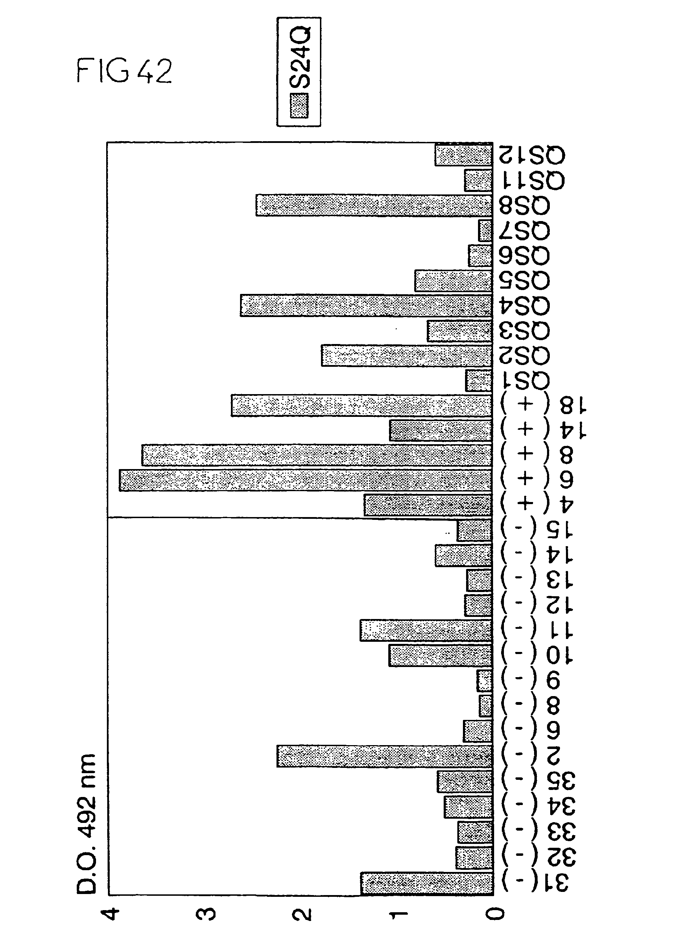

Dans la figure 42, les résultats de chaque sérum testé avec un anticorps anti-IgM, sont représentés. Chaque barre verticale représente la densité optique (D.O à 492 nm) nette d'un sérum testé. L'axe des ordonnées indique la D.O. nette au sommet des barres verticales. Les 15 premières barres verticales situées à gauche de la ligne verticale coupant l'axe des abscisses représentent les sérums de 15 témoins sains (donneurs de sang) et les barres verticales situées à droite de la ligne pointillée verticale, représentent les sérums de 15 cas de SEP testés.

La moyenne des D.O. des SEP testées est de : 1,6.

La moyenne des D.O. nettes des témoins est de : 0,7.

L'écart type des témoins négatifs est de : 0,6.

Le seuil de positivité théorique peut être calculé selon la formule :

valeur seuil = (moyenne des D.O. des témoins négatifs) + (3 x écart-type des D.O. des témoins négatifs)

La valeur seuil est donc égale à 0,7+ (3 x 0,6) = 2,5 ;

Les résultats négatifs représentent un "bruit de fond" non spécifique de la présence d'anticorps spécifiquement dirigés contre un épitope du peptide.

Selon cette analyse et comme montré à la figure 42 et dans le tableau 4 correspondant, le test IgM est spécique de la SEP, puisqu'aucun témoin n'a une D.O. nette au-dessus du seuil. 6 des 15 sérums SEP produisent un résultat IgM positif

La différence de séroprévalence entre les SEP et la population témoin est extrèmement significative : test "chi-2", $p<0{,}002$.

Ces résultats sont en faveur d'un rôle étiopathogénique de MSRV-1 dans la SEP.

Ainsi, la détection des anticorps IgM et IgG contre le peptide S24Q permet d'évaluer, seul ou en combinaison avec d'autres peptides MSRV-1, l'évolution d'une infection par MSRV-1 et/ou de la réactivation virale de MSRV-1.

**TABLEAU N°4**

| TEMOINS | SEP |
|---|---|
| 1,449 | 0,974 |
| 0,371 | 6,117 |
| 0,448 | 2,883 |
| 0,456 | 1,945 |
| 0,885 | 1,787 |
| 2,235 | 0,273 |
| 0,301 | 1,766 |
| 0,138 | 0,668 |
| 0,16 | 2,603 |
| 1,073 | 0,802 |
| 1,366 | 0,245 |

(suite)

| | TEMOINS | SEP |
|---|---|---|
| | 0,283 | 0,147 |
| | 0,262 | 2,441 |
| | 0,585 | 0,287 |
| | 0,356 | 0,589 |
| | | |
| **Moyenne** | **0,7** | |
| **Ecart Type** | **0,6** | |
| **Valeur seuil** | **2,5** | |

Il est possible, grâce aux nouvelles découvertes effectuées et aux nouvelles méthodes mises au point par les inventeurs, de permettre la réalisation perfectionnée de tests diagnostiques de l'infection et/ou de la réactivation MSRV-1, et d'évaluer une thérapie dans la SEP et/ou la PR, sur la base de son efficacité à "négativer" la détection de ces agents dans les fluides biologiques des patients. De plus, la détection précoce chez des personnes ne présentant pas encore de signes neurologiques de SEP, ou de signes rhumatologiques de PR, pourrait permettre d'instaurer un traitement d'autant plus efficace sur l'évolution clinique ultérieure qu'il précèderait le stade lésionnel qui correspond à l'apparition des troubles cliniques. Or, à ce jour, un diagnostic de SEP ou de PR ne peut être établi avant l'installation d'une symptomatologie lésionnelle et, donc, aucun traitement n'est instauré avant l'émergence d'une clinique évocatrice de lésions déjà conséquentes. Le diagnostic d'une infection et/ou réactivation de MSRV-1 et/ou MSRV-2 chez l'homme est donc déterminant et la présente invention en fournit les moyens.

Il est ainsi possible, outre de réaliser un diagnostic de l'infection et/ou de la réactivation MSRV-1, d'évaluer une thérapie dans la SEP sur la base de son efficacité à "négativer" la détection de ces agents dans les fluides biologiques des patients.

**BIBLIOGRAPHIE**

**(1)** Norrby E., Prog. Med. Virol., 1978; 24, 1-39.
**(2)** Johnson R.T., "Handbook of clinical neurology, 47 Demyelinating diseases", Vinken P. et Bruyn G.W., eds. Amsterdam, Elsevier Science Publishing, 1985, 319-336.
**(3)** Perron H. et coll., Res. Virol. 1989, 140, 551-561.
**(4)** Perron H. et coll., "Current concepts in multiple sclerosis" Wiethölter et coll., eds. Amsterdam, Elsevier, 1991, 111-116.
**(5)** Perron H. et coll., The Lancet 1991, 337, 862-863.
**(6)** Perron H. et coll., J. Gen. Virol. 1993, 74, 65-72.
**(7)** Fields et Knipe, Fondamental Virology 1986, Rev Press N.Y.
**(8)** Nielsen P.E. et coll, Science 1991; 254, 1497-1500.
**(9)** Maniatis et al, Molecular Cloning, Cold Spring Harbor, 1982.
**(10)** Southern. E.M., J. Mol. Biol. 1975, 98, 503.
**(11)** Dunn A.R. et Hassel J.A., Cell 1977, 12, 23.
**(12)** Shih et coll., J. Virol. 1989, 63, 64-75.
**(13)** Perron H. et coll., Res. Vir. 1992, 143, 337-350.
**(14)** Meyerhans et coll., Cell 1989, 58, 901-910.
**(15)** Linial M.L. and Miller A.D., "Current topics in microbiology and immunobiology. Retroviruses, stratégies of replication" vol. 157, 125-152; Swanstrom R. et Vogt P.K., éditeurs, Springer-Verlag, Heidelberg 1990.
**(16)** Lori F. et coll., J. Virol. 1992, 66, 5067-5074.
**(17)** Sambrook J., Fritsch E.F., et Maniatis T., Molecular cloning, a laboratory manual. Cold Spring Harbor Laboratory Press, 1989.
**(18)** La Mantia et coll., Nucleic Acids Research 1991, 19, 1513-1520.
**(19)** Gonzalez-Quintial R, Baccala R, Pope R M and Thoeofilopoulos N, J. Clin. Invest, vol. 97, Number 5, pp1335-1343, 1996.

**(20)** Chomzynski P. et N. Sacchi, Analytical Biochemistry 1987, 162, 156-159.
**(21)** F. Mallet et coll., Journal of Clinical Microbiology 1993; 31, 1444-1449.
**(22)** G. Barany and R.B. Merrifielsd, 1980, In the Peptides, 2, 1-284, Gross E and Meienhofer J, Eds Academic Press, New York.
**(23)** Poser et al, Gbers G.C. eds. The diagnosis of multiple sclerosis Thieme Stratton Inc, New York 1984 : 225-229.
**(24)** La Mantia et coll., Nucleic Acid Research 1989, 17, 5913-22.
**(25)** PLAZA, A ; KONO, D.H ; THEOFILOPOULOS, A.N. NEW HUMAN Vβ GENES AND POLYMORPHIC VARIANTS. J. Imm; 147(12): 4360-4365, 1991.

## Revendications

1. Fragment nucléotidique comprenant, ou consistant en une séquence nucléotidique choisie dans le groupe consistant en :

   (a) SEQ ID NO 46, SEQ in NO 51, SEQ ID NO 52, SEQ ID NO 53 et SEQ ID NO 56 ;
   (b) les séquences complémentaires auxdites séquences génomiques.

2. Sonde nucléique de détection d'un agent pathogène et/ou infectant associé à la sclérose en plaques, **caractérisée en ce que** sa séquence nucléotidique consiste en une séquence de 100 monomères contigus d'un fragment choisi parmi les fragments dont les séquences nucléotidiques sont identifiées en SEQ ID NO 46, SEQ ID NO 51, SEQ ID NO 52, SEQ ID NO 53 ou **en ce que** sa séquence nucléotidique consiste en une séquence de 30 ou 100 monomères contigus du fragment dont la séquence nucléotidique est identifiée en SEQ ID NO 56 ; et les séquences nucléotidiques complémentaires desdites séquences.

**3.** Amorce pour l'amplification par polymérisation d'un ARN ou d'un ADN d'un matériel viral, **caractérisée en ce que** sa séquence nucléotidique consiste en une séquence de 30 monomères contigus du fragment dont la séquence nucléotidique est identifiée en SEQ ID NO 56 ; et la séquence nucléotidique complémentaire de ladite séquence.

**4.** Amorce pour l'amplification par polymérisation d'un ARN ou d'un ADN d'un matériel viral, **caractérisée en ce que** sa séquence nucléotidique est identique à SEQ ID NO : 49.

**5.** ARN ou ADN, et notamment vecteur de réplication, comprenant un fragment selon la revendication 1.

**6.** Anticorps mono- ou polyclonal dirigé contre le virus MSRV-1, **caractérisé en ce qu'**il est obtenu par réaction immunologique d'un organisme humain ou animal, à un agent immunogène constitué par un polypeptide antigénique codé par un fragment nucléotidique selon la revendication 1, ledit polypeptide formant ou comprenant un déterminant antigénique reconnu par des sera de patients infectés par le virus MSRV-1, et/ou chez lesquels le virus MSRV-1 a été réactivé.

**7.** Réactif de détection du virus MSRV-1, ou d'une exposition audit virus, **caractérisé en ce qu'**il comprend, à titre de substance réactive, un polypeptide codé par un fragment nucléotidique selon la revendication 1, ledit polypeptide formant ou comprenant un déterminant antigénique reconnu par des sera de patients infectés par le virus MSRV-1, et/ou chez lesquels le virus MSRV-1 a été réactivé, ou un anticorps dudit peptide

**8.** Composition diagnostique, prophylactique, ou thérapeutique, comprenant un polypeptide codé par un fragment nucléotidique selon la revendication 1, ledit polypeptide formant ou comprenant un déterminant antigénique reconnu par des sera de patients infectés par le virus MSRV-1, et/ou chez lesquels le virus MSRV-1 a été réactivé, ou un anticorps dudit peptide, selon la revendication 6.

**9.** Composition immunothérapeutique active, notamment composition vaccinale, selon la revendication 8.

**10.** Composition diagnostique, prophylactique, ou thérapeutique, notamment pour inhiber l'expression d'au moins un agent pathogène et/ou infectant associé à la sclérose en plaques, comprenant un fragment nucléotidique selon la revendication 1.

**11.** Procédé pour détecter un agent pathologique et/ou infectant associé à la sclérose en plaques, dans un échantillon biologique, **caractérisé en ce que** l'on met en contact un ARN et/ou un ADN présumé appartenir ou provenant dudit agent pathologique et/ou infectant, ou leur ARN et/ou ADN complémentaire, avec une composition comprenant un fragment nucléotidique selon la revendication 1.

**12.** Procédé pour détecter la présence ou l'exposition à un agent pathologique et/ou infectant associé à la sclérose en plaques, dans un échantillon biologique, **caractérisé en ce que** l'on met en contact ledit. échantillon avec un polypeptide codé par un fragment nucléotidique selon la revendication 1, ledit polypeptide formant ou comprenant un déterminant antigénique reconnu par des sera de patients infectés par le virus MSRV-1, et/ou chez lesquels le virus MSRV-1 a été réactivé, ou un anticorps dudit peptide, selon la revendication 6.

**13.** Dispositif de détection du virus MSRV-1, comprenant un réactif selon la revendication 7, supporté par un support solide, immunologiquement compatible avec ledit réactif, **caractérisé en ce qu'**il comprend un moyen de mise en contact d'un échantillon biologique, tel qu'un échantillon de sang ou de liquide céphalo-rachidien, susceptible de contenir des anticorps anti-MSRV-1, avec ledit réactif, dans des conditions permettant une éventuelle réaction immunologique, et des moyens de détection du complexe immun formé avec ledit réactif.

**14.** Procédé de détection d'anticorps anti-MSRV-1, dans un échantillon biologique, tel qu'un échantillon de sang ou de liquide céphalo-rachidien, **caractérisé en ce qu'**on met en contact ledit échantillon et un réactif selon la revendication 7, consistant en un anticorps, dans des conditions permettant une éventuelle réaction immunologique, et on détecte ensuite la présence d'un complexe immun formé avec ledit réactif.

**15.** utilisation d'une sonde nucléique pour la détection in vitro d'un agent pathogène et/ou infectant associé à la sclérose en plaques, ladite sonde comprenant de 10 à 100 monomères contigus d'un fragment tel que défini à la revendication 1.

**16.** Utilisation d'une amorce pour l'amplification par polymérisation d'un ARN ou d'un ADN d'un matériel viral associé à la sclérose en plaques, ladite amorce comprenant une séquence nucléotidique de 10 à 30 monomères contigus, identique à la séquence nucléotidique d'un fragment tel que défini à la revendication 1.

## Claims

**1.** Nucleotide fragment comprising, or consisting of, a nucleotide sequence selected from the group consisting of:

(a) SEQ ID NO 46, SEQ ID NO 51, SEQ ID NO 52, SEQ ID NO 53 and SEQ ID NO 56;
(b) the sequences which are complementary to said genomic sequences.

**2.** Nucleic acid probe for detecting a pathogenic and/or infectious agent which is associated with multiple sclerosis, **characterized in that** its nucleotide sequence consists of a sequence of 100 contiguous monomers of a fragment selected among the fragments whose nucleotide sequences are shown in SEQ ID NO 46, SEQ ID NO 51, SEQ ID NO 52, SEQ ID NO 53 or **in that** its nucleotide sequence consists of a sequence of 30 or 100 contiguous monomers of the fragment whose nucleotide sequence is shown in SEQ ID NO 56; and the complementary nucleotide sequences of said sequences.

**3.** Primer for the amplification by polymerization of an RNA or a DNA of a viral material, **characterized in that** its nucleotide sequence consists of a sequence of 30 contiguous monomers of the fragment whose nucleotide sequence is shown in SEQ ID NO 56; and the nucleotide sequence which is complementary to said sequence.

**4.** Primer for the amplification by polymerization of an RNA or a DNA of a viral material, **characterized in that** its nucleotide sequence is identical to SEQ ID NO 49.

**5.** RNA or DNA, in particular replication vector, comprising a fragment according to Claim 1.

**6.** Mono- or polyclonal antibody against the MSRV-1 virus, **characterized in that** it is obtained by immunological reaction of a human or animal organism to an immunogenic agent composed of an antigenic polypeptide encoded by a nucleotide fragment according to Claim 1, said polypeptide forming, or comprising, an antigenic determinant which is recognized by sera from patients who are infected with the MSPV-1 virus and/or in which the MSRV-1 virus has been reactivated.

**7.** Reagent for detecting the MSRV-1 virus or exposure to said virus **characterized in that** it comprises, by way of reactive substance, a polypeptide encoded by a nucleotide fragment according to Claim 1, said polypeptide forming, or comprising, an antigenic determinant which is recognized by sera from patients who are infected with the MSRV-1 virus and/or in which the MSRV-1 virus has been reactivated, or an antibody of said peptide.

**8.** Diagnostic, prophylactic or therapeutic composition comprising a polypeptide encoded by a nucleotide fragment according to Claim 1, said polypeptide forming, or comprising, an antigenic determinant which is recognized by sera from patients who are infected with the MSRV-1 virus and/or in which the MSRV-1 virus has been reactivated, or an antibody of said peptide, according to Claim 6.

**9.** Immunotherapeutically active composition, in particular vaccine composition, according to Claim 8.

**10.** Diagnostic, prophylactic or therapeutic composition, in particular for inhibiting the expression of at least one pathogenic and/or infectious agent associated with multiple sclerosis, comprising a nucleotide fragment according to Claim 1.

**11.** Method of detecting, in a biological sample, a pathologic and/or infectious agent associated with multiple sclerosis, **characterized in that** an RNA and/or a DNA, to which it is assumed that it belongs, or originates from said pathologic and/or infectious agent, or their complementary RNA and/or DNA, are brought into contact with a composition comprising a nucleotide fragment according to Claim 1.

**12.** Method of detecting, in a biological sample, the presence of, or exposure to, a pathologic and/or infectious agent associated with multiple sclerosis, **characterized in that** said sample is brought into contact with a polypeptide encoded by a nucleotide fragment according to Claim 1, said polypeptide forming, or comprising, an antigenic determinant which is recognized by sera from patients who are infected with the MSRV-1 virus and/or in which the MSRV-1 virus has been reactivated, or an antibody of said peptide, according to Claim 6.

**13.** Device for detecting MSRV-1 virus, comprising a reagent according to Claim 7 which is supported by a solid carrier which is immunologically compatible with said reagent, **characterized in that** the device comprises a means of bringing a biological sample, such as a blood or cerebrospinal liquid sample, which may contain anti-MSRV-1 antibodies, into contact with said reagent under conditions which permit a possible immunological reaction, and means of detecting the immune complex formed with said reagent.

**14.** Method of detecting anti-MSRV-1 antibodies in a biological sample, such as a blood sample or a cerebrospinal fluid sample, **characterized in that** said sample is brought into contact with a reagent according to Claim 7, consisting of an antibody, under conditions which permit a possible immunological reaction, and that the preserce of an immune complex formed with said reagent is subsequently detected.

**15.** Use of a nucleic acid probe for the in-vitro-detection of a pathogenic and/or infectious agent associated with multiple sclerosis, said probe comprising 10 to 100 contiguous monomers of a fragment as defined in Claim 1.

**16.** Use of a primer for the amplification by polymerization of an RNA or a DNA of a viral material associated with multiple sclerosis, said primer comprising a nucleotide sequence of 10 to 30 contiguous monomers which is identical to the nucleotide sequence of a fragment as defined in Claim 1.

## Patentansprüche

**1.** Nukleotidfragment, das eine Nukleotidsequenz aus der Gruppe

(a) SEQ ID NR. 46, SEQ ID NR. 51, SEQ ID NR. 52, SEQ ID NR. 53 und SEQ ID NR. 56;
(b) die zu diesen genomischen Sequenzen komplementären Sequenzen

enthält bzw. aus solch einer Nukleotidsequenz besteht.

**2.** Nukleinsäuresonde für den Nachweis eines pathogenen und/oder infektiösen Agens, das mit multipler Sklerose assoziiert ist, **dadurch gekennzeichnet, daß** ihre Nukleotidsequenz aus einer Sequenz von 100 aufeinanderfolgenden Monomeren eines Fragments aus der Reihe der Fragmente, deren Nukleotidsequenzen in SEQ ID NR. 46, SEQ ID NR. 51, SEQ ID NR. 52, SEQ ID NR. 53 angegeben sind, besteht, oder **dadurch**, daß ihre Nukleotidsequenz aus einer Sequenz von 30 oder 100 aufeinanderfolgenden Monomeren des Fragments, dessen Nukleotidsequenz in SEQ ID NR. 56 angegeben ist, besteht; sowie den zu diesen Sequenzen komplementären Nukleotidsequenzen.

**3.** Primer für die Amplifikation mittels Polymerisation einer RNA oder einer DNA eines Virusmaterials, **dadurch gekennzeichnet, daß** seine Nukleotidsequenz aus einer Sequenz von 30 aufeinanderfolgenden Monomeren des Fragments, dessen Nukleotidsequenz in SEQ ID NR. 56 angegeben ist, besteht; und der zu dieser Sequenz komplementären Nukleotidsequenz.

**4.** Primer für die Amplifikation mittels Polymerisation einer RNA oder einer DNA eines Virusmaterials, **dadurch gekennzeichnet, daß** seine Nukleotidsequenz mit der SEQ ID NR. 49 identisch ist.

**5.** RNA oder DNA, insbesondere Replikationsvektor, die/der ein Fragment nach Anspruch 1 enthält.

**6.** Monoklonaler oder Polyklonaler Antikörper gegen das MSRV-I-Virus, **dadurch gekennzeichnet, daß** er durch immunologische Reaktion eines menschlichen oder tierischen Organismus auf ein immunogenes Agens erhalten wird, das aus einem antigenen Polypeptid besteht, das von einem Nukleotidfragment nach Anspruch 1 kodiert wird, wobei das Polypeptid eine antigene Determinante, die von Seren von mit MSRV-1-Virus infizierten Patienten und/oder von Patienten, bei denen das MSRV-1-Virus reaktiviert worden ist, erkannt wird, bildet oder enthält.

**7.** Nachweisreagens für das MSRV-1-Virus oder für Kontakt mit diesem Virus, **dadurch gekennzeichnet, daß** es als Reagenswirkstoff ein Polypeptid, das von einem Nukleotidfragment nach Anspruch 1 kodiert wird, wobei das Polypeptid eine antigene Determinante, die von Seren von mit MSRV-1-Virus infizierten Patienten und/oder von Patienten, bei denen das MSRV-1-Virus reaktiviert worden ist, erkannt wird, bildet oder enthält, oder einen Antikörper gegen dieses Peptid enthält.

**8.** Diagnostische, prophylaktische oder therapeutische Zusammensetzung, die ein Polypeptid, das von einem Nukleotidfragment nach Anspruch 1 kodiert wird, wobei das Polypeptid eine antigene Determinante, die von Seren von mit MSRV-1-Virus infizierten Patienten und/oder bei denen das MSRV-1-Virus reaktiviert worden ist, erkannt wird, bildet oder enthält, oder einen Antikörper gegen dieses Peptid nach Anspruch 6 enthält.

**9.** Immuntherapeutisch aktive Zusammensetzung, insbesondere Impfstoffzusammensetzung, nach Anspruch B.

**10.** Diagnostische, prophylaktische oder therapeutische Zusammensetzung, insbesondere zum Hemmen der Expression mindestens eines pathogenen und/oder infektiösen Agens, das mit multipler Sklerose assoziiert ist, die ein Nukleotidfragment nach Anspruch L enthält.

**11.** Verfahren zum Nachweisen eines pathologischen und/oder infektiösen Agens, das mit multipler Sklerose assoziiert ist, in einer biologischen Probe, **dadurch gekennzeichnet, daß** man eine RNA und/oder eine DNA, von der angenommen wird, daß sie zu diesem pathologischen und/oder infektiösen Agens gehört oder von diesem stammt, oder deren komplementäre RNA und/oder DNA, mit einer Zusammensetzung, die ein Nukleotidfragment nach Anspruch 1 enthält, in Kontakt bringt.

**12.** Verfahren zum Nachweisen des Vorhandenseins oder des Kontakts mit einem pathologischen und/oder infektiösen Agens, das mit multipler Sklerose assoziiert ist, in einer biologischen Probe, **dadurch gekennzeichnet, daß** man die Probe mit einem Polypeptid in Kontakt bringt, das von einem Nukleotidfragment nach Anspruch 1 kodiert wird, wobei das Polypeptid eine antigene Determinante, die von Seren von mit MSRV-1-Virus infizierten Patienten und/oder von Patienten, bei denen das MSRV-1-Virus reaktiviert worden ist, erkannt wird, bildet oder enthält, oder einen Antikörper gegen dieses Peptid nach Anspruch 6 enthält.

**13.** Vorrichtung zum Nachweisen des MSRV-1-Virus, das ein Reagens nach Anspruch 7 enthält, das sich auf einem festen Träger, der immunologisch mit dem Reagens kompatibel ist, befindet, **dadurch gekennzeichnet, daß** sie ein Mittel zum in-Kontaktbringen einer biologischen Probe, wie einer Blutprobe oder einer Cerebrospinalflüssigkeitsprobe, die anti-MSRV-1-Antikörper enthalten kann, mit dem Reagens, und zwar unter Bedingungen, die eine mögliche Immunreaktion ermöglichen, sowie Mittel zum Nachweisen des mit dem Reagens gebildeten Immunkomplexes enthält.

**14.** Verfahren zum Nachweisen von anti-MSRV-1-Antikörpern in einer biologischen Probe, wie einer Blutprobe oder einer Cerebrospiralflüssigkeitsprobe, **dadurch gekennzeichnet, daß** man die Probe und ein Reagens nach Anspruch 7, das aus einem Antikörper besteht, in Kontakt bringt, und zwar unter Bedingungen, die eine mögliche Immunreaktion ermöglichen, und man anschließend das Vorliegen eines mit dem Reagens gebildeten Immunkomplexes nachweist.

**15.** Verwendung einer Nukleinsäuresonde zum in-vitro-Nachweisen eines pathogenen und/oder infektiösen Agens, das mit Mutipler Sklerose assoziiert ist, wobei die Sonde 10 bis 100 aufeinanderfolgende Monomere eines Fragments wie es in Anspruch 1 definiert ist enthält.

**16.** Verwendung eines Primers für die Amplifikation durch Polymerisation einer RNA oder einer DNA eines Virusmaterials, das mit Mutipler Sklerose assoziiert ist, wobei der Primer eine Nukleotidsequenz von 10 bis 30 aufeinanderfolgenden Monomeren, die mit der Nukleotidsequenz eines Fragments wie es in Anspruch 1 definiert ist identisch ist, enthält.

FIG. 1

Consensus GTTTAGGGAT ANCCCTCATC TCTTTGGTCA GGTACTGGCC CAAGATCTAG 50

Consensus GCCACTTCTC AGGTCCAGSN ACTCTGTYCC TTCAG 85

SEQ ID NO3 (POL MSRV-1B)

Consensus GTTCAGGGAT AGCCCCCATC TATTTGGCCA GGCACTAGCT CAATACTTGA 50

Consensus GCCAGTTCTC ATACCTGGAC AYTCTYGTCC TTGGGT 86

SEQ ID NO4 (POL MSRV-1B)

Consensus GTTCARRGAT AGCCCCCATC TATTTGGCCW RGYATTAGCC CAAGACTTGA 50

Consensus GYCAATTCTC ATACCTGGAC ACTCTTGTCC TTYRG 85

SEQ ID NO5 (POL MSRV-1B)

Consensus GTTCAGGGAT AGCTCCCATC TATTTGGCCT GGCATTAACC CGAGACTTAA 50

Consensus GCCAGTTCTY ATACGTGGAC ACTCTTGTCC TTTGG 85

SEQ ID NO6 (POL MSRV-1B)

Consensus GTGTTGCCAC AGGGGTTTAR RGATANCYCY CATCTMTTTG GYCWRGYAYT

Consensus RRCYCRAKAY YTRRGYCAVT TCTYAKRYSY RGSNAYTCTB KYCCTTYRGT

Consensus ACATGGATGA C

SEQ ID NO7 (POL MSRV-1B)

FIG. 2

CONSENSUS A SEQ ID NO 3

```
GTTTAGGGATAGCCC TCATCTCTTGGTCA GGTACTGGCCCAAGA TCTAGGCCACTTCTC   60
V . G . P  S S L W S  G T G P R  S R P L L
 F R D S P  H L F G Q  V L A Q D  L G H F S
  L G I A L  I S L V R  Y W P K I  . A T S Q

AGGTCCAGGCACTCT GTTCCTTCAG                                        85
R S R H S  V P S
 G P G T L  F L Q
  V Q A L C  S F
```

CONSENSUS B SEQ ID NO 4

```
GTTCAGGGATAGCCC CCATCTATTTGGCCA GGCACTAGCTCAATA CTTGAGCCAGTTCTC   60
V Q G . P  P S I W P  G T S S I  L E P V L
 F R D S P  H L F G Q  A L A Q Y  L S Q F S
  S G I A P  I Y L A R  H . L N T  . A S S H

ATACCTGGACACTCT TGTCCTTCGGT                                       86
I P G H S  C P S
 Y L D T L  V L R
  T W T L L  S F G
```

CONSENSUS C SEQ ID NO 5

```
GTTCAGGGATAGCCC CCATCTATTTGGCCA GGCATTAGCCCAAGA CTTGAGTCAATTCTC   60
V Q G . P  P S I W P  G I S P R  L E S I L
 F R D S P  H L F G Q  A L A Q D  L S Q F S
  S G I A P  I Y L A R  H . P K T  . V N S H

ATACCTGGACACTCT TGTCCTTCAG                                        85
I P G H S  C P S
 Y L D T L  V L Q
  T W T L L  S F
```

CONSENSUS D SEQ ID NO 6

```
GTTCAGGGATAGCTC CCATCTATTTGGCCT GGCATTAACCCGAGA CTTAAGCCAGTTCTC   60
V Q G . L  P S I W P  G I N P R  L K P V L
 F R D S S  H L F G L  A L T R D  L S Q F S
  S G I A P  I Y L A W  H . P E T  . A S S H

ATACGTGGACACTCT TGTCCTTTGG                                        85
I R G H S  C P L
 Y V D T L  V L W
      T L L  S F
```

FIG. 3

Consensus TTGGATCCAG TGYTGCCACA GGGCGCTGAA GCCTATCGCG TGCAGTTGCC 50

Consensus GGATGCCGCC TATAGCCTCT ACGTGGATGA CCTSCTGAAG CTTGAG 96

SEQ ID NO 11

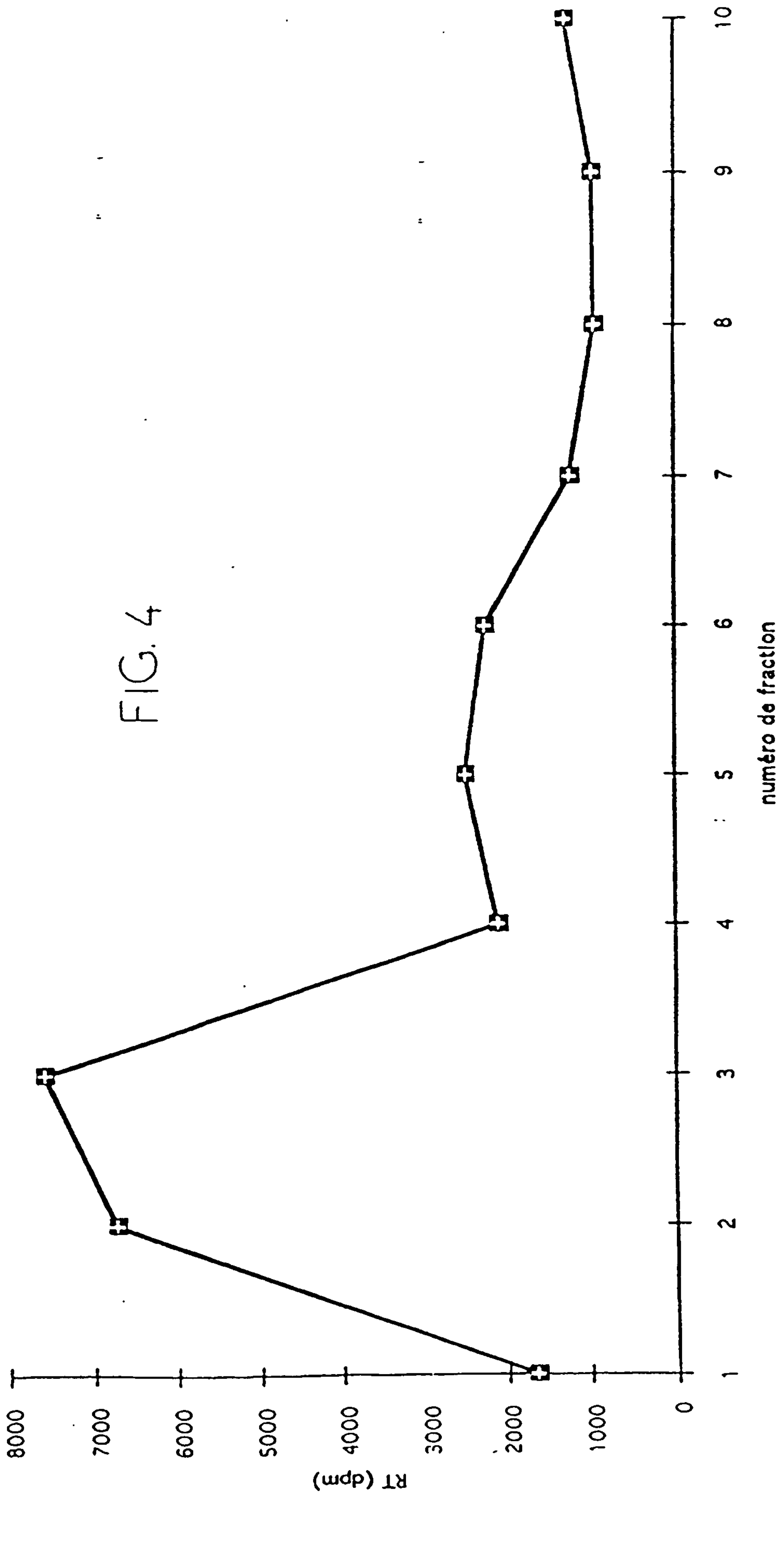
RT (dpm)
8000
7000
6000
5000
4000
3000
2000
1000
0
1
2
3
4
5
6
7
8
9
10
numéro de fraction

FIG. 4

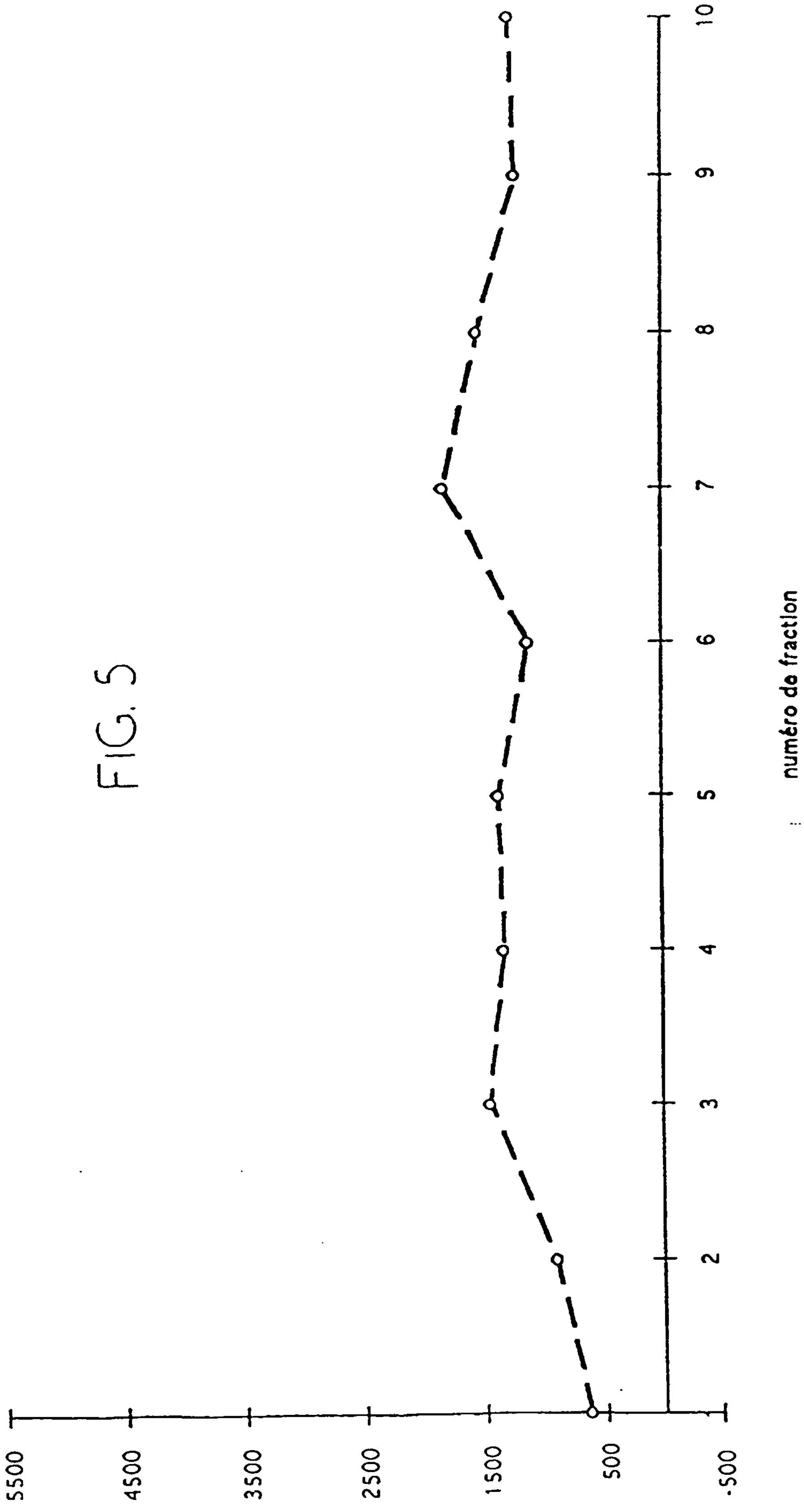
5500
4500
3500
2500
1500
500
-500
RT (dpm)
2
3
4
5
6
7
8
9
10
numéro de fraction

FIG. 5

FIG. 6

CAAGCCACCC AAGAACTCTT AAATTTCCTC ACTACCTGTG GCTACAAGGT 50
TTCCAAACCA AAGGCTCAGC TCTGCTCACA GGAGATTAGA TACTTAGGGT 100
TAAAATTATC CAAAGGCACC AGGGGCCTCA GTGAGGAACG TATCCAGCCT 150
ATACTGGGTT ATCCTCATCC CAAAACCCTA AAGCAACTAA GAGGGTTCCT 200
TAGCATGATC AGGTTTCTGC CGAAAACAAG ATTCCCAGGT ACAACCAAAA 250
TAGCCAGACC ATTATATACA CTAATTAAGG AAACTCAGAA AGCCAATACC 300
TATTTAGTAA GATGGACACC TAAACAGAAG GCTTTCCAGG CCCTAAAGAA 350
GGCCCTAACC CAAGCCCCAG TGTTCAGCTT GCCAACAGGG CAAGATTTTT 400
CTTTATATGG CACAGAAAAA ACAGGAATCG CTCTAGGAGT CCTTACACAG 450
GTCCGAGGGA TGAGCTTGCA ACCCGTGGCA TACCTGAATA AGGAAATTGA 500
TGTAGTGGCA AAGGGTTGGC CTCATNGTTT ATGGGTAATG GNGGCAGTAG 550
CAGTCTNAGT ATCTGAAGCA GTTAAAATAA TACAGGGAAG AGATCTTNCT 600
GTGTGGACAT CTCATGATGT GAACGGCATA CTCACTGCTA AAGGAGACTT 650
GTGGTTGTCA GACAACCATT TACTTAANTA TCAGGCTCTA TTACTTGAAG 700
AGCCAGTGCT GNGACTGCGC ACTTGTGCAA CTCTTAAACC C 741

SEQ ID NO9 (PSJ 17)

TCAGGGATAGCCCCCATCTATTTGGCCAGGCATTAGCCCAAGACTTGAGTC
AATTCTCATACCTGGACACTCTTGTCCTTCAGTACATGGATGATTTACTTT
TAGTCGCCCGTTCAGAAACCTTGTGCCATCAAGCCACCCAAGAACTCTTAA
CTTTCCTCACTACCTGTGGCTACAAGGTTTCCAAACCAAAGGCTCGGCTCT
GCTCACAGGAGATTAGATACTNAGGGCTAAAATTATCCAAAGGCACCAGG
GCCCTCAGTGAGGAACGTATCCAGCCTATACTGGCTTATCCTCATCCCAAA
ACCCTAAAGCAACTAAGAGGGTTCCTTGGCATAACAGGTTTCTGCCGAAA
ACAGATTCCCAGGTACASCCCAATAGCCAGACCATTATATACACTAATTA
NGGAAACTCAGAAAGCCAATACCTATTTAGTAAGATGGACACCTACAGAA
GTGGCTTTCCAGGCCCTAAAGAAGGCCCTAACCCAAGCCCCAGTGTTCAGC
TTGCCAACAGGGCAAGATTTTTCTTTATATGCCACAGAAAAAACAGGAAT
AGCTCTAGGAGTCCTTACGCAGGTCTCAGGGATGAGCTTGCAACCCGTGGT
ATACCTGAGTAAGGAAATTGATGTAGTGGCAAAGGGTT

SEQ ID NO 8 (MOO3-POO4)

FIG. 7

FIG. 8

SEQ ID NO 2 (F11-1)

```
         10        20        30         40        50        60         70
          *         *         *          *         *         *          *
CCC TTT GCC ACT ACA TCA ATT TTA GGA GTA AGG AAA CCC AAC GGA CAG TGG AGG TTA GTG CAA GAA CTC AGG
 P   F   A   T   T   S   I   L   G   V   R   K   P   N   G   Q   W   R   L   V   Q   E   L  R>
___a___a___a___a___a___a___a___a__TRANSLATION OF F11-1 (A)___a___a___a___a___a___a___a___a___>

       80        90        100        110       120        130       140
        *         *          *          *         *          *         *
ATT ATC AAT GAG GCT GTT GTT CCT CTA TAC CCA GCT GTA CCT AAC CCT TAT ACA GTG CTT TCC CAA ATA CCA
 I   I   N   E   A   V   V   P   L   Y   P   A   V   P   N   P   Y   T   V   L   S   Q   I  P>
___a___a___a___a___a___a___a___a__TRANSLATION OF F11-1 (A)___a___a___a___a___a___a___a___a___>

  150       160       170        180        190       200       210
   *         *         *          *           *         *         *
GAG GAA GCA GAG TGG TTT ACA GTC CTG GAC CTT AAG GAT GCC TTT TTC TGC ATC CCT GTA CGT CCT GAC TCT
 E   E   A   E   W   F   T   V   L   D   L   K   D   A   F   F   C   I   P   V   R   P   D  S>
___a___a___a___a___a___a___a___a__TRANSLATION OF F11-1 (A)___a___a___a___a___a___a___a___a___>

 220       230       240        250       260       270       280
  *         *         *          *         *         *         *
CAA TTC TTG TTT GCC TTT GAA GAT CCT TTG AAC CCA ACG TCT CAA CTC ACC TGG ACT GTT TTA CCC CAA GGG
 Q   F   L   F   A   F   E   D   P   L   N   P   T   S   Q   L   T   W   T   V   L   P   Q  G>
___a___a___a___a___a___a___a___a__TRANSLATION OF F11-1 (A)___a___a___a___a___a___a___a___a___>

290
 *
TTC AAG GGA
 F   K   G>
___a___a___>
```

FIG. 9

SEQ ID NO 1 (MSRV-1 pol)

```
        10          20          30          40          50          60          70
CCC TTT GCC ACT ACA TCA ATT TTA GGA GTA AGG AAA CCC AAC GGA CAG TGG AGG TTA GTG CAA GAA CTC AGG
 P   F   A   T   T   S   I   L   G   V   R   K   P   N   G   Q   W   R   L   V   Q   E   L   R>
__a__a__a__a__a__a__a__TRANSLATION OF MSRV-1 POL • (A)__a__a__a__a__a__a__a__>
        80          90         100         110         120         130         140
ATT ATC AAT GAG GCT GTT GTT CCT CTA TAC CCA GCT GTA CCT AAC CCT TAT ACA GTG CTT TCC CAA ATA CCA
 I   I   N   E   A   V   V   P   L   Y   P   A   V   P   N   P   Y   T   V   L   S   Q   I   P>
__a__a__a__a__a__a__a__TRANSLATION OF MSRV-1 POL • (A)__a__a__a__a__a__a__a__>
       150         160         170    A    180         190         200         210
GAG GAA GCA GAG TGG TTT ACA GTC CTG GAC CTT AAG|GAT GCC TTT TTC TGC ATC CCT GTA CGT CCT GAC TCT
 E   E   A   E   W   F   T   V   L   D   L   K | D   A   F   F   C   I   P   V   R   P   D   S>
__a__a__a__a__a__a__a__TRANSLATION OF MSRV-1 POL • (A)__a__a__a__a__a__a__a__>
       220         230         240         250         260         270         280
CAA TTC TTG TTT GCC TTT GAA GAT CCT TTG AAC CCA ACG TCT CAA CTC ACC TGG ACT|GTT TTA CCC CAA GGG|
 Q   F   L   F   A   F   E   D   P   L   N   P   T   S   Q   L   T   W   T | V   L   P   Q   G>|
__a__a__a__a__a__a__a__TRANSLATION OF MSRV-1 POL • (A)__a__a__a__a__a__a__a__>
290         300         310         320         330|  B   340         350         360
TTC AGG GAT AGC CCC CAT CTA TTT GGC CAG GCA TTA GCC CAA|GAC TTG AGT CAA TTC TCA TAC CTG GAC ACT
 F   R   D   S   P   H   L   F   G   Q   A   L   A   Q | D   L   S   Q   F   S   Y   L   D   T>
__a__a__a__a__a__a__a__TRANSLATION OF MSRV-1 POL • (A)__a__a__a__a__a__a__a__>
       370         380         390         400         410         420         430
CTT GTC CTT CAG|TAC ATG GAT GAT|TTA CTT TTA GTC GCC CGT TCA GAA ACC TTG TGC CAT CAA GCC ACC CAA
 L   V   L   Q | Y   M   D   D | L   L   L   V   A   R   S   E   T   L   C   H   Q   A   T   Q>
__a__a__a__a__a__a__a__TRANSLATION OF MSRV-1 POL • (A)__a__a__a__a__a__a__a__>
       440         450         460         470         480         490         500
GAA CTC TTA ACT TTC CTC ACT ACC TGT GGC TAC AAG GTT TCC AAA CCA AAG GCT CGG CTC TGC TCA CAG GAG
 E   L   L   T   F   L   T   T   C   G   Y   K   V   S   K   P   K   A   R   L   C   S   Q   E>
__a__a__a__a__a__a__a__TRANSLATION OF MSRV-1 POL • (A)__a__a__a__a__a__a__a__>
       510         520         530         540         550         560         570
ATT AGA TAC TNA GGG CTA AAA TTA TCC AAA GGC ACC AGG GCC CTC AGT GAG GAA CGT ATC CAG CCT ATA CTG
 I   R   Y   X   G   L   K   L   S   K   G   T   R   A   L   S   E   E   R   I   Q   P   I   L>
__a__a__a__a__a__a__a__TRANSLATION OF MSRV-1 POL • (A)__a__a__a__a__a__a__a__>
       580         590         600         610         620         630         640
GCT TAT CCT CAT CCC AAA ACC CTA AAG CAA CTA AGA GGG TTC CTT GGC ATA ACA GGT TTC TGC CGA AAA CAG
 A   Y   P   H   P   K   T   L   K   Q   L   R   G   F   L   G   I   T   G   F   C   R   K   Q>
__a__a__a__a__a__a__a__TRANSLATION OF MSRV-1 POL • (A)__a__a__a__a__a__a__a__>
650         660         670         680         690         700         710         720
ATT CCC AGG TAC ASC CCA ATA GCC AGA CCA TTA TAT ACA CTA ATT ANG GAA ACT CAG AAA GCC AAT ACC TAT
 I   P   R   Y   X   P   I   A   R   P   L   Y   T   L   I   X   E   T   Q   K   A   N   T   Y>
__a__a__a__a__a__a__a__TRANSLATION OF MSRV-1 POL • (A)__a__a__a__a__a__a__a__>
       730         740         750         760         770         780         790
TTA GTA AGA TGG ACA CCT ACA GAA GTG GCT TTC CAG GCC CTA AAG AAG GCC CTA ACC CAA GCC CCA GTG TTC
 L   V   R   W   T   P   T   E   V   A   F   Q   A   L   K   K   A   L   T   Q   A   P   V   F>
__a__a__a__a__a__a__a__TRANSLATION OF MSRV-1 POL • (A)__a__a__a__a__a__a__a__>
       800         810         820         830         840         850         860
AGC TTG CCA ACA GGG CAA GAT TTT TCT TTA TAT GCC ACA GAA AAA ACA GGA ATA GCT CTA GGA GTC CTT ACG
 S   L   P   T   G   Q   D   F   S   L   Y   A   T   E   K   T   G   I   A   L   G   V   L   T>
__a__a__a__a__a__a__a__TRANSLATION OF MSRV-1 POL • (A)__a__a__a__a__a__a__a__>
       870         880         890         900         910         920         930
CAG GTC TCA GGG ATG AGC TTG CAA CCC GTG GTA TAC CTG AGT AAG GAA ATT GAT GTA GTG GCA AAG GGT TGG
 Q   V   S   G   M   S   L   Q   P   V   V   Y   L   S   K   E   I   D   V   V   A   K   G   W>
__a__a__a__a__a__a__a__TRANSLATION OF MSRV-1 POL • (A)__a__a__a__a__a__a__a__>
       940         950         960         970         980         990        1000
CCT CAT NGT TTA TGG GTA ATG GNG GCA GTA GCA GTC TNA GTA TCT GAA GCA GTT AAA ATA ATA CAG GGA AGA
 P   H   X   L   W   V   M   X   A   V   A   V   X   V   S   E   A   V   K   I   I   Q   G   R>
__a__a__a__a__a__a__a__TRANSLATION OF MSRV-1 POL • (A)__a__a__a__a__a__a__a__>
1010        1020        1030        1040        1050        1060        1070        1080
GAT CTT NCT GTG TGG ACA TCT CAT GAT GTG AAC GGC ATA CTC ACT GCT AAA GGA GAC TTG TGG TTG TCA GAC
 D   L   X   V   W   T   S   H   D   V   N   G   I   L   T   A   K   G   D   L   W   L   S   D>
__a__a__a__a__a__a__a__TRANSLATION OF MSRV-1 POL • (A)__a__a__a__a__a__a__a__>
      1090        1100        1110        1120        1130        1140        1150
AAC CAT TTA CTT AAN TAT CAG GCT CTA TTA CTT GAA GAG CCA GTG CTG NGA CTG CGC ACT TGT GCA ACT CTT
 N   H   L   L   X   Y   Q   A   L   L   L   E   E   P   V   L   X   L   R   T   C   A   T   L>
__a__a__a__a__a__a__a__TRANSLATION OF MSRV-1 POL • (A)__a__a__a__a__a__a__a__>
AAA CCC
 K   P>
__a__>
```

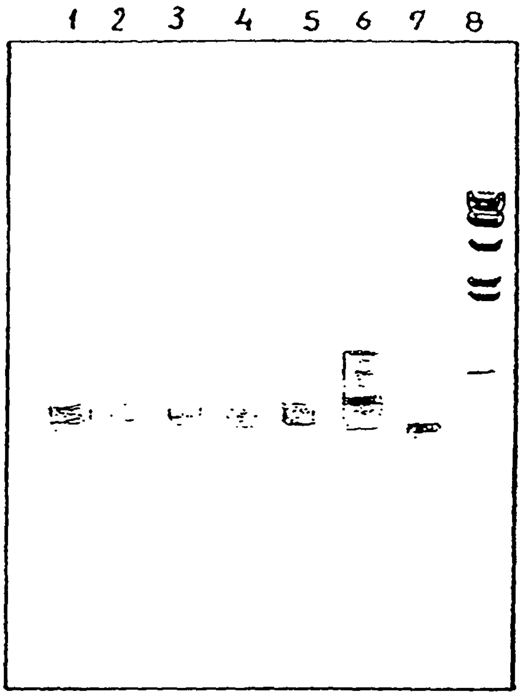


FIG. 10

FIG. 11

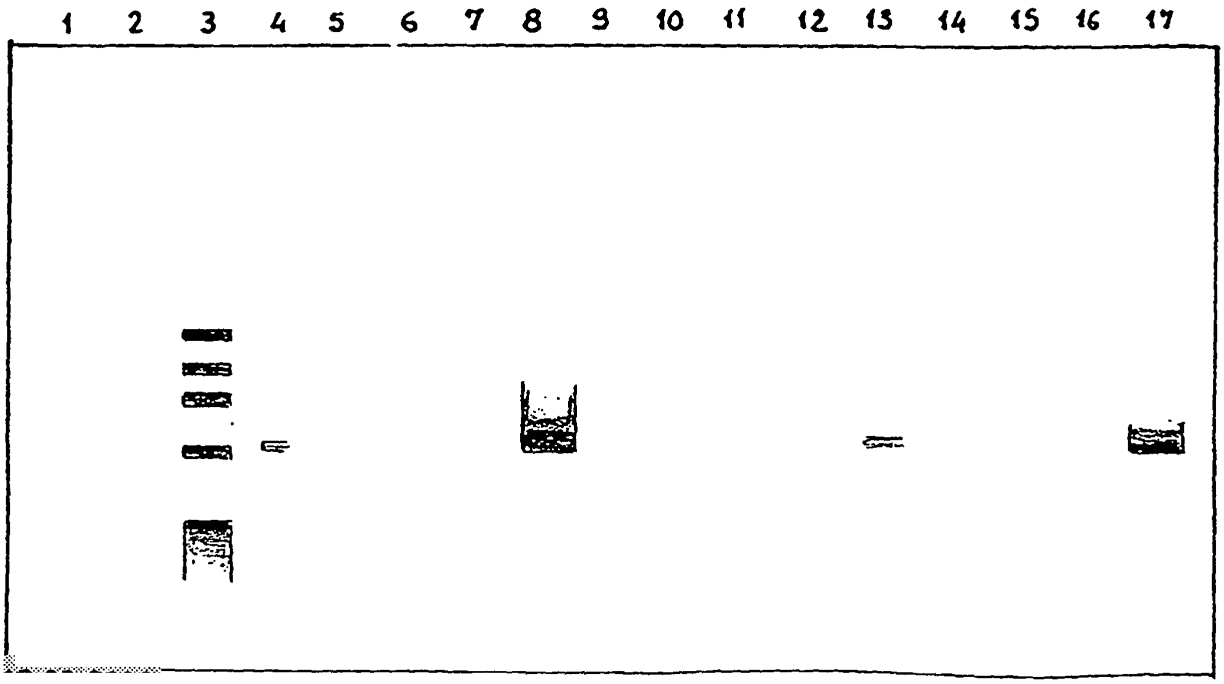

FIG. 12

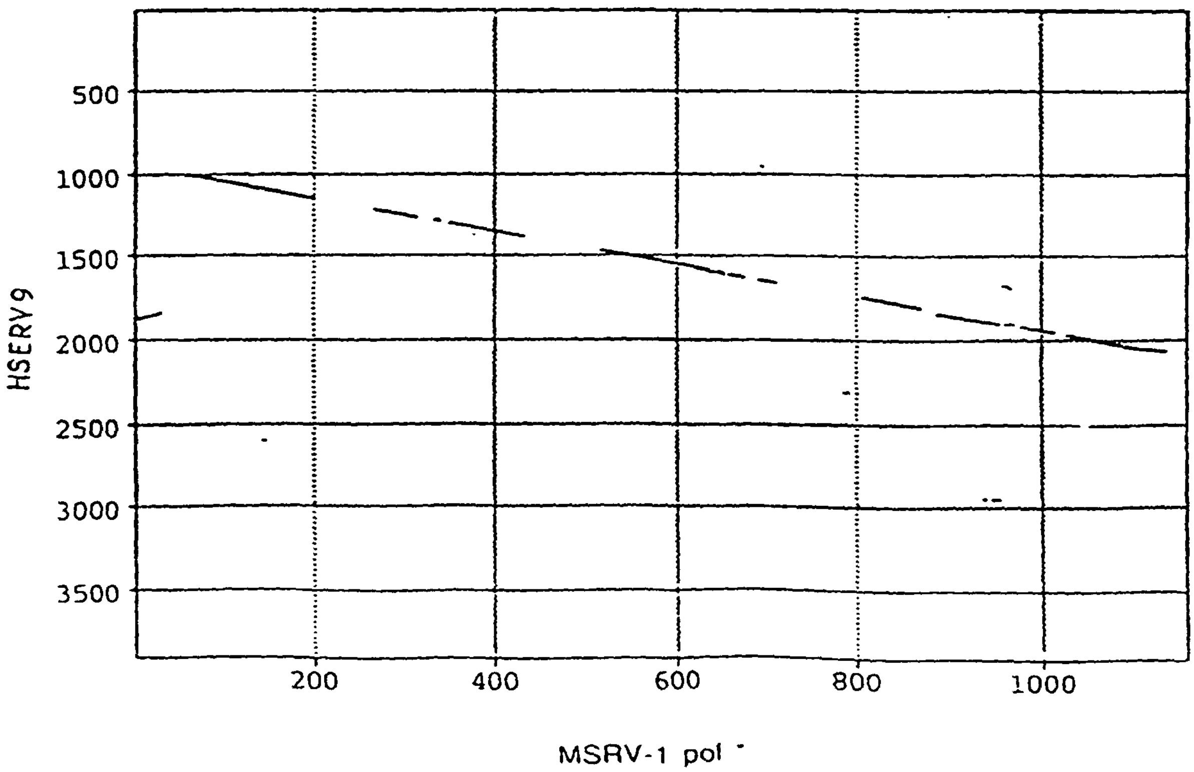
500
1000
1500
2000
2500
3000
3500
HSERV9
200
400
600
800
1000
MSRV-1 pol

FIG. 13

SEQ ID NO 46 (FBd3)

GTGCTGATTGGTGTATTTACAATCCTTTATCTAATCCGAAATGCCCATGTTG
CAATATGGAAAGAAAGGGAGTTCCTAACCTCTGGGGGAACCCCCATTAAA
TACCACAAGTAAATCATGGAGTTATTGCACACAGTGCAAAAACTCAAGGA
GGTGGAAGTCTTACACTGCCAAAGCCATCAGAAAAGGGAAGAGGGGAGAA
GAGCAGCATAAGTGGCTACAGAGGCAAGGAAAGACTAGCAGAAAGGAAA
GAGAGAAAGAGACAGAAAGTCAGAGAGAGAGAGAGGAAGAGACAGAGCA
CAAAGAGGGAGTCAGAGAGAGAGAGAGACAGAGAGTCAGAGAGAAGGAA
AGAGAGAGAGGAAGAGACAAAGAATGAATCAAACAGAGAGACAGAAAGT
CAGAGAGAGAGAGAGAGAGGAAGAGACAGAGAAAAAGAGGGAGTCAGAA
AAAGAGAGACCAAAGAAGAAGTCCAAAGAGAAAGAAAGAGAGATGGAAG
TAGTAAAGGAAAAACAGTGTACCCTATTCCTTTAAAAGCCGGGGTAAATTT
AAAACCTATAATTGATAACTGAAGGTCTTCTCTGTAACCCTGTAACACTCC
AATACCACCTTGTTGTCAAGTGTAAACAAGGGCGTAGCCCAAAAGCACTG
AGGCCACTAACAACCCATAGCCTTCCTATCAAAATTCCTTAACCCAGCAGG
TTTCCTAACAGGGGATCTAAATCTTAATTAATTACCATACAATGGTCCAAC
CAGACTTAGGAGGAATTCCCTTCAGGACGGGAAGATAGATGCTTCCTCCCA
GGCGATTAAGGGAGAAAGACACAATGGGTATTCAGTAAGTGCCAAGGGGA
ACACTTGTAGAAGCAAAGTTAGGAAAATTGCCAAATAATTGGTTTGCTCAA
GAGTTGTTTGCACTCAGCCAAACCTTGAAGTACTTGCAGAATCAGAAAGGA
GCCATCTATACCAATTCTAAGTTAATATGGACTGAAGGAGGTTTTATTAAT
ACCAAAGAGAAATTAAAATCCCAAACTTATAAGGTTTTCAACCAAAGTAA
AGTTTGCTAAAAGTTAACAGCGTAACATGTATTATCCTACTACCACACACT
CTCAAAGGATTTCTCAGACAGTTTGCAAGAAATAATGATATCTATCCTTAC
TCTACAATCCCAAATAGACTCTTTGGCAGCAGTGACTCTCCAAAACCGTCA
AGGCCTAGACCTCCTCACTGCTGAGAAAGGAGGACTCTGCACCTTCTTAAG
GGAAGAGTGTTGTCTTTACACTAACCAGTCAGGGATAGTATGAGATGCTGC
CCGGCATTTACAGAAAAAGGCTTCTGAAATCAGACAACGCCTTTCAAATTC
CTATACCAACCTCTGGAGTTGGGCAACATGGTTTCTTCCCTTTCTATGTCCC
ATGGCTGCCATCTTGCTATTACTCGCCTTTGGGCCCTGTATTTTTAACCTCC
TTGTCAAATTTGTTTCTTCTAGGATCGAGGCCATCAAGCTACAGATGGTCTT
ACAAATGGAACCCCAAATGAGCTCAACTATCAACTTCTACTGAGGACCCCT
AGACCAACCCCCTGGCCCTTTCACTGGCCTAAAGAGTTCCCGTCTGGAGGA
CACTACCACTGCAGGGCCCCATCTTTGCCCCTATCCAGAAGGAAGTAGCTA
GAGCAGTCATTGCCCAATTCCCAAGAGCAGCTGGGGTGTCCCGTTTAGAGT
GGGGATTGAGAGGTGAAGCCAGCTGGACTTCTGGGTCGGGTGGGGACTTG
GAGAACTTTTGTGTCTAGCTAAAGGATTGTAAATGCAACAATCAGTGCTCT
GTGTCTAGCTAAAGGATTGTAAATACACCAATCAGCAC

FIG. 14

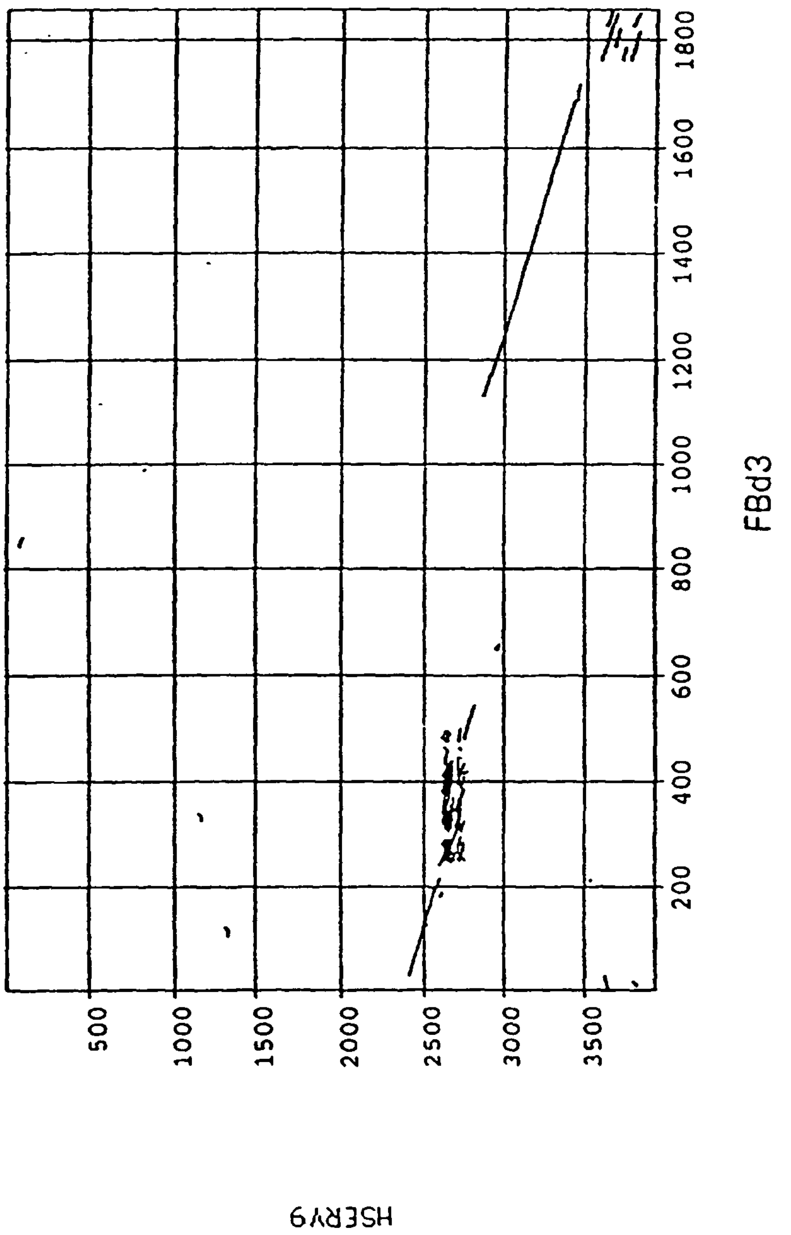

200
400
600
800
1000
1200
1400
1600
1800
FBd3
500
1000
1500
2000
2500
3000
3500
HSERY9

FIG. 15

SEQ ID NO 51 ( t pol)

GGCTGCTAAAGGAGACTTGTGGTTGTCAGACAATCGCCTACTTAGGTACCA
GGCCTTATTACTTGAGGGACTGGTGCTTCAGATGCGCACTTGTGCAGCTCT
TAACCCAAACTTATGCTGCCCAGAAGGATCTTTTAGAGGTCCCCTTAGCCA
ACCCTGACCTCAACCTATATATATACTGATGGAAGTTCGTTTGTAGAAAAG
GGATTACAAAGGGNAGGATATNCCATAGGTTAGTGATAAAGCAGTACTTG
AAAGTAAGCCTCTTCCCCCCAGGGACCAGCGCCCCCGTTAGCAGAACTAGT
GGCACTGACCCCGAGCCTTAGAACTTGGAAAGGGAGGAGGATAAATGTGT
ATACAGATAGCAAGTATGCTTATCTAATCCGAAATGCCCATGTTG

SEQ ID NO 52 (JLBc1)

TCAGGGATAGCCCCCATCTATTTGGTCAGGCACTGGCCCAAGATCTAGGGA
CATGCCACTTTTAAGAGCCATTTCTCAAGTCCAGGTACTCTGGTCCTTCGGT
ATGTGGATGATTTACTTTTGGCTACCAGTTCAGTAGCCTCATGCCAGCAGG
CTACTCTAGATCTCTTGAACTTTCTAGCTAATCAAGGGTACAAGGCATCTA
GGTTGAAGGCCCAGCTTTGCCTACAGCAGGTCAAATATCTAGGCCTAATCT
TAGCCAGAGGGACCAGGGCACTCAGCAAGGAACAAATACAGCCTATACTG
GCTTATCCTCACCCTAAGACATTAAAACAGTTGCGGGGGTTCCTTGGAATC
ACTGGCTTTTTGGTGACTATGGATTCCCAGATACAGCAAGATTGGCAGGCC
CCTCTATACTGTAATCAAGGAGACTCACGAGGGCAAGTACTCATCTAGTAG
AATGGGAACTAGGGACAGAAACAGCCTTCAAAACCTTAAAGCAGGCCCTA
GTACAATCTCCAGCTTTAAGCCTTCCCACAGGACAAAACTTCTCTTTATAC
ATCACAGAGAGGGCAGAGATAGCTCTTGGTGTCCTTATTCAGACTCATGGG
ACTACCCCACAACCAGTGGCACACCTAAGTAAGGAAATTGATGTAGTAGC
AAAAGGCTGGCCTCACTGTTTATGGGTAGCTGTGGTGGTGGCTGTCTTAGT
GTCAGAAGCTATCAAAATAATACAAGGAAAGGATCTCACTGTCTGGACTA
CTCATGATGTAATGGCATACTAGGTGCCAAAAGAAGTTTATGGGTATCAGA
CAACCACCTGCTTAGATACCAGGGACTACTCCTGGAGGATTGGGCTTCAAG
TGCGTTTTTTGTGGCCTCAACCCTGCCACTTTTCCTCCAGAGGATGGAGAG
CCGCTTGAGCATGCTTGCCAACAGGTTGTAGGCCAGAATTATTCCACCCGA
GATGATCTCTTAGAGTACCCTTAGCTAATCCTGACCTTAACCTATATACCA
ATGGAAGTTCATTTGTGGAAAACGGGATATGAAGGGCAGGTTATGTCATAG
TTAGTGATGTAATCATACTTGCAAGTAAGCCTCTTACCCCAGGGGCCAGCA
CTCAGTTAGCAGAACTAGTCACACTTACCTTAACCTTAGAACTGGGAAAGG
GAAAAAGAATAAATATGTATACAGATAGTAAGTATGCTTATCTAATCCTAC
ATGCCCATGCTGCAATATGGAAGGAAAGGGAGTTCCTAACCCCTGGGGGA
ACCCCCATTAAATACCACAAGGYAAATCATGGAGTTATTGCACGCAGTGC
AAAAACTCAAGGAGGTGGCAGTCTTACACTGCCGAAGCYATCAAAAAGGG
GAAGGAGAGGGGAGAACAGCAGCATAAGTGGTTGGCAGAGGCAGTGAAA
GACCAGCAGAGAGAAGGAGAGAGACAACGTCAACGACAGAAGGAAAGAA
GAGGAGGAGACAGAGAGGAAGAGACAGAGAGACAGTTAGTCCAAGAGAG
AGACAGAGAGAGGAAGAGACAGACAGAAAGTCCAAGAGAGAAGGAAAGA
GAGGAAGAGACCAAGGAGTCCNAGAGAGAGAAAGAGATAGAAGTAGTAA
AGAAAAAACATTGTACCCTATTCCTTTAAAAGCCGGGGTATATTTAAAACC
TATAATTGATAATTGAGTTCTTGCACCCTCCTCCAGGGGATYGCTGGGAGG
AAACCCTCAACCGATATGTGAAAATTGTGGGTCGTCCCTATGTCTCAATTA
CCAGCCAATACCCCCTTGTTTTTAGTGTGAACGAGGGTGTAGAGCGCAGAC
AGGGAGACCTCTGACAATCCATACCCTTCCTATCCAAAATCCTTAACCCAG
CAGGTTTTCTAAAAGGGGATCTAAATCTTAATTAATTACCATACAAAGGTC
AAACCAGATCTAGGAGGAACTTCCTTCAGGACAGGATGATAGATGGTTCCT
CCCAGGCGATTAAAGAAAATAAAAAGACACATGGGCAGCCAGTAAGTGAT
AAGGGAACACTAGTAGAAGCAGTTAGGAGAAGTTGCCTAATAATTGGTCT
ACTCCAAATGTGTGAGTTGTTCGCACTCAGCCCAAATCTTAAAGTACTTAC
AGAATTAGGGAGGAGCCATTTACACCAATTCTAAGTTAATATGGACTGGAT
GAGGTTTTATTAATAGCGAAGGAGAATTAAATCCTAAACTNACAAGGTTTT
CAACTAAAGTAAATTTTACTAAAAGCTAACAGTGTAACATGCATTATCCTA
CTACAACACACTCTCANAGGATTCCTCAGACAGTTTACAAGAAATAACAA
AATCTATCTGGTAAGGATAGTAACTACAATCCCAAATACATTCTTTGGCAG
CAGTGACTCTC

FIG.16

SEQ ID NO 53 (JLBc2)

TCAGGGATAGCCCCCATCTATTTGATCAGGCACTAGCCCAAGATCTAGGCC
ACTTCTGAAGTCCAGGCATTCTAGTCCTTCAGTATGTGGATGATTTACTTTT
GGCTACCAGTTTGGAAGCCTCATGCCAGCAGGCTACTTGAGATCTCTTGAA
CTTTCTAGCTAATCAAGGGTGTATGGCATCTAAATTGAAAGTCCAGCTCTG
CCTACAACAAGTCAAATATCTAGGCCTAATCTTAGATAGAAGAACCAGGG
CCCTCAGCAAGGAATGAATAAAGCCTATGCTGGCTTATCGGCACCCTAAGA
CATTAAAACAATTGTGGGGGTTCCTTGGAATCACTGGCTTTTGCCGACTAT
GGATCCCTGGATAGAGTGAGATAGCCAGGCCCCCTCTATTACTCTTATCAA
GGAGACCCAGAGGGCAAATACTTATCTAGTATTATGGGNACCAGAGGCAG
AAAAAGCCTTCCAAACCTTAAAGGAGACCCTAGTACAAGCTCCAGCTTTAA
GCCTTCCCACAGGACAAANCTTCTCTTTATATGTCACAGAGAGAGCAGGAA
TAGCTCCTGGAGTCCTTACTCAGACTTTTGGACGACCCCACGGCCAGTGGC
RTACCTAAGTAAGGAAATTGATGTAGTAGCAAAAGGCTGGCCTCACTGTTT
ATGGGTAGTTGCGGCTGTGGCAGTCTTACTGTCAAAGGCTATCAAAATAAT
ACAAGGAAAGGATTTCACTATCTGGACTACTCATGAGGAAAATGGCATATT
AGGTGCCAAAGGAAGTTTTTGGCTATCAGACAACCACCTGCTCAGATTCCA
GGCACTACTGATTGAGAGACCAGTGCTTTAAATATGTATGTGTGTGTGTGG
CCCTCAACCCTGCCACTGTTCTCCCAGAAGATGGAGAACCAATGAAGCATT
ACTGTCAACAAATTAGAGTCCAGAGTTATGCTGCCTGAGAGGATCTCTTAG
AAGTCCCCTTAGCTAATCCTGACCTTAACCTATATGCTGATGGAAGTTCAC
TTGTGGAGAATGGGATACGAAAAGCACATTATGCCATAGTTAGTGAGGTA
ACAGTACTTGAAAGTAAGCCTATTCCCCCATGGACCAGAGCCCAGTTAGCA
GAACTAGTGGCACTTACCCAAGCCTTAGAACTAGGAAAGGGAAAAATAAT
AAATGTGTATACAGATAGCAAGTATGCTTATCTAATCCTACATGCCCATGC
TGCAGTATGGAAAGAAAGGGAGTTCCTAACCTCTGGGGGAACCCCCATTA
AATACCACAAGGCAAATCATGGAGTTATTGCATGTAGTGCAAAACCTCAA
GTAGGTGGCAGTTTTACACTGCCTGAAGCTATGGGGAAGGAGAGAGGAGA
ACAGCAGCATAAGTGGCTAGCAGAGGCAGCGAAAGACTAGCAGAGAGGA
GAGGTAGGGGAAAGACAGAAAGTCAAAGAAAAGAAGTCAAAGACAGACA
GAGAAAGAGACAGAGGGAGCCAGAGAGAAAGAAAAGAGAGAACGAAAGA
GACAGAATGTCAAAGAACAGAAGAGAGAGGCAGCGCCAGAAGAGTTAAG
AAAGTGAGAAAGAGAGATGGAAATAGTAAAGAAAAAACAGTGTACCCTAT
TCCTTTAAAAGCCAGGGTAAATTTAAAACGTATAATTTTATAATTGGAAGG
TCTTCTCCATAACCCTATAACATTAAAATACCACCTTGTTGTCAGTGTAAAC
AAGAGCATAGCCCAAAAGCACTGAGGCCACTGACAACCCATAGCCTTCCT
ATCAAAAATCCTTAACTCTGCAGGTTTCCTAACAGGGGATCTAAATCTCAA
CTAATCACCATACAATGGTCCGACCAGACCTAGGAGCGACTCCCCTCAGG
ACAGAAGGATGGATGGTTCCTCCCAGGCCATTAAGGGAAAGAGACACAAT
GGGTATTCAGTAAGTGATAAGGGAACTCTTGTAGAAGCAGTTAGGAAGATT
GCCTAATATTTGGTCTGCTCAAATGTGCCAGCTGTTTGCACTCAGCTAAAC
CTTAAATTACTTACAGAATTAGGAAGGAGCCATCTATACCAATTCTGAGTT
AATATGAGCTGAACAAGTTCTTATTAATAGCAAAGAATCATTGAAATCTCA
AACTTGCAAAGTTTTCAACAAAAGTAAAGTTTGCTGAAAGTTAGCAGTGTA
ACATGTATTATCCTAACTTCTAATCTTGTGGAAATCAGACCCTATCAGTGC
CCCTCAAAGCTGAAGTCCATCAGCATATGGCCATACAACTAATACCCCTAT
TTATAGGGTTAGGAATGGCCACTGCTACAGGAATGGGAGTAACAGGTTTAT
CTACTTCATTATCCTATTACCACACACTCTTAAAGGATTTCTCAGACAGTTT
ACAAGAAATAACAAAATCTATCCTTACTCTNTARTCCCAAATAGRTTCTTT
GGCAGCAGTGACTCTC

FIG. 17

FIG. 18

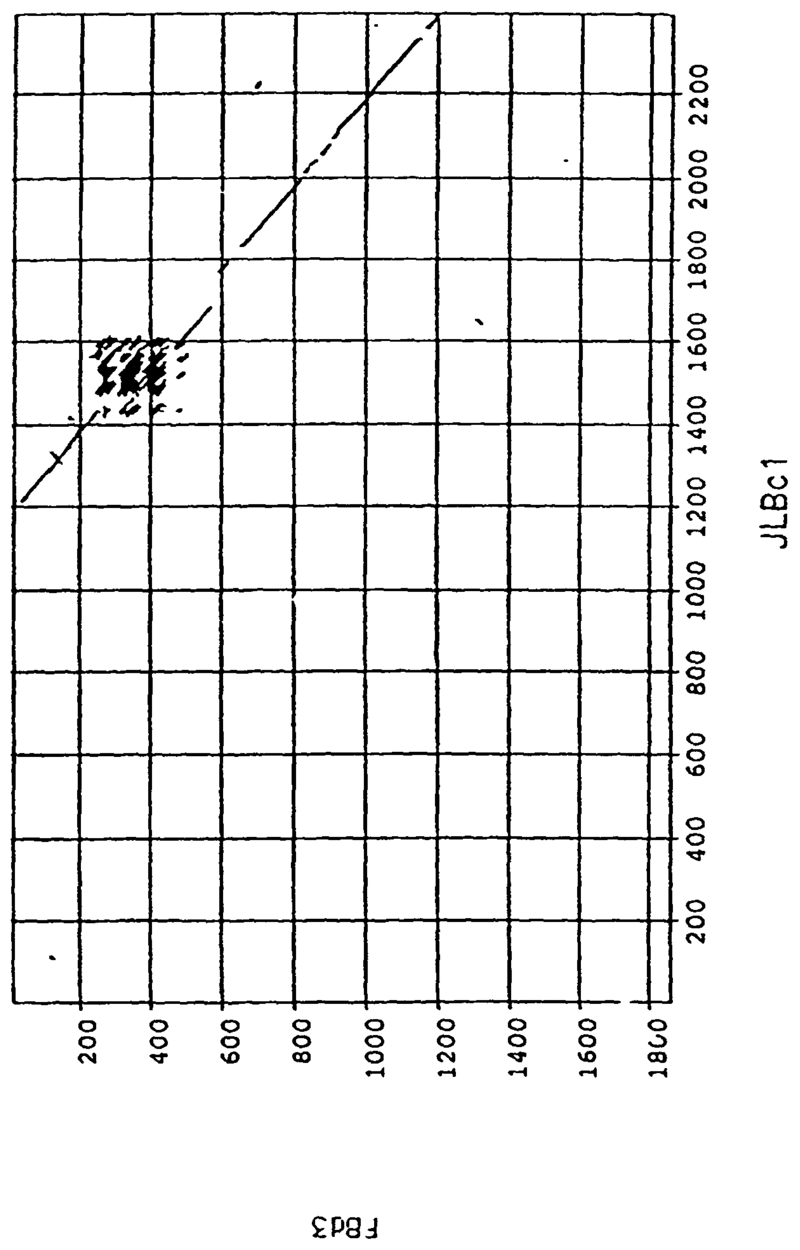
200 400 600 800 1000 1200 1400 1600 1800 2000 2200
JLBc1
200 400 600 800 1000 1200 1400 1600 1800
FBd3

FIG. 19

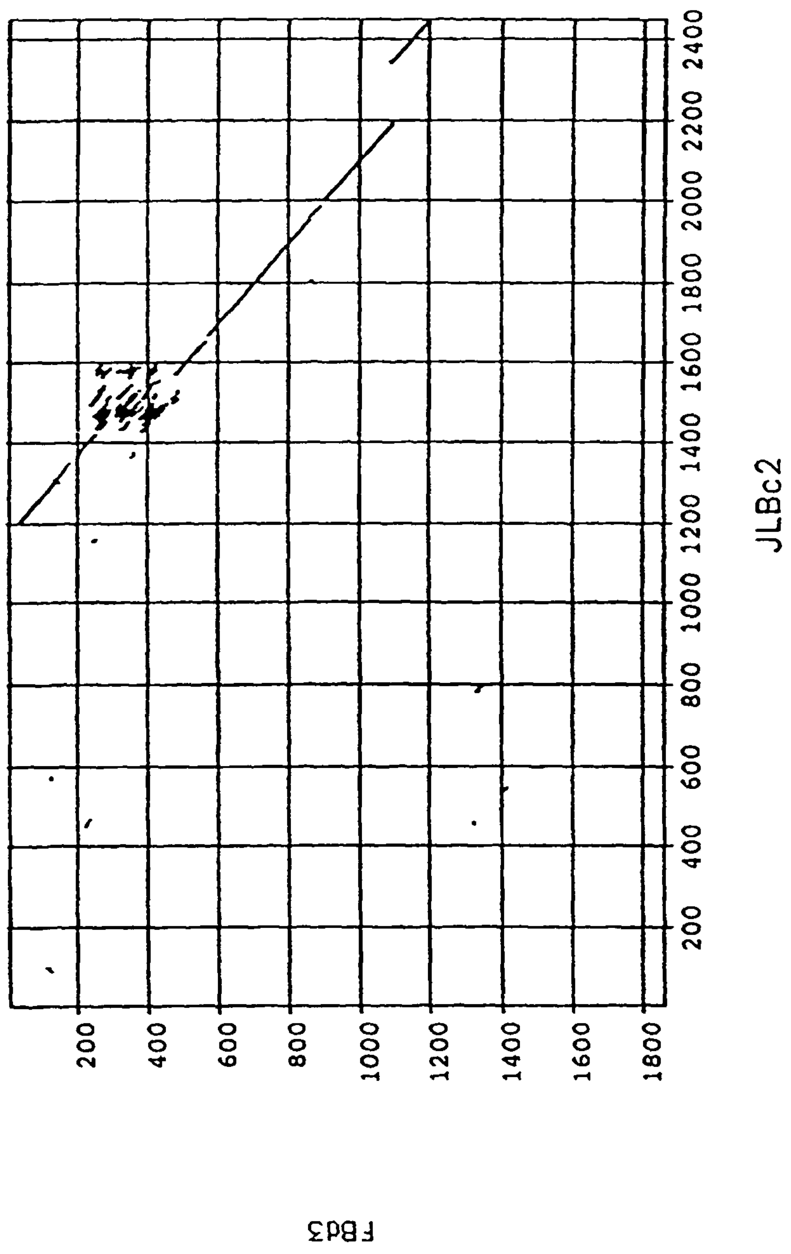
200
400
600
800
1000
1200
1400
1600
1800
2000
2200
2400
JLBc2
FBd3

FIG. 20

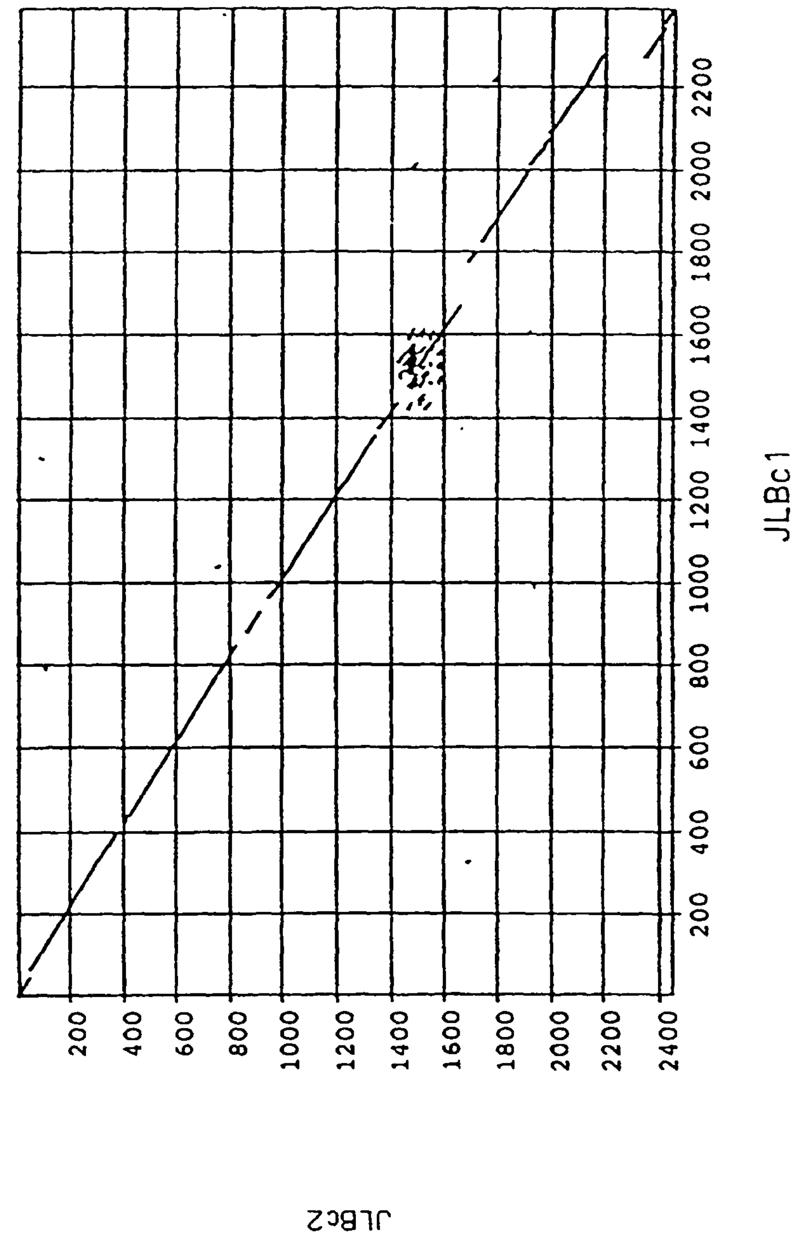

FIG. 21

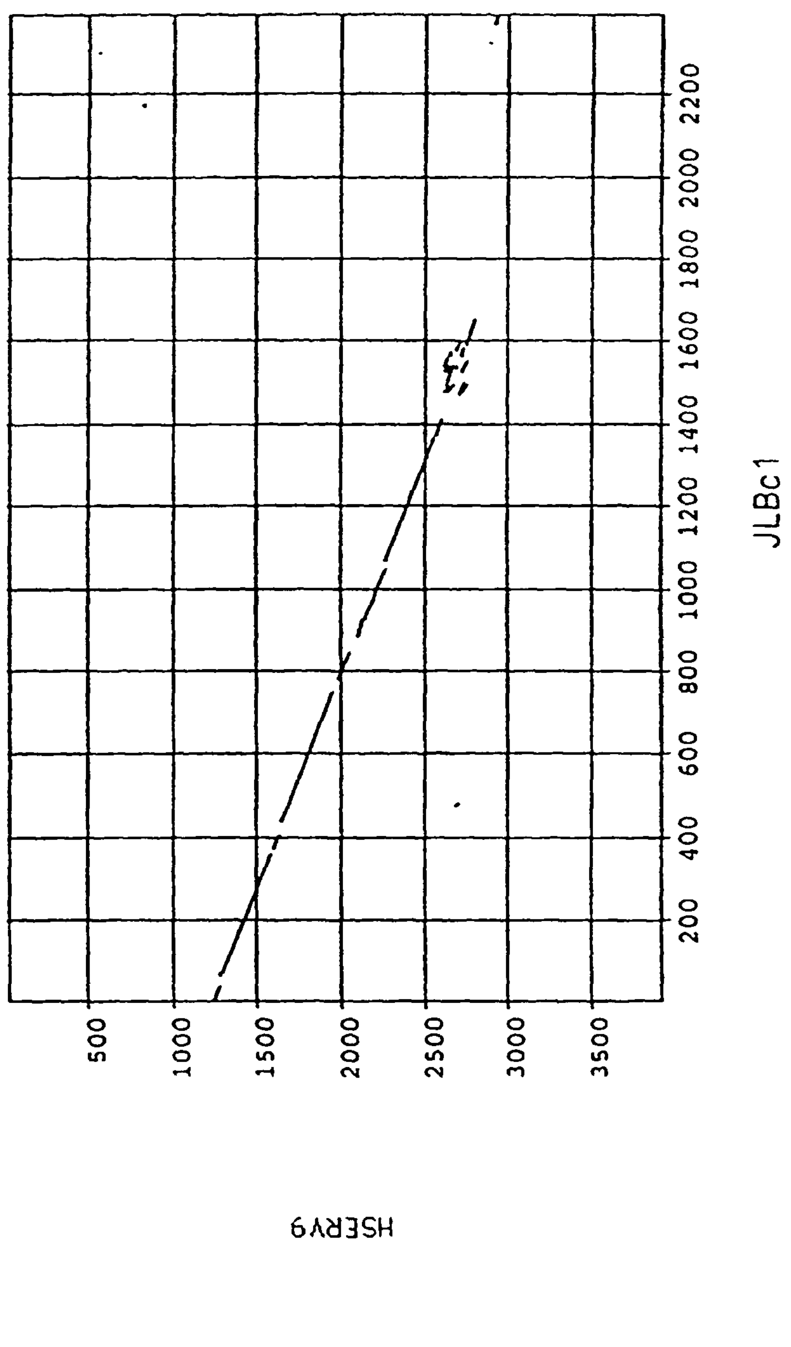
500
1000
1500
2000
2500
3000
3500
200
400
600
800
1000
1200
1400
1600
1800
2000
2200
JLBc1
HSERY9

FIG. 22

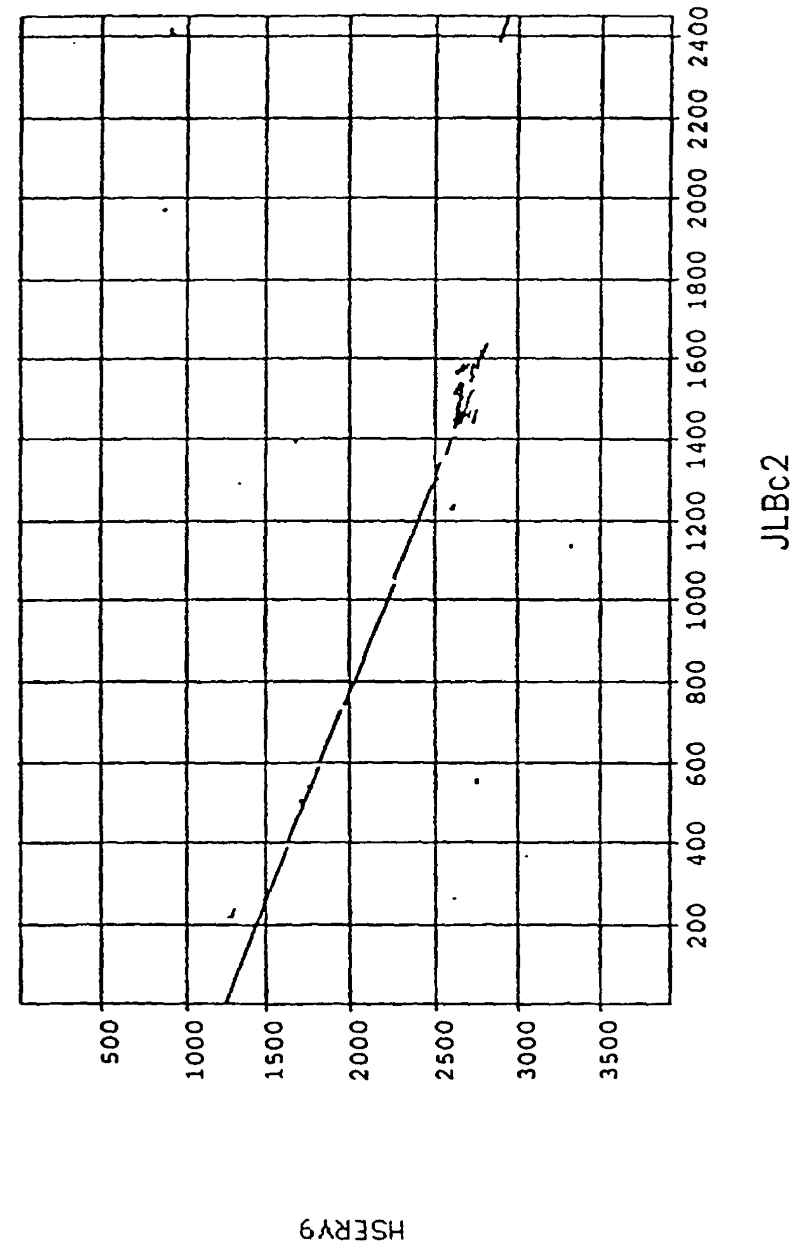
200
400
600
800
1000
1200
1400
1600
1800
2000
2200
2400
JLBc2
500
1000
1500
2000
2500
3000
3500
HSERY9

```
   1 TTCCTGAGTT CTTGCACTAA CCTCAAATGA GAGAAGTGCC GCCATAACTG CAACCCAAGA
  61 GTTTGGCGAT CCCTGGTATC TCAGTCAGGT CAATGACAGG ATGACAACAG AGGAAAGATA
 121 ATGATTCCCC ACAGGCCAGC AGGCAGTTCC CAGTGTAGAC CCTCATTAGG ACACAGAATC
 181 AGAACATGGA GATTGGTGCC GCAGACATTT GCTAACTTGC GTGCTAGAAG GACTAAGGAA
 241 AACTAGGAAG ATATGAATTA TTCAATGATG TCCACTATAA CACAGGGGAA AGGAAGAAAA
 301 TCCTACTGCC TTTCTGGAGA GACTAAGGGA GGCATTGAGG AAGCATACCA GGCAAGTGGA
 361 CATTGGAGGC TCTGGAAAAG GGAAAAGTTG GGAAAAGTAT ATGTCTAATA GGGCTTGCTT
 421 CCAGTGTGGT CTACAAGGAC ACTTTAAAAA AGATTGTCCA ATAGAAATAA GCCACCACCT
 481 CGTCCATGCC CCTTATGTCA AGGGAATCAC TGGAAGGCCC ACTGCCCCAG GGGATGAAGG
 541 TCCTCTGAGT CAGAAGCCAC TAACCAGATG ATCCAGCAGC AGGACTGAGG GTGCCCGGGG
 601 CAAGCGCCAG CCCATGCCAT CACCCTCACA GAGCCCCAGG TATGCTTGAC CATTGAGGGT
 661 CAGAAGGGTA CTGTCTCCTG GACACTGGCG GGCCTTCTCA GTCTTACTTT CCTGTCCTGG
 721 ACAACTGTCC TCCAGATCTG TCACTGTCCG AGGGGTCCTA GGACAGCCAG TCACTAGATA
 781 CTTCTCCCAG CCACTAAGTT GTGACTGGGG AACTTTACTC TTCCACATGC TTTTCTAATT
 841 ATGCCTGAAA GCCCCACTCT CTTGTTAGGG GAGAGACATT CTAGCAAAAG CAGGGGCCAT
 901 TATACATGTG AATATAGGAG AAGGAACAAC TGTTTGTTGT CCCCTGCTTG AGGAAGGAAT
 961 TAATCCTGAA GTCCGGGCAA CAGAAGGACA ATATGGACAA GCAAAGAATG CCCGTCCTGT
1021 TCAAGTTAAA CTAAAGGATT CCACCTCCTT TCCCTACCAA AGGCAGTACC CCCTCAGACC
1081 CGAGACCCAA CAAGAACTCC AAAAGATTGT AAAGGACCTA AAAGCCCAAG GCCTAGTAAA
1141 ACCAAGCAAT AGCCCTTGCA AGACTCCAAT TTTAGGAGTA AGGAAACCCA ACGGAC
```

SEQ ID NO 56 (GM3)

FIG. 23

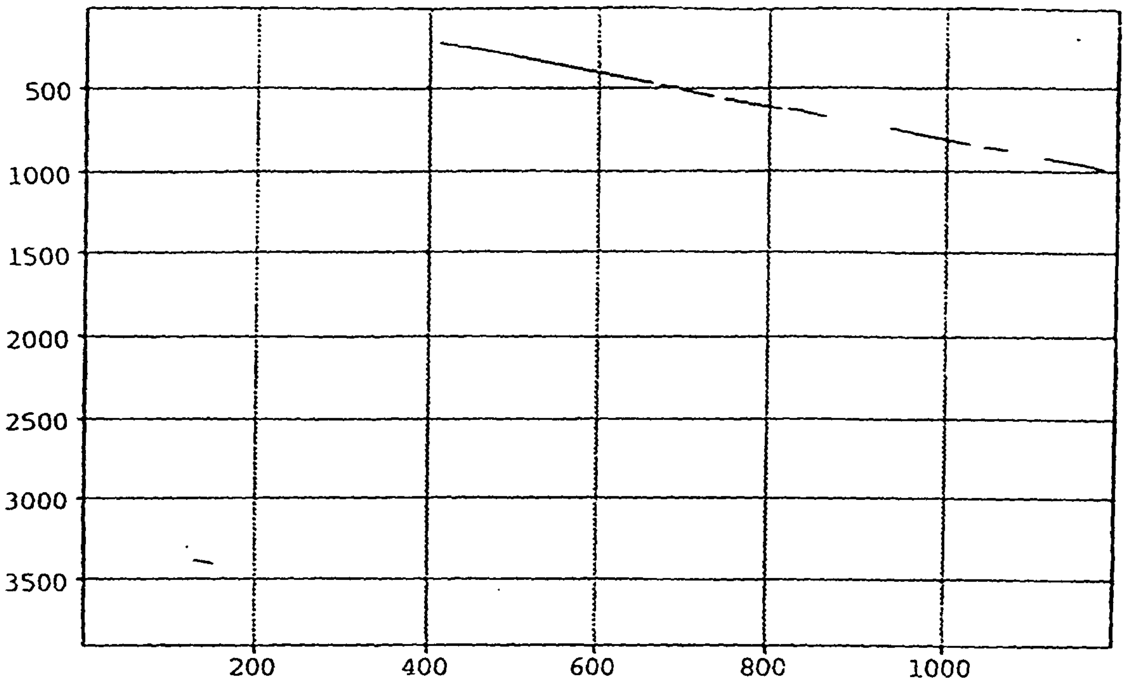
500
1000
1500
2000
2500
3000
3500
200
400
600
800
1000
COMPLETE G/M3

FIG. 24

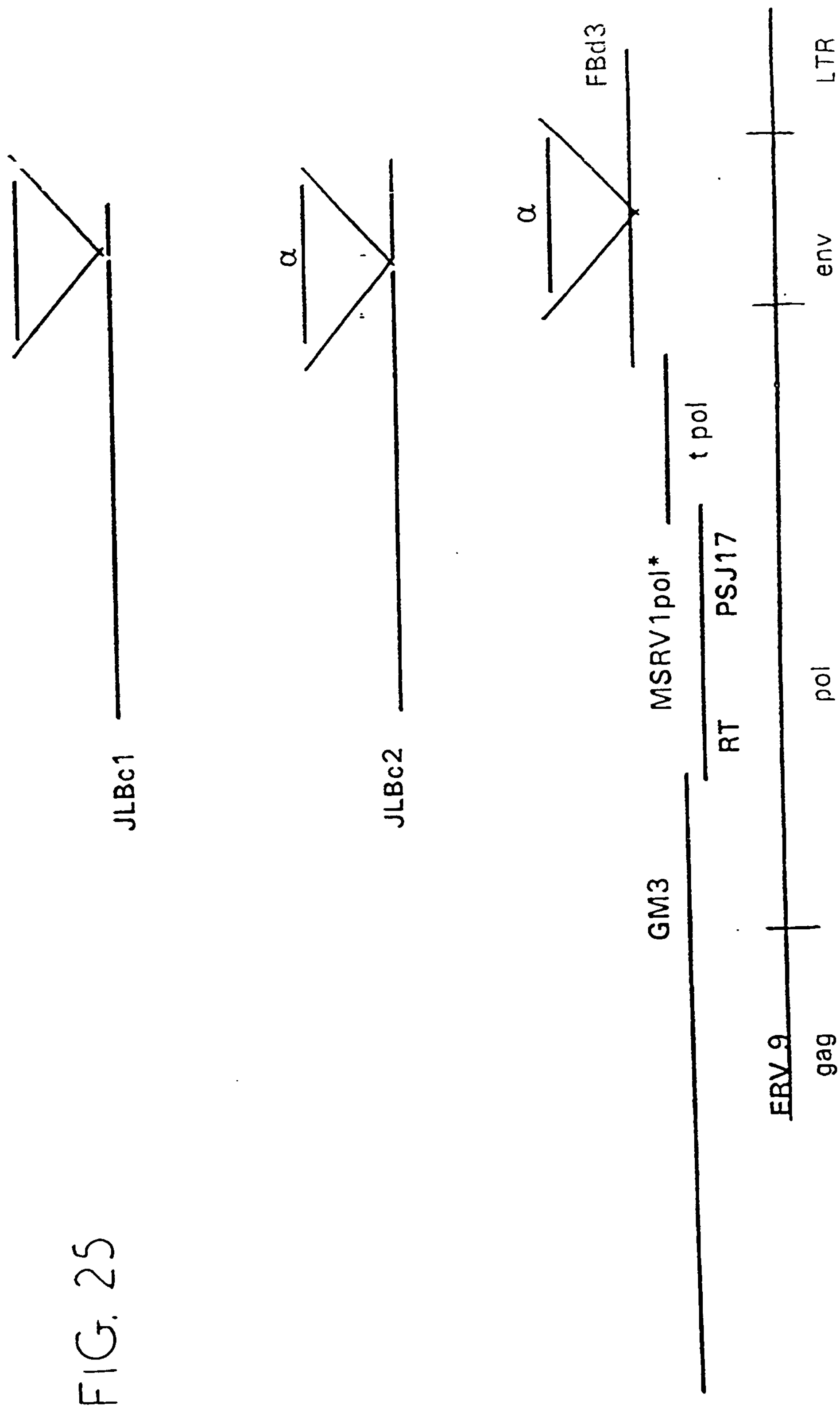
α'
JLBc1
α
JLBc2
α
FBd3
t pol
MSRV1pol*
PSJ17
RT
GM3
ERV 9
gag
pol
env
LTR

FIG. 25

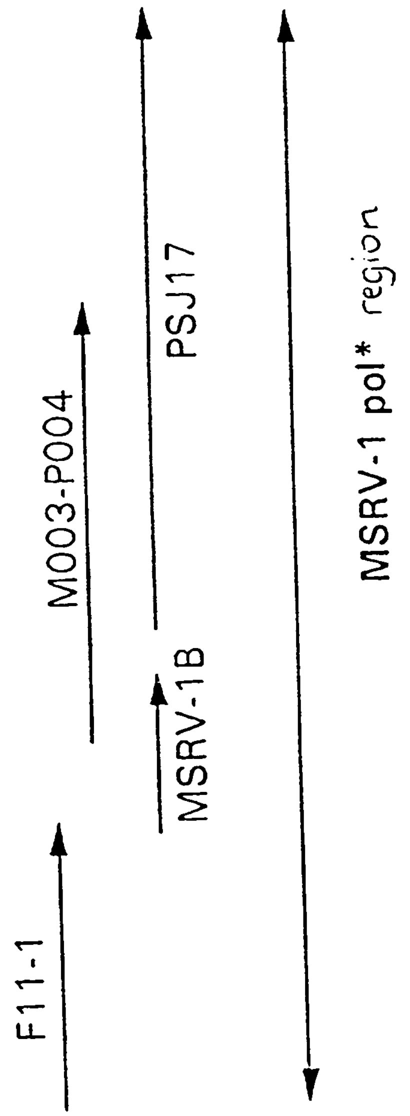


FIG. 26

FIG. 27a

SEQ ID NO 57 (POL)

```
ATG ATC CAG CAG CAG GAC NGA GGG TGC CCG GGG CAA GCG CCA GCC CAT GCC ATC ACC CTC ACA GAG CCC CAG GTA TGC TTG ACC ATT GAG 90
M   I   Q   Q   Q   D   X   G   C   P   G   Q   A   P   A   H   A   I   T   L   T   E   P   Q   V   C   L   T   I   E

GGT CAG AAG GGT NAC TGT CTC CTG GAC ACT GGC GGN GCC TTC TCA GTC TTA CTT TCC TGT CCT GGA CAA CTG TCC TCC AGA TCT GTC ACT 180
G   Q   K   G   X   C   L   L   D   T   G   G   A   F   S   V   L   L   S   C   P   G   Q   L   S   S   R   S   V   T

GTC CGA GGG GTC CTA GGA CAG CCA GTC ACT AGA TAC TTC TCC CAG CCA CTA AGT TGT GAC TGG GGA ACT TTA CTC TTC CCA CAT GCT TTT 270
V   R   G   V   L   G   Q   P   V   T   R   Y   F   S   Q   P   L   S   C   D   W   G   T   L   L   F   P   H   A   F

CTA ATT ATG CCT GAA AGC CCC ACT CTC TTG TTG GGG AGA GAC ATT CTA GCA AAA GCA GGG GCC ATT ATA CAT GTG AAT ATA GGA GAA GGA 360
L   I   M   P   E   S   P   T   L   L   L   G   R   D   I   L   A   K   A   G   A   I   I   H   V   N   I   G   E   G

ACA ACT GTT TGT TGT CCC CTG CTT GAG GAA GGA ATT AAT CCT GAA GTC CGG GCA ACA GAA GGA CAA TAT GGA CAA GCA AAG AAT GCC CGT 450
T   T   V   C   C   P   L   L   E   E   G   I   N   P   E   V   R   A   T   E   G   Q   Y   G   Q   A   K   N   A   R

CCT GTT CAA GTT AAA CTA AAG GAT TCC ACC TCC TTT CCC TAC CAA AGG CAG TAC CCC CTC AGA CCC GAG ACC CAA CAA GAA CTC CAA AAG 540
P   V   Q   V   K   L   K   D   S   T   S   F   P   Y   Q   R   Q   Y   P   L   R   P   E   T   Q   Q   E   L   Q   K

ATT GTA AAG GAC CTA AAA GCC CAA GGC CTA GTA AAA CCA AGC AAT AGC CCT TGC AAG ACT CCA ATT TTA GGA GTA AGG AAA CCC AAC GGA 630
I   V   K   D   L   K   A   Q   G   L   V   K   P   S   N   S   P   C   K   T   P   I   L   G   V   R   K   P   N   G

CAG TGG AGG TTA GTG CAA GAA CTC AGG ATT ATC AAT GAG GCT GTT GTT CCT CTA TAC CCA GCT GTA CCT AAC CCT TAT ACA GTG CTT TCC 720
Q   W   R   L   V   Q   E   L   R   I   I   N   E   A   V   V   P   L   Y   P   A   V   P   N   P   Y   T   V   L   S

CAA ATA CCA GAG GAA GCA GAG TGG TTT ACA GTC CTG GAC CTT AAG GAT GCC TTT TTC TGC ATC CCT GTA CGT CCT GAC TCT CAA TTC TTG 810
Q   I   P   E   E   A   E   W   F   T   V   L   D   L   K   D   A   F   F   C   I   P   V   R   P   D   S   Q   F   L

TTT GCC TTT GAA GAT CCT TTG AAC CCA ACG TCT CAA CTC ACC TGG ACT GTT TTA CCC CAA GGG TTC AGG GAT AGC CCC CAT CTA TTT GGC 900
F   A   F   E   D   P   L   N   P   T   S   Q   L   T   W   T   V   L   P   Q   G   F   R   D   S   P   H   L   F   G

CAG GCA TTA GCC CAA GAC TTG AGT CAA TTC TCA TAC CTG GAC ACT CTT GTC CTT CAG TAC ATG GAT GAT TTA CTT TTA GTC GCC CGT TCA 990
Q   A   L   A   Q   D   L   S   Q   F   S   Y   L   D   T   L   V   L   Q   Y   M   D   D   L   L   L   V   A   R   S

GAA ACC TTG TGC CAT CAA GCC ACC CAA GAA CTC TTA ACT TTC CTC ACT ACC TGT GGC TAC AAG GTT TCC AAA CCA AAG GCT CGG CTC TGC 1080
E   T   L   C   H   Q   A   T   Q   E   L   L   T   F   L   T   T   C   G   Y   K   V   S   K   P   K   A   R   L   C
```

FIG. 27b

SEQ ID NO 57 (POL)

TCA CAG GAG ATT AGA TAC TNA GGG CTA AAA TTA TCC AAA GGC ACC AGG GCC CTC AGT GAG GAA CGT ATC CAG CCT ATA CTG GCT TAT CCT 1170

S Q E I R Y X G L K L S K G T R A L S E E R I Q P I L A Y P

CAT CCC AAA ACC CTA AAG CAA CTA AGA GGG TTC CTT GGC ATA ACA GGT TTC TGC CGA AAA CAG ATT CCC AGG TAC ASC CCA ATA GCC AGA 1260

H P K T L K Q L R G F L G I T G F C R K Q I P R Y X P I A R

CCA TTA TAT ACA CTA ATT ANG GAA ACT CAG AAA GCC AAT ACC TAT TTA GTA AGA TGG ACA CCT ACA GAA GTG GCT TTC CAG GCC CTA AAG 1350

P L Y T L I X E T Q K A N T Y L V R W T P T E V A F Q A L K

AAG GCC CTA ACC CAA GCC CCA GTG TTC AGC TTG CCA ACA GGG CAA GAT TTT TCT TTA TAT GCC ACA GAA AAA ACA GGA ATA GCT CTA GGA 1440

K A L T Q A P V F S L P T G Q D F S L Y A T E K T G I A L G

GTC CTT ACG CAG GTC TCA GGG ATG AGC TTG CAA CCC GTG GTA TAC CTG AGT AAG GAA ATT GAT GTA GTG GCA AAG GGT TGG CCT CAT NGT 1530

V L T Q V S G M S L Q P V V Y L S K E I D V V A K G W P H X

TTA TGG GTA ATG GNG GCA GTA GCA GTC TNA GTA TCT GAA GCA GTT AAA ATA ATA CAG GGA AGA GAT CTT NCT GTG TGG ACA TCT CAT GAT 1620

L W V M X A V A V X V S E A V K I I Q G R D L X V W T S H D

GTG AAC GGC ATA CTC ACT GCT AAA GGA GAC TTG TGG TTG TCA GAC AAC CAT TTA CTT AAN TAT CAG GCT CTA TTA CTT GAA GAG CCA GTG 1710

V N G I L T A K G D L W L S D N H L L X Y Q A L L L E E P V

CTG NGA CTG CGC ACT TGT GCA ACT CTT AAA CCC AAA CTT ATG CTG CCC AGA AGG ATC TTT NTA GAG GTC CCC TTA GCC AAC CCT GAC CTC 1800

L X L R T C A T L K P K L M L P R R I F X E V P L A N P D L

AAC TAT ATA TAT ACT GAT GGA AGT TCG TTT GTA GAA AAG GGA TTA CAA AGG GNA GGA TAT NCC ATA GGT GTT AGT GAT AAA GCA GTA CTT 1890

N Y I Y T D G S S F V E K G L Q R X G Y X I G V S D K A V L

GAA AGT AAG CCT CTT CCC CCC CAG GGA CCA GCG CCC CCG TTA GCA GAA CTA GTG GCA CTG ACC CCG CGA GCC TTA GAA CTT TGG AAA GGG 1980

E S K P L P P Q G P A P P L A E L V A L T P R A L E L W K G

AGG AGG ATA AAT GTG TAT ACA GAT AGC AAG TAT GCT TAT CTA ATC CGA AAT GCC CAT GTT GTT TAT CTA ATC CGA AAT GCC CAT GTT GCA 2070

R R I N V Y T D S K Y A Y L I R N A H V V Y L I R N A H V A

ATA TGG AAA GAA AGG GAG TTC CTA ACC TCT GGG GGA ACC CCC ATT AAA TAC CAC AAG TTA ATC ATG GAG TTA TTG CAC ACA GTG CAA AAA 2160

I W K E R E F L T S G G T P I K Y H K L I M E L L H T V Q K

FIG. 27c

SEQ ID NO 57 (POL)

```
CTC AAG GAG GTG GAA GTC TTA CAC TGC CAA AGC CAT CAG AAA AGG GAA AGG GGA GAA GAG CAG CAT AAG TGG CTA CAG AGG CAA GGA AAG 2250
L   K   E   V   E   V   L   H   C   Q   S   H   Q   K   R   E   R   G   E   E   Q   H   K   W   L   Q   R   Q   G   K

ACT AGC AGA AAG GAA AGA GAG AAA GAG ACA GAA AGT CAG AGA GAG AGA GAG GAA GAG ACA GAG CAC AAA GAG GGA GTC AGA GAG AGA GAG 2340
T   S   R   K   E   R   E   K   E   T   E   S   Q   R   E   R   E   E   E   T   E   H   K   E   G   V   R   E   R   E

AGA CAG AGA GTC AGA GAG AAG•GAA AGA GAG AGA GGA AGA GAC AAA GAA TGA
R   Q   R   V   R   E   K   E   R   E   R   G   R   D   K   E   .                                                       2391
```

FIG. 28

GATGCCTTTTTCTGCATCCCTGTACGTCCTGACTCTCAATTCTTGTTTGCCTTTGAAG
ATCCTTTGAACCCAACGTCTCAACTCACCTGGACTGTTTTACCCCAAGGGTTCAGGGA
TAGCCCCATCTATTTGGCCAGGCATTAGCCCAAGATGCCTTTTGCATCCCTGTACGTG
ACTCTCAATTCTTGTTTGCCTTTGCCTTTGAAGATGCTTTGAACCCAACGTCTCAACT
CACCTGGACTGTTTTACGCCAAGGGTTCAGGGATAGCCCCCATCTATTTGGC
CAGGCATTAGCCCAA SEQ ID NO 40

Asp-Ala-Phe-Phe-Cys-Ile-Pro-Val-Arg-Pro-Asp-Ser-Gln-Phe-
Leu-Phe-Ala-Phe-Glu-Asp-Pro-Leu-Asn-Pro-Thr-Ser-Gln-Leu-
Thr-Trp-Thr-Val-Leu-Pro-Gln-Gly-Phe-Arg-Asp-Ser-Pro-His-
Leu-Phe-Gly-Gln-Ala-Leu-Ala-Gln

SEQ ID NO 39 (POL2B)

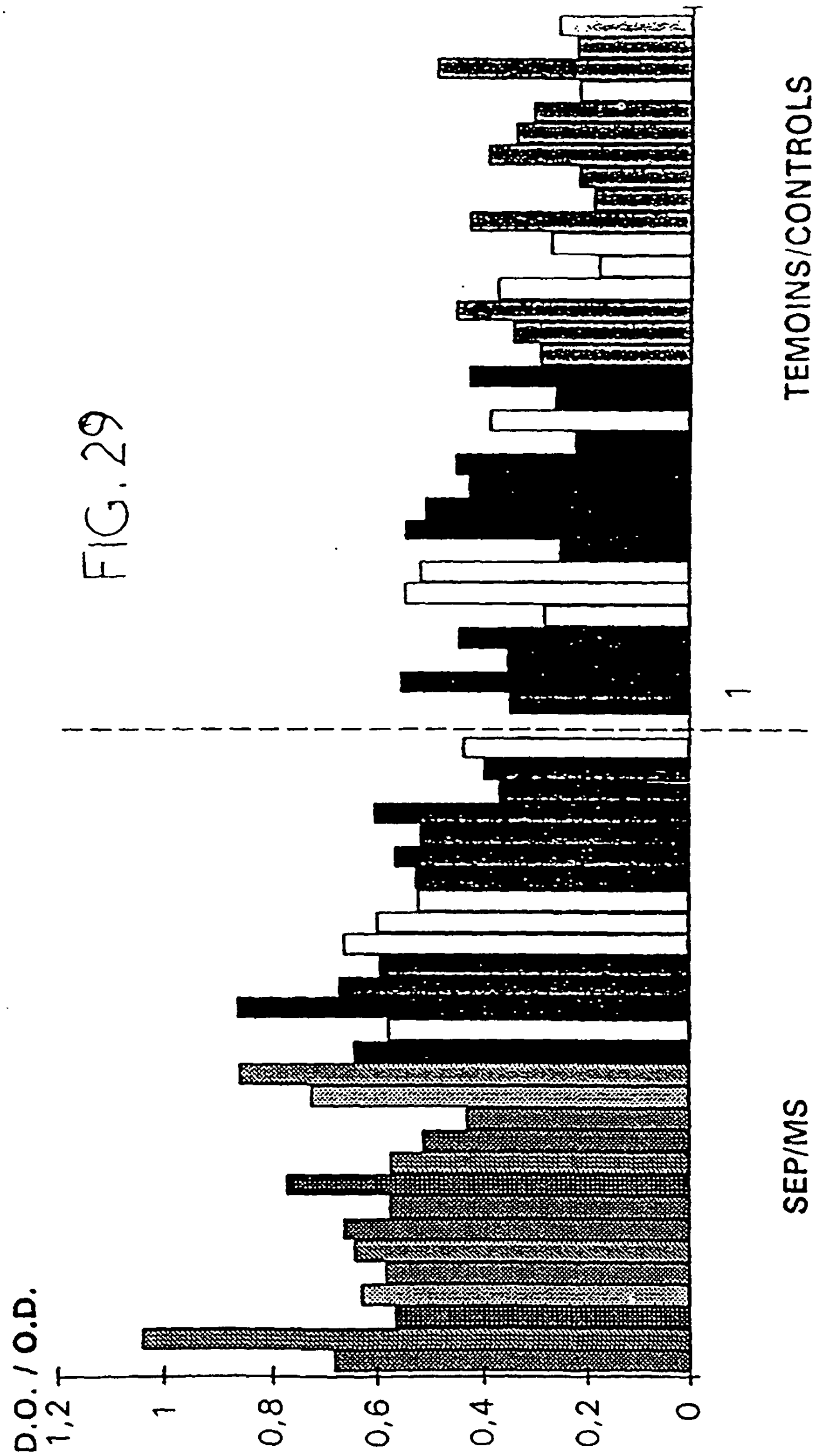


FIG. 29

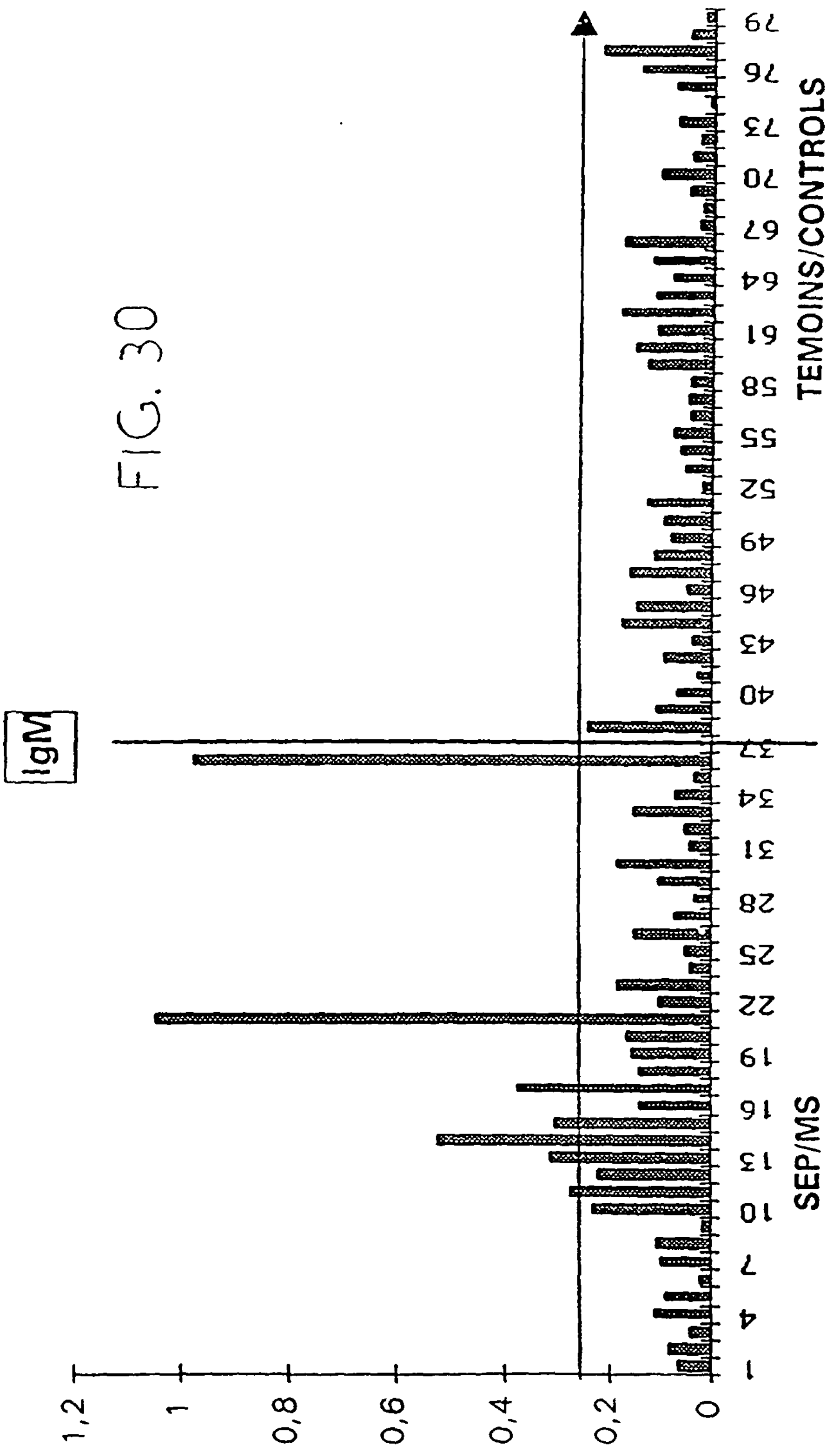
IgM
1,2
1
0,8
0,6
0,4
0,2
0
1
4
7
10
13
16
19
22
25
28
31
34
37
40
43
46
49
52
55
58
61
64
67
70
73
76
79
SEP/MS
TEMOINS/CONTROLS

FIG. 30

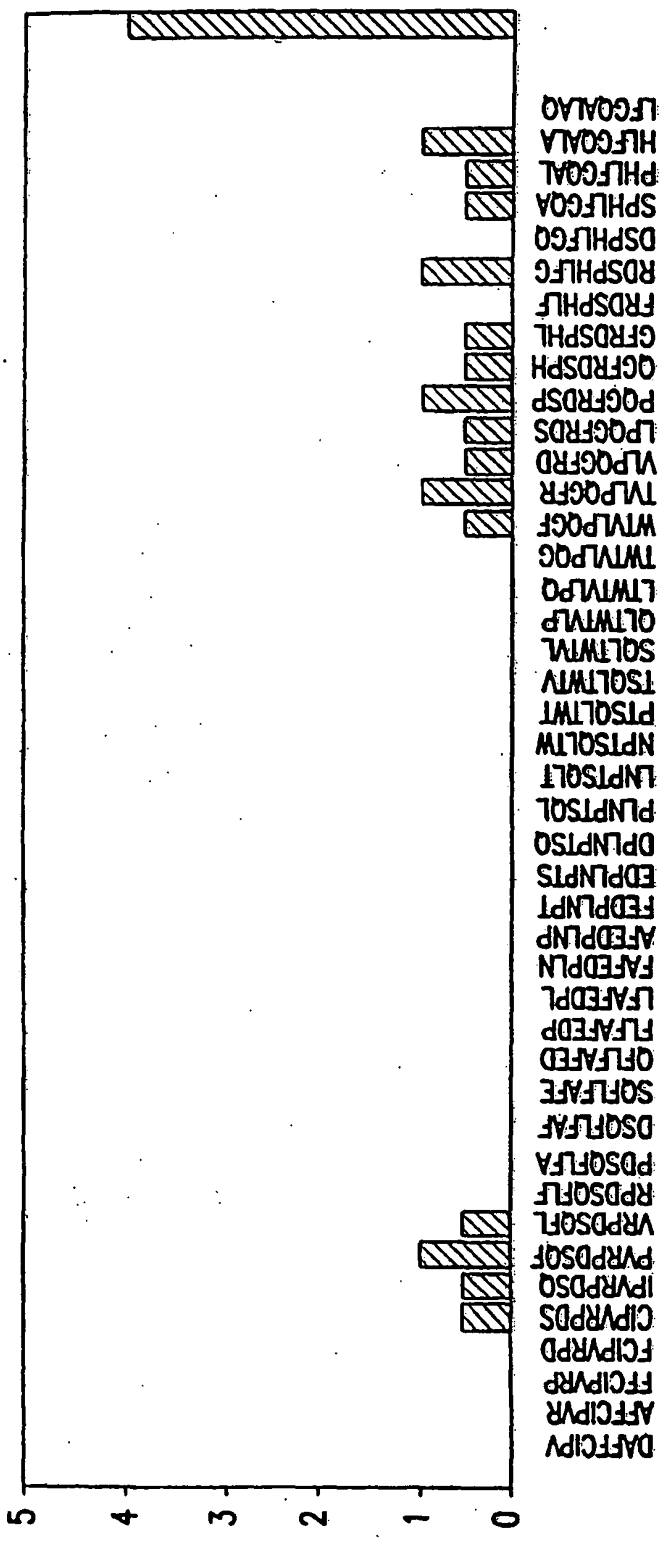
LFGQALQ
HLFGQALA
PHLFGQAL
SPHLFGQA
DSPHLFGQ
RDSPHLFG
FRDSPHLF
GFRDSPHL
QGFRDSPH
PQGFRDSP
LPQGFRDS
VLPQGFRD
TVLPQGFR
WTVLPQGF
TWTVLPQG
LTWTVLPQ
QLTWTVLP
SQLTWTVL
TSQLTWTV
PTSQLTWT
NPTSQLTW
LNPTSQLT
PLNPTSQL
DPLNPTSQ
EDPLNPTS
FEDPLNPT
AFEDPLNP
FAFEDPLN
LFAFEDPL
FLFAFEDP
QFLFAFED
SQFLFAFE
DSQFLFAF
PDSQFLFA
RPDSQFLF
VRPDSQFL
PVRPDSQF
IPVRPDSQ
CIPVRPDS
FCIPVRPD
FFCIPVRP
AFFCIPVR
DAFFCIPV
5
4
3
2
1
0

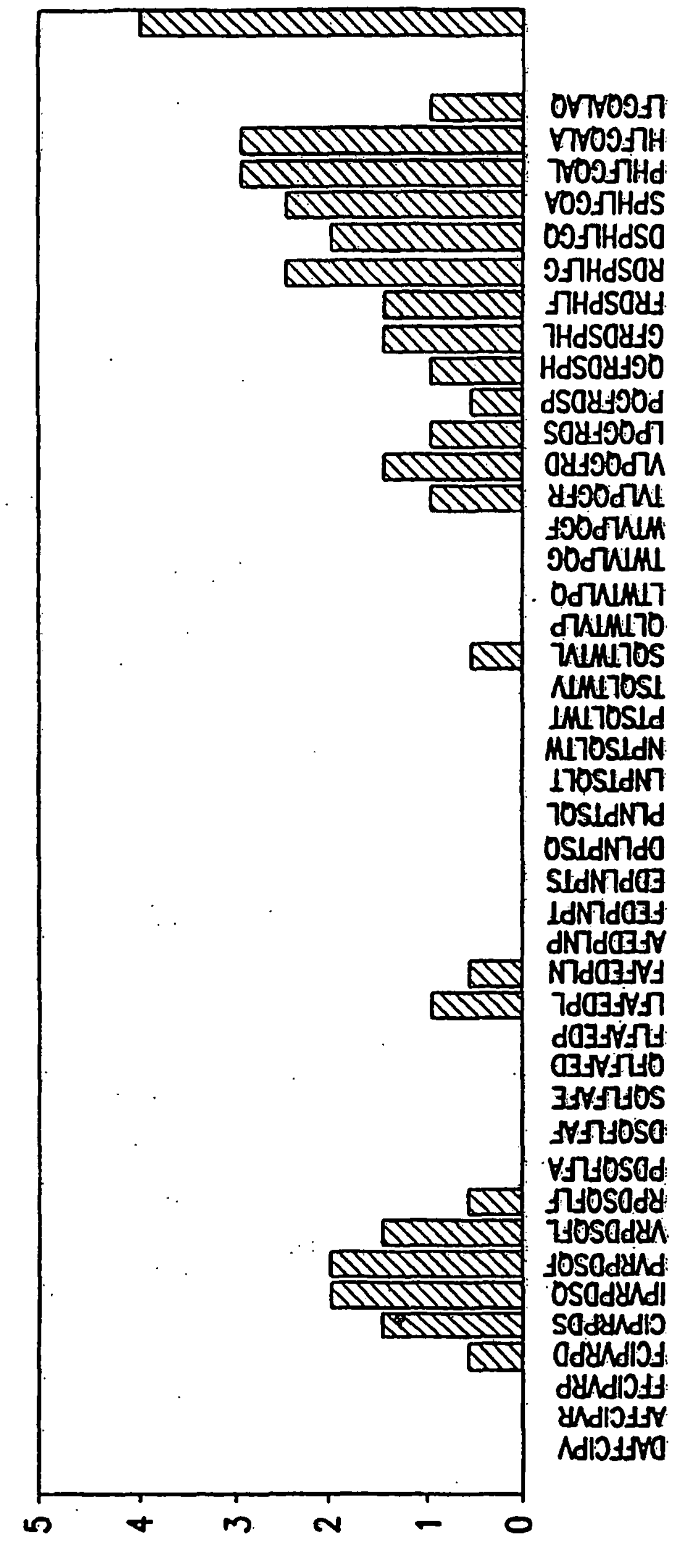
LFGQALQ
HLFGQALA
PHLFGQAL
SPHLFGQA
DSPHLFGQ
RDSPHLFG
FRDSPHLF
GFRDSPHL
QGFRDSPH
PQGFRDSP
LPQGFRDS
VLPQGFRD
TVLPQGFR
WTVLPQGF
TWTVLPQG
LTWTVLPQ
QLTWTVLP
SQLTWTVL
TSQLTWTV
PTSQLTWT
NPTSQLTW
LNPTSQLT
PLNPTSQL
DPLNPTSQ
EDPLNPTS
FEDPLNPT
AFEDPLNP
FAFEDPLN
LFAFEDPL
FLFAFEDP
QFLFAFED
SQFLFAFE
DSQFLFAF
PDSQFLFA
RPDSQFLF
VRPDSQFL
PVRPDSQF
IPVRPDSQ
CIPVRPDS
FCIPVRPD
FFCIPVRP
AFFCIPVR
DAFFCIPV
0
1
2
3
4
5

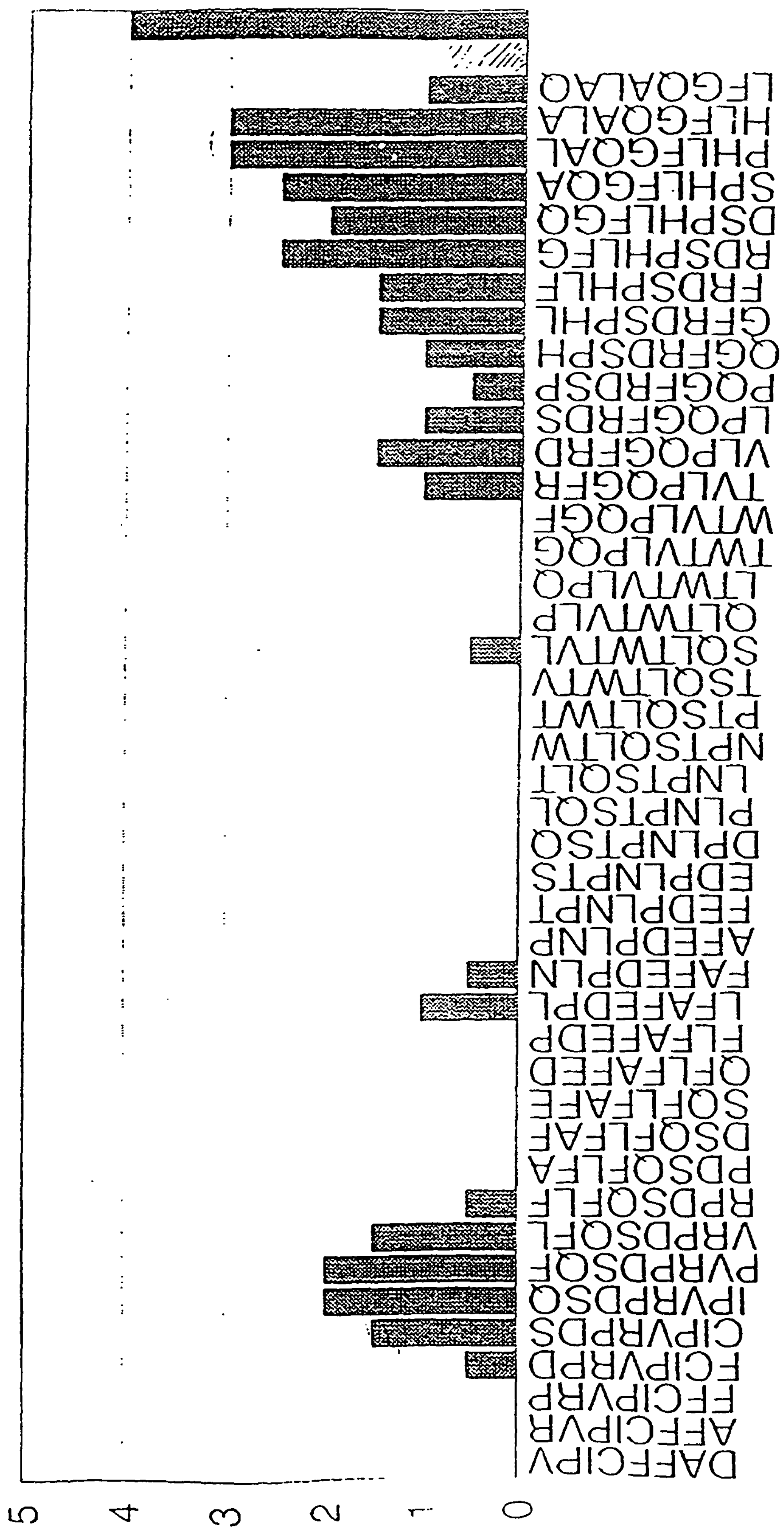
5
4
3
2
1
0
DAFFCIPV
AFFCIPVR
FFCIPVRP
FCIPVRPD
CIPVRPDS
IPVRPDSQ
PVRPDSQF
VRPDSQFL
RPDSQFLF
PDSQFLFA
DSQFLFAF
SQFLFAFE
QFLFAFED
FLFAFEDP
LFAFEDPL
FAFEDPLN
AFEDPLNP
FEDPLNPT
EDPLNPTS
DPLNPTSQ
PLNPTSQL
LNPTSQLT
NPTSQLTW
PTSQLTWT
TSQLTWTV
SQLTWTVL
QLTWTVLP
LTWTVLPQ
TWTVLPQG
WTVLPQGF
TVLPQGFR
VLPQGFRD
LPQGFRDS
PQGFRDSP
QGFRDSPH
GFRDSPHL
FRDSPHLF
RDSPHLFG
DSPHLFGQ
SPHLFGQA
PHLFGQAL
HLFGQALA
LFGQALQ

FIG. 33

FIG. 34

Cys-Ile-Pro-Val-Arg-Pro-Asp-Ser-Gln-Phe-Leu SEQ ID NO 41

Val-Leu-Pro-Gln-Gly-Phe-Arg-Asp-Ser-Pro-His-Leu-Phe-Gly-
Gln-Ala-Leu-Ala SEQ ID NO 42

Leu-Phe-Ala-Phe-Glu-Asp-Pro-Leu SEQ ID NO 43
Phe-Ala-Phe-Glu-Asp-Pro-Leu-Asn SEQ ID NO 44

FIG35

```
         10         20         30         40         50
1234567890 1234567890 1234567890 1234567890 1234567890
CTTCCCCAAC TAATAAGGAC CCCCCTTTCA ACCCAAACAG TCCAAAAGGA     50
L  P  Q  L   I  R  T   P  L  S   T  Q  T  V   Q  K  D
 F  P  N   .  .  G  P   P  F  Q   P  K  Q   S  K  R  T
  S  P  T   N  K  D   P  P  F  N   P  N  S   P  K  G

CATAGACAAA GGAGTAAACA ATGAACCAAA GAGTGCCAAT ATTCCCTGGT    100
 I  D  K   G  V  N  N   E  P  K   S  A  N   I  P  W  L
  .  T  K   E  .  T   M  N  Q  R   V  P  I   F  P  G
H  R  Q  R   S  K  Q   .  T  K   E  C  Q  Y   S  L  V

TATGCACCCT CCAAGCGGTG GGAGAAGAAT TCGGCCCAGC CAGAGTGCAT    150
 C  T  L   Q  A  V   G  E  E  F   G  P  A   R  V  H
Y  A  P  S   K  R  W   E  K  N   S  A  Q  P   E  C  M
 M  H  P   P  S  G  G   R  R  I   R  P  S   Q  S  A  C

GTACCTTTTT CTCTCTCACA CTTGAAGCAA ATTAAAATAG ACNTAGGTNA    200
V  P  F  S   L  S  H   L  K  Q   I  K  I  D   X  G  X
 Y  L  F   L  S  H  T   .  S  K   L  K  .   T  .  V  N
  T  F  F   S  L  T   L  E  A  N   .  N  R   X  R  X

ATTNTCAGAT AGCCCTGATG GYTATATTGA TGTTTTACAA GGATTAGGAC    250
 X  S  D   S  P  D  G   Y  I  D   V  L  Q   G  L  G  Q
  X  Q  I   A  L  M   X  I  L  M   F  Y  K   D  .  D
I  X  R  .   P  .  W   L  Y  .   C  F  T  R   I  R  T

AATCCTTTGA TCTGACATGG AGAGATATAA TATTACTGCT AAATCAGACG    300
 S  F  D   L  T  W   R  D  I  I   L  L  L   N  Q  T
N  P  L  I   .  H  G   E  I  .   Y  Y  C  .   I  R  R
 I  L  .   S  D  M  E   R  Y  N   I  T  A   K  S  D  A

CTAACCTCAA ATGAGAGAAG TGCTGCCATA ACTGGAGCCC GAGAGTTTGG    350
L  T  S  N   E  R  S   A  A  I   T  G  A  R   E  F  G
 .  P  Q   M  R  E  V   L  P  .   L  E  P   E  S  L  A
  N  L  K   .  E  K   C  C  H  N   W  S  P   R  V  W

CAATCTCTGG TATCTCAGTC AGGTCAATGA TAGGATGACA ACGGAGGAAA    400
 N  L  W   Y  L  S  Q   V  N  D   R  M  T   T  E  E  R
  I  S  G   I  S  V   R  S  M  I   G  .  Q   R  R  K
Q  S  L  V   S  Q  S   G  Q  .   .  D  D  N   G  G  K

GAGAACGATT CCCCACAGGG CAGCAGGCAG TTCCCAGTGT AGCTCCTCAT    450
 E  R  F   P  T  G   Q  Q  A  V   P  S  V   A  P  H
E  N  D  S   P  Q  G   S  R  Q   F  P  V  .   L  L  I
 R  T  I   P  H  R  A   A  G  S   S  Q  C   S  S  S  L

TGGGACACAG AATCAGAACA TGGAGATTGG TGCCGCAGAC ATTTA         495
W  D  T  E   S  E  H   G  D  W   C  R  R  H   L
 G  T  Q   N  Q  N  M   E  I  G   A  A  D   I
  G  H  R   I  R  T   W  R  L  V   P  Q  T   F
```

FIG 36

```
          10         20         30         40         50
1234567890 1234567890 1234567890 1234567890 1234567890
CTTCCCCAAC TAATAAGGAC CCCCCTTTCA ACCCAAACAG TCCAAAAGGA    50
L  P  Q  L   I  R  T   P  L  S   T  Q  T  V   Q  K  D

CATAGACAAA GGAGTAAACA ATGAACCAAA GAGTGCCAAT ATTCCCTGGT   100
 I  D  K   G  V  N  N   E  P  K   S  A  N   I  P  W  L

TATGCACCCT CCAAGCGGTG GGAGAAGAAT TCGGCCCAGC CAGAGTGCAT   150
  C  T  L   Q  A  V   G  E  E  F   G  P  A   R  V  H

GTACCTTTTT CTCTCTCACA CTTGAAGCAA ATTAAAATAG ACCTAGGTAA   200
V  P  F  S   L  S  H   L  K  Q   I  K  I  D   L  G  K

ATTCTCAGAT AGCCCTGATG GYTATATTGA TGTTTTACAA GGATTAGGAC   250
 F  S  D   S  P  D  G   Y  I  D   V  L  Q   G  L  G  Q

AATCCTTTGA TCTGACATGG AGAGATATAA TATTACTGCT AAATCAGACG   300
  S  F  D   L  T  W   R  D  I  I   L  L  L   N  Q  T

CTAACCTCAA ATGAGAGAAG TGCTGCCATA ACTGGAGCCC GAGAGTTTGG   350
L  T  S  N   E  R  S   A  A  I   T  G  A  R   E  F  G

CAATCTCTGG TATCTCAGTC AGGTCAATGA TAGGATGACA ACGGAGGAAA   400
 N  L  W   Y  L  S  Q   V  N  D   R  M  T   T  E  E  R

GAGAACGATT CCCCACAGGG CAGCAGGCAG TTCCCAGTGT AGCTCCTCAT   450
  E  R  F   P  T  G   Q  Q  A  V   P  S  V   A  P  H

TGGGACACAG AATCAGAACA TGGAGATTGG TGCCGCAGAC ATTTACAACT   500
W  D  T  E   S  E  H   G  D  W   C  R  R  H   L  Q  L

TGCGTGCTAN AAGGACTNAG GAAAACTAGG AAGACTANGA ATTATTCAAN   550
 A  C  X   K  D  X  G   K  L  G   R  L  X   I  I  Q  X

GATGTCCACT ANNACACAGG GGAAAGGAAG AAAATCCTAC TGCCTTTCTG   600
  C  P  L   X  H  R   G  K  E  E   N  P  T   A  F  L

GAGAGACTAA GGGAGGCATT GAGGAAGCAT ACCAGGCAAG TGGACATTGG   650
E  R  L  R   E  A  L   R  K  H   T  R  Q  V   D  I  G

AGGCTCTGGA AAAGGGAAAA GTTGGGCAAA TTATATGCCT AATAGGGCTT   700
 G  S  G   K  G  K  S   W  A  N   Y  M  P   N  R  A  C

GCTTCCAGTG CAGTCTACAA GGACGCTTTA GAAAAGATTG TCCAAGTAGA   750
  F  Q  C   S  L  Q   G  R  F  R   K  D  C   P  S  R

AATAAGCCGC CCCTCGTCCA TGCCCCTTAT GTCAAGGGAA TCACTGGAAG   800
N  K  P  P   L  V  H   A  P  Y   V  K  G  I   T  G  R

GCCTACTGCC CCAGGGGACG AAGGTCCTCT GAGTCAGAAG CCACTAACCT   850
 P  T  A   P  G  D  E   G  P  L   S  Q  K   P  L  T  .

GA                                                       852
```

FIG 37

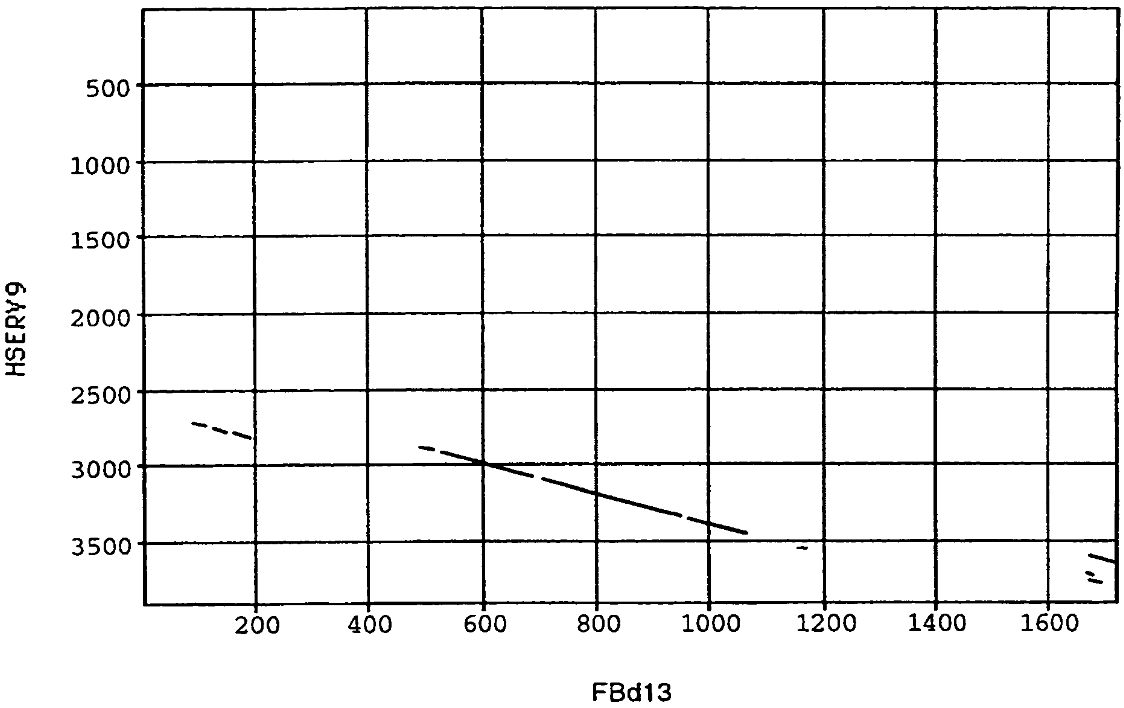
500
1000
1500
2000
2500
3000
3500
HSERY9
200
400
600
800
1000
1200
1400
1600
FBd13

FIG 38
a

```
        10         20         30         40         50
1234567890 1234567890 1234567890 1234567890 1234567890
AAGGAAACTC AGAAAGCCAA TACCCATTTA GTAAGATGGA CACCAGAAGC     50
K  E  T  Q   K  A  N   T  H  L   V  R  W  T   P  E  A
 R  K  L   R  K  P  I   P  I  .   .  D  G   H  Q  K  Q
  G  N  S   E  S  Q   Y  P  F  S   K  M  D   T  R  S

AGAAGCAGCT TTCCAGGCCC TAAAGAAATC CCTAACCCAA GCCCCAGTGT    100
 E  A  A   F  Q  A  L   K  K  S   L  T  Q   A  P  V  L
  K  Q  L   S  R  P   .  R  N  P   .  P  K   P  Q  C
R  S  S  F   P  G  P   K  E  I   P  N  P  S   P  S  V

TAAGCTTGCC AACGGGGCAA GACTTTTCTT TATATGTCAC AGAAAAACAG    150
  S  L  P   T  G  Q   D  F  S  L   Y  V  T   E  K  Q
.  A  C  Q   R  G  K   T  F  L   Y  M  S  Q   K  N  R
 K  L  A   N  G  A  R   L  F  F   I  C  H   R  K  T  G

GAATAGCTCT AGGAGTCCTT ACACAGGTCC AAGGGACAAG CTTGCAACCT    200
E  .  L  .   E  S  L   H  R  S   K  G  Q  A   C  N  L
 N  S  S   R  S  P  Y   T  G  P   R  D  K   L  A  T  C
  I  A  L   G  V  L   T  Q  V  Q   G  T  S   L  Q  P

GTGGCATACC TGAGTAAGGA AACTGATGTA NTGGCAAAGG GTTGGCCTCA    250
 W  H  T   .  V  R  K   L  M  X   W  Q  R   V  G  L  I
  G  I  P   E  .  G   N  .  C  X   G  K  G   L  A  S
V  A  Y  L   S  K  E   T  D  V   X  A  K  G   W  P  H

TTGTTTACAG GTAGGGCAGC AGTAGCAGTC TTAGTTTCTG AAACAGTTAA    300
  V  Y  R   .  G  S   S  S  S  L   S  F  .   N  S  .
L  F  T  G   R  A  A   V  A  V   L  V  S  E   T  V  K
 C  L  Q   V  G  Q  Q   .  Q  S   .  F  L   K  Q  L  K

AATAATACAG GGAAGAGATC TTACTGTGTG GACATCTCAT GATGTGAACG    350
N  N  T  G   K  R  S   Y  C  V   D  I  S  .   C  E  R
 I  I  Q   G  R  D  L   T  V  W   T  S  H   D  V  N  G
  .  Y  R   E  E  I   L  L  C  G   H  L  M   M  .  T

GCATACTCAC TGCTAAAGAG GACTTGTGGC TGTCAGACAA CCATTTACTT    400
 H  T  H   C  .  R  G   L  V  A   V  R  Q   P  F  T  .
  I  L  T   A  K  E   D  L  W  L   S  D  N   H  L  L
A  Y  S  L   L  K  R   T  C  G   C  Q  T  T   I  Y  L

AAATAGCAGG TTCTATTACT TGAAGTGCCA GTGCTGCGAC TGCACATTTG    450
  I  A  G   S  I  T   .  S  A  S   A  A  T   A  H  L
K  .  Q  V   L  L  L   E  V  P   V  L  R  L   H  I  C
 N  S  R   F  Y  Y  L   K  C  Q   C  C  D   C  T  F  V

TGCAACTCTT AACCCAGCCA CATTTCTTCC AGACAATGAA GAAAAGATAG    500
C  N  S  .   P  S  H   I  S  S   R  Q  .  R   K  D  R
 A  T  L   N  P  A  T   F  L  P   D  N  E   E  K  I  E
  Q  L  L   T  Q  P   H  F  F  Q   T  M  K   K  R  .
```

```
          10         20         30         40         50
 1234567890 1234567890 1234567890 1234567890 1234567890
FIG38b
 AACATAACTG TCAACAAGTA ATTGCTCAAA CCTATGCTGC TCGAGGGGAC   550
  T . L  S T S N  C S N  L C C  S R G P
   H N C  Q Q V  I A Q T  Y A A  R G D
 N I T V  N K .  L L K  P M L L  E G T

 CTTCTAGAGG TTCCCTTGAC TGATCCCGAC CTCAACTTGT ATACTGATGG   600
  S R G  S L D  . S R P  Q L V  Y . W
 L L E V  P L T  D P D  L N L Y  T D G
  F . R  F P . L  I P T  S T C  I L M E

 AAGTTCCTTG GCAGAAAAAG GACTTTGAAA AGCGGGGTAT GCAGTGATCA   650
 K F L G  R K R  T L K  S G V C  S D Q
  S S L  A E K G  L . K  A G Y  A V I S
   V P W  Q K K  D F E K  R G M  Q . S

 GTGATAATGG AATACTTGAA AGTAATCGCC TCACTCCAGG AACTAGTGCT   700
  . . W  N T . K  . S P  H S R  N . C S
   D N G  I L E  S N R L  T P G  T S A
 V I M E  Y L K  V I A  S L Q E  L V L

 CACCTGGCAG AACTAATAGC CCTCACTTGG GCACTAGAAT TAGGAGAAGG   750
  P G R  T N S  P H L G  T R I  R R R
 H L A E  L I A  L T W  A L E L  G E G
  T W Q  N . . P  S L G  H . N  . E K E

 AAAAAGGGTA AATATATATT CAGACTCTAA GTATGCTTAC CTAGTCCTCC   800
 K K G K  Y I F  R L .  V C L P  S P P
  K R V  N I Y S  D S K  Y A Y  L V L H
   K G .  I Y I  Q T L S  M L T  . S S

 ATGCCCATGC AGCAATATGG AGAGAGAGGG AATTCCTAAC TTCTGAGGGA   850
  C P C  S N M E  R E G  I P N  F . G N
   A H A  A I W  R E R E  F L T  S E G
 M P M Q  Q Y G  E R G  N S . L  L R E

 ACACCTATCA ACCATCAGGG AAGCCATTAG GAGATTATTA TTGGCTGTAC   900
   T Y Q  P S G  K P L G  D Y Y  W L Y
 T P I N  H Q G  S H .  E I I I  G C T
  H L S  T I R E  A I R  R L L  L A V Q

 AGAAACCTAA AGAGGTGGCA GTCTTACACT GCCAGGGTCA TCAGGAAGAA   950
 R N L K  R W Q  S Y T  A R V I  R K K
  E T .  R G G S  L T L  P G S  S G R R
   K P K  E V A  V L H C  Q G H  Q E E

 GAGGAAAGGG AAATAGAAGG CAATCGCCAA GCGGATATTG AAGCAAAAAA   1000
  R K G  K . K A  I A K  R I L  K Q K K
   G K G  N R R  Q S P S  G Y .  S K K
 E E R E  I E G  N R Q  A D I E  A K K
```

FIG 38C

```
          10         20         30         40         50
1234567890 1234567890 1234567890 1234567890 1234567890
AGCCGCAAGG CAGGACTCTC CATTAGAAAT GCTTATAGAA GGACCCCTAG   1050
  P  Q  G   R  T  L  H  .  K  C  L  .  K   D  P  .
S  R  K  A   G  L  S  I  R  N  A  Y  R  R   T  P  S
 A  A  R  Q  D  S  P   L  E  M  L  I  E   G  P  L  V

TATGGGGTAA TCCCCTCTGG GAAACCAAGC CCCAGTACTC AGCAGGAAAA   1100
Y  G  V  I   P  S  G  K  P  S  P  S  T  Q   Q  E  K
 M  G  .   S  P  L  G   N  Q  A  P  V  L   S  R  K  N
  W  G  N   P  L  W  E  T  K  P  Q  Y  S   A  G  K

ATAGAATAGG AAACCTCACA AGGACATACT TTCCTCCCCT CCAGATGGCT   1150
 .  N  R   K  P  H  K  D  I  L   S  S  P  P  D  G  .
  R  I  G   N  L  T   R  T  Y  F   P  P  L  Q  M  A
I  E  .  E   T  S  Q  G  H  T  F  L  P  S   R  W  L

AGCCACTGAG GAAGGAA                                       1167
  P  L  R   K  E
S  H  .  G   R
 A  T  E   E  G
```

FIG 39 a

```
        10         20         30         40         50
1234567890 1234567890 1234567890 1234567890 1234567890
AACTTGCGTG CTAGAAGGAC TAAGGAAAAC TAGGAAGACT ATGAATTATT   50
N L R A  R R T  K E N  . E D Y  E L F
 T C V  L E G L  R K T  R K T  M N Y S
  L A C  . K D  . G K L  G R L  . I I

CAATGATGTC CACTATAACA CAGGGGAAAG GAAGAAAATC CTACTGCCTT  100
 N D V  H Y N T  G E R  K K I  L L P F
  M M S  T I T  Q G K G  R K S  Y C L
Q . C P  L . H  R G K  E E N P  T A F

TCTGGAGAGA CTAAGGGAGG CATTGAGGAA GCATACCAGG CAAGTGGACA  150
  W R D  . G R  H . G S  I P G  K W T
S G E T  K G G  I E E  A Y Q A  S G H
 L E R  L R E A  L R K  H T R  Q V D I

TTGGAGGCTC TGGAAAAGGG AAAAGTTGGG CAAATTGAAT GCCTAATAGG  200
L E A L  E K G  K V G  Q I E C  L I G
 W R L  W K R E  K L G  K L N  A . . G
  G G S  G K G  K S W A  N . M  P N R

GCTTGCTTCC AGTGCAGTCT ACAAGGACGC TTTAGAAAAG ATTGTCCAAG  250
 L A S  S A V Y  K D A  L E K  I V Q V
  L L P  V Q S  T R T L  . K R  L S K
A C F Q  C S L  Q G R  F R K D  C P S

TAGAAATAAG CCGCCCCTCG TCCATGCCCC TTATGTCAAG GGAATCACTG  300
  E I S  R P S  S M P L  M S R  E S L
. K . A  A P R  P C P  L C Q G  N H W
 R N K  P P L V  H A P  Y V K  G I T G

GAAGGCCTAC TGCCCCAGGG GACGAAGGTC CTCTGAGTCA GAAGCCACTA  350
E G L L  P Q G  T K V  L . V R  S H .
 K A Y  C P R G  R R S  S E S  E A T N
  R P T  A P G  D E G P  L S Q  K P L

ACCTGATGAT CCAGCAGCAG GACTGAGGGT GCCCGGGGCA AGTGCCAGCC  400
 P D D  P A A G  L R V  P G A  S A S P
  L M I  Q Q Q  D . G C  P G Q  V P A
T . . S  S S R  T E G  A R G K  C Q P

CATGCCATCA CCCTCAGAGC CCCGGGTATG TTTGACCATT GAGAGCCAGG  450
  C H H  P Q S  P G Y V  . P L  R A R
H A I T  L R A  P G M  F D H .  E P G
 M P S  P S E P  R V C  L T I  E S Q E

AAGTTAACTG TCTCCTGGAC ACTGGCGCAG CCTTCTCAGT CTTACTTTCC  500
K L T V  S W T  L A Q  P S Q S  Y F P
 S . L  S P G H  W R S  L L S  L T F L
  V N C  L L D  T G A A  F S V  L L S
```

FIG 39b

```
         10         20         30         40         50
1234567890 1234567890 1234567890 1234567890 1234567890
TGTCCCAGAC AATTGTCCTC CAGATCTGTC ACTATCCGAG GGGTCCTAAG   550
 V  P  D   N  C  P  P   D  L  S   L  S  E   G  S  .  D
  S  Q  T   I  V  L   Q  I  C  H   Y  P  R   G  P  K
C  P  R  Q   L  S  S   R  S  V   T  I  R  G   V  L  R

ACAGCCAGTC ACTACATACT TCTCTCAGCC ACTAAGTTGT GACTGGGGAA   600
  S  Q  S   L  H  T   S  L  S  H   .  V  V   T  G  E
T  A  S  H   Y  I  L   L  S  A   T  K  L  .   L  G  N
 Q  P  V   T  T  Y  F   S  Q  P   L  S  C   D  W  G  T

CTTTACTCTT TTCACATGCT TTTCTAATTA TGCCTGAAAG CCCCACTCCC   650
L  Y  S  F   H  M  L   F  .  L   C  L  K  A   P  L  P
 F  T  L   F  T  C  F   S  N  Y   A  .  K   P  H  S  L
  L  L  F   S  H  A   F  L  I  M   P  E  S   P  T  P

TTGTTAGGGA GAGACATTTT AGCAAAAGCA GGGGCCATTA TACACCTGAA   700
 C  .  G   E  T  F  .   Q  K  Q   G  P  L   Y  T  .  T
  V  R  E   R  H  F   S  K  S  R   G  H  Y   T  P  E
L  L  G  R   D  I  L   A  K  A   G  A  I  I   H  L  N

CATAGGAAAA GGAATACCCA TTTGCTGTCC CCTGCTTGAG GAAGGAATTA   750
  .  E  K   E  Y  P   F  A  V  P   C  L  R   K  E  L
H  R  K  R   N  T  H   L  L  S   P  A  .  G   R  N  .
 I  G  K   G  I  P  I   C  C  P   L  L  E   E  G  I  N

ATCCTGAAGT CTGGGCAATA GAAGGACAAT ATGGACAAGC AAAGAATGCC   800
I  L  K  S   G  Q  .   K  D  N   M  D  K  Q   R  M  P
 S  .  S   L  G  N  R   R  T  I   W  T  S   K  E  C  P
  P  E  V   W  A  I   E  G  Q  Y   G  Q  A   K  N  A

CGTCCTGTTC AAGTTAAACT AAAGGATTCT GCCTCCTTTC CCTACCAAAG   850
 V  L  F   K  L  N  .   R  I  L   P  P  F   P  T  K  G
  S  C  S   S  .  T   K  G  F  C   L  L  S   L  P  K
R  P  V  Q   V  K  L   K  D  S   A  S  F  P   Y  Q  R

GAAGTACCCT CTTAGACCCG AGGCCCTACA AGGACTCAAA AGATTGTTAA   900
  S  T  L   L  D  P   R  P  Y  K   D  S  K   D  C  .
E  V  P  S   .  T  R   G  P  T   R  T  Q  K   I  V  K
 K  Y  P   L  R  P  E   A  L  Q   G  L  K   R  L  L  R

GGACCTAAAA GCCCAAGGCC TAGTAAAACC ATGCAGTAGC CCCTGCAATA   950
G  P  K  S   P  R  P   S  K  T   M  Q  .  P   L  Q  Y
 D  L  K   A  Q  G  L   V  K  P   C  S  S   P  C  N  T
  T  .  K   P  K  A   .  .  N  H   A  V  A   P  A  I

CTCCAATTTT AGGAGTAAGG AAACCCAACG GACAGTGGAG GTTAGTGCAA  1000
 S  N  F   R  S  K  E   T  Q  R   T  V  E   V  S  A  R
  P  I  L   G  V  R   K  P  N  G   Q  W  R   L  V  Q
L  Q  F  .   E  .  G   N  P  T   D  S  G  G   .  C  K
```

FIG 39
C

```
         10         20         30         40         50
1234567890 1234567890 1234567890 1234567890 1234567890
GATCTCAGGA TTATTAATGA GGCTGTTTTT CCTCTATACC CAGCTGTATC   1050
 S  Q  D  Y  .  .  G  C  F  S  S  I  P  S  C  I
D  L  R  I  I  N  E  A  V  F  P  L  Y  P  A  V  S
 I  S  G  L  L  M  R  L  F  F  L  Y  T  Q  L  Y  L

TAGCCCTTAT ACTCTGCTTT CCCTAATACC AGAGGAAGCA GAGTAGTTTA   1100
.  P  L  Y  S  A  F  P  N  T  R  G  S  R  V  V  Y
 S  P  Y  T  L  L  S  L  I  P  E  E  A  E  .  F  T
  A  L  I  L  C  F  P  .  Y  Q  R  K  Q  S  S  L

CAGTCCTGGA CCTTAAGGAT GCCTCTTTCT GCATCCCTGT ACATCCTGAT   1150
 S  P  G  P  .  G  C  L  F  L  H  P  C  T  S  .  F
  V  L  D  L  K  D  A  S  F  C  I  P  V  H  P  D
Q  S  W  T  L  R  M  P  L  S  A  S  L  Y  I  L  I

TCTCAATTCT TGTTTGTCTT TGAAGATCCT TTGAACCCAA TGTCTCAATT   1200
  S  I  L  V  C  L  .  R  S  F  E  P  N  V  S  I
S  Q  F  L  F  V  F  E  D  P  L  N  P  M  S  Q  F
 L  N  S  C  L  S  L  K  I  L  .  T  Q  C  L  N  S

CACCTGGACT GTTTTACCCC AGGGGTTCCG GGATAGCCCC CATCTATTTG   1250
H  L  D  C  F  T  P  G  V  P  G  .  P  P  S  I  W
 T  W  T  V  L  P  Q  G  F  R  D  S  P  H  L  F  G
  P  G  L  F  Y  P  R  G  S  G  I  A  P  I  Y  L

GCCAGGCATT AGCCCAAGAC TTGAGCCAAT TCTCATACCT GGACATCTTG   1300
 P  G  I  S  P  R  L  E  P  I  L  I  P  G  H  L  V
  Q  A  L  A  Q  D  L  S  Q  F  S  Y  L  D  I  L
A  R  H  .  P  K  T  .  A  N  S  H  T  W  T  S  C

TCCTTCGGTA TGGGATGATT TAATTTTAGC CACCCGTTCA GAAACCTTGT   1350
  L  R  Y  G  M  I  .  F  .  P  P  V  Q  K  P  C
S  F  G  M  G  .  F  N  F  S  H  P  F  R  N  L  V
 P  S  V  W  D  D  L  I  L  A  T  R  S  E  T  L  C

GCCATCAAGC CACCCAAGCG TTCTTAAATT TCCTCACTCC GTGTGGCTAC   1400
A  I  K  P  P  K  R  S  .  I  S  S  L  R  V  A  T
 P  S  S  H  P  S  V  L  K  F  P  H  S  V  W  L  Q
  H  Q  A  T  Q  A  F  L  N  F  L  T  P  C  G  Y

AAGGTTTCCA AACCAAAGGC TCAGCTCTGC TCACAGCAGG TTAAATACTT   1450
 R  F  P  N  Q  R  L  S  S  A  H  S  R  L  N  T  .
  G  F  Q  T  K  G  S  A  L  L  T  A  G  .  I  L
K  V  S  K  P  K  A  Q  L  C  S  Q  Q  V  K  Y  L

AGGGTTAAAA TTATCCAAAG GCACCAGGGC CCTCTGTGAG GAATGTATCC   1500
  G  .  N  Y  P  K  A  P  G  P  S  V  R  N  V  S
R  V  K  I  I  Q  R  H  Q  G  P  L  .  G  M  Y  P
 G  L  K  L  S  K  G  T  R  A  L  C  E  E  C  I  Q
```

FIG 39d

```
        10         20         30         40         50
1234567890 1234567890 1234567890 1234567890 1234567890
AACCTGTACT GGCTTATCTT CATCCCAAAA CCCTAAAGCA ACTAAGAAGG   1550
N L Y W  L I F  I P K  P . S N  . E G
 T C T  G L S S  S Q N  P K A  T K K V
  P V L  A Y L  H P K T  L K Q  L R R

TCCTTGGCAT AACAGGTTTC TGCCGAA                            1577
 P W H  N R F L  P
  L G I  T G F  C R
S L A .  Q V S  A E
```

FIG 40

```
         10         20         30         40         50
1234567890 1234567890 1234567890 1234567890 1234567890
TCCAGCAGCA GGACTGAGGG TGCCCGGGGC AAGTGCCAGC CCATGCCATC    50
S  S  S  R  T  E  G  A  R  G  K  C  Q  P  M  P  S

ACCCTCAGAG CCCCGGGTAT GTTTGACCAT TGAGAGCCAG GAAGTTAACT   100
 P  S  E  P  R  V  C  L  T  I  E  S  Q  E  V  N  C

GTCTCCTGGA CACTGGCGCA GCCTTCTCAG TCTTACTTTC CTGTCCCAGA   150
  L  L  D  T  G  A  A  F  S  V  L  L  S  C  P  R

CAATTGTCCT CCAGATCTGT CACTATCCGA GGGGTCCTAA GACAGCCAGT   200
Q  L  S  S  R  S  V  T  I  R  G  V  L  R  Q  P  V

CACTACATAC TTCTCTCAGC CACTAAGTTG TGACTGGGGA ACTTTACTCT   250
 T  T  Y  F  S  Q  P  L  S  C  D  W  G  T  L  L  F

TTTCACATGC TTTTCTAATT ATGCCTGAAA GCCCCACTCC CTTGTTAGGG   300
  S  H  A  F  L  I  M  P  E  S  P  T  P  L  L  G

AGAGACATTT TAGCAAAAGC AGGGGCCATT ATACACCTGA ACATAGGAAA   350
R  D  I  L  A  K  A  G  A  I  I  H  L  N  I  G  K

AGGAATACCC ATTTGCTGTC CCCTGCTTGA GGAAGGAATT AATCCTGAAG   400
 G  I  P  I  C  C  P  L  L  E  E  G  I  N  P  E  V

TCTGGGCAAT AGAAGGACAA TATGGACAAG CAAAGAATGC CCGTCCTGTT   450
  W  A  I  E  G  Q  Y  G  Q  A  K  N  A  R  P  V

CAAGTTAAAC TAAAGGATTC TGCCTCCTTT CCCTACCAAA GGAAGTACCC   500
Q  V  K  L  K  D  S  A  S  F  P  Y  Q  R  K  Y  P

TCTTAGACCC GAGGCCCTAC AAGGACTCAA AAGATTGTTA AGGACCT      547
 L  R  P  E  A  L  Q  G  L  K  R  L  L  R  T
```

FIG 41

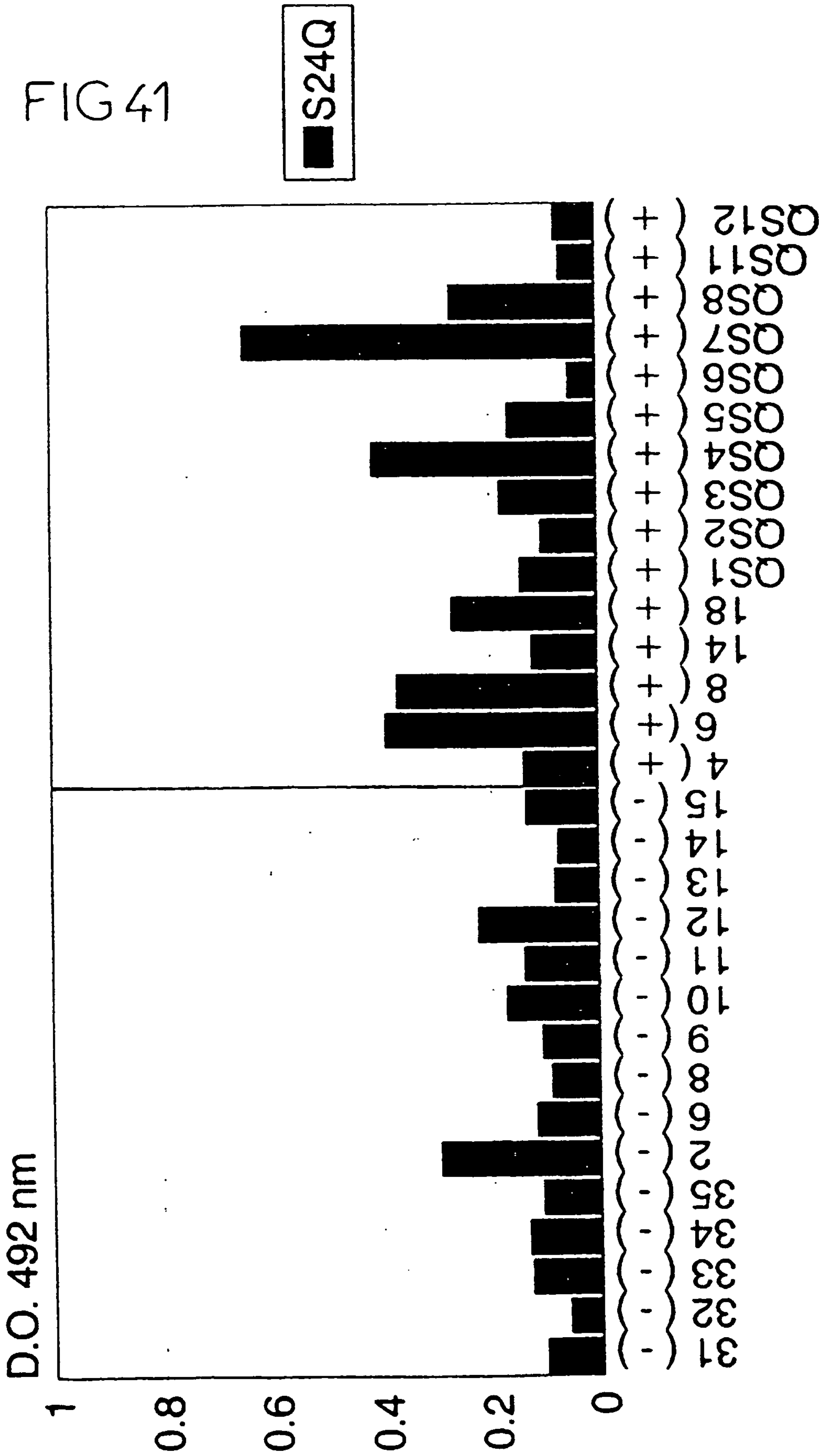

FIG 42

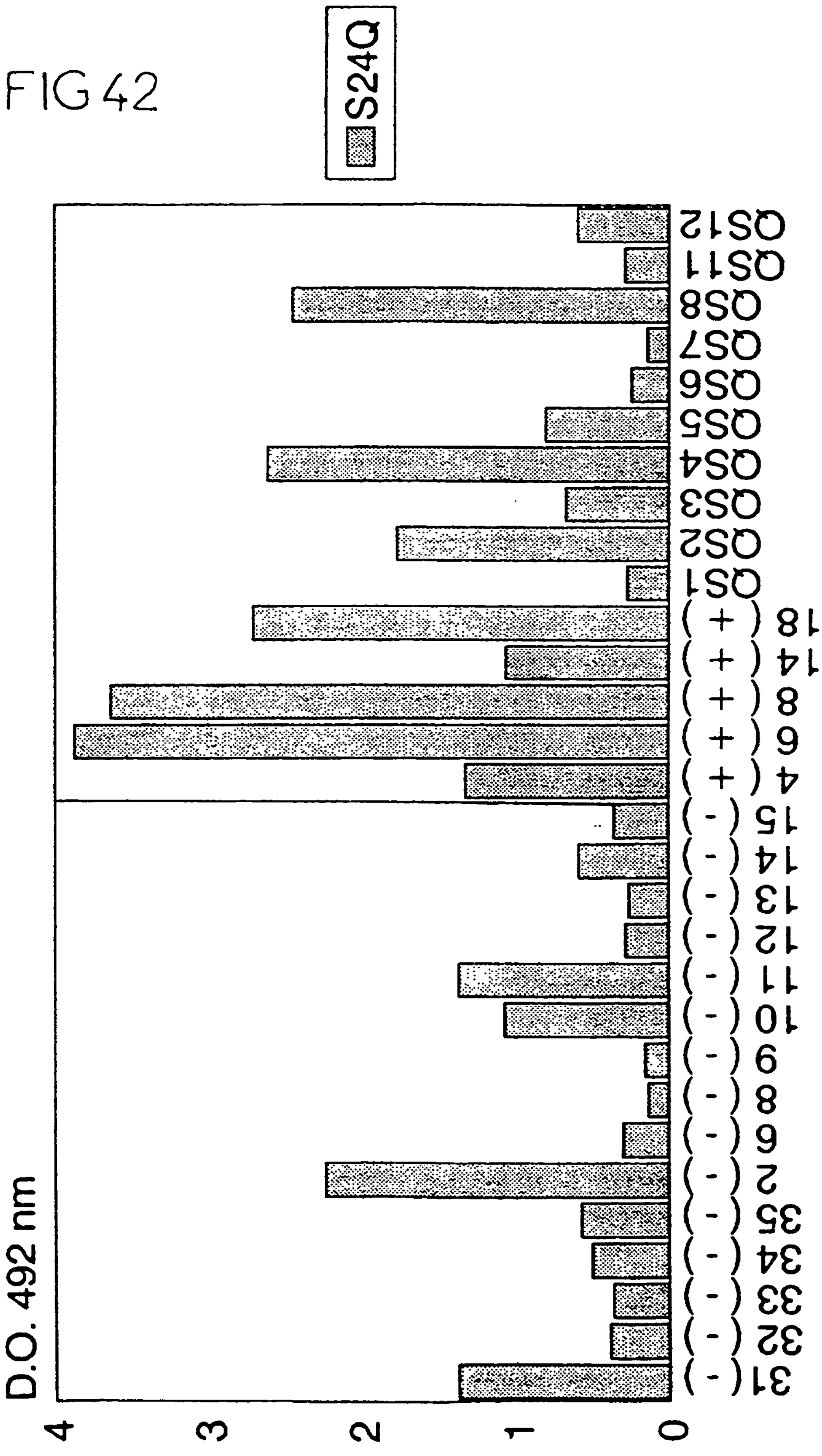

S24Q
D.O. 492 nm
0
1
2
3
4
31 ( - )
32 ( - )
33 ( - )
34 ( - )
35 ( - )
2 ( - )
6 ( - )
8 ( - )
9 ( - )
10 ( - )
11 ( - )
12 ( - )
13 ( - )
14 ( - )
15 ( - )
4 ( + )
6 ( + )
8 ( + )
14 ( + )
18 ( + )
QS1
QS2
QS3
QS4
QS5
QS6
QS7
QS8
QS11
QS12

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9320188 A
- FR 92072201
- FR 93010817
- FR V92072202
- FR V93010816
- FR 2716198
- FR 9502960
- FR 9204322
- FR 2689519
- FR 9213447
- FR 2689521
- FR 9213443
- FR 2689520
- FR 9401529
- FR 2715936
- FR 9401531
- FR 2715939
- FR 9401530
- FR 9401532
- FR 2715937
- FR 9414322
- FR 2727428
- FR 9415810
- FR 2728585
- WO 9320189 A
- EP 0569272 A
- FR 2663040 A


### Littérature non-brevet citée dans la description


- **BARANY G ; MERRIFIELSD R.B.** Peptides. Academic Press, 1980, vol. 2, 1-284
- **BERG et al.** *J. Ann. Chem. Soc,* 1989, vol. 111, 8024-8026
- **NORRBY E.** *Prog. Med. Virol,* 1978, vol. 24, 1-39
- Demyelinating diseases. **JOHNSON R.T.** Handbook of clinical neurology. Elsevier Science Publishing, 1985, vol. 47, 319-336
- **PERRON H.** *Res. Virol,* 1989, vol. 140, 551-561
- **PERRON H.** Current concepts in multiple sclerosis. Elsevier, 1991, 111-116
- **PERRON H.** *The Lancet,* 1991, vol. 337, 862-863
- **PERRON H.** *J. Gen. Virol,* 1993, vol. 74, 65-72
- **FIELDS ; KNIPE.** Fondamental Virology. Rev Press, 1986
- **NIELSEN P.E.** *Science,* 1991, vol. 254, 1497-1500
- **MANIATIS et al.** *Molecular Cloning*
- **SOUTHERN. E.M.** *J. Mol. Biol.,* 1975, vol. 98
- **DUNN A.R ; HASSEL J.A.** *Cell,* 1977, vol. 12
- **SHIH.** *J. Virol,* 1989, vol. 63, 64-75
- **PERRON H.** *Res. Vir,* 1992, vol. 143, 337-350
- **MEYERHANS.** *Cell,* 1989, vol. 58, 901-910
- **LINIAL M.L ; MILLER A.D.** Current topics in microbiology and immunobiology. Retroviruses, stratégies of replication. Springer-Verlag, 1990, vol. 157, 125-152
- **LORI F.** *J. Virol,* 1992, vol. 66, 5067-5074
- **SAMBROOK J ; FRITSCH E.F ; MANIATIS T.** Molecular cloning, a laboratory manual. Cold Spring Harbor Laboratory Press, 1989
- **LA MANTIA.** *Nucleic Acids Research,* 1991, vol. 19, 1513-1520
- **GONZALEZ-QUINTIAL R ; BACCALA R ; POPE R M ; THOEOFILOPOULOS N.** *J. Clin. Invest,* 1996, vol. 97 (5), 1335-1343
- **CHOMZYNSKI P ; N. SACCHI.** *Analytical Biochemistry,* 1987, vol. 162, 156-159
- **F. MALLET.** *Journal of Clinical Microbiology,* 1993, vol. 31, 1444-1449
- **G. BARANY ; R.B. MERRIFIELSD.** Peptides. Academic Press, 1980, vol. 2, 1-284
- **POSER et al.** The diagnosis of multiple sclerosis. Thieme Stratton Inc, 1984, 225-229
- **LA MANTIA.** *Nucleic Acid Research,* 1989, vol. 17, 5913-22
- **PLAZA, A ; KONO, D.H.** THEOFILOPOULOS, A.N. NEW HUMAN Vβ GENES AND POLYMORPHIC VARIANTS. *J. Imm,* 1991, vol. 147 (12), 4360-4365